# EUROPEAN PATENT APPLICATION

(11) **EP 1 715 043 A2**
(43) Date of publication of application: **25.10.2006**
(21) Application number: 06002432.0
(22) Date of filing: 23.12.1999
(51) Int. Cl.: C12N 15/12, C07K 14/47, C12N 5/10, C07K 16/18, C12N 15/62, C12Q 1/68, G01N 33/50, G01N 33/53, A61K 38/02, A61K 48/00

(54) **Compounds for immunotherapy and diagnosis of colon cancer and methods for their use**

(30) Priority: 23.12.1998 US 221298; 02.07.1999 US 347496; 22.09.1999 US 401064; 19.11.1999 US 444242; 02.12.1999 US 454150
(62) Divisional of application: 99967625.7
(71) Applicant: CORIXA CORPORATION, Seattle, WA 98101 (US)
(72) Inventor: Xu, Jiangchun, Seattle, WA 98101 (US); Lodes, Michael, Seattle, WA 98101 (US); Secrist, Heather, Seattle, WA 98101 (US); Benson, Darin, Seattle, WA 98101 (US); Meagher, Madeleine, Joy, Seattle, WA 98101 (US); Stolk, John, Seattle, WA 98101 (US); Wang, Tongtong, Seattle, WA 98101 (US); Yuqiu, Jiang, Seattle, WA 98101 (US)
(74) Representative: Thornton, Neil

(57) **Abstract**

Compositions and methods for the therapy and diagnosis of cancer, such as colon cancer, are disclosed. Compositions may comprise one or more colon tumor proteins, immunogenic portions thereof, or polynucleotides that encode such portions. Alternatively, a therapeutic composition may comprise an antigen presenting cell that expresses a colon tumor protein, or a T cell that is specific for cells expressing such a protein. Such compositions may be used, for example, for the prevention and treatment of diseases such as colon cancer. Diagnostic methods based on detecting a colon tumor protein, or mRNA encoding such a protein, in a sample are also provided.

SEQUENCE LISTING

<110> Corixa Corporation

<120> COMPOUNDS FOR IMMUNOTHERAPY AND
DIAGNOSIS OF COLON CANCER AND METHODS FOR THEIR USE

<130> 210121.471PC

<140> PCT
<141> 1999-12-23

<160> 486

<170> FastSEQ for Windows Version 3.0

<210> 1
<211> 458
<212> DNA
<213> Homo sapien

<220>
<221> misc_feature
<222> (1)...(458)
<223> n = A,T,C or G

<400> 1
ncaggtctgg cggcacctgt gcactcagcc gtcgatacac tggtcgattg ggacagggaa 60
gacgatgtgg ttttcaggga ggcccagaga tttggagaag cggatgaagt tctcctttag 120
ttccgaagtc agctccttgg ttctcccgta gagggtgatc ttgaagtact ccctgttttg 180
agaaactttc ttgaagaaca ccatagcatg ctggttgtag ttggtgctca ccactcggac 240
gaggtaactc gttaatccag ggtaactctt aatgttgccc agcgtgaact cgccgggctg 300
gcaacctgga acaaaagtcc tgatccagta gtcacacttc tttttcctaa acaggacgga 360
ggtgacattg tagctcttgt cttctttcag ctcatagatg gtggcataca tcttttgcgg 420
gtctttgtct tctctgagaa ttgcattccc tgccagga 458

<210> 2
<211> 423
<212> DNA
<213> Homo sapien

<400> 2
cagggtccat aggtgatccg caactctcga gcatttatat acaatagcaa atcatccagt 60
gtgttgtaca gtctataata ctccaacagt ctcccatctg tattcaatgg cgccacccaa 120
tacagtcctt tgtttggatg ctggggagag taatccctac cccaagcacc atatagataa 180
gaaaaccctc tccagttgag ctgaaccaca gacggtttgc tgatgttcac cacaccacca 240
tgaccacagc tccctggagt gggaggaggg tggacgacag gggtgttttg atctttagag 300
gcttcacact ctttcagctt ggtcttcaga gccacgattt ctcggcgaat ggcaaggaca 360
ttgtttttgt ctagtgtctc aagcttctct accaagagag tcatatttct tatctccacc 420
tcc 423

<210> 3
<211> 538

<212> DNA
<213> Homo sapien

<400> 3
ggtctgtcca atggcaacag gaccctcact ctaytcartg tcacaagraa tgayrcagsa 60
msctayraat gtgaaaycca gaacccagtg agtgccarsc gcagtgayyc agtcatcctg 120
aatgtcctct atggcccrga tgmccccacc atttcccctc taaacacatm ttaccgwyca 180
ggggaaaatc tgaacctctc ctgccacgca gcctctaacc cacctgcaca gtactcttgg 240
tttrtcaatg ggactttcca gcaatccacm caagagctct ttatccccaa catcactgtg 300
aataatagyg gatcctatac gtgccaagcc cataactcag mcactggcct caataggacc 360
acagtcacga cgatcacagt ctatgcaaga gccacccaaa cccttcatca ccagcaacaa 420
ctccaacccc gtggaggatg aggatgctgt agccttaacc tgtgaacctg agattcagaa 480
cacaacctac ctgtggtggg taaataatca gagcctcccg gtcagtccca ggctgcag 538

<210> 4
<211> 309
<212> DNA
<213> Homo sapien

<400> 4
tggtaascca aaaagatgct ggggcagatt gtggacaagt agaagaacct ccttcccctc 60
tgcgaacatt gaacggcgtg gattcaatag tgagcttggc agtggtgggc gggttccaga 120
aggttagaag tgaggctgtg agcaggagcc cctgccaggg gatvcacgca mtctgtgggg 180
aggggctgag rggdgwcycc atggtctctg ctgtctgctc tgtcctcctc tgtggagaag 240
agcttgagct ccaggaacgc tttgrtcavg gctgcctgtg acctytgctc tgbtctgcct 300
gcccgggcg 309

<210> 5
<211> 412
<212> DNA
<213> Homo sapien

<400> 5
gtccaatggc aacaggaccc ctcacttcta ttcaatgtca caagaaatga cgcaagagcc 60
tatgtatgtg gaatccagaa ctkcagtgag tgcaaaccgc agtgacccag tcaccctgga 120
tgtcctctat gggccagaca scccccatca tttccccccc agactcgtct tacctttcgg 180
gagcgaacct caacctctcc tgccactcgg cctctaaccc atccccgcag tattcttggc 240
kgtatcaatg ggataccgca gcaacacaca caagttctct ttatcgccaa aatcacgcca 300
aataataacg ggacctatgc ctgttttgtc tctaacttgg ctactggccc gcaataattc 360
catagtcaag agcatcacag tcttctgcat ctggaacttc tcctggtctt ct 412

<210> 6
<211> 332
<212> DNA
<213> Homo sapien

<400> 6
gtgcaagggc tttacaaaaa ctgtgccagt krcttctyca tgwsrcwrga tctgacttka 60
ttsaygttkt atgagsysya saatmctgaw gctcmttyts sakgrwsttc kgsatmrgca 120
gtsrattcsa catttgggrt akrtymtctc tsgaagysam tgtcakgcag tgrcayccwr 180
gkktcwgcwt gcwgtgrgtt amcakcmwtr ywtagkgsgm ayatrattta ramrgtayak 240
cymtctcmct cytycmccay wtgcwcaass mkcacacctc ggccgcgacc acgctaagcc 300
cgaattccag cacactggcg gccgttacta gt 332

<210> 7

<211> 401
<212> DNA
<213> Homo sapien

<400> 7
tggtgttgtt ggcgccagtt ccctggacct ggaacagccg tgtggagggc ccggtctcca 60
agttgttagt tcgggaggtg cctccctggt agaccaccat gcgtcccttg aagatggaca 120
taagatgagg tggctccttg cccattggga cccggatctg gactggttca ccattgtact 180
tctggtccag gatgacggct tgataagctg atgctgtaat ttcatcttgg ctggcctggc 240
tgccctgcca aacgtagagc aggtaatgct gcttctcgcc gatgaaggta ggtgtaagag 300
cagcaggtaa gcaagttcgc ccccatagaa gtgggcctag ccacttggaa ttccagcaca 360
ctggcggccc gttactagtg ggatcccgag ctcggtacca a 401

<210> 8
<211> 1151
<212> DNA
<213> Homo sapien

<400> 8
   ctctctccat aaaactcagc actttacaga tgtagaatat ataagcatgc caaatttact 60
   tatctgccac atacaaagca tcattccagg tgctagtgag gggaaaaaaa agttggagat 120
   ttggtccctc gaggagctcc agatattaat ctacctaact aagtccccag gtttcttcca 180
   ggcatggaag aattagtggt gctacatgga tgaggactag tcattgggca atatttcctg 240
   tacaaagaat ccctagacgc catactgagt tttaagttcc ttaattccta atttaaggct 300
   tctagtgaag cctcctcaca gtaggcttca ctaggcccac agtgccccta gacctctgac 360
   aatcccaccc tagacagact ttattgcaaa atgcgcctga agaggcagat gattcccaag 420
   agaactcacc aaatcaagac aaatgtccta gatctccagt gtggtagaac tatgcaccta 480
   aacattgctg caaaatgaac acacttttag acacccctgc agatatctaa gtaagtggag 540
   aagactattt tttcaacaaa cattttctct ttcaccctaa ctcctaaaca gcttactggg 600
   gcttctgcaa gacagaaaga tcataattca gaaggtaacc atcgttatag acataaagtt 660
   tctggtcaaa agggttatag ttaatgctct gcactttttc ctgcatctta tgcattacaa 720
   tgtctagttt gccctctttc cctgtgtttg tgtcataata gtaaaaaatc tcttctgttc 780
   tggtgtttca tagtacgggt ggcatacaga accccacata ccatgaaggc gttagaagca 840
   gatggtttat actgcttggt ataccaagtg tttagcacct gaagtgtggt gtcattgagt 900
   ttactaatca ccatgttacc agtgctggct tcagttgaat aaataaccca caatccattc 960
   tcatccacag caaagtcaat atcttgccaa gcaacattag catatgaaaa gcggttatta 1020
   taggcagcat tagggagagt ttgagtcaca gcaatcgtgt tggtggtcag gttaactctg 1080
   gcaatattcc cggtgttgta catgttgacg tacatgttgt tgttgtaaac tgctgtacca 1140
   ctaccttgga c 1151

<210> 9
<211> 604
<212> DNA
<213> Homo sapien

<220>
<221> misc_feature
<222> (1)...(604)
<223> n = A,T,C or G

<400> 9
ctgtgcaagg gctttacaaa aactgtgcca ggacttccca tgaggctgga ttgcttgatt 60
catgttttat gagccccaca atactgaagc tccttttcca gggacttggc ataggcagtc 120
aattccacat ttgggatagg tcctctctgg aagtgaatgt caggcagtga catccaagtt 180
tctgcatgca gtgggttaac agccatgttt agggggaaca tgatttaaaa agtacatctc 240

tctccctcct cccccacatg cacaaggctc acatctcatt atggtgkcgg cccatgtcac 300
attaaagtgt gatacttkgg ttttgaaaac attcaaacag tctctgtgga aatctggaga 360
gaaattggcg gagagctgcc gtggtgcatt cctcctgtag tgcttcaagn taatgcttca 420
tcctttntta ataacttttg atagacaggg gctagtcgca cagacctctg ggaagccctg 480
gaaaacgctg atgcttgttt gaagatctca agcgcagagt ctgcaagttc atcccctctt 540
tcctgaggtc tgttggctgg aggctgcaga acattggtga tgacatggac cacgccattt 600
gtgg 604

<210> 10
<211> 473
<212> DNA
<213> Homo sapien

<400> 10
tcgagaagat ccctagtgag actttgaacc gtatcctggg cgacccagaa gccctgagag 60
acctgctgaa caaccacatc ttgaagtcag ctatgtgtgc tgaagccatc gttgcggggc 120
tgtctgtgga gaccctggag ggcacgacac tggaggtggg ctgcagcggg gacatgctca 180
ctatcaacgg gaaggcgatc atctccaata aagacatcct agccaccaac ggggtgatcc 240
actacattga tgagctactc atcccagact cagccaagac actatttgaa ttggctgcag 300
agtctgatgt gtccacagcc attgaccttt tcagacaagc cggcctcggc aatcatctct 360
ctggaagtga gcggttgacc ctcctgggct cccctgaatt ctgtattcaa agatggaacc 420
cctccaattg atgcccatac aaggaatttg cttcggaacc acataattaa aga 473

<210> 11
<211> 411
<212> DNA
<213> Homo sapien

<220>
<221> misc_feature
<222> (1)...(411)
<223> n = A,T,C or G

<400> 11
tcctcattgg tcggggccaa aagcgtgtac tggccgttac cttcaagcat cgtgttgagc 60
cctgatgcag ccacagcagc ccgaagggtc tcaaaggtgt cctcgatctc aatgatctgc 120
tggatgttgt tggtgatggt ggagatgacc ttatcgatga ggtgcaccac cccgttggct 180
gcatggtggt cggctttyar carccgggca cagttcacag ttacaatccc attaggatag 240
tggtggatct nggatgttgg aattctggta catagnaggt gaggggtcat gcccgtgttt 300
cagctcatca gtcaggactc gcctgcccac catatggtaa gcsgragggc atttgagcag 360
ctcaatgttt gacattgctg gaccagggga gttccagcac ttctangang a 411

<210> 12
<211> 560
<212> DNA
<213> Homo sapien

<400> 12
tacttgcctg gagatwgcyt tykckwtmtg ytcwrawgtc cgtggataca gaaatctctg 60
caggcaagtt gctccagagc atattgcagg acaagcctgt aacgaatagt taaattcacg 120
gcatctggat tcctaatcct tttccgaaat ggcaggtgtg agtgcctgta taaaatattc 180
tatgtttacc ttcaacttct tgttctggct atgtggtatc ttgatcctag cattagcaat 240
atgggtacga gtaagcaatg actctcaagc aatttttggt tctgaagatg taggctctag 300
ctcctacgtt gctgtggaca tattgattgc tgtaggtgcc atcatcatga ttctggcttt 360
cctgggatgc tgcggtgcta taaaagaaag tcgctgcatg cttctgttgt ttttcatagg 420

cttgcttctg atcctgctcc tgcaggtggg cgacaggtat cctaggagct gttttcaaat 480
ctaagtctga tcgcattgtg aatgaaactc tctatgaaaa cacaaagctt ttgagcgcca 540
caggggaaag tgaaaaacaa 560

<210> 13
<211> 150
<212> DNA
<213> Homo sapien

<400> 13
gggcaggctg tctttttaaa atgtctcggc tagctagacc acagatatct tctagacata 60
ttgaacacat ttaagatttg agggatataa gggaaaatga tatgaatgtg tatttttact 120
caaaataaaa gtaactgttt acgttggtga 150

<210> 14
<211> 403
<212> DNA
<213> Homo sapien

<400> 14
ctgctgcctg tggcgtgtgt gggctggatc ccttgaaggc tgagtttttg agggcagaaa 60
gctagctatg ggtagccagg tgttacaaag gtgctgctcc ttctccaacc cctacttggt 120
ttccctcacc ccaagcctca tgttcatacc agccagtggg ttcagcagaa cgcatgacac 180
cttatcacct ccctccttgg gtgagctctg aacaccagct ttggcccctc cacagtaagg 240
ctgctacatc aggggcaacc ctggctctat cattttcctt ttttgccaaa aggaccagta 300
gcataggtga gccctgagca ctaaaaggag gggtccctga agctttccca ctatagtgtg 360
gagttctgtc cctgaggtgg gtacagcagc cttggttcct ctg 403

<210> 15
<211> 688
<212> DNA
<213> Homo sapien

<220>
<221> misc_feature
<222> (1)...(688)
<223> n = A,T,C or G

<400> 15
caaagcacat tttaatcatt tattttaaaa gggggagtaa agcatttaaa ctgccaatcc 60
tatagactag gacttgaaca tcaaaggaaa aatagacaaa gactagatga taaagtcatt 120
caaaagcaca gaagcacatc acatacacca gcaaggtttc caactactgc actgattaac 180
tagatactct caatagcttt tctatagctc gtcctagaaa aaaaaattaa attttcattt 240
tcttacaagt tccaggctta aacaaaggca aaaattacat gcaacaactg atacactcat 300
aagttgcaca tatgctccaa ggtctttatt agataacaat aaatgctagc actttgtcac 360
tgccatcaga ttttccttat agtcttagag tcatgtaaat aaaagttcca taatgaaatt 420
aaagaaaatt aatttttcta atcttagatc agttccatag aaaactatta atttttttaa 480
agtaggcagt agaagggggt tggtgggggg tggaattggt tagtaagtct ggttctaatc 540
ttctgagctg cctttggaag gaagttatga ggtagaagat tctactgact tttagtaagg 600
tggacaatga gagaaaagaa aaagcaggtg cctcatcnnc agatccttnt ggtatttatn 660
tgccangtnc nanntaatnc atanaaag 688

<210> 16
<211> 408
<212> DNA

<213> Homo sapien

<400> 16
caggtcatca agatgactta caggatgtaa tagggagagc tgtcgagatt ggtgttaaaa 60
agtttatgat tacaggtgga aatctacaag acagtaaaga tgcactgcat ttggcacaaa 120
caaatggtat gtttttcagt acagttggat gtcgtcctac aagatgtggt gaatttgaaa 180
agaataaccc tgatctttac ttaaaggagt tgctaaatct tgctgaaaac aataaaggga 240
aagttgtggc aataggagaa tgcggacttg attttgaccc gactgcagtt ttgtcccaaa 300
gatactcaac tcaaatattt tgaaaaacag tttgaactgt cagaacaaac aaaattacca 360
atgtttcttc attgtccgaa actcacatgc tgaatttttg gacataat 408

<210> 17
<211> 407
<212> DNA
<213> Homo sapien

<400> 17
ggtcctgggg aggccctagg ggagcaccgt gatggagagg acagagcagg ggctccagca 60
ccttctttct ggactggcgt tcacctccct gctcagtgct tgggctccac gggcaggggt 120
cagagcactc cctaatttat gtgctatata aatatgtcag atgtacatag agatctattt 180
tttctaaaac attcccctyc ccactcctct cccacagagt gctggactgt tccaggccct 240
ccagtgggct gatgctggga cccttaggat ggggctccca gctcctttct cctgtgaatg 300
gaggcagaag acctccaata aagtgccttc tgggcttttt ctaacctttg tcttagctac 360
ctgtgtactg aaatttgggc ctttggatcg aatatggtca agaggtt 407

<210> 18
<211> 405
<212> DNA
<213> Homo sapien

<400> 18
tgaagagtca acttgggcct ggaggactga taaagtttgt gattttgagg gcctctaaaa 60
gtattaaagc agcggcagcc gctgcacgca gacatgaggg ctaggttaaa acagtaagat 120
caagttgttt ggacagaaag gctacagagt gtggtcctgg ctcttgtgta agaattacga 180
ccacgctaac catgcctagg aaggaaagga gttattgttt tgtagaaagg tgctggggtt 240
tgagagatca gtcggacacg attggcaggg agagcacgtg tgtttttatg agaattatgc 300
ccgagatagg taacagatga ggaagaaatt tgggcttgat tgaagtaatg ggggctgtct 360
gtgaagcttt gcagcagtac agcctaggta atttgctgag cctaa 405

<210> 19
<211> 401
<212> DNA
<213> Homo sapien

<400> 19
tcctgacatt cctgccttct tatattaata agacaaataa aacaaaatag tgttgaagtg 60
ttggggcagc gaaaattttt ggggggtggt atggagagat aatgggcgat gtttctcagg 120
gctgcttcaa gcgggattag gggcggcgtg ggagcctaga gtgggagaga ttaagctgaa 180
gggaggtctt gtggtaaggg gtgatatcat ggggatgtta gaagaaacat ttgtcgtata 240
gaatgattgg tgatggcctg gatacggttt tggatgattt gagaagctaa atggaagata 300
caaggtccga ataaaaggag gagaaaaatg ggtattaaat gtctaagaat tgggaggacc 360
taggacatct gattagagag tgcctaagga gattcagcat a 401

<210> 20
<211> 331

<212> DNA
<213> Homo sapien

<400> 20
aggtccagct ctgtctcata cttgactcta aagtcatcag cagcaagacg ggcattgtca 60
atctgcagaa cgatgcgggc attgtccaca gtatttgcga agatctgagc cctcaggtcc 120
tcgatgatct tgaagtaatg gctccagtct ctgacctggg gtcccttctt ctccaagtgc 180
tcccggattt tgctctccag cctccggttc tcggtctcca ggctcctcac tctgtccagg 240
taagaggcca ggcggtcgtt caggctttgc atggtctcct tctcgttctg gatgcctccc 300
attcctgcca gacccccggc tatcccggtg g 331

<210> 21
<211> 346
<212> DNA
<213> Homo sapien

<220>
<221> misc_feature
<222> (1)...(346)
<223> n = A,T,C or G

<400> 21
ggtccaccac ttgtacccga tatggacttc cggcttctct gtccaatgga gccacactaa 60
agatctcacc agtcacgtgg tcaattttaa gccaacctct tgtgtctccc ctcagtgaat 120
agcttatgtc cagaccttct ggatccttgg cagtcacatt gcccacttta gtgcctatag 180
ctacatcctc actgactttc gcttggaata cgtgttggga aaattgaggt gcttcattca 240
catctgtcac aataagncgt gaacttggca aaagaacttg cattgtactt cacaccaaac 300
actagaggct caggattttc tgctttgaac acaatgttgg aaacag 346

<210> 22
<211> 360
<212> DNA
<213> Homo sapien

<220>
<221> misc_feature
<222> (1)...(360)
<223> n = A,T,C or G

<400> 22
gaagactccc tctctcggaa gccggatccc gagccgggca ggatggatca ccaccagccg 60
gggactgggc gctaccaggt gcttcttaat gaagaggata actcagaatc atcggctata 120
gagcagccac ctacttcaaa cccagcaccc gcagattgtg caggctgcgt cttcagcacc 180
agcacttgaa actgactctt cccctccacc atatagtagt attactggtg gaagtaccta 240
caacttcaga tacagaagtt tacggtgagt tttatcccgt gccacctccc tatagcgttg 300
ctacctctct tcctacnwta cgatgaaagc tgagaaggct aaagctgctg caatggcatg 360

<210> 23
<211> 251
<212> DNA
<213> Homo sapien

<400> 23
ggcggagctc cacgacgagc tggaaaagga accttttgag gatggctttg caaatgggga 60
agaaagtact ccaaccagag atgctgtggt cacgtatact gcagaaagta aaggagtcgt 120

gaagtttggc tggatcaagg gtgtattagt acgttgtatg ttaaacattt ggggtgtgat 180
gcttttcatt agattgtcat ggattgtggg tcaagctgga ataggtctat cagtccttgt 240
aataatgatg g 251

<210> 24
<211> 421
<212> DNA
<213> Homo sapien

<220>
<221> misc_feature
<222> (1)...(421)
<223> n = A,T,C or G

<400> 24
caggtctttc ccaggtgttg actccagctc cagcttcagc tccagctcca ggtcgggctc 60
cagctccagc cgcagcttar gcagcgggag gttctgtgtc ccagttgttt tccaatttca 120
ccggctcccg tggatgamcg ygggacctgy caswgctcct gtktycctgc yagsacacca 180
cnytttyccg tggacacrar kggaacckct tggaattcac agctyatgtt ctttctcara 240
agtttgagaa agaactttct aaagtgaggg aatatgtcca attaattagt gtgtatgaaa 300
agaaactgtt aaacctaact gtccgaattg acatcatgga raaaggatac catttcttac 360
actgaactgg acttcgagct gatcaaggta gaagtgaagg agatggaaaa actggtcata 420
c 421

<210> 25
<211> 381
<212> DNA
<213> Homo sapien

<220>
<221> misc_feature
<222> (1)...(381)
<223> n = A,T,C or G

<400> 25
gaactttttg tttctttatt ttcaatattt gtcttattaa tatttttctt attttataat 60
gcaattacaa caatttagga nacaaaacaa tataaacaaa agaatgttaa atagtttttt 120
ttaaaaaata gcttgttgct tgcaanaaag tccatataat cttattcccc cccaaatata 180
attttatact ttgcactaaa ccaaaatagc ttatggaaaa ttagtattaa atagctaaac 240
acagaaaacc tacagctata aataacataa aatacagttt aactttaatg ngatgcttaa 300
acaaagcaaa ctatgatgca atatgaatca acttcattaa ttggacaagt ccagnggagg 360
cacaaattag ataagcacta a 381

<210> 26
<211> 401
<212> DNA
<213> Homo sapien

<220>
<221> misc_feature
<222> (1)...(401)
<223> n = A,T,C or G

<400> 26
ggaaaaggga ctggcctctc tgaagagtga gatgagggaa gtggaaggag agctggaaag 60

gaaggagctg gagtttgaca cgaatatgga tgcagtacag atggtgatta cagaagccca 120
gaaggttgat accagaagcc aagaacgctg gggttacaat ccaagacaca ctcaacacat 180
tagacgggct cctgcattct gatggaccaa ccttttcang tggtaagatt gaagangggg 240
cctgggctta cctgggaagc aaaaactttt cccganccaa ggaacccagg attcaaccan 300
gcnacttgcn ggccaaggaa ggcanaactn ggaanaaaag gccccttaag caaaagggnc 360
accttcattt gctnggaaan cagcctttan ttggaatctt g 401

<210> 27
<211> 383
<212> DNA
<213> Homo sapien

<220>
<221> misc_feature
<222> (1)...(383)
<223> n = A,T,C or G

<400> 27
aattgcaact ggacttttat tgggcagtta cnacaacnaa tgttttcana aaaatatttg 60
gaaaaaatat accacttcat agctaagtct tacagagaan aggatttgct aataaaactt 120
aagttttgaa aattaagatg cnggtanagc ttctgaacta atgcccacag ctccaaggaa 180
nacatgtcct atttagttat tcaaatacca gttgagggca ttgtgattaa gcaaacaata 240
tatttgttan aactttgntt ttaaattact gntncttgac attacttata aaggagnctc 300
taactttcga tttctaaaac tatgtaatac aaaagtatan ntttccccat tttgataaaa 360
gggccnanga tactgantag gaa 383

<210> 28
<211> 401
<212> DNA
<213> Homo sapien

<220>
<221> misc_feature
<222> (1)...(401)
<223> n = A,T,C or G

<400> 28
ggtcgcgttt cccctggctc acagtctgcc attatttgca tttttaaatg aagaaaagtt 60
taacgtggat ggatggacag tttacaatcc agtggaagaa tacaggaggc agggcttgcc 120
caatcaccat tggagaataa cttttattaa taagtgctat gagctctgcg acacttaccc 180
tgctcttttg gtggttccgt atcgtgcctc anatgatgac ctccggagag ttgcaacttt 240
taggtcccga aatcgaattc cagtgctgtc atggattcat ccagaaaata agacggtcat 300
tgtgcgttgc agtcagcctc ttgtcggtat gagtgggaaa cgaaataaag atgatgagaa 360
atatctcgat gttatcaggg agactaataa acaaatttct a 401

<210> 29
<211> 401
<212> DNA
<213> Homo sapien

<400> 29
atatgagttt gccatctcca tggatgccat ttcaatgcct tcagggtaat cattctctcc 60
ccaaagactg cccacggggt catcactcct gtgacgaaat gagggctgga ttgaagatgt 120
tctgctgagc acccccctgg tcatctttgg ggtctcagaa gagccataat catgaccatt 180
ctcagcatct gaataatcag gttctctcca agtgcttggc aagttctgat tgtcctcagc 240

actgggatag tctggctccc caaaaaaggg tggagagtta ggttgaatgt cagcgcctgg 300
ataatcaggc tttcccagag agtctgcgta tggattgatt ctaaaacttg tatgttccag 360
attctttctg gatcctggat ggttcaaatt ggctctgggt c 401

<210> 30
<211> 401
<212> DNA
<213> Homo sapien

<400> 30
cctgaactat ttattaaaaa catgaccact cttggctatt gaagatgctg cctgtatttg 60
agagactgcc atacataata tatgacttcc tagggatctg aaatccataa actaagagaa 120
actgtgtata gcttacctga acaggaatcc ttactgatat ttatagaaca gttgatttcc 180
cccatcccca gtttatggat atgctgcttt aaacttggaa gggggagaca ggaagtttta 240
attgttctga ctaaacttag gagttgagct aggagtgcgt tcatggtttc ttcactaaca 300
gaggaattat gctttgcact acgtccctcc aagtgaagac agactgtttt agacagactt 360
tttaaaatgg tgccctacca ttgacacatg cagaaattgg t 401

<210> 31
<211> 297
<212> DNA
<213> Homo sapien

<400> 31
acctccatta atgccaggtg ttcctcctct gatgccagga atgccaccag ttatgccagg 60
catgccacct ggattgcatc atcagagaaa atacacccag tcattttgcg gtgaaaacat 120
aatgatgcca atgggtggaa tgatgccacc tggaccagga ataccacctc tgatgcctgg 180
aatgccacca ggtatgcccc cacctgttcc acgtcctgga attcctccaa tgactcaagc 240
acaggctgtt tcagcgccag gtattcttaa tagaccacct gcaccaacag caactgt 297

<210> 32
<211> 401
<212> DNA
<213> Homo sapien

<400> 32
caaacctgga gccaaaaagg acacaaagga ctctcgaccc aaactgcccc agaccctctc 60
cagaggttgg ggtgaccaac tcatctggac tcagacatat gaagaagctc tatataaatc 120
caagacaagc aacaaaccct tgatgattat tcatcacttg ggtgagtgcc cacacagtca 180
agctttaaag aaagtgtttg ctgaaaataa agaaatccag aaattggcag agcagtttgt 240
cctcctcaat ctggtttatg aaacaactga caaacacctt tctcctgatg gccagtatgt 300
ccccaggatt atgtttgttg acccatctct gacagttaga gcccgatatc actggaagat 360
attcaaaccg tctctatgct tacgaacctg cagatacagc t 401

<210> 33
<211> 401
<212> DNA
<213> Homo sapien

<400> 33
agcagaggga caggaatcat tcggccactg ttcagacggg agccacaccc ttctccaatc 60
caagcctggc cccagaagat cacaaagagc caaagaaact ggcaggtgtc cacgcgctcc 120
aggccagtga gttggttgtc acttactttt tctgtgggga agaaattcca taccggagga 180
tgctgaaggc tcagagcttg accctgggcc actttaaaga gcagctcagc aaaaagggaa 240
attataggta ttacttcaaa aaagcaagcg atgagtttgc ctgtggagcg gtgtttgagg 300

agatctggga ggatgagacg gtgctcccga tgtatgaagg ccggattctg ggcaaagtgg 360
agcggatcga ttgagccctg gggtctggct ttggtgaact g 401

<210> 34
<211> 401
<212> DNA
<213> Homo sapien

<400> 34
aacaatggct atgaaggcat tgtcgttgca atcgacccca atgtgccaga agatgaaaca 60
ctcattcaac aaataaagga catggtgacc caggcatctc tgtatctgtt tgaagctaca 120
ggaaagcgat tttatttcaa aaatgttgcc attttgattc ctgaaacatg gaagacaaag 180
gctgactatg tgagaccaaa acttgagacc tacaaaaatg ctgatgttct ggttgcttga 240
gtctactcct ccaggtaatg atgaacccta cactgagcag atggggcaac tgtggagaga 300
aggggtgaaa ggatcccacc tcactcctga tttcattgca ggaaaaaagt tagcttgaat 360
atggaccaca aggtaagggc atttgtccat gaatggggct c 401

<210> 35
<211> 401
<212> DNA
<213> Homo sapien

<220>
<221> misc_feature
<222> (1)...(401)
<223> n = A,T,C or G

<400> 35
catttcttcc tactagactg cccccttgat ccactggcag aaatgatggc accaccttgt 60
cttcaggtgg tgctccttca ttattccaag gatgcagcat ctctatggtg ccaggtatgg 120
gggtaaagcc tttggcgccc tttccgcaat ggcacatcag cagtaaaagt ggtaccaata 180
gcangaacag aaagggcaaa atcatgancg caattgctgc gggtcccaag cccacatagg 240
aatcatgctg ngcttccctg canccgctgc catgcaagac actnacaaac tgngantgta 300
aggacctgct tttcaggaca actaaaaccc tgattgnctg aaatcaggaa ctgaatttca 360
cttctcccaa gctttttctc actttggtgc aacancacac t 401

<210> 36
<211> 401
<212> DNA
<213> Homo sapien

<400> 36
   cctgctagaa tcactgccgc tgtgctttcg tggaaatgac agttccttgt tttttttgtt 60
   tctgtttttg ttttacatta gtcattggac cacagccatt caggaactac cccctgcccc 120
   acaaagaaat gaacagttgt agggagaccc agcagcacct ttcctccaca caccttcatt 180
   ttgaagttcg ggtttttgtg ttaagttaat ctgtacattc tgtttgccat tgttacttgt 240
   actatacatc tgtatatagt gtacggcaaa agagtattaa tccactatct ctagtgcttg 300
   actttaaatc agtacagtac ctgtacctgc acggtcaccc gctccgtgtg tcgccctata 360
   ttgagggctc aagctttccc ttgttttttg aaaggggttt a 401

<210> 37
<211> 401
<212> DNA
<213> Homo sapien

<220>
<221> misc_feature
<222> (1)...(401)
<223> n = A,T,C or G

<400> 37
cnnctntgna atggantnnt tgnctaaaan ganttgatga tgatgaanat ccctangang 60
antaagcatg gancntgatc ntttnctnng cactccttta cgacacggaa acangnatca 120
ncatgatggt accaganacc ttatcaccna cgcgcacnga nctgactnat tccaaagagt 180
tgnggttacg gncatccggt cattgctcgt gcccattgct gcagggctga tnctactggt 240
gcttattatg ntggccctga ggatgctcca caatgaatat aagcatgctg catgatcagc 300
ggcaacanat gctctgccgt ttgcactaca tctttcacgg acacnatntc gaanacgggc 360
acnttgcana gttagacttg gaatgcatgg ngccggncan n 401

<210> 38
<211> 401
<212> DNA
<213> Homo sapien

<400> 38
aattggctca ctctctcaag gcaagcactg tctcaaggca gtctcaaggc agagatgaca 60
cagcaaaaaa cagaggggga gaaaaaagtc tattattggc ttgtgattta caaaagccaa 120
agtcctttag ataaaaggcc aggagtcgta ccaacataga taccaaatcc aggagaacac 180
agaccagcga taagagggac gcttccccat gacccagacc agcctaaagc ccctgtgggg 240
gcagccagtg gggagctgtc agaccttgga catggtggtc tttgagaatg ggtctgccct 300
tctctccctg accagttggg atagacacct gactggaatc cttgacactg gcaggtgttt 360
ctatgaacag agaggactgt gcctgtcttc ctgaatccca a 401

<210> 39
<211> 401
<212> DNA
<213> Homo sapien

<220>
<221> misc_feature
<222> (1)...(401)
<223> n = A,T,C or G

<400> 39
tctggtangg agcaattcta ttatttggca ttgcatggct gggttgaatt aaaacaggga 60
gtgagaacag gtgagtctag aagtccaact ctgaaaagga ccactgtaca tttgaacaca 120
cggctgtgtt aaagatgctg ctaatgtcag tcactgggtg cactaaagga tctcttattt 180
tatgtaaaac gttgggaatg acaagatana actgatactc tggtaagtta ccctctgaag 240
ctacttcttg tgaaatacta atgacagcat catcctgcca agcgaaagag gcaggcataa 300
gcaaggacaa attaaaaggg ggtaagagcc ttatcatgat gaggagtctt gttttgacat 360
cttgggaaaa gctgtccata gtgtgaagtc gtcaatttct c 401

<210> 40
<211> 401
<212> DNA
<213> Homo sapien

<400> 40
tctggtcacc caactcttgt ggaagagggg aattgagatc gagtactgaa tatctggcag 60
agaggctgga atccttcagc cccagagccc agggaccact ccagtagatg cagagagggg 120

cctgcccagg ggtcagggca gtgggtatca ctggtgacat caagaatatc agggctgggg 180
aggcatcttt gtttcctggt gccctcctca aagttgctga cactttgggg acgggaaggg 240
gtagaagtag ggctgctcct tttggagctg gagggaatag acctggagac agagttgagg 300
cagtcgggct gtccaggttc taagcatcac agcttctgca ctgggctctg aggagattct 360
cagccagagg atcccagcct cctcctccct caaatgtcaa g 401

<210> 41
<211> 401
<212> DNA
<213> Homo sapien

<220>
<221> misc_feature
<222> (1)...(401)
<223> n = A,T,C or G

<400> 41
ctggactaaa aatgtccact atggggtgca ctctacagtt tttgaaatgc taggaggcag 60
aaggggcaga gagtaaaaaa catgacctgg tagaaggaag agaggcaaag gaaactaggt 120
ggggaggatc aattagagag gaggcacctg ggatccacct tcttccttan gtcccctcct 180
ccatcagcaa aggagcactt ctctaatcat gccctcccga agactggctg ggagaaggtt 240
taaaaacaaa aaatccagga gtaagagcct taggtcagtt tgaaattgga gacaaactgt 300
ctggcaaagg gtgcganagg gagcttgtgc tcangagtcc agcccgtcca gcctcggggt 360
gtangtttct gaagtgtgcc attggggcct caccttctct g 401

<210> 42
<211> 310
<212> DNA
<213> Homo sapien

<400> 42
ggttcgacaa atccccaaaa atggcaaatt aagccctgtg acaaaataag ttattggatc 60
atacagaaat agcccaaatc tggaaatttt gaattaaaat tgtaatcctg taaaacaagt 120
tttggggtga atggatttct ttaataccaa taatattttt aattcccacc acagatggat 180
ttgctgaata tgctaatgct gtgaatgaga aaacaatttt ggggtaggta tacccacaag 240
taatctgatg acaaaataaa ccacagactg atgtcaaatg gacaaaaaac tgaaaatatg 300
ctgtgagaaa 310

<210> 43
<211> 401
<212> DNA
<213> Homo sapien

<400> 43
aggtcactta cacttgtgac cagtgtgggg cagagaccta ccagccgatc cagtctccca 60
ctttcatgcc tctgatcatg tgcccaagcc aggagtgcca aaccaaccgc tcaggagggc 120
ggctgtatct gcagacacgg ggctccagat tcatcaaatt ccaggagatg aagatgcaag 180
aacatagtga tcaggtgcct gtgggaaata tccctcgtag tatcacggtg ctggtagaag 240
gagagaacac aaggattgcc cagcctggag accacgtcag cgtcactggt attttcttgc 300
caatcctgcg cactgggttc cgacaggtgg tacagggttt actctcagaa acctacctgg 360
aagcccatcg gattgtgaag atgaacaaga gtgaggatga t 401

<210> 44
<211> 401
<212> DNA

<213> Homo sapien

<400> 44
atccctgtaa gtctattaaa tgtaaataat acatacttta caacttctct tagtcggccc 60
ttggcagatt aaatctttgc aaaattccat atgtgctatt gaaaaatgaa ataaaacctc 120
agatgtctga attcttattt caaatacagt tatataatta ttttaaatta caatatacaa 180
tttctgttaa atacaactgt taagggattc tgagaacaat tataagatta taataatata 240
tacaaactaa cttctgaaat gacatgggtt gtttccttcc caccctccta ccctctcaaa 300
gagtttttgc atttgctgtt cctggttgca aaaggcaaaa gaaaatctaa aaatagtctg 360
tgtgtgtcca cgacatgctc gctcctttga gaatctcaaa c 401

<210> 45
<211> 401
<212> DNA
<213> Homo sapien

<220>
<221> misc_feature
<222> (1)...(401)
<223> n = A,T,C or G

<400> 45
gtgcctgctg cctggcagcc tggccctgcc gctgcctcag gaggcgggag gcatgagtga 60
gctacagtgg gaacaggctc aggactatct caagagattt tatctctatg actcagaaac 120
aaaaaatgcc aacagtttag aagccaaact caaggagatg caaaaaattc tttggcctac 180
ctatactgga atggtaaact cccgcgtcat anaaataatg caanaagccc agatgtggag 240
tgccagatgt tgcagaatac tcactatttc caaatagccc aaaatggact tccaaagtgg 300
tcacctacag gatcgtatca tatactcgag acttaccgca tattacagtg gatcgattag 360
tgtcaaaggc tttaaacatg tggggcaaag agatccccct g 401

<210> 46
<211> 401
<212> DNA
<213> Homo sapien

<220>
<221> misc_feature
<222> (1)...(401)
<223> n = A,T,C or G

<400> 46
gtcagaattg tctttctgaa aggaagcact cggaatcctt ccgaactttc caagtccatc 60
catgattcan agatactgcc ttctctctct ctgggatttt atgtgtttct gatagtgaat 120
tgttgatgta tttgctactt tgcttctttt ctctttcaag acttgatcat tttatatgct 180
gnttggagaa aaaaagaact tttggtagca aggaggtttc aagaaatgat tttggatttt 240
ctgctgcgga atttctcggc acctacctgt agtatggggc acttggtttg gttgcagagt 300
aagaaggtgg aagaatgagc tgtacttggt taagcagttg aaaccttttt tgagcaggat 360
ctgtaaaagc ataattgaat ttgtttcacc cccgtggatt c 401

<210> 47
<211> 401
<212> DNA
<213> Homo sapien

<400> 47

ggtctgcagc aatgcacttc aaccatacat actgcttcca ctagctaata ccaaatgcag 60
gttctcagat ccagacaaat ggaggaaaag aacatttatg cttccgtttc agaaagccaa 120
gtcgtagttt tggcccttcc tttctctaaa gtttattccc aaaaacaggt agcattcctg 180
attgggcaga gaagaggata ttttcagccc acatctgctg caggtatgtc attttctccc 240
atcttcactg tgactagtaa agatctcacc acttctcttt ggaatttcca actttgcttg 300
tgattgaatg tcacttcgtg aatttgtatt atgtcagatc acttggcatt gctcttccat 360
atgcatcaag ttgccaggca ctaaacccaa tgttcatgaa c 401

<210> 48
<211> 430
<212> DNA
<213> Homo sapien

<400> 48
acataacttg taaacttttt ctgcttgggg gctgtaacag acagaagagt aaagactaca 60
aggatttctt gaagatgctt caatgaaaat catcatttcc tctttagtca tcccaagtct 120
tggtttgaaa aacttgggca tggacttata cagaccttga accaccactg acttatcatt 180
gggtggcaga ccttgaaacc aagctctctg tgttacttct gaaagtgcat caattctgat 240
ttggctaaga acagaagaca aatactggga tcgtgattct gtgttatact ctagccacag 300
catagcagct tctcgaacgg tttcttcctt ttctacattt aaattgtcac tactgagaat 360
atctatcagt aggtcatgtg acagacctgc cccgggccgg gcccgctcga tgcttgccga 420
atatcatggt 430

<210> 49
<211> 57
<212> DNA
<213> Homo sapien

<220>
<221> misc_feature
<222> (1)...(57)
<223> n = A,T,C or G

<400> 49
ggtattaaca atatcangca ctcattcttc ccctcttatg aaanggatna attttta 57

<210> 50
<211> 327
<212> DNA
<213> Homo sapien

<220>
<221> misc_feature
<222> (1)...(327)
<223> n = A,T,C or G

<400> 50
gatggnggtn tccacaagan tnaangtncn tattaantan nncttgtaga nccacttnna 60
ttaattgnnn tatgnntgnc cttctggtgg ntgtngaagc ttcatatnnt ntttggacat 120
cattacacgt cttagctctt tnaagnacaa ctttaatgct atatgaattt tgccattttn 180
gctaacactg gtatgctccn ngcatccacc atnccacntg gaattattta ttncnttcat 240
attaatnttt tgtttaccaa atctnacttg acccgaacga aactttctgn gtattttang 300
gccccnccat tcttactttt caagcct 327

<210> 51

<211> 236
<212> DNA
<213> Homo sapien

<400> 51
cgtctcgaag aagcgctgca ggccgatgat ggactgcacg tctgccttgt cctcagttaa 60
cttgttgaat tgcttgaaca tgcggcccac atcctgggca aactcctgtg gggagctgta 120
gggaggtgac aacttctcct ggaggcgggc acggatcagg gtcagatcca gggtgccacc 180
gggctggtcc agggagaagg tggagtcgta gccagacctg cccgggcggc cgctcg 236

<210> 52
<211> 291
<212> DNA
<213> Homo sapien

<220>
<221> misc_feature
<222> (1)...(291)
<223> n = A,T,C or G

<400> 52
ctcacatcct gggtccggct gtagagctgc accatggtgc tgagcgcccc ctccagctcc 60
ttgtagatgt aaaggacggc gaaggagctg tagtctgtgt ccacgatgcg cacgtccagg 120
tagcccaagg ccgggactct gaagttgtcc ctcggagccc accttcangt actcgggcat 180
ccacctggtt acagccnttc gncctcggna actccatntg gactttacag gccgccctcc 240
tctgtgggcc tgatggncct tgcaggacat nggaacacgg gagctcnctt t 291

<210> 53
<211> 95
<212> DNA
<213> Homo sapien

<220>
<221> misc_feature
<222> (1)...(95)
<223> n = A,T,C or G

<400> 53
gtctgtgcag tttctgacac ttgttgttga acatggntaa atacaatggg tatcgctgan 60
cactaagttg tanaanttaa caaatgtgct gnttg 95

<210> 54
<211> 66
<212> DNA
<213> Homo sapien

<220>
<221> misc_feature
<222> (1)...(66)
<223> n = A,T,C or G

<400> 54
cctnaatnat ntnaatggta tcaatnnccc tgaangangg gancggngga agccggnttt 60
gtccgg 66

<210> 55
<211> 265
<212> DNA
<213> Homo sapien

<220>
<221> misc_feature
<222> (1)...(265)
<223> n = A,T,C or G

<400> 55
atctttcttc tcagtgcctt ggccntgttg agtctatctg gtaacactgg agctgactcc 60
ctgggaagag aggccaaatg ttacaatgaa cttaatggat gcaccaagat atatgaccct 120
gtctgtggga ctgatggaaa tacttatccc aatgaatgcc gtgttatgtt tttgaaaatc 180
ggaaacgcca gacttctatc ctcattcaaa aatctgggcc ttnctgaaaa ccagggtttt 240
naaaatccca ttcnggtcnc cggcg 265

<210> 56
<211> 420
<212> DNA
<213> Homo sapien

<220>
<221> misc_feature
<222> (1)...(420)
<223> n = A,T,C or G

<400> 56
gagcggccgc ccgggcaggt cctcgcggtg acctgatggg atttcaaaac cttggttctc 60
agcaaggccc agatttttga atgangatag aagtctggcg tttccgattt tcaaaacata 120
acacgcattc attgggataa gtatttccat cagtcccaca gacngggtca tatatcttgg 180
gtgcatccat taagttcntt tgttaacatt tgggcctctc tttcccangg gaattcagct 240
cccagttgtt taccaanatt naactccacc ggggccaaag gcncttgaaa aaaaaaanaa 300
ttccttgttt accttccttg ggcttnaagt tctggcgtcc aaaagttcaa tttgaaaact 360
gcaccgcact taccacgtct cttcnagaan cctggggaca cctcggccgc gaccacgcta 420

<210> 57
<211> 170
<212> DNA
<213> Homo sapien

<400> 57
gaagcggagt tgcagcgcct ggtggccgcc gagcagcaga aggcgcagtt tactgcacag 60
gtgcatcact tcatggagtt atgttgggat aaatgtgtgg agaagccagg gaatcgccta 120
gactctcgca ctgaaaattg tctctccaga cctcggccgc gaccacgcta 170

<210> 58
<211> 193
<212> DNA
<213> Homo sapien

<400> 58
attttcagtg cgagagtcta ggcgattccc tggcttctcc acacatttat cccaacataa 60
ctccatgaag tgatgcacct gtgcagtaaa ctgcgccttc tgctgctcgg cggccaccag 120
gcgctgcaac tccgcttcat cggcttcgcc cagctccgcc attgttcgcc acctgcccgg 180

gcggccgctc gaa                                                          193

<210> 59
<211> 229
<212> DNA
<213> Homo sapien

<400> 59
cgcaactctc gagcatttat atacaatagc aaatcatcca gtgtgttgta cagtctataa      60
tactccaaca gtctcccatc tgtattcaat ggcgccaccc aatacagtcc tttgtttgga     120
tgctggggag agtaatccct accccaagca ccatatagat aagaaaaccc tctccagttg     180
agctgaacca cagacggttt gctgatacct gcccgggcgg ccgctcgaa                 229

<210> 60
<211> 340
<212> DNA
<213> Homo sapien

<400> 60
tcgagcggcc gcccgggcag gtcctctaaa gatcaaaaca cccctgtcgt ccaccctcct      60
cccactccag ggaagctgtg gtcatggtgg tgtggtgaac atcagcaaac cgtctgtggt     120
tcagctcaac tggagagggt tttcttatct atatggtgct tggggtaggg attactctcc     180
ccagcatcca aacaaaggac tgtattgggt ggcgccattg aatacagatg ggaaactgtt     240
ggagtattat aaactggtac aacacactgg atgatttgct attgtatata aatgctcgag     300
aattgcggat cacctatgga cctcggccgc gaccacgctg                           340

<210> 61
<211> 179
<212> DNA
<213> Homo sapien

<220>
<221> misc_feature
<222> (1)...(179)
<223> n = A,T,C or G

<400> 61
tttttgtgac ggacgnttgg agtacatgtc ccaggatcac atccagcagc tagagtggct      60
gggacaagct ggcggnggcc aagcactgtt gaaacnatag gggtctgggn gnactcgggt     120
tnaagtggtt ggtccgantn ttnataacct tgtcngaacc nancatctcg gttgncang      179

<210> 62
<211> 78
<212> DNA
<213> Homo sapien

<220>
<221> misc_feature
<222> (1)...(78)
<223> n = A,T,C or G

<400> 62
agggcgttcg taacgggaat gccgaagcgt gggaaaaagg gagcggtggc nggaagacgg      60
ggatgagctt angacaga                                                    78

<210> 63
<211> 410
<212> DNA
<213> Homo sapien

<220>
<221> misc_feature
<222> (1)...(410)
<223> n = A,T,C or G

<400> 63
cccagttact tggggaggct gaggcaggga gaatcctttg aacccggngg gtgggaggtt 60
gcagtgagcc cgagatagca ccattgcact tccancatgg ggtggacaga gtgagactct 120
atctcaaaaa aaaagaaaag aaaaggaaag agattagatt aagattaagt acctacttcc 180
tntcccattt caagtcctga aaatagagga tcagaaatgt tgaggaattc tttaggatag 240
aaagggagat gggattttac ttatggggaa agaccgcaaa taaagactgn aacttaacca 300
cattccccaa gtgnaaggtg ttacccaaga agtaggaacc cttttggctn ttaccttacc 360
ttccngaaaa aaacttattn cttaaaatgg aaacccttaa agcccgggca 410

<210> 64
<211> 199
<212> DNA
<213> Homo sapien

<220>
<221> misc_feature
<222> (1)...(199)
<223> n = A,T,C or G

<400> 64
cttgttctca aaaaggtcaa agggagcccg acgaggaata aatagcaatg ccctgaattc 60
caactgacct tctacagaaa agtgcttgac tgccaagtgg tcttcccagt cattagtgag 120
gctcttgtag aattctccat actcctcttg ggngangnca tnagggtttn nggcccaaat 180
aggntgggcc tngttaagt 199

<210> 65
<211> 125
<212> DNA
<213> Homo sapien

<220>
<221> misc_feature
<222> (1)...(125)
<223> n = A,T,C or G

<400> 65
agcggtacag ttctgtcctg gcatcatcat tcattgtagt atggtcaata ggtgccatga 60
aactcagtag cttgctaagg acatgaaacc gaagtttcct gcctttgctg gcctngtngn 120
gggta 125

<210> 66
<211> 204
<212> DNA
<213> Homo sapien

<400> 66
attcagaatt ctggcatcgg tatttctata aagtccatca gttagagcag gagcaggccc 60
ggagggacgc cctgaagcag cgggcggaac agagcatctc tgaagagccc ggctgggagg 120
aggaggaaga ggagctcatg ggcatttcac ccatatctcc aaaagaggca aaggttcctg 180
tggacctcgg ccgcgaccac gcta 204

<210> 67
<211> 383
<212> DNA
<213> Homo sapien

<220>
<221> misc_feature
<222> (1)...(383)
<223> n = A,T,C or G

<400> 67
tcagggcctc caggcagcca gttttgcagg anattcagca cctagngtct tcctgcctna 60
cgctcccaag aacctgctcc tgcaggggga acatcagaac tcgtccttga tgtcaaaatg 120
gggctggtct tnaggcttga agtccaggtt agggctgcca tcctcattga gaattctccg 180
ggcagtgtan ccgacgatgg ggtatttggc tttgtacact ttggtgaaaa cctnatccag 240
ggcctccagt tccttggccg tganacccgt antgtcatgg gtgaggtctg caggatccaa 300
ggacatcttg gctacccctc tagtggagtc cttccccgtc aaggcattgt aaggggctcc 360
tcgtccataa aactcctttt cgg 383

<210> 68
<211> 99
<212> DNA
<213> Homo sapien

<400> 68
tcacatctcc tttttttttt aactttttca aatttttgtg ttaaatagaa ggctaaaggg 60
ttagatttaa gtttctgcta cattgaccct atttaccta 99

<210> 69
<211> 37
<212> DNA
<213> Homo sapien

<220>
<221> misc_feature
<222> (1)...(37)
<223> n = A,T,C or G

<400> 69
gagaaggacn tacggncctg ntantanang aatctcc 37

<210> 70
<211> 222
<212> DNA
<213> Homo sapien

<220>
<221> misc_feature
<222> (1)...(222)

<223> n = A,T,C or G

<400> 70
gtgggtcatt tttgctgtca ccagcaacgt tgccacgacg aacatccttg acagacacat 60
tcttgacatt gaagcccaca ttgtccccag gaagagcttc actcaaagct tcatggcgca 120
tttcgacaga ttttacttcc gttgtaacgt tgactggagc aaaggtgacc accataccgg 180
gtttgagaac acccantcac ctgccccggg cggccgctcg aa 222

<210> 71
<211> 428
<212> DNA
<213> Homo sapien

<220>
<221> misc_feature
<222> (1)...(428)
<223> n = A,T,C or G

<400> 71
caggagtatt ttgtagaaaa gccagaagag cattagtaga tgtatggaaa tatacggtag 60
ggcacacgct gacagtactt ttcccaagcc acgccgtatt tcttcttaca gtggtactcg 120
tcacgagctt ctcggtggac aagcaacatg gtgaaataaa ttatgtagaa ataaggcaga 180
atgtggttaa aaccacatgg gagggaccac gccaaggcca tgatgagatc acccaagtaa 240
ttggggtggc gaacaaagcc ccaccatcca gaaactagaa naatttttcc cgttgaaata 300
tgaatggntt ttaaatgtgc aagctttgga tcactgggaa ttttcccgaa tgcctttttc 360
tganaattgc accttnggaa gantccttac cccaagnttc agaccattat ttnaaaagcn 420
ttggaact 428

<210> 72
<211> 264
<212> DNA
<213> Homo sapien

<220>
<221> misc_feature
<222> (1)...(264)
<223> n = A,T,C or G

<400> 72
gaataaagag cttactggaa tccagcaggg ttttctgccc aaggatttgc aagctgaagc 60
tctctgcaaa cttgatagga gagtaaaaag ccacaataga gcagtttatg aagatcttgg 120
aggagattga cacacttgat cctgccagaa aatttcaaag acagtagatt gaaaaggaaa 180
ggctttggta aaaaaaggtt caggcattcc tagccgantg tgacacagtg gagcanaaca 240
tctgcangag actgancggc tgca 264

<210> 73
<211> 442
<212> DNA
<213> Homo sapien

<220>
<221> misc_feature
<222> (1)...(442)
<223> n = A,T,C or G

<400> 73
ggcgaatccg gcgggtatca gagccatcag aaccgccacc atgacggtgg gcaagagcag 60
caagatgctg cagcatattg attacaggat gaggtgcatc ctgcaggacg gccggatctt 120
cattggcacc ttcaaggctt ttgacaagca catgaatttg atcctctgtg actgtgatga 180
gttcagaaag atcaagccaa agaacttcaa acaagcagaa agggaagaga agcgagtcct 240
cggtctggng ctgctgccaa gggagaatct ggtctcaatg acngtagaag gaccttcttc 300
caaagatact ggnattgctc gagttccact tgctggaact tcccggggcc caaggatcgc 360
aaggcttctg gcaaaagaaa tccanacttn ggccgggacc acctaancca attcacacac 420
tggcggccgt actagtggat cc 442

<210> 74
<211> 337
<212> DNA
<213> Homo sapien

<220>
<221> misc_feature
<222> (1)...(337)
<223> n = A,T,C or G

<400> 74
ggtagcagcg tctccagagc ctgatctggg gtcccagata cccaggcagc agcagccctg 60
gaggtaaagg gcaagctccc caatgtgagg ggagacccca ttcctggtca gccaggcttt 120
cagaggagat agcaggtcga gggagccaac gaagaagaga ctgccancag gggaaggact 180
gtcccgccaa ggacagaact gattcagggg ggtcaatgct cctctagaga agagccacac 240
agaactgggg ggtccaggaa ccatgaanct tggctgtggt ctaaggagcc aggaatctgg 300
acagtgttct gggtcatacc aggattctgg aattgta 337

<210> 75
<211> 588
<212> DNA
<213> Homo sapien

<220>
<221> misc_feature
<222> (1)...(588)
<223> n = A,T,C or G

<400> 75
catgatgagt tctgagctac ggaggaaccc tcatttcctc aaaagtaatt tatttttaca 60
gcttctggtt tcacatgaaa ttgtttgcgc tactgagact gttactacaa actttttaag 120
acatgaaaag gcgtaatgaa aaccatcccg tccccattcc tcctcctctc tgagggactg 180
gagggaagcc gtgcttctga ggaacaactc taattagtac acttgtgttt gtagatttac 240
actttgtatt atgtattaac atggcgtgtt tatttttgta tttttctctg gttgggagta 300
tgatatgaag gatcaagatc ctcaactcac acatgtagac aaacattagc tctttactct 360
ttctcaaccc cttttatgat tttaataatt ctcacttaac taattttgta agcctgagat 420
caataagaaa tgttcaggag agangaaaga aaaaaaatat atgttcccca tttatattta 480
gagagagacc cttantcttg cctgcaaaaa gtccaccttt catagtagta ngggccacat 540
attacattca gttgctatag gncagcactg aactgcatta cctgggca 588

<210> 76
<211> 196
<212> DNA
<213> Homo sapien

<400> 76
   gcggtatcac agcctggccc ccatgtacta tcgggggccc caggctgcca tcgtggtcta 60
   tgacatcacc aacacagata catttgcacg ggccaagaac tgggtgaagg agctacagag 120
   gcaggccagc cccaacatcg tcattgcact cgcgggtaac aaggcagacc tggacctgcc 180
   cgggcggccg ctcgaa 196

<210> 77
<211> 458
<212> DNA
<213> Homo sapien

<220>
<221> misc_feature
<222> (1)...(458)
<223> n = A,T,C or G

<400> 77
agtagagatg gggtttcact gtgttaacca ggatggtctt gatctcctgg cctcgtgatc 60
tgcccgcctc ggcctcccaa agtgttggga ttacaggcgt gaaccaccgc acccggccag 120
aaatgttagt ttttccctat tctctctcct ttttcctatt atatacttgg tcaaccagac 180
agccatccta ccccanaatg gtaatgcctc ttcattcctc atatgaggga ataaaagaga 240
aaaaagcttt tggaaaacat ccacttatct aatcatccca aatatgtaat caaaagtata 300
caactcatgt gaagaataca ctggtaaaat gttantatag gccaaggtat cttgaattcc 360
tatatagaaa gctggtaaat gcccttttgg ctggaaccgc catcttccnn taattcnccc 420
aaaatgacca aacacaaagg gnaagangan aagccccc 458

<210> 78
<211> 464
<212> DNA
<213> Homo sapien

<220>
<221> misc_feature
<222> (1)...(464)
<223> n = A,T,C or G

<400> 78
tccgcaaatt tcctgccggc aaggtcccag catttgaggg tgatgatgga ttctgtgtgt 60
ttgagagcaa cgccattgcc tactatgtga gcaatgagga gctgcgggga agtactccag 120
aggcagcagc ccaggtggtg cagtgggtga gctttgctga ttccgatata gtgcccccag 180
ccagtacctg ggtgttcccc accttgggca tcatgcacca caacaaacag gccactgaga 240
atgcaaagga ggaagtgagg cgaattctgg ggctgctgga tgcttacttg aagacgagga 300
cttttctggt gggcgaacga gtgacattgg ctgacatcac agttgtctgc accctgttgt 360
ggctctataa gcaggntcta gaaccttctt ttcgcangac cttcggccgg accacgctta 420
acccaaattc cacacacttg cnggccgtac taanggaatc ccac 464

<210> 79
<211> 380
<212> DNA
<213> Homo sapien

<220>
<221> misc_feature
<222> (1)...(380)
<223> n = A,T,C or G

<400> 79
ctgtatgacc agtttttcca tctccttcac ttctaccttg atcagctcga agtccagttc 60
agtgtaagaa atggtatcct tctccatgat gtcaattcgg acagttaggt ttaacagttt 120
cttttcatac acactaatta attggacata ttccctcact ttanaaagtt ctttctcaaa 180
cttctganaa aagaacatga actgtgaatt ccaagcgttc ccactctgtc cacgggaaaa 240
ggtggtgtct ggcagggaaa cagaacactg gcaggtccac ggtcatccac ggagccggtg 300
aaattgggaa aacaactggg acacagaacc tccgctgcct aagctgcggn tgggagcttg 360
gaacccgacc tggaactgga 380

<210> 80
<211> 360
<212> DNA
<213> Homo sapien

<220>
<221> misc_feature
<222> (1)...(360)
<223> n = A,T,C or G

<400> 80
tcgagcggcc gcccgggcag gtcctcagag agctgtttgt tncgcttctt caaaaactcc 60
tattctccac ttctgctaaa ggactggatg acatcaattg tgatagcaat atttgtgggt 120
gttctgtcan ncancatcgc actcctgaac aaagtagatg ttggattgga tcagtctctt 180
tccacccaga tgactcctan atggtggatn atttcaaatc catcantcag tacctgcatg 240
cgnggtccgc ctgtgtnctt tgtcctgcag gangggcnct actacacttc ttccnagggg 300
canaacatgg tgtgcngcgg ccatgggctg gcaacantga ttcnctgctg cacccanatn 360

<210> 81
<211> 440
<212> DNA
<213> Homo sapien

<220>
<221> misc_feature
<222> (1)...(440)
<223> n = A,T,C or G

<400> 81
acgtggtccg gcgagtctga cctgcagata tgaactcctt gggaaaccta cattctgcct 60
cagacatact gggggcaaat ggctttaaaa gtctggctca gggagccaag attacagaaa 120
nccgttgagt cnccatacat ggacactgac aaaggaactg aagatatcca aacaagccct 180
cctggtcccg ngcctgcata aagatcggga ncggaacggt accngacgtc tgtggtcagg 240
ggttgtggaa aattggaaaa aaccagtcct gcccacattg acagggaagc ctcaacggaa 300
attgaacaga tngtcttatc accagtctcc cctcctggat cntgtctcgg ctcnggggan 360
tcagtgatca gtcctttcag gtggaagaag caaagaagat caacaanaag cngatcctct 420
cacctgntac cagcatatgg 440

<210> 82
<211> 264
<212> DNA
<213> Homo sapien

<220>
<221> misc_feature

<222> (1)...(264)
<223> n = A,T,C or G

<400> 82
agcgtggtcg cggccgangt cctgacattc ctgccttctt atattaatta tacnaataaa 60
acaaaatagt gttgaagtgt tggagcggcg aaaatttttg gggggtggta tggacagaga 120
atgggcgatn ttctcanggc tgcttcaagt gggattgggg cngcgtggga tcatncagtg 180
gganagattn cnctgaccgg antctnttgg tanggatnat cttgtgggga tgtgcaagag 240
ncattcgtct cctgaatgan tggt 264

<210> 83
<211> 410
<212> DNA
<213> Homo sapien

<220>
<221> misc_feature
<222> (1)...(410)
<223> n = A,T,C or G

<400> 83
ancgtggtcg cggccgangt ccacagttgt gggagagcca gccattgtgg gggcagctcc 60
acaggtaaga ctcgtgtcct gagcagcgca catcatccag gacaatgggt cctgagccct 120
gaccaaaccg ggcatttcct ggggctgaca tggcccagcc acagcccant tgcctgcaga 180
cgaaattggc atcattggtg tcccagtant catcacacac ggtgccccag gaacctccgg 240
tatangaact ccactcggcc tcnanacctg tcgcctccat tccncagcct cagggggcaa 300
actgggattc agatccttct gtgggtacag gtggtgatat cctgacaggc caactttctg 360
gcctgagtgt tgactgangc tgggcagacc tgcccgggcg gccgctcgaa 410

<210> 84
<211> 320
<212> DNA
<213> Homo sapien

<220>
<221> misc_feature
<222> (1)...(320)
<223> n = A,T,C or G

<400> 84
tcgaacggcc gcccgggcag gtctgcccca ggtgtatcca tttgccgccg atctctatca 60
naaggagctg gctaccctgc nncgacgaan tcctgaanat aatctcaccc ncccagatct 120
ctctgtcgca atggagatgt cgtcatcggt ggncctgatc acagggcatt ggactcagag 180
anangtnanc acagtgtnga agcgattgan nnagttcagt tgctggtctt acccgatntt 240
ggaaggaagg aaaacgtgtt angacgtatc tcgatgnant tgaccaaanc tgaangctnc 300
agggggcatc gcaaaganan 320

<210> 85
<211> 218
<212> DNA
<213> Homo sapien

<220>
<221> misc_feature
<222> (1)...(218)

<223> n = A,T,C or G

<400> 85
tcgagcggcc gcccgggcag gtctgctgcc cgtgctggtg ccattgcccc atgtgaagtc 60
actgtgccag cccagaacac tggtctcggg cccgagaaga ctcctttctc caggctntan 120
gtatcaccac taaaatctcc aggggcacca tnganatcct gggtgtccgc aatgttgcca 180
atgtctgtcc gcnnattggc tacccaactg ttgcatca 218

<210> 86
<211> 283
<212> DNA
<213> Homo sapien

<220>
<221> misc_feature
<222> (1)...(283)
<223> n = A,T,C or G

<400> 86
tcgacttctt gtgaaggttt tgganaaata tgtatcagtt cgttttattt gggtattcaa 60
taatatcctt ggtgataatg ctgactccat ggcttctgac cccaaaaatt gaccctgctg 120
ccactggttg tagccctgag attgattttt gtagccacga ttgtttcctc gtcctctgaa 180
gtnctggttg tanttccctc tgtngggcat tcccctctgt tgtanttccc tctgtttgan 240
taactaccac ggccaggaaa aacaggggca cgaaggtatg gat 283

<210> 87
<211> 179
<212> DNA
<213> Homo sapien

<220>
<221> misc_feature
<222> (1)...(179)
<223> n = A,T,C or G

<400> 87
agcgtggtcc cggccgatgt ctttctgtgt aagtgcataa cactccacat acttgacatc 60
cttcangtca cgggccagct nttcagcant ctctggagtg ataggctact gtntgttctn 120
ggcaagtgtc tcaanaatac aggggtcntc tctgagatga ntttcagtcc cgaaccctc 179

<210> 88
<211> 512
<212> DNA
<213> Homo sapien

<220>
<221> misc_feature
<222> (1)...(512)
<223> n = A,T,C or G

<400> 88
tcgagcggcc gcccgggcag gtcctancan agaatcacca aatttatgga gagttaacag 60
gggtttaaca ggaangaagt gcctttagta agttctcaag ccagangctg gaggcagcag 120
ctaaatcaga ggacaggatc ctcagtgaaa gtgagccatt cggggtggca tgtcactcca 180
ggaataagca caacttanaa acaaatgatt tcgtangata gcacagtgac attggtgcac 240

ttgtgaacct gaggccactg tgtcaaactg tgcactggtt gtgaataggg aganccaaaa 300
attatgtcct actgggtaat gagctttcaa tgggctcgat cctctcacnc tgaaagctct 360
gtagagcagc tcagaaccac aaccactccc aacattgacc cttctggggg tactgtctgt 420
ggcacccaca ggaaggagct ggagatcccc attaggactg tccacccaca cttgaagcca 480
caaaactgca cctcggccgc gaccaccgct ta 512

<210> 89
<211> 358
<212> DNA
<213> Homo sapien

<220>
<221> misc_feature
<222> (1)...(358)
<223> n = A,T,C or G

<400> 89
tcgagcgggc cgcccgggca ggtctgccag tccccatccc agacattctt tgcatctaag 60
ctgangtctg aactgagtgg ggtgggctgg tgtttccatc ctcacaactc cagtgagccg 120
ggtgtggccg tggcctgcgt ctctctggcg gttagtgatg ttggcatcat ccaccttttt 180
caaaacaaaa gcactggact gaagaanaat cccnccctgt ntccacccag tccatggttt 240
ttaataaaag ggttatnnaa gttgancaag ncatcaccac acacaancct aagaacnttt 300
ttcatcnntc cccaaaacaa acccncaccc tgggaactcc gggcgcgaac cacgccta 358

<210> 90
<211> 250
<212> DNA
<213> Homo sapien

<220>
<221> misc_feature
<222> (1)...(250)
<223> n = A,T,C or G

<400> 90
cgagcggccg cccgggcagg tctggatggg gagacggact ggaactgcgg cttcccgtgg 60
cctgcacgca caaggctccc cacggccgcc gaccttcttc agattcgatc gtatgtgtac 120
gcacnaagag ccaaatattg acattcacaa cttcgtggga atnttacccc anaagactgc 180
gaccccccga tcaggcgana gcctgagcat agaagaacac cgctgtgggc ttggcactgt 240
gggncccatc 250

<210> 91
<211> 133
<212> DNA
<213> Homo sapien

<220>
<221> misc_feature
<222> (1)...(133)
<223> n = A,T,C or G

<400> 91
tcgagcggcc gnccgggcag gtcccgggtg gttgtttgcc gaaatgggca agttcntnaa 60
ncctgggaag gtggtgcntg tnctggctgg acgctactcc ggacgcnaag ctgtcntcgt 120
gangancatt gat 133

<210> 92
<211> 232
<212> DNA
<213> Homo sapien

<220>
<221> misc_feature
<222> (1)...(232)
<223> n = A,T,C or G

<400> 92
agcgtggtcg cggccgangt ctgtcacttt gcgggggtag cggtcaattc cagccaccag 60
agcatggctg taggggcgat ctgaggtgcc atcatcaatg ttcttcacga tgacaagctt 120
tgcgtccgga gtagcgtcca gccaggacaa gcaccacctt cccacgtntt cangaactng 180
cccatttcgg cataaccacc cgggacctgc ccgggcggnc gctcgaaaag cc 232

<210> 93
<211> 480
<212> DNA
<213> Homo sapien

<220>
<221> misc_feature
<222> (1)...(480)
<223> n = A,T,C or G

<400> 93
agcgtgggtc gcggccgang tctgtangct caccggccag agaagaccac tgtgagcatt 60
ttgccgtata tcctgccctg ccatttgttc actttttaaa ctaaaatagg aacatccgac 120
acacaccgtt tgcatcgtct tctcccttga tattttaagc attttcccat gtcgtgagtt 180
tctcagaaac atgtttttaa caattgtact atttagtcat ngtccattta ctataattta 240
tctgaccatt tccctactgt taaaatactt aagacggttt ctgatttttc cactatttaa 300
ataatgctgt gatgaatatc tttaaaatct tctgatttct tacttttttc ccccttagat 360
gcctggaagt ggtattttga ggtgaaagag tttgttcatt ttgaanatat ttctgtctct 420
ctctcgacct gatgtgtana cgctcacttc cagttagcag aaccacctta gtttgtgtct 480

<210> 94
<211> 472
<212> DNA
<213> Homo sapien

<220>
<221> misc_feature
<222> (1)...(472)
<223> n = A,T,C or G

<400> 94
tcgagcggnc gcccgggcag ggtctgatgt cantcacaac ttgaagggat gccaatgatg 60
taccaatccn atgtgaaatc tctcctctta tctcctatgc tgganaaggg attacaaagt 120
tatgtggcng ataannaatt ccatgcacct ctantcatcg atgagaatgg agttcatgan 180
ctggtgaacn atggtatctg aacccgatac cangttttgt ttgccacgat angantagct 240
tttatttttg atagaccaac tgtgaaccta ccacacgtct tggacnactg anntctaact 300
atccncaggg ttttattttg cttgttgaac tcttncagct nttgcaaact tcccaagatc 360
canatgactg antttcagat agcattttta tgattcccan ctcattgaag gtcttatnta 420

tntcnttttt tccaagccaa ggagaccatt ggacctcggc cgcgaccacc tn 472

<210> 95
<211> 309
<212> DNA
<213> Homo sapien

<220>
<221> misc_feature
<222> (1)...(309)
<223> n = A,T,C or G

<400> 95
tcgagcggcc gcccgggcag agtgtcgagc cagcgtcgcc gcgatggtgt tgttggagag 60
cgagcagttc ctgacggaac tgaccagact tttccanaag tgccggacgt cgggcancgt 120
ctatatcacc ttgaagaant atgacggtcg aaccaaaccc attccaaaga aangtactgt 180
gganggcttt gancccgcag acaacnagtg tctgttaaga actaccgatn ggaaanaana 240
anatcagcac tgtgggtgag ctccnaggga agttaataan tttcggatgg gcttattcna 300
acctcctta 309

<210> 96
<211> 371
<212> DNA
<213> Homo sapien

<220>
<221> misc_feature
<222> (1)...(371)
<223> n = A,T,C or G

<400> 96
tcgagcggcc gcccgggcag gtccaccact cacctactcc ccgtctctat agatttgcct 60
gttctgggca gttctcagca atggaatcct actgtgtatc tttttgtgac tggttcttta 120
actcagcatc acattttcaa ggttcatcca tgctgcagcc tggctccgta ctggtgacag 180
tacttcattt ctctctccct tttgttcaga ccaaggtctc cctctgtccc caaggctaaa 240
gtgcagttgg tgtgatcatg gctcactgca gcctcaaact cctggactca aacagtcctc 300
ccatctcagc ctcccaaagt gctgatntta taagttgcaa gccctgcacc cagcctgtat 360
ctccagtttg t 371

<210> 97
<211> 430
<212> DNA
<213> Homo sapien

<220>
<221> misc_feature
<222> (1)...(430)
<223> n = A,T,C or G

<400> 97
tcgancggcc gcccgggcag gtttnttttn tttntttttt nnnngntagt atttaaagan 60
atttattaaa tcatcttatc accaaaatgg aaacatnttc caactagaaa catgcnacca 120
tcatcttccc cagtccagtc ncaangtcca atattttnct tgcctctgca gataaaaagt 180
tcnnattttt atacccactc ttactccccc ccaaaatttt aattcngtcc tnccctaaaa 240
ttncnccggg taacaantta ccaaaatggc naaccaatta ttttaaanaa aagttgcncn 300

ttnaaaangg aaactttntg gcaanttanc ctcttttccc ttcccacccc ccantttaag 360
gggaaaacaa tggcactttg ctcttgcttn aacccaaaat tgtcttccaa aaactattaa 420
aaatgttnaa 430

<210> 98
<211> 307
<212> DNA
<213> Homo sapien

<220>
<221> misc_feature
<222> (1)...(307)
<223> n = A,T,C or G

<400> 98
tcnaacggcc gcccnggcnn gtctngcngc acctgtgcct canccgtcga tacctggtcg 60
attgggacan ggaanacaat ntggttttca gggaggccac anatttggag aaacggatga 120
attctccttt attccgaant cagctccttg gtctccgtag anggtgatct tgaaattctc 180
ctgttttgaa aactttcttg aanaaacctt acctgctggt tgtatttggt ctcccactcg 240
gacaagtact cgttatccnn ggtactctta atgtgcccac gtnaactccc cgggntggca 300
actggaa 307

<210> 99
<211> 207
<212> DNA
<213> Homo sapien

<220>
<221> misc_feature
<222> (1)...(207)
<223> n = A,T,C or G

<400> 99
   gtccnggacc gatgttgcna aganntttct tggtccanta ggttcnaaaa aatgataanc 60
   naggtntanc acgtgaagat ntntatanag tcttantnaa aacncntaga tctgnatgac 120
   gataantcga anacnggggg aggggntgag gngaggtggn gtganggaag anntgttgat 180
   aaaagannna gntgataaga anngagc 207

<210> 100
<211> 200
<212> DNA
<213> Homo sapien

<220>
<221> misc_feature
<222> (1)...(200)
<223> n = A,T,C or G

<400> 100
acntnnacta gaantaacag ncnttctang aacactacca tctgtnttca catgaaatgc 60
cacacacata naaactccaa catcaatttc attgcacaga ctgactgtaa ttaattttgt 120
cacaggaatc tatggactga atctaatgcn nccccaaatg ttgttngttt gcaatntcaa 180
acatnnttat tccancagat 200

<210> 101

<211> 51
<212> DNA
<213> Homo sapien

<220>
<221> misc_feature
<222> (1)...(51)
<223> n = A,T,C or G

<400> 101
tcgagcggcc gcccgggcag gtctgaccag tgganaaatg cccagttatt g 51

<210> 102
<211> 385
<212> DNA
<213> Homo sapien

<220>
<221> misc_feature
<222> (1)...(385)
<223> n = A,T,C or G

<400> 102
aacgtggtcg cggccgaagt ccatggtgct gggattaatc cactgtgacn gtgactctga 60
gttgagttgt ttttcaatct tctccaagcc tgtggactca tcctccacat ccttgggtag 120
taggatgaac atgctgaaga tgctnatttt gaaaaggaac tctatgaatc ttacaattga 180
atactgtcaa tgtttcccca tnacagaacg tggnccccca aggttccatc atctgcactg 240
ggtttgggtg ttctgtcttg gttgactctt gaaaagggac atttcttttt gttttcttga 300
attcanggaa attttcttca tccactttgc ccacaaaagt taggcagcat ttaaccccca 360
anggattttg ggtctgggtc cttcc 385

<210> 103
<211> 189
<212> DNA
<213> Homo sapien

<220>
<221> misc_feature
<222> (1)...(189)
<223> n = A,T,C or G
<400> 103
agcgtggtcg cggccgaagt ctgcagcctg ggactgaccg ggaagctctg attatttacc 60
caccacaggt angttgtgtt ctgaatctca agttcacagg ttaaggctac agcatcctca 120
tcctccacgg ggttggantt gttgctggtg atgaanggtt tggggtggct ctgcataact 180
gttgatctc 189

<210> 104
<211> 181
<212> DNA
<213> Homo sapien

<220>
<221> misc_feature
<222> (1)...(181)
<223> n = A,T,C or G

<400> 104
tcgagcggcc gcccgggcag gtccaggtct ccaccaangc accaccgtgg gaagctggta 60
attgatgccc accttgaagc cnntggggca ccatccncca actggatgct gcgcttggtt 120
ttgatggtgg caatggcaca ttgactcttt tgggaaccac ttcaccacgg tacaacaggc 180
a 181

<210> 105
<211> 327
<212> DNA
<213> Homo sapien

<220>
<221> misc_feature
<222> (1)...(327)
<223> n = A,T,C or G

<400> 105
tcgagcggcc gcccgggcag gtcttctgtg gagtctgcgt gggcatcgtg ggcagtgggg 60
ctgccctggc cgatgctcan aaccccagcc tctttgtaaa gattctcatc gtgganatct 120
ttggcagcgc cattggcctc tttggggtca tcgtcgcaat tcttcanacc tccanaatga 180
anatgggtga ctanataata tgtgtgggtn gggccgtgcc tcacttttat ttattgctgg 240
ttttcctggg acagaactcg ggcgcgaaca cgcttanccg aattccaaca cactggcggg 300
cgttactagt ggatccgagc tcggtac 327

<210> 106
<211> 268
<212> DNA
<213> Homo sapien

<220>
<221> misc_feature
<222> (1)...(268)
<223> n = A,T,C or G

<400> 106
agcgtggtcg cggccgangt ctggcgtgtg ccacatcggt cccacctcgc tttacaaaac 60
agtcctgaac ttnatctaat aaaattattg tacacnacat ttacattaga aaaaganagc 120
tgggtgtang aaaccgggcc tggtgttccc tttaagcgaa ngtggctcca cagttggggc 180
atcgtcgctt cctcnaagca aaaacgccaa tgaaccccna agggggaaaa aggaatgaag 240
gaactgnccn gggangnccg ctccgaaa 268

<210> 107
<211> 353
<212> DNA
<213> Homo sapien

<220>
<221> misc_feature
<222> (1)...(353)
<223> n = A,T,C or G

<400> 107
tcgagcggcc gcccgggcag gtggccaggc catgttatgg gatctcaacg aaggcaaaca 60
cctttacacn ctagatggtg gggacatcat caacgccctg tgcttcagcc ctaaccgcta 120

ctggctgtgt gctgccgcag gccccagcat caagatctgg gatttanagg gaaagatcnt 180
tgtnnatgaa ctgaancnta aattatcagt tccannacca ngcaaaaacc acccngtgca 240
ctccctggcc tggtctgctg atgggacctc gggcgcgaac acgctnancc caattccanc 300
acactgggcg gncgttacta ntggatccga actcnggtac caancttggc gtt 353

<210> 108
<211> 360
<212> DNA
<213> Homo sapien

<220>
<221> misc_feature
<222> (1)...(360)
<223> n = A,T,C or G

<400> 108
agcgtggtcg cggccgaagt cctggcctca catgaccctg ctccagcaac ttgaacagga 60
naagcagcag ctacatcctt aaggtccgga aagttagatg aagatttgga tcctgcattg 120
ncctgcctcc cacctatctc tcccnaatta taaacagcct ccttgggaag cagcagaatt 180
taaaaactct cccnctgccc tnttgaacta cacaccnacc gggaaaacct ttttcanaat 240
ggcacaaaaa tncnagggaa tgcatttcca tgaangaana aactgggtta cccaaaatta 300
ttgggttggg gaaatccngg gggggttttn aaaaaagggc aanccnccaa anaaaaaaac 360

<210> 109
<211> 101
<212> DNA
<213> Homo sapien

<220>
<221> misc_feature
<222> (1)...(101)
<223> n = A,T,C or G

<400> 109
atcgtggtcn cggccgaagt cctgtgtcct ggatgggccg tgtgcancga atccgttggc 60
gactcctaac taccaanaaa angactctcg gaagaaattt c 101

<210> 110
<211> 300
<212> DNA
<213> Homo sapien

<220>
<221> misc_feature
<222> (1)...(300)
<223> n = A,T,C or G

<400> 110
ccanggaaac ccagagtcac atgagatagg gtggctttcg ggacaggggg tcagangaat 60
ggtacatgga tctcagcccc tgatggacac ggaacaggtg tggtcagaac tcccangatt 120
ctgcatccan gatccagtct ctatagaagt tatggatcat tccttcattt cattcccccc 180
ttcatgaaaa aacttctgaa caagcctttt ttctcacttt ggggccctgt ttggcncaag 240
gtnttnantt ggggaaaaaa aaacaaatcc nttccnttan ccctccgtgg ggaatgacct 300

<210> 111

<211> 366
<212> DNA
<213> Homo sapien

<220>
<221> misc_feature
<222> (1)...(366)
<223> n = A,T,C or G

<400> 111
cgagcggccg cccgggcagg tccttgtgtt gccatctgtt ancattgatt tctggaatgg 60
aacanctttc tcaaagtttg gtcttgctan tcatgaagtc atgtcagtgt cttaagtcac 120
tgctgctcac ttccttaccc agggaatata ctgcataagt ttctgaacac ctgttttcan 180
tattcactgt tcctctcctg cccaaaattg gaagggacct catttaaaaa tcaaatttga 240
atcctgaaan aaaaacngga aatntttctc ttggaatttg gaatagaatt attcanttga 300
ataacatgtt ttttcccctt gccttgctct tcncaanaac atctggacct cggccgcgac 360
acctta 366

<210> 112
<211> 405
<212> DNA
<213> Homo sapien

<220>
<221> misc_feature
<222> (1)...(405)
<223> n = A,T,C or G

<400> 112
ctgactncta aacttctaat tcnatcaana taactactct ccttccgtct tncagagtgt 60
tcacaataaa tctgtgaatc tggcatacac agttgctgga aaattgttct tcctccacna 120
aaaggtcaat tgttcnccnc atgaaanaag ataaattgtt catccatcac tnctgaacca 180
tccaaaacgc cggcggaatt attnccccgt tattatgggg aacggaattt tnaataaatt 240
tgggaangaa tggggctttt attgttttgt tttccccctt tcttggcatt gattgggccg 300
caatgggccc cctcgctcan aanntgcccc ggggccggcc gctccaaaac cgaaattccc 360
anccacactt ggcgggccgt tactanttgg atccgaactc ggtta 405

<210> 113
<211> 401
<212> DNA
<213> Homo sapien

<400> 113
ggatagaaga gtatatgggt ttggcaccac ggggtggata ggcaaaacat ttggttgata 60
aggcgcagat tctgaactaa cttgtaaggc ttgtctggtt ttaggacagg taaaatgggg 120
gaatggtaag gagagtttat aggttttagg agcccatgct gtagcaggca agtgataaca 180
ggctttaatc ctttcaaagc atgctgtggg atgagatatt ggcatttgag cggggtaagg 240
gtgattaggt tttaatgaga tggtaagggg tgcatgatcc ggtccgccaa ggaagggaag 300
tagaggtatc ttatacttgt ggggttaagg tgggggggat ataagaggga ggacgccaaa 360
ggaggctttg gattaggaat aaggggcggc aatgagatgc a 401

<210> 114
<211> 401
<212> DNA
<213> Homo sapien

<220>
<221> misc_feature
<222> (1)...(401)
<223> n = A,T,C or G

<400> 114
angtccacag gangcangag gccaggctcc gtcccancca gtccatgatg ttgaagagga 60
ggaagcagca catggggttg aagaactgac tccacttccc aggactggtg gagctggtca 120
ccatggctgt ggtggcgggg aagacggaca gggtgacttc tggaagacag tgaagactga 180
aggttttcct ggcttctggg gctcatctgg ctctgattcc ggctccttct ccaggtcaag 240
atccagggtt cagagctact ttcttggggg actactnggg aatcccgttc tcatctgggg 300
gtngaggggg gacggggnaa gggncatgct tgtgacccag gtttcccacc tcggcccgcg 360
accacgctaa ggcccgaatt ncagcacact tggcggcccg t 401

<210> 115
<211> 401
<212> DNA
<213> Homo sapien

<400> 115
atccctgtaa gtctattaaa tgtaaataat acatacttta caacttctct tagtcggccc 60
ttggcagatt aaatctttgc aaaattccat atgtgctatt gaaaaatgaa ataaaacctc 120
agatgtctga attcttattt caaatacagt tatataatta ttttaaatta caatatacaa 180
tttctgttaa atacaactgt taagggattc tgagaacaat tataagatta taataatata 240
tacaaactaa cttctgaaat gacatgggtt gtttccttcc caccctccta ccctctcaaa 300
gagtttttgc atttgctgtt cctggttgca aaaggcaaaa gaaaatctaa aaatagtctg 360
tgtgtgtcca cgacatgctc gctcctttga gaatctcaaa c 401

<210> 116
<211> 301
<212> DNA
<213> Homo sapien

<220>
<221> misc_feature
<222> (1)...(301)
<223> n = A,T,C or G

<400> 116
ngatttaatt gnnagcttct ttttaatgga atnnttggct aaaatgaatt gatgattatg 60
aatatcccta ggaggagtta gcatggannn tgatcatttt cttngnactc ctttangaca 120
nggaaacagg natcagcatg anggtancan aaaccttatn accnangcgc acganctgac 180
ttcttccaaa gagttgnggt tccgggcagc ggtcattgcc gtgcccattg ctggagggct 240
gattctagtg ntgcttatta tgctggccct gaggatgctt ccaanatgaa aataagangc 300
t 301

<210> 117
<211> 383
<212> DNA
<213> Homo sapien

<220>
<221> misc_feature
<222> (1)...(383)

<223> n = A,T,C or G

<400> 117
aattgcaact ggacttttat tgggcagtta cnacaacnaa tgttttcana aaaatatttg 60
gaaaaaatat accacttcat agctaagtct tacagagaan aggatttgct aataaaactt 120
aagttttgaa aattaagatg cnggtanagc ttctgaacta atgcccacag ctccaaggaa 180
nacatgtcct atttagttat tcaaatacca gttgagggca ttgtgattaa gcaaacaata 240
tatttgttan aactttgntt ttaaattact gntncttgac attacttata aaggagnctc 300
taactttcga tttctaaaac tatgtaatac aaaagtatan ntttccccat tttgataaaa 360
gggccnanga tactgantag gaa 383

<210> 118
<211> 301
<212> DNA
<213> Homo sapien

<400> 118
   ctgctagaat cactgccgct gtgctttcgt ggaaatgaca gttccttgtt ttttttgttt 60
   ctgtttttgt tttacattag tcattggacc acagccattc aggaactacc ccctgcccca 120
   caaagaaatg aacagttgta gggagaccca gcagcacctt tcctccacac accttcattt 180
   tgaagttcgg gtttttgtgt taagttaatc tgtacattct gtttgccatt gttacttgta 240
   ctatacatct gtatatagtg tacggcaaaa gagtattaat ccactatctc tagtgcttga 300
   c 301

<210> 119
<211> 401
<212> DNA
<213> Homo sapien

<400> 119
taaggacatg gacccccggc tgattgcatg gaaaggaggg gcagtgttgg cttgtttgga 60
tacaacacag gaactgtgga tttatcagcg agagtggcag cgctttggtg tccgcatgtt 120
acgagagcgg gctgcgtttg tgtggtgaat ggggaggaaa tgtcactgcc gaagaccaaa 180
aacaagcttc ttggtataaa agactcttac agaatatgtg tattgtaatt tattgatctg 240
gatgcttaag tgtcatggac agtaaatgaa tttgaacttt atgtttgagg acatgacatt 300
gggtttgaaa atataaactg cttttgagca gtttaagtca gggcatttga gaataaaata 360
ggaactttct cttcagtttg taaaactctc ttgccctctc t 401

<210> 120
<211> 301
<212> DNA
<213> Homo sapien

<400> 120
tccagagata ccacagtcaa acctggagcc aaaaaggaca caaaggactc tcgacccaaa 60
ctgccccaga ccctctccag aggttggggt gaccaactca tctggactca gacatatgaa 120
gaagctctat ataaatccaa gacaagcaac aaacccttga tgattattca tcacttgggt 180
gagtgcccac acagtcaagc tttaaagaaa gtgtttgctg aaaataaaga aatccagaaa 240
ttggcagagc agtttgtcct cctcaatctg gtttatgaaa caactgacaa acacctttct 300
c 301

<210> 121
<211> 2691
<212> DNA
<213> Homo sapien

<400> 121
gcttgcccgt cggtcgctag ctcgctcggt gcgcgtcgtc ccgctccatg gcgctcttcg 60
tgcggctgct ggctctcgcc ctggctctgg ccctgggccc cgccgcgacc ctggcgggtc 120
ccgccaagtc gccctaccag ctggtgctgc agcacagcag gctccggggc cgccagcacg 180
gccccaacgt gtgtgctgtg cagaaggtta ttggcactaa taggaagtac ttcaccaact 240
gcaagcagtg gtaccaaagg aaaatctgtg gcaaatcaac agtcatcagc tacgagtgct 300
gtcctggata tgaaaaggtc cctggggaga agggctgtcc agcagcccta ccactctcaa 360
acctttacga gaccctggga gtcgttggat ccaccaccac tcagctgtac acggaccgca 420
cggagaagct gaggcctgag atggaggggc ccggcagctt caccatcttc gcccctagca 480
acgaggcctg ggcctccttg ccagctgaag tgctggactc cctggtcagc aatgtcaaca 540
ttgagctgct caatgccctc cgctaccata tggtgggcag gcgagtcctg actgatgagc 600
tgaaacacgg catgaccctc acctctatgt accagaattc caacatccag atccaccact 660
atcctaatgg gattgtaact gtgaactgtg cccggctcct gaaagccgac caccatgcaa 720
ccaacggggt ggtgcacctc atcgataagg tcatctccac catcaccaac aacatccagc 780
agatcattga gatcgaggac acctttgaga cccttcgggc tgctgtggct gcatcagggc 840
tcaacacgat gcttgaaggt aacggccagt acacgctttt ggccccgacc aatgaggcct 900
tcgagaagat ccctagtgag actttgaacc gtatcctggg cgacccagaa gccctgagag 960
acctgctgaa caaccacatc ttgaagtcag ctatgtgtgc tgaagccatc gttgcggggc 1020
tgtctgtaga gaccctggag ggcacgacac tggaggtggg ctgcagcggg gacatgctca 1080
ctatcaacgg gaaggcgatc atctccaata aagacatcct agccaccaac ggggtgatcc 1140
actacattga tgagctactc atcccagact cagccaagac actatttgaa ttggctgcag 1200
agtctgatgt gtccacagcc attgaccttt tcagacaagc cggcctcggc aatcatctct 1260
ctggaagtga gcggttgacc ctcctggctc ccctgaattc tgtattcaaa gatggaaccc 1320
ctccaattga tgcccataca aggaatttgc ttcggaacca cataattaaa gaccagctgg 1380
cctctaagta tctgtaccat ggacagaccc tggaaactct gggcggcaaa aaactgagag 1440
tttttgttta tcgtaatagc ctctgcattg agaacagctg catcgcggcc cacgacaaga 1500
gggggaggta cgggaccctg ttcacgatgg accgggtgct gaccccccca atggggactg 1560
tcatggatgt cctgaaggga gacaatcgct ttagcatgct ggtagctgcc atccagtctg 1620
caggactgac ggagaccctc aaccgggaag gagtctacac agtctttgct cccacaaatg 1680
aagccttccg agccctgcca ccaagagaac ggagcagact cttgggagat gccaaggaac 1740
ttgccaacat cctgaaatac cacattggtg atgaaatcct ggttagcgga ggcatcgggg 1800
ccctggtgcg gctaaagtct ctccaaggtg acaagctgga agtcagcttg aaaaacaatg 1860
tggtgagtgt caacaaggag cctgttgccg agcctgacat catggccaca aatggcgtgg 1920
tccatgtcat caccaatgtt ctgcagcctc cagccaacag acctcaggaa agaggggatg 1980
aacttgcaga ctctgcgctt gagatcttca aacaagcatc agcgttttcc agggcttccc 2040
agaggtctgt gcgactagcc cctgtctatc aaaagttatt agagaggatg aagcattagc 2100
ttgaagcact acaggaggaa tgcaccacgg cagctctccg ccaatttctc tcagatttcc 2160
acagagactg tttgaatgtt ttcaaaacca agtatcacac tttaatgtac atgggccgca 2220
ccataatgag atgtgagcct tgtgcatgtg ggggaggagg gagagagatg tactttttaa 2280
atcatgttcc ccctaaacat ggctgttaac ccactgcatg cagaaacttg gatgtcactg 2340
cctgacattc acttccagag aggacctatc ccaaatgtgg aattgactgc ctatgccaag 2400
tccctggaaa aggagcttca gtattgtggg gctcataaaa catgaatcaa gcaatccagc 2460
ctcatgggaa gtcctggcac agtttttgta aagcccttgc acagctggag aaatggcatc 2520
attataagct atgagttgaa atgttctgtc aaatgtgtct cacatctaca cgtggcttgg 2580
aggcttttat ggggccctgt ccaggtagaa aagaaatggt atgtagagct tagatttccc 2640
tattgtgaca gagccatggt gtgtttgtaa taataaaacc aaagaaacat a 2691

<210> 122
<211> 683
<212> PRT
<213> Homo sapien

<400> 122

<210> 123
<211> 1205
<212> DNA
<213> Homo sapien

<400> 123
ccagtcagca gagggacagg aatcattcgg ccactgttca gacgggagcc acacccttct      60
ccaatccaag cctggcccca gaagatcaca aagagccaaa gaaactggca ggtgtccacg     120
cgctccaggc cagtgagttg gttgtcactt actttttctg tggggaagaa attccatacc     180
ggaggatgct gaaggctcag agcttgaccc tgggccactt taaagagcag ctcagcaaaa     240
agggaaatta taggtattac ttcaaaaaag caagcgatga gtttgcctgt ggagcggtgt     300
ttgaggagat ctgggaggat gagacggtgc tcccgatgta tgaaggccgg attctgggca     360
aagtggagcg gatcgattga gccctgcggt ctggctttgg tgaactgttg gagcccgaag     420
ctcttgtgaa ctgtcttggc tgtgagcaac tgcgacaaaa catttgaagg gaaaattaaa     480
ccaatgaaga agacaaagtc taaggaagaa tcggccagtg ggccttcggg agggcggggg     540
gaggttgatt ttcatgattc atgagctggg tactgactga gataagaaaa gcctgaacta     600
tttattaaaa acatgaccac tcttggctat tgaagatgct gcctgtattt gagagactgc     660
catacataat atatgacttc ctagggatct gaaatccata aactaagaga aactgtgtat     720
agcttacctg aacaggaatc cttactgata tttatagaac agttgatttc ccccatcccc     780
agtttatgga tatgctgctt taaacttgga agggggagac aggaagtttt aattgttctg     840
actaaactta ggagttgagc taggagtgcg ttcatggttt cttcactaac agaggaatta     900
tgctttgcac tacgtccctc caagtgaaga cagactgttt tagacagact ttttaaaatg      960
gtgccctacc attgacacat gcagaaattg gtgcgttttg tttttttttc ctatgctgct    1020
ctgttttgtc ttaaaggtct tgaggattga ccatgttgcg tcatcatcaa cattttgggg    1080

gttgtgttgg atgggatgat ctgttgcaga gggagaggca gggaaccctg ctccttcggg 1140
ccccaggttg atcctgtgac tgaggctccc cctcatgtag cctccccagg cccagggccc 1200
tgagg 1205

<210> 124
<211> 583
<212> DNA
<213> Homo sapien

<400> 124
ccaagaagca gtggccttat tgcatcccaa accacgcctc ttgaccaggc tgcctccctt 60
gtggcagcaa cggcacagct aattctactc acagtgcttt taagtgaaaa tggtcgagaa 120
agaggcacca ggaagccgtc ctggcgcctg gcagtccgtg ggacgggatg gttctggctg 180
tttgagattc tcaaaggagc gagcatgtcg tggacacaca cagactattt ttagattttc 240
ttttgccttt tgcaaccagg aacagcaaat gcaaaaactc tttgagaggg taggagggtg 300
ggaaggaaac aaccatgtca tttcagaagt tagtttgtat atattattat aatcttataa 360
ttgttctcag aatcccttaa cagttgtatt taacagaaat tgtatattgt aatttaaaat 420
aattatataa ctgtatttga aataagaatt cagacatctg aggttttatt tcatttttca 480
atagcacata tggaattttg caaagattta atctgccaag ggccgactaa gagaagttgt 540
aaagtatgta ttatttacat ttaatagact tacagggata agg 583

<210> 125
<211> 783
<212> DNA
<213> Homo sapien

<400> 125
tcaaccatac atactgcttc cactagctaa taccaaatgc aggttctcag atccagacaa 60
atggaggaaa agaacattta tgcttccgtt tcagaaagcc aagtcgtagt tttggccctt 120
cctttctcta aagtttattc ccaaaaacag gtagcattcc tgattgggca gagaagagga 180
tattttcagc ccacatctgc tgcaggtatg tcattttctc ccatcttcac tgtgactagt 240
aaagatctca ccacttctct ttggaatttc caactttgct tgtgattgaa tgtcacttcg 300
tgaatttgta ttatgtcaga tcacttggca ttgctcttcc atatgcatca agttgccagg 360
cactgttgcg ctgtcgggcc cactggaatc cacgggggtg aaacaaattc aattatgctt 420
ttacagatcc tgctcaaaaa aggtttcaac tgcttaacca agtacagctc attcttccac 480
cttcttactc tgcaaccaaa ccaagtgccc catactacag gtaggtgccg agaaattccg 540
cagcagaaaa tccaaaatca tttctgaaac ctccttgcta acaaaagttc tttttttctc 600
caaacagcat ataaaatgat caagtcttga aagagaaaag aagcaaagta gcaaatacat 660
caacaattca ctatcagaaa cacataaaat cccagagaga gagaaggcag tatctctgaa 720
tcatggatgg acttggaaag ttcggaagga ttccgagtgc ttcctttcag aaagacaatt 780
ctg 783

<210> 126
<211> 604
<212> DNA
<213> Homo sapien

<400> 126
   cctgctagaa tcactgccgc tgtgctttcg tggaaatgac agttccttgt tttttttgtt 60
   tctgtttttg ttttacatta gtcattggac cacagccatt caggaactac cccctgcccc 120
   acaaagaaat gaacagttgt agggagaccc agcagcacct ttcctccaca caccttcatt 180
   ttgaagttcg ggtttttgtg ttaaagttaa tctgtacatt ctgtttgcca ttgttacttg 240
   tactatacat ctgtatatag tgtacggcaa aagagtatta atccactatc tctagtgctt 300
   gactttaaat cagtacagta cctgtacctg cacggtcacc cgctccgtgt gtcgccctat 360
   attgagggct caagctttcc cttgtttttt gaaaggggtt tatgtataaa tatattttat 420
   gcctttttat tacaagtctt gtactcaatg acttttgtca tgacattttg ttctacttat 480
   actgtaaatt atgcattata aagagttcat ttaaggaaaa ttacttggta caataattat 540
   tgtaattaav agatgtagcc tttattaaaa ttttatattt ttcaaaaaaa aaaaaaaaaa 600
   aaaa 604

<210> 127
<211> 417
<212> DNA
<213> Homo sapien

<400> 127
ctgagcctct gtcaccagag aaggctgagg ccccaatggc acacctcaga aacctacacc 60
ccgaggctgg acggctggac tcctgagcac aagctccctc tcgcaccctt tgccagacag 120
tttgtctcca atttcaaact gacctaaggc tcttactcct ggattttttg tttttaaacc 180
ttctcccagc cagtcttcgg gagggcatga ttagagaagt gctcctttgc tgatggagga 240
ggggacctaa ggaagaaggt ggatcccagg tgcctcctct ctaattgatc ctccccacct 300
agtttccttt gcctctcttc cttctaccag gtcatgtttt ttactctctg ccccttctgc 360
ctcctagcat ttcaaaaact gtagagtgca ccccatagtg gacattttta gtccagg 417

<210> 128
<211> 657
<212> DNA
<213> Homo sapien

<400> 128
ccacactgaa atgcagttta atgtggaaac ttttctaaat acatattgta gcatctttgg 60
acatcaacgt gtggcctgaa atttttatta ttgttccctc ttctcctcca ttaaaaaaaa 120
aatctccttg tggtatttag tcatttacca ttaacacata ttatggctta aaaagggcca 180
tcccttcctt ttctgagctg gagttcttca cgctcacctt tgatgcatgg ccttagctgg 240
ttactttgcc ttggtttggt catgaacatt ggggttagtg gcctggcaac ttgaatgcat 300
atggaaagaa caatgccaag tgatctgaca taatacaaat tccgaagtga cattcaatca 360
caagcaaagt tggaaattcc aaagagaagt ggtgagatct ttactagtca cagtgaagat 420
gggagaaaat gacatacctg cagcagatgt gggctgaaaa tatcctcttc tctgcccaat 480
caggaatgct acctgttttt gggaataaac tttagagaaa ggaagggcca aaactacgac 540
ttggctttct gaaacggaag cataaatgtt cttttcctcc atttgtctgg atctgagaac 600
ctgcatttgg tattagctag tggaagcagt atgtatggtt gaagtgcatt gctgcag 657

<210> 129
<211> 1220
<212> DNA
<213> Homo sapien

<400> 129
cgcgtgctcg gctcacacca acaaggcaag ccaaaggcgc ccctccccag agggatccct 60
aacgtgccca gcatgtagat tctggactaa cagacaacat acattcaccg ctggtcaccc 120
agatcctcat tcaaacccac tgctggcaca tccctttcct tactttgccc tgtgctacca 180
gccacggaag gagcctctct tgttttttct ataaaatggg taggcaggag aaaagcaggt 240
gccctaagat tgctctaagg cccagcatgt ggttacagtt ctctgacttg cagaacctgc 300
caggtgtatg gctacaagtt atcctcgtgc tgatctgtct cattactaag ttaatggaga 360
agacagaaag gtaaaaatca cgtgtagcaa gaacaactct tatttcacaa actcaggtat 420
gaaacgaaac gcctgtcctt catggaactg cttttagctc ctgtcttttc aaaatggcag 480
agggagttcc tacacacact ttttccctgg aggccaaggt ctaggggtag aaaggggagg 540
ggtggggcta ccaggtagca gttgacaacc caaggtcaga ggagtggccc tcagtgtcat 600
ctgtccacag tgatacctgc caagatgacc actgacccac atctggtctt agtcattggt 660
ctcctcagat ttctggggcc acctgcaagc cccattccat tcctacagat ctctcagcca 720

cctgtaagtc ctttgtgaag atgtgggtga cacaggggga caggaaaacc catttctcaa 780
cccagatcca tgtctccact gcttctactc tgggttggga ttcaggaaga caggcacagt 840
cctctctgtt catagaaaca cctgccagtg tcaaggattc cagtcaggtg tctatcccaa 900
ctggtcaggg agagaagggc agacccattc tcaaagacca ccatgtccaa ggtctgacag 960
ctccccactg gctgccccca caggggcttt aggctggtct gggtcatggg gaagcgtccc 1020
tcttatcgct ggtctgtgtt ctcctggatt tggtatctat gttggtacga ctcctggcct 1080
tttatctaaa ggactttggc ttttgtaaat cacaagccaa taatagactt ttttctcccc 1140
ctctgttttt tgctgtgtca tctctgcctt gagactgcct tgagacagtg cttgccttga 1200
gagagtgagc caattaacag 1220

<210> 130
<211> 1274
<212> DNA
<213> Homo sapien

<400> 130
ccatatgagt ttgccatctc catggatgcc atttcaatgc cttcagggta atcattctct 60
ccccaaagac tgcccacggg gtcatcactc ctgtgacgaa atgagggctg gattgaagat 120
gttctgctga gcacccccct ggtcatcttt ggggtctcag aagagccata atcatgacca 180
ttctcagcat ctgaataatc aggttctctc caagtgcttg gcaagttctg attgtcctca 240
gcactgggat agtctggctc cccaaaaaag ggtggagagt taggttgaat gtcagcgcct 300
ggataatcag gctttcccag agagtctgcg tatggattga ttctaaaact tgtatgttcc 360
agattctttc tggatcctgg atggttcaaa ttggctctgg gtccaggatg atcagagttg 420
ctctgagctc cagggtagtc cggttctaag gagccaaaat gatctggatg tgttctggag 480
cctgcatagt ttccactgct gctggagcct gcaaaatcag gatttcgttg agatccaggg 540
tagtctggtt gtctggatga tgctcggtgg tagggatgac tctgaaattc actataatct 600
ggctctggta gagaggtagg atggtctggg cttgttctag aggctgcaga gtatgcattg 660
cttctggtgc cagaatagtc tggattactc agagatctag gataatttgg ttctgccaga 720
gacccaggat agtctggacg tgttctggag gctacagagt atggattgct cctggtgccg 780
gggtaatctg gattgttcag aggacctgga acatctggat aaccttgagt tttcaaatac 840
ccctgcgtac ggttctgaga ccctgaatag tcagggtaat ctgggtcttc ctcagaccag 900
ttattcctgt agtaggcaga catgttggta tggactcttc accctggagt ggtaaactgt 960
cccagcattt gcaattactc agggatcttt tttttttcac ttttttgccc ttattgttct 1020
tgctttgtcc caagtagatg caaatgttgt gcaaaccaac ttgatcttaa gatgttgtta 1080
agaacactgg agtcacgtgt ccatgggtcc ttcaggctgg cttttgatgg gagctgggat 1140
gcagatgatt tacggagggt tataatctgt gatgctggtc tgaagtctga atattccaag 1200
ttgctgactg caggcagagc ctcatgtcct cctggcgctc ctgttgccgc tgcttgcgct 1260
ggccctcggg tcga 1274

<210> 131
<211> 554
<212> DNA
<213> Homo sapien

<220>
<221> misc_feature
<222> (1)...(554)
<223> n = A,T,C or G

<400> 131
ctgtaattct gccttttcta ccttcattcc atccttcctc tgcccagata aagkccagca 60
gaaattcctc ctttctacct ctctgggact ctgagacagg aaatcttcaa ggaggagttt 120
ttccctcccc actattctta ttctcaaccc ccagaggaac caaggctgct gtacccacct 180
cagggacaga actccacact atagtgggaa agcttcaggg acccctcctt ttagtgctca 240
gggctcacct atgctactgg tccttttggc aaaaaaggaa aatgatagag ccagggttgc 300

ccctgatgta gcagccttac tgtggagggg ccaaagctgg tgttcagagc tcacccaagg 360
agggaggtga taaggtgtca tgcgttctgc tgaacccact ggntggtatg aacatgaggc 420
ttggggtgag ggaaaccaag taggggttgg agaaggagca gcacctttgt macacctggc 480
tacccatagc tagctttctg ccctcaaaaa ctcagccttc aagggatcca gcccacacac 540
gccacaggca gcag 554

<210> 132
<211> 787
<212> DNA
<213> Homo sapien

<400> 132
ctggtcaccc aactcttgtg gaagagggga attgagatcg agtactgaat atctggcaga 60
gaggctggaa tccttcagcc ccagagccca gggaccactc cagtagatgc agagaggggc 120
ctgcccaggg gtcagggcag tgggtatcac tggtgacatc aagaatatca gggctgggga 180
ggcatctttg tttcctggtg ccctcctcaa agttgctgac actttgggga cgggaagggg 240
tagaagtagg gctgctcctt ttggagctgg agggaataga cctggagaca gagttgaggc 300
agtcgggctg tccaggttct aagcatcaca gcttctgcac tgggctctga ggagattctc 360
agccagagga tcccagcctc ctcctccctc aaatgtcagt ccaagcaaat accaaagcaa 420
cgcatcgatt ttgtggaagt caattagaga tgtggggagc tatcggagac aagcactatt 480
gtaccttttc acctccacac ttgtcacaag cagggactgt ctcctcccca ctttgcttgc 540
cacgcctgcc atggcttgag ctggggtgag gagtggtctt tatcttcttt gggagatcct 600
gactggttgc gcacttgcta agggcaggaa gtctggaggg ctgcaggaat ggtgccgttg 660
ataaacaggt ggacttataa tcatcatgca ctgcaattgt agaacatagt ctcctgcctt 720
ttctcatttg tataattgtc tgggtcaata ttctcccaat attgggaggg gctctgcagc 780
cctccag 787

<210> 133
<211> 219
<212> DNA
<213> Homo sapien

<220>
<221> misc_feature
<222> (1)...(219)
<223> n = A,T,C or G

<400> 133
tactgctcta agttttgtna aatttttcat attttaattt caagcttatt ttggagagat 60
aggaaggtca tttccatgta tgcataataa tcctgcaaag tacaggtact ttgtctaaga 120
aacattggaa gcaggttaaa tgttttgtaa actttgaaat atatggtcta atgtttaagc 180
agaattggaa nagactaata tcggttaaca aataacaac 219

<210> 134
<211> 234
<212> DNA
<213> Homo sapien

<400> 134
gattttaaaa acatcatgac tttgaactga aaaacataca cgtttagcac acaaatattg 60
taatatgaat gaactccaac tccatttgaa aacatgtgaa tcaaagtaca gttttagaag 120
ttagtaattc acatttaagc aagttagcgc cttgctgaat acagcctttg taaaaaagag 180
acttagtgca tattttaatg gtacattgtg gttttgtacc atttggttga gttg 234

<210> 135

<211> 414
<212> DNA
<213> Homo sapien

<400> 135
ctccagcctg gctatatccg gtcccgctat aacctgggca tcagctgcat caacctcggg 60
gctcaccggg aggctgtgga gcactttctg gaggccctga acatgcagag gaaaagccgg 120
ggcccccggg gtgaaggagg tgccatgtcg gagaacatct ggagcaccct gcgtttggca 180
ttgtctatgt taggccagag cgatgcctat ggggcagccg acgcgcggga tctgtccacc 240
ctcctaacta tgtttggcct gccccagtga cagtgggacg ggctgccctg tgagtgtcca 300
cctggggatt aaatatgtct tcaacaaggg aggcctggct tctacaatgg tttaggtaaa 360
ggggcctttg aagtagttct ggccaggctt gcaatacaca caacacaaga gcca 414

<210> 136
<211> 461
<212> DNA
<213> Homo sapien

<400> 136
gaagtgatta ataggtttat ttgcatatac acagagaaga gtcagcattg ttgggtgaga 60
agaggcaggc tgtgaggagg taaggcttca gcagaggaag gcaccttgac agacaacacg 120
agactcctat taaatcagca cagttgcaaa cttcacctgc ctcaagccaa cagctcattg 180
aactcatatg tcgattgaga atcatttaca aaaccaggag agaaacaatg ggaagagcaa 240
cggtctctca tccctggacc tgacactcaa aacattatgt acaggatgca ggaacaaaat 300
ctgtctgatc agtgccctct cctgctggga aaaacaccca tcacggaaga atttggggat 360
taaatatgtc ttcaacaagg gaggcctggc ttctacaatg gtttaggtaa aggggccttt 420
gaagtagttc tggccaggct tgcaatacac acaacacaag a 461

<210> 137
<211> 269
<212> DNA
<213> Homo sapien

<400> 137
atagcaaatg gacacaaatt acaaatgtgt gtgcgtggga cgaagacatc tttgaaggtc 60
atgagtttgt tagtttaaca tcatatattt gtaatagtga aacctgtact caaaatataa 120
gcagcttgaa actggcttta ccaatcttga aatttgacca caagtgtctt atatatgcag 180
atctaatgta aaatccagaa cttggactcc atcgttaaaa ttatttatgt gtaacattca 240
aatgtgtgca ttaaatatgc ttccacagt 269

<210> 138
<211> 452
<212> DNA
<213> Homo sapien

<220>
<221> misc_feature
<222> (1)...(452)
<223> n = A,T,C or G

<400> 138
ctccatggga ggcaaaatat agagaattta tggtgcccaa ctcttatgta atcactggac 60
taatcttccc tggtaactat gcaacatttg gacagaaagg cacacaaaaa agtttaaata 120
tttcatgtgc caatctggaa aaaaataatt taaatcaaca gaacagacag tacatctaca 180
caaatgagga aagcagaaaa gatacctcac attcatttat ctcaggtttc aaagtggctt 240

caatgctaaa gtaaatgtat taacatttgg aaaatacaag acaatttttt tgtttgtttt 300
caattttttt agctctatac aatgattaca acataagaca aaaaaaaaaa aaaaacacaa 360
aaaacaaaac aaaaaaggag ttcaggactt gttatcagtg tccaagtggc taanaactgg 420
ttcccataac aagcattgaa agttaaggcc cc 452

<210> 139
<211> 474
<212> DNA
<213> Homo sapien

<400> 139
tgtgcctcat tgaggttaca attgaaacag atgtgagcac ctgagagact ttccctgatt 60
atattcctcc acaaaccact gtaccatatt accttatttt atcttcttga aattcttatt 120
cattggcttg tttgttgtct ctttgcatta gatatatgta agctccttgg cataaatttg 180
acattggtag gggactgaca ttctaacctg gcccaggccc taggagagag ataactccac 240
aaagcagcac atactatctt aggttagcag ggagctaact caccatgtag cagatgaaaa 300
aaaccaaacc cagcactgtg cataaatacc acttgccaag aagtcaggtc ctcggcaacc 360
gagaatcaac ctcagcacaa acgcaggtgg ctgggctctg ttccccctta gccaccacct 420
cagcctctcc cctcccctgc cccaagtgcc caagagcttg gctctctgtg cttt 474

<210> 140
<211> 487
<212> DNA
<213> Homo sapien

<400> 140
cttccctgcc tcgtgttcct gagaaacgga ttaatagccc tttatccccc tgcaccctcc 60
tgcaggggat ggcactttga gccctctgga gccctcccct tgctgagcct tactctcttc 120
agactttctg aatgtacagt gccgttggtt gggatttggg gactggaagg gaccaaggac 180
actgacccca agctgtcctg cctagcgtcc agcgtcttct aggagggtgg ggtctgcctg 240
tcctggtgtg gttggtttgg ccctgtttgc tgtgactacc cccccccctc cccgaaccga 300
gggacggctg cctttgtctc tgcctcagat gccacctgcc ccgcccatgc tccccatcag 360
cagcatccag actttcagga agggcagggc cagccagtcc agaaccgcat ccctcagcag 420
ggactgataa gccatctctc ggagggcccc ctaataccca agtggagtct ggttcacacc 480
ctggggg 487

<210> 141
<211> 248
<212> DNA
<213> Homo sapien

<220>
<221> misc_feature
<222> (1)...(248)
<223> n = A,T,C or G

<400> 141
ttaaagatgg ggaaatgagg cctgnaaata gaaaagattt gcctagagtc acacacactg 60
tcaggtcagg tagagtcaaa atcaggcacc ccgactcaca gactgcttca cattgccatc 120
agagattgtc ctgcaacaat attatgttta gttctactgc agaatgataa ctggatctta 180
ccccctttgc ctgatctggc cacaaacttg tttttcaggt ctttccatta ggctctcttc 240
agctaatt 248

<210> 142
<211> 173

<212> DNA
<213> Homo sapien

<400> 142
tactaagatt gtccaagcct ccctcttaaa actttctttc cctttagagg aatcattact 60
tcgtattaaa agtttctact tccttgtaga atatctacat ccaatgggcc atggcacaaa 120
atttaagtct agaaagaatc ttaaaggctc atcttatagt aaccagaggc agg 173

<210> 143
<211> 511
<212> DNA
<213> Homo sapien

<220>
<221> misc_feature
<222> (1)...(511)
<223> n = A,T,C or G

<400> 143
cctcgtcaga ggggtggttc ctggtnacct gtactccacg gacctcggtg aagcaaaagc 60
ttcagggcag agggaatgag gcaacccagt ggcagccccg ctgggccccg tggctcctgc 120
tctcctattg gacgtagagg caggggagag acttctctat acaaatattc tcatcacaga 180
agggatgatc cttgctgctc tgccgtaggg tttttgatgc tgagctatgc tgcacatgac 240
gttaacctaa agaacttgga ctgagctttt aaaaaaggac agcaaacaat tttataatcc 300
ttaaagtgta atagacggtt acactagtgc agggtattgg ggaggctctt tgggtgtgga 360
ggctgtcact tgtatttatt gtgactctaa atctttgata gtaaaacaaa tgtaaaaaga 420
aatgtttgcc accagatggg aatagaagtt ccaataagca ggctggaatg ggtggctata 480
cgttgtatca cgaggaagtt ttagactctg a 511

<210> 144
<211> 190
<212> DNA
<213> Homo sapien

<400> 144
cattcttctg tcacatgcca attcagttgt caatcccatt gtctatgctt accggaaccg 60
agacttccgc tacacttttc acaaaattat ctccaggtat cttctctgcc aagcagatgt 120
caagagtggg aatggtcagg ctggggtaca gcctgctctc ggtgtgggcc tatgatctag 180
gctctcgcct 190

<210> 145
<211> 169
<212> DNA
<213> Homo sapien

<400> 145
gatgtggtta tctcctcaga tggccagttt gccctctcag gctcctggga tggaaccctg 60
cgcctctggg atctcacaac gggcaccacc acgaggcgat ttgtgggcca taccaaggat 120
gtgctgagtg tggccttctc ctctgacaac cggcagattg tctctggat 169

<210> 146
<211> 511
<212> DNA
<213> Homo sapien

<400> 146
atctagagaa gatttgggaa acacatgata gctatggtta aatacttaac agggcaatca 60
cagggaagat gactagattt cctaacatcc atgagtgaaa tttatagaag tatactctct 120
gacttgatat aaaggaagat tttaaaaaac atgactgttc aggagtgttc aagtagggtc 180
agatgaccag tgattgggaa tacttcgtaa gcaggagcaa gtaagatctg agccactgtt 240
ctatcggtag ggtgtctgtg gtattccttg gtcaaagaag tactctaagc aacttcagtc 300
tcacgaatta ctatcaccct cgtgggcata catgatggtt accctaaaga ggaagtttca 360
gaaggcagta atattggatc ctggaatagt cagacaggag ccttcatgca gatacccttt 420
tcagttctcc atacacccat tcacaagtgg tcacaaaaac acccagtacc tttacttggc 480
tttacccact taacaatatg ctcaatatga g 511

<210> 147
<211> 421
<212> DNA
<213> Homo sapien

<220>
<221> misc_feature
<222> (1)...(421)
<223> n = A,T,C or G

<400> 147
gaccagttga gttcttcctg gctattgtat aatccacagc cacactgtga aagcaaatct 60
ggccagttag caacacaggg agaatctgcc tgaactgacc aaaggtgtcc atacttcatg 120
tcagtgagaa tttcacctcc atcatgttct aaagagccaa caacagattc tagggcactg 180
caaaatgctt cagcaattaa ttgaagttct gtttgagtac attcatcatc tttgagaatg 240
ctttctgggt cgttgtgagt cttgtgtctg atatatgcag ccaaatgagt ttcagtacag 300
ccacctccca acaaagccca tggttccttg agtgttaact gcaggacatg cagtgccgtc 360
tgacacgtga gcttcagctc atcccangca gtgtcatttc tgttgcagag aagccaagct 420
g 421

<210> 148
<211> 237
<212> DNA
<213> Homo sapien

<400> 148
acacaccact gttggccttc catctgggtt aagtcaactg tgagtagaaa ccgaagataa 60
cagttttgta ttcataatgg ccttttcata ctccaagtac ttttgagcac agagcctctt 120
gcttctgacc tggcacttgg aacacagata tatatatctt ttgttctgtc cctgggaaac 180
tgatatttgt gtaagacaac caccagatat tttctctaat aaaatcttct aaaatta 237

<210> 149
<211> 168
<212> DNA
<213> Homo sapien

<400> 149
agagaaagtt aaagtgcaat aatgtttgaa gacaataagt ggtggtgtat cttgtttcta 60
ataagataaa cttttttgtc tttgctttat cttattaggg agttgtatgt cagtgtataa 120
aacatactgt gtggtataac aggcttaata aattctttaa aaggagag 168

<210> 150
<211> 68
<212> DNA

<213> Homo sapien

<220>
<221> misc_feature
<222> (1)...(68)
<223> n = A,T,C or G

<400> 150
ggtggggttt ggcagagatg antttaagtg ctgtggccag aagcgggggg ggggtttggt 60
ggaaattt 68

<210> 151
<211> 421
<212> DNA
<213> Homo sapien

<400> 151
aggtgacacg tattcgggat gaaagtataa tagtcattcc ttcaaccctt gcatttatgg 60
actctggaaa tcgaagatcc acagtgagta aagatgttcg tccaaagaca aaaaatagaa 120
acagctcaac aaagcgagag acaaaaaaac aaaatggcac tgtggctctg cctttgaagt 180
ctgggctcca gcagagggct gatcttccca caggagacga gacggcctat gacactctcc 240
agaactgttg tcagtgccga attttacttc ccttgcccat tctaaatgag caccaggaga 300
agtgccagag gttagctcac caaaagaaac tccagtgggg ctggtgagat ggctcagcgg 360
gtaagagcac ccgactgctc ttccgaaggt ccggagttca aatcccagca accacatggt 420
g 421

<210> 152
<211> 507
<212> DNA
<213> Homo sapien

<220>
<221> misc_feature
<222> (1)...(507)
<223> n = A,T,C or G

<400> 152
gaattcggca cnagctcgtg ccgccagggt nggtccnttt tttgctccgc ctcgccanga 60
cttcctacag ctatcgccag tcgtcggcca cgtcntcctt cngaggcctg ggcggcggct 120
ccgtgcgttn tgggccgggg gtcgcctttc nctcncccag cattcacggg ggctccggcg 180
gccgcggcgt atccgtgtcc tccgcccgct ntgtgtcctc gtcctcctcn ggggcctacg 240
gctngctgct acngcggctt cctgaccgct tccnacgggc tgctggcngg caacgagaag 300
ctaaccatgc agaacctnaa cnaccgcctg gcctcctacc tgnacaaggt gcgcnccctg 360
taggcggcca acggcnagct agaggtgaag atccnctact gggtaccaga agcaggggcc 420
tgggccctgc ccgactacag ccactnctnc acnaccatgc agtacctgcn ggganaagat 480
tntngggngc caccatngag aactgca 507

<210> 153
<211> 513
<212> DNA
<213> Homo sapien

<400> 153
gaattcggca cgaggtggct cagatgtcca ctactgggag tatggtcgaa ttgggaattt 60
tattgtgaaa aagcccatgg tgctgggaca tgaagcttcg ggaacagtcg aaaaagtggg 120

atcatcggta aagcacctaa aaccaggtga tcgtgttgcc atcgagcctg gtgctccccg 180
agaaaatgat gaattctgca agatgggccg atacaatctg tcaccttcca tcttcttctg 240
tgccgcgccc cccgatgacg ggaacctctg ccggttctat aagcacaatg cagccttttg 300
ttacaagctt cctgacaatg tcacctttga ggaaggcgcc ctgatcgagc cactttctgt 360
ggggatccat gcctgcagga gaggcggagt taccctggga cacaaggtcc ttgtgtgtgg 420
agctgggcca atcgggatgg tcactttgct cgtggccaaa gcaatgggag cagctcaagt 480
agtggtgact gatctgtctg ctacccgatt gtc 513

<210> 154
<211> 507
<212> DNA
<213> Homo sapien

<220>
<221> misc_feature
<222> (1)...(507)
<223> n = A,T,C or G

<400> 154
ggcacgagct cgtgccgaat tcggcncgag cagacacaat ggtaagaatg gtgcctgtcc 60
tgctgtctct gctgctgctt ctgggtcctg ctgtccccca ggagaaccaa gatggtcgtt 120
actctctgac ctatatctac actgggctgt ccaagcatgt tgaagacgtc cccgcgtttc 180
aggcccttgg ctcactcaat gacctccagt tctttagata caacagtaaa gacaggaagt 240
ctcagcccat gggactctgg agacaggtgg aaggaatgga ggattggaag caggacagcc 300
aacttcagaa ggccagggag gacatcttta tggagaccct gaaagacatc gtggagtatt 360
acaacgacag taacgggtct cacgtattgc agggaaggtt tggttgtgag atcgagaata 420
acagaagcag cggagcattc tggaaatatt actatgatgg aaaggactac attgaattca 480
acaaagaaat cccagcctgg gtcccct 507

<210> 155
<211> 507
<212> DNA
<213> Homo sapien

<220>
<221> misc_feature
<222> (1)...(507)
<223> n = A,T,C or G

<400> 155
ggcacgagga gacctaaggg ctgagtntcg ggaacaggag aaagctctgt tggccctcca 60
gcagcagtgt gctgagcagg cacaggagca tgaggtggag accagggccc tgcaggacag 120
ctggctgcag gcccaggcag tgctcaagga acgggaccag gagctggaag ctctgcgggc 180
agaaagtcag tcctcccggc atcaggagga ggctgcccgg gcccgggctg aggctctgca 240
ggaggccctt ggcaaggctc atgctgccct gcaggggaaa gagcagcatc tcctcgagca 300
ggcagaattg agccgcagtc tggaggccag cactgcaacc ctgcaagcct ccctggatgc 360
ctgccaggca cacagtcggc agctggagga ggctctgagg atacaagaag gtgagatcca 420
ggaccaggat ctccgatacc aggaggatgt gcagcagctg cagcaggcac ttgcccagag 480
ggatgaagag ctgagacatc agcagga 507

<210> 156
<211> 509
<212> DNA
<213> Homo sapien

<220>
<221> misc_feature
<222> (1)...(509)
<223> n = A,T,C or G

<400> 156
ggcacgagga cagagagaac cctgtngaaa gagcgttacc aggaggtcct ggacaaacag 60
aggcaagtgg agaatcagct ccaagtgcaa ttaaagcagc ttcagcaaag gagagaagag 120
gaaatgaaga atcaccagga gatattaaag gctattcagg atgtgacaat aaagcgggaa 180
gaaacaaaga agaagataga gaaagagaag aaggagtttt tgcagaagga gcaggatctg 240
aaagctgaaa ttgagaagct ttgtgagaag ggcagaagag aggtgtggga aatggaactg 300
gatagactca agaatcagga tggcgaaata aataggaaca ttatggaaga gactgaacgg 360
gcctggaagg cagagatctt atcactagag agccggaaag agttactggt actgaaacta 420
gaagaagcag aaaaagaggc agaattgcac cttacttacc tcaagtcaac tcccccaaca 480
ctggagacag ttcgttccaa acaggagtg 509

<210> 157
<211> 507
<212> DNA
<213> Homo sapien

<400> 157
ggcacgaggg cagccctcct accggcgcac gtggtgccgc cgctgctgcc tcccgctcgc 60
cctgaaccca gtgcctgcag ccatggctcc cggccagctc gccttattta gtgtctctga 120
caaaaccggc cttgtggaat ttgcaagaaa cctgaccgct cttggtttga atctggtcgc 180
ttccggaggg actgcaaaag ctctcaggga tgctggtctg gcagtcagag atgtctctga 240
gttgacggga tttcctgaaa tgttgggggg acgtgtgaaa actttgcatc ctgcagtcca 300
tgctggaatc ctagctcgta atattccaga agataatgct gacatggcca gacttgattt 360
caatcttata agagttgttg cctgcaatct ctatcccttt gtaaagacag tggcttctcc 420
aggtgtaagt gttgaggagg ctgtggagca aattgacatt ggtggagtaa ccttactgag 480
agctgcagcc aaaaaccacg ctcgagt 507

<210> 158
<211> 507
<212> DNA
<213> Homo sapien

<220>
<221> misc_feature
<222> (1)...(507)
<223> n = A,T,C or G

<400> 158
ggcacgagtc gagctgtgcc tattcgngtc aatccaagag tgagtaatgt gaagtctgtc 60
tacaaaaccc acattgatgt cattcattat cggaaaacgg atgcaaaacg tctgcatggc 120
cttgatgaag aagcagaaca gaaacttttt tcagagaaac gtgtggaatt gcttaaggaa 180
ctttccagga aaccagacat ttatgagagg cttgcttcag ccttggctcc aagcatttat 240
gaacatgaag atataaagaa gggaattttg cttcagctct ttggcgggac aaggaaggat 300
tttagtcaca ctggaagggg caaatttcgg gctgagatca acatcttgct gtgtggcgac 360
cctggtacca gcaagtccca gctgctgcag tacgtgtaca acctcgtccc caggggccag 420
tacacgtntg ggaagggctc cagtgcannt ggcctnactg cntacgtaat gaaagaccct 480
gagacaaggn anctggnnct gnnacag 507

<210> 159
<211> 508

<212> DNA
<213> Homo sapien

<220>
<221> misc_feature
<222> (1)...(508)
<223> n = A,T,C or G

<400> 159
ggcacnanaa accaggatta tggtnnggat ccaaagattg ctaatgcaat aatgaaggca 60
gcagatgagg tagctgaagg taaattaaat gatcattttc ctctcgtggt atggcagact 120
ggatcaggaa ctcagacaaa tatgaatgta aatgaagtca ttagcaatag agcaattgaa 180
atgttaggag gtgaacttgg cagcaagata cctgtgcatc ccaacgatca tgttaataaa 240
agccagagct caaatgatac ttttcccaca gcaatgcaca ttgctgctgc aatagaagtt 300
catgaagtac tgttaccagg actacagaag ttacatgatg ctcttgatgc aaaatccaaa 360
gagtttgcac agatcatcaa gattggacgt actcatactc aggatgctgt tccacttact 420
cttgggcagg aatttagtgg ttatgttcaa caagtaaaat atgcaatgac aagaataaaa 480
gctgccatgc caagaatcta tgagctcg 508

<210> 160
<211> 508
<212> DNA
<213> Homo sapien

<220>
<221> misc_feature
<222> (1)...(508)
<223> n = A,T,C or G

<400> 160
ggcacgagct tggagcaaag tcatctnaag gaattagagg acacacttca ggttaggcac 60
atacaagagt ttgagaaggt tatgacagac cacagagttt ctttggagga attaaaaaag 120
gaaaaccaac aaataattaa tcaaatacaa gaatctcatg ctgaaattat ccaggaaaaa 180
gaaaaacagt tacaggaatt aaaactcaag gtttctgatt tgtcagacac gagatgcaag 240
ttagaggttg aacttgcgtt gaaggaagca gaaactgatg aaataaaaat tttgctggaa 300
gaaagcagag cccagcagaa ggagaccttg aaatctcttc ttgaacaaga gacagaaaat 360
ttgagaacag aaattagtaa actcaaccaa aagattcagg ataataatga aaattatcag 420
gtgggcttag cagagctaag aactttaatg acaattgaaa aagatcagtg tatttccgag 480
ttaattagta gacatgaaga agaatcta 508

<210> 161
<211> 507
<212> DNA
<213> Homo sapien

<400> 161
ggcacgagcg ctaccggcgc ctcctctgcg gccactgagc cggagccggc ctgagcagcg 60
ctctcggttg cagtacccac tggaaggact taggcgctcg cgtggacacc gcaagcccct 120
cagtagcctc ggcccaagag gcctgctttc cactcgctag ccccgccggg ggtccgtgtc 180
ctgtctcggt ggccggaccc gggcccgagc ccgagcagta gccggcgcca tgtcggtggt 240
gggcatagac ctgggcttcc agagctgcta cgtcgctgtg gcccgcgccg gcggcatcga 300
gactatcgct aatgagtata gcgaccgctg cacgccggct tgcatttctt ttggtcctaa 360
gaatcgttca attggagcag cagctaaaag ccaggtaatt tctaatgcaa agaacacagt 420
ccaaggattt aaaagattcc atggccgagc attctctgat ccatttgtgg aggcagaaaa 480
atctaacctt gcatatgata ttgtgca 507

<210> 162
<211> 507
<212> DNA
<213> Homo sapien

<220>
<221> misc_feature
<222> (1)...(507)
<223> n = A,T,C or G

<400> 162
ggcacgagca gctgtgcacc gacatgntct cagtgtcctg agtaagacca aagaagctgg 60
caagatcctc tctaataatc ccagcaaggg actggccctg ggaattgcca aagcctggga 120
gctctacggc tcacccaatg ctctggtgct actgattgct caagagaagg aaagaaacat 180
atttgaccag cgtgccatag agaatgagct actggccagg aacatccatg tgatccgacg 240
aacatttgaa gatatctctg aaaaggggtc tctggaccaa gaccgaaggc tgtttgtgga 300
tggccaggaa attgctgtgg tttacttccg ggatggctac atgcctcgtc agtacagtct 360
acagaattgg gaagcacgtc tactgctgga gaggtcacat gctgccaagt gcccagacat 420
tgccacccag ctggctggga ctaagaaggt gcagcaggag ctaagcaggc cgggcatgct 480
ggagatgttg ctccctggcc agcctga 507

<210> 163
<211> 460
<212> DNA
<213> Homo sapien

<400> 163
ggcacgagaa ataactttat ttcattgtgg gtcgcggttc ttgtttgtgg atcgctgtga 60
tcgtcacttg acaatgcaga tcttcgtgaa gactctgact ggtaagacca tcaccctcga 120
ggttgagccc agtgacacca tcgagaatgt caaggcaaag atccaagata aggaaggcat 180
ccctcctgac cagcagaggc tgatctttgc tggaaaacag ctggaagatg ggcgcaccct 240
gtctgactac aacatccaga aagagtccac cctgcacctg gtgctccgtc tcagaggtgg 300
gatgcaaatc ttcgtgaaga cactcactgg caagaccatc acccttgagg tggagcccag 360
tgacaccatc gagaacgtca aagcaaagat ccaggacaag gaaggcattc ctcctgacca 420
gcagaggttg atctttgccg gaaagcagct ggaagatggg 460

<210> 164
<211> 462
<212> DNA
<213> Homo sapien

<400> 164
ggcacgagcc ggatctcatt gccacgcgcc cccgacgacc gcccgacgtg cattcccgat 60
tccttttggt tccaagtcca atatggcaac tctaaaggat cagctgattt ataatcttct 120
aaaggaagaa cagacccccc agaataagat tacagttgtt ggggttggtg ctgttggcat 180
ggcctgtgcc atcagtatct taatgaagga cttggcagat gaacttgctc ttgttgatgt 240
catcgaagac aaattgaagg gagagatgat ggatctccaa catggcagcc ttttccttag 300
aacaccaaag attgtctctg gcaaagacta taatgtaact gcaaactcca agctggtcat 360
tatcacggct ggggcacgtc agcaagaggg agaaagccgt cttaatttgg tccagcgtaa 420
cgtgaacatc tttaaattca tcattcctaa tgttgtaaaa ta 462

<210> 165
<211> 462
<212> DNA

<213> Homo sapien

<400> 165
ggcacgagga agccatgagc agcaaagtct ctcgcgacac cctgtacgag gcggtgcggg 60
aagtcctgca cgggaaccag cgcaagcgcc gcaagttcct ggagacggtg gagttgcaga 120
tcagcttgaa gaactatgat ccccagaagg acaagcgctt ctcgggcacc gtcaggctta 180
agtccactcc ccgccctaag ttctctgtgt gtgtcctggg ggaccagcag cactgtgacg 240
aggctaaggc cgtggatatc ccccacatgg acatcgaggc gctgaaaaaa ctcaacaaga 300
ataaaaaact ggtcaagaag ctggccaaga agtatgatgc gtttttggcc tcagagtctc 360
tgatcaagca gattccacga atcctcggcc caggtttaaa taaggcagga aagttccctt 420
ccctgctcac acacaacgaa aacatggtgg ccaaagtgga tg 462

<210> 166
<211> 459
<212> DNA
<213> Homo sapien

<220>
<221> misc_feature
<222> (1)...(459)
<223> n = A,T,C or G

<400> 166
ggcacgagag ggacctgtnt gaatggntcc actagggttn anntgnctct tacttttaac 60
cantnaaatn gacctgcccg tgaanangcg ggcntgacac annaanacga gaagacccta 120
tggagcttta atttattaat gcanacagna cctaacaaac ccacangtcc taaactacca 180
agcctgcatt aaaaatttcg gntggggcna cctcnnagca naacccaacc tccgagcaac 240
tcatgctaag acttcaccag tcaaagctga actactatac tcaattgatc caataacttg 300
accaacagan caagntaccc tagggataac ancacaatcc tattctagac cccttatnac 360
caatangntt tacacctcna tngnggaacc aggacatccg atggggcagn cgttattaaa 420
gttngttgnt aacnataaag tctacgtgat ctgagttag 459

<210> 167
<211> 464
<212> DNA
<213> Homo sapien

<220>
<221> misc_feature
<222> (1)...(464)
<223> n = A,T,C or G

<400> 167
gaattgggac caacganaan cntgcggntc ttnttttgcn tccanngccc agctnattgc 60
tcagacacac atggggaagg tnaaggtcgg gagtcaacng atttggtngt attgnagcgt 120
ttggtcacca gngctgcttt taactctggn aaagtggata ttgttgtcat naatgacccc 180
tncattgacc tnaactacat ggtttacatg ttccaatatg attccaccca tggcaaattc 240
catngcaccg tnaaggctga gaacgggaag cttgtnatca atggaaatcc catcaccatc 300
tttcangaac ganatccntn caaaaatcaa anttgggggc gatgcttggc cncttgaagt 360
accgttcaan gggaannncc ccactttggc cgntntttnc aancccaccc caatttgggn 420
aaaaaaaaag gggnntttgg gggggggcct tttanntttt tttt 464

<210> 168
<211> 462
<212> DNA

<213> Homo sapien

<220>
<221> misc_feature
<222> (1)...(462)
<223> n = A,T,C or G

<400> 168
ggcacgaggn nnaacctncg gggctggggc agcacgcctt gngcaancct gcactgcact 60
gaagacccgg tgccggaagc cgnnggcngc nacatgcagn aactgaacca gctgggcgcg 120
cancagttct cagacctgac agaggtgctt ttacacttcc taactgatcc anantangtg 180
gaaatattnt tngttnatnt catntgaatn atccancncc aatcatanca nntttnattn 240
cctcataanc nttgagaana gcnnccttnt gnttncanan ggtgctntga anangagtct 300
cacangcaan caggtccaag cggatttnnt aactntgggt cttantgang agaaagncac 360
ttacttttct gaaancngga agcagaatgc tcccaccctt gctcgatggg ccatacgtca 420
agactctgat gattaaccag ctttanatat ggacnggaaa tt 462

<210> 169
<211> 460
<212> DNA
<213> Homo sapien

<220>
<221> misc_feature
<222> (1)...(460)
<223> n = A,T,C or G

<400> 169
ggcacgaggg acagcagacn agacagtcac agcagccttg acaaaacgtt cctggaactc 60
aagntcttnt ncncaaagga ggacagagca nacagcagag accatggant ctncctcggc 120
ccctccccac agatggtgca tcccctggca naggctcctg ctcacagcct cacttctaac 180
cttctggaac ccgcccacca ctgccaagct cactattgaa tccacgccgt tcaatgnntc 240
ntaggggaag gaggngcttt ctactnttnc acaatctgan ccccttcttn tttggttact 300
ancatggctc tncatgtnaa aatactggna tggntaacct gtcaaattta taggnantnt 360
gctaattggg aaactnccnn tngtctaccc caggggnccc agattcctnn gttcncataa 420
cnattaattt aacccctaat gncaanccct tngttaaaga 460

<210> 170
<211> 508
<212> DNA
<213> Homo sapien

<220>
<221> misc_feature
<222> (1)...(508)
<223> n = A,T,C or G

<400> 170
ggcacgaggg ggatttttag gtggtcnggt gtggtatcag gaataatgtg ggaggccaga 60
ttgaagtcca ggccaggaac aatggtaatt gtgggactta agaaagtgtg agtacagctg 120
aatgagccgg ggagcagaaa gtatatgcgt caggtatgag gaagaaaata gattttggaa 180
gttatgagaa atgtagagag tgagttgagc atagtttgtg attttgaggg cctctaacag 240
tattaaagca gcggcagcgg ctgcacacag acatgatggc taggctaaaa caggaaggtc 300
aagttgtttg gacagaaagg ctacagggtg cagtcctggc tcttgtgtaa gaattctgac 360
cacactaacc atgcctagga aggaaaggag ttgttctttt gtaagggatt gaggtttggg 420

agattaatcg gacacgatca gcagggagag cacctgtgtt tttatgagaa ttatgctgag 480
ataggtaaca gatgaggatg aaatttgg 508

<210> 171
<211> 507
<212> DNA
<213> Homo sapien

<220>
<221> misc_feature
<222> (1)...(507)
<223> n = A,T,C or G

<400> 171
ggcacgagac cagccactag cgcagnctcg agcgatggcc tatgtccccg caccgggcta 60
ccagcccacc tacaacccga cgctgcctta ctaccagccc atcccgggcg ggctcaacgt 120
gggaatgtct gtttacatcc aaggagtggc cagcgagcac atgaagcggt tcttcgtgaa 180
ctttgtggtt gggcaggatc cgggctcaga cgtcgccttc cacttcaatc cgcggtttga 240
cggctgggac aaggtggtct tcaacacgtt gcagggcggg aagtggggca gcgaggagag 300
gaagaggagc atgcccttca aaaagggtgc cgcctttgag ctggtcttca tagtcctggc 360
tgagcactac aaggtggtgg taaatggaaa tcccttctat gagtacgggc accggcttcc 420
cctacagatg gtcacccacc tgcaagtgga tggggatctg caacttcaat caatcaactt 480
catcggaggc cagcccctcc ggcccca 507

<210> 172
<211> 409
<212> DNA
<213> Homo sapien

<400> 172
ggcacgagct ggagtgtctg ctgccacccc ctcgtcctct gcagaaatgt ctgtcaccta 60
cgatgactct gtgggagtgg aagtgtccag cgacagcttc tgggaggttg ggaactacaa 120
acggactgtg aagcggattg acgatggcca ccgcctgtgt ggtgacctca tgaactgtct 180
gcatgagcgg gcacgcatcg agaaggcgta tgcacagcag ctcactgagt gggcccgacg 240
ctggaggcag ctggtagaga agggaccaca gtatgggacc gtggagaagg cctggatagc 300
tgtcatgtct gaagcagaga gggtgagtga actgcacctg gaagtgaagg catcactgat 360
gaatgaagac tttgagaaga tcaagaactg gcagaaggaa gcctttcac 409

<210> 173
<211> 409
<212> DNA
<213> Homo sapien

<400> 173
ggcacgaggg cagctagagg aagagtccaa ggccaagaac gcactggccc acgccctgca 60
gtcagctcgc catgactgtg acctgctgcg ggaacagtat gaagaggagc aggaagccaa 120
ggctgagctg cagagggcca tgtccaaggc caacagcgag gtagcccagt ggaggacgaa 180
atatgagacg gatgccatcc agcgcacaga ggagctggaa gaggccaaga agaagctggc 240
tcagcgtctg caggatgctg aggaacatgt agaagctgtg aattccaaat gcgcttctct 300
tgaaaagacg aagcagcgac ttcagaatga agtggaggac ctcatgattg acgtggagag 360
gtctaatgct gcctgcgctg cgcttgataa gaagcagagg aactttgac 409

<210> 174
<211> 407
<212> DNA

<213> Homo sapien

<400> 174
ggcacgagcc ggggcggggc gcggcgctcc ggctcgaggc attcggagct gcgggagccg 60
ggctggcagg agcaggatgg cggcggcggc ggctgcaggc gaggcgcgcc gggtgctggt 120
gtacggcggc aggggcgctc tgggttctcg atgcgtgcag gcttttcggg cccgcaactg 180
gtgggttgcc agcgttgatg tggtggagaa tgaagaggcc agcgctagca tcattgttaa 240
aatgacagac tcgttcactg agcaggctga ccaggtgact gctgaggttg gaaagctctt 300
gggtgaagag aaggtggatg caattctttg cgttgctgga ggatgggccg ggggcaatgc 360
caaatccaag tctctcttta agaactgtga cctgatgtgg aagcaga 407

<210> 175
<211> 407
<212> DNA
<213> Homo sapien

<400> 175
ggcacgagct tgcccgtcgg tcgctagctc gctcggtgcg cgtcgtcccg ctccatggcg 60
ctcttcgtgc ggctgctggc tctcgccctg gctctggccc tgggccccgc cgcgaccctg 120
gcgggtcccg ccaagtcgcc ctaccagctg gtgctgcagc acagcaggct ccggggccgc 180
cagcacggcc ccaacgtgtg tgctgtgcag aaggttattg gcactaatag gaagtacttc 240
accaactgca agcagtggta ccaaaggaaa atctgtggca aatcaacagt catcagctac 300
gagtgctgtc ctggatatga aaaggtccct ggggagaagg gctgtccagc agccctacca 360
ctctcaaacc tttacgagac cctgggagtc gttggatcca ccaccac 407

<210> 176
<211> 409
<212> DNA
<213> Homo sapien

<400> 176
ggcacgagtg gtgccaaaac gggaccatgc cctcctggag gagcagagca agcagcagtc 60
caacgagcac ctgcgccgcc agttcgccag ccaggccaat gttgtggggc cctggatcca 120
gaccaagatg gaggagatcg ggcgcatctc cattgagatg aacgggaccc tggaggacca 180
gctgagccac ctgaagcagt atgaacgcag catcgtggac tacaagccca acctggacct 240
gctggagcag cagcaccagc tcatccagga ggccctcatc ttcgacaaca agcacaccaa 300
ctataccatg gagcacatcc gcgtgggctg ggagcagctg ctcaccacca ttgcccgcac 360
catcaacgag gtggagaacc agatcctcac ccgcgacgcc aagggcatc 409

<210> 177
<211> 408
<212> DNA
<213> Homo sapien

<400> 177
ggcacgaggt ccaggtaact gcaaaaacaa tggctcagca tgaagaactg atgaagaaaa 60
ctgaaacaat gaatgtagtt atggagacca ataaaatgct aagagaagag aaggagcagg 120
tttcaaaaat ggcatcagtc cgtcagcatt tggaagaaac aacacagaaa gcagaatcac 180
agttgttgga gtgtaaagca tcttgggagg aaagagagag aatgttaaag gatgaagttt 240
ccaaatgtgt atgtcgctgt gaagatctgg agaaacaaaa cagattactt catgatcaga 300
tcgaaaaatt aagtgacaag gtcgttgcct ctgtgaagga aggtgtacaa ggtccactga 360
atgtatctct cagtgaagaa ggaaaatctc aagaacaaat tttggaaa 408

<210> 178
<211> 92

<212> DNA
<213> Homo sapien

<400> 178
ggcacgagaa gaaattaaga gctaaagaca aggagaatga aaatatggtt gcaaagctga 60
acaaaaaagt taaagagcta gaagaggaga tg 92

<210> 179
<211> 411
<212> DNA
<213> Homo sapien

<400> 179
ggcacgagga gacacgccac ctataccaca gttctcagaa tgaattagct aagttggaat 60
cagaacttaa gagtctcaaa gaccagttga ctgatttaag taactcttta gaaaaatgta 120
aggaacaaaa aggaaacttg gaagggatca taaggcagca agaggctgat attcaaaatt 180
ctaagttcag ttatgaacaa ctggagactg atcttcaggc ctccagagaa ctgaccagta 240
ggctgcatga agaaataaat atgaaagagc aaaagattat aagcctgctt tctggcaagg 300
aagaggcaat ccaagtagct attgctgaac tgcgtcagca acatgataaa gaaattaaag 360
agctggaaaa cctgctgtcc caggaggaag aggagaatat tgttttagaa g 411

<210> 180
<211> 411
<212> DNA
<213> Homo sapien

<400> 180
ggcacgaggt tgttcggagc gggcgagcgg agttagcagg gctttactgc agagcgcgcc 60
gggcactcca gcgaccgtgg ggatcagcgt aggtgagctg tggccttttg cgaggtgctg 120
cagccatagc tacgtgcgtt cgctacgagg attgagcgtc tccacccatc ttctgtgctt 180
caccatctac ataatgaatc ccagtatgaa gcagaaacaa gaagaaatca aagagaatat 240
aaagactagt tctgtcccaa gaagaactct gaagatgatt cagccttctg catctggatc 300
tcttgttgga agagaaaatg agctgtccgc aggcttgtcc aaaaggaaac atcggaatga 360
ccacttaaca tctacaactt ccagccctgg ggttattgtc ccagaatcta g 411

<210> 181
<211> 411
<212> DNA
<213> Homo sapien

<400> 181
ggcacgaggc gggacagggc gaagcggcct gcgcccacgg agcgcgcgac actgcccgga 60
agggaccgcc acccttgccc cctcagctgc ccactcgtga tttccagcgg cctccgcgcg 120
cgcacgatgc cctcggccac cagccacagc gggagcggca gcaagtcgtc cggaccgcca 180
ccgccgtcgg gttcctccgg gagtgaggcg gccgcgggag ccggggccgc cgcgccggct 240
tctcagcacc ccgcaaccgg caccggcgct gtccagaccg aggccatgaa gcagattctc 300
ggggtgatcg acaagaaact tcggaacctg gagaagaaaa agggtaagct tgatgattac 360
caggaacgaa tgaacaaagg ggaaaggctt aatcaagatc agctggatgc c 411

<210> 182
<211> 411
<212> DNA
<213> Homo sapien

<400> 182

ggcacgagcc gacatggagc tgttcctcgc gggccgccgg gtgctggtca ccggggcagg 60
caaaggtata gggcgcggca cggtccaggc gctgcacgcg acgggcgcgc gggtggtggc 120
tgtgagccgg actcaggcgg atcttgacag ccttgtccgc gagtgcccgg ggatagaacc 180
cgtgtgcgtg gacctgggtg actgggaggc caccgagcgg gcgctgggca gcgtgggccc 240
cgtggacctg ctggtgaaca acgccgctgt cgccctgctg cagcccttcc tggaggtcac 300
caaggaggcc tttgacagat cctttgaggt gaacctgcgt gcggtcatcc aggtgtcgca 360
gattgtggcc aggggcttaa tagcccgggg agtcccaggg gccatcgtga a 411

<210> 183
<211> 409
<212> DNA
<213> Homo sapien

<400> 183
ggcacgagcc tacactctgg ccagagatac cacagtcaaa cctggagcca aaaaggacac 60
aaaggactct cgacccaaac tgccccagac cctctccaga ggttggggtg accaactcat 120
ctggactcag acatatgaag aagctctata taaatccaag acaagcaaca aacccttgat 180
gattattcat cacttggatg agtgcccaca cagtcaagct ttaaagaaag tgtttgctga 240
aaataaagaa atccagaaat tggcagagca gtttgtcctc ctcaatctgg tttatgaaac 300
aactgacaaa cacctttctc ctgatggcca gtatgtcccc aggattatgt ttgttgaccc 360
atctctgaca gttagagccg atatcactgg aagatattca aatcgtctc 409

<210> 184
<211> 410
<212> DNA
<213> Homo sapien

<400> 184
ggcacgaggt cattccagca ccaacaggat ccaagccaga ttgattgggc tgcattggcc 60
caagcttgga ttgcccaaag agaagcttca ggacagcaaa gcatggtaga acaaccacca 120
ggaatgatgc caaatggaca agatatgtct acaatggaat ctggtccaaa caatcatggg 180
aatttccaag gggattcaaa cttcaacaga atgtggcaac cagaatgggg aatgcatcag 240
caacccccac acccccctcc agatcagcca tggatgccac caacaccagg cccaatggac 300
attgttcctc cttctgaaga cagcaacagt caggacagtg gggaatttgc ccctgacaac 360
aggcatatat ttaaccagaa caatcacaac tttggtggac cacccgataa 410

<210> 185
<211> 411
<212> DNA
<213> Homo sapien

<220>
<221> misc_feature
<222> (1)...(411)
<223> n = A,T,C or G

<400> 185
ggcacgagca cagatgtagt ttctctgcgg cgtgtgcgtt ttccctcctc ccccgccctc 60
agggtccacg gccaccatgg cgtattaggg gcagcagtgc ctgcggcagc attggccttt 120
gcagcggcgg cagcagcacc aggctctgca gcggcaaccc ccagcggctt aagccatggc 180
gcttctcacg gcattcagca gcagcgttgc tgtaaccgac aaagacacct tcgaattaag 240
cacattcctc gattccagca aagcaccgca acatgaccga aatgagcttc ctgagcagcg 300
aggtgttggt gggggacttg atgtccccct tcgacccgtc gggtttgggg gctgaagaaa 360
gcctangtct cttagatgat tacctggagg tggccaagca cttcaaacct c 411

<210> 186
<211> 410
<212> DNA
<213> Homo sapien

<400> 186
ggcacgagct tctagtcccg ccatggccgc tctcacccgg gacccccagt tccagaagct 60
gcagcaatgg taccgcgagc accgctccga gctgaacctg cgccgcctct tcgatgccaa 120
caaggaccgc ttcaaccact tcagcttgac cctcaacacc aaccatgggc atatcctggt 180
ggattactcc aagaacctgg tgacggagga cgtgatgcgg atgctggtgg acttggccaa 240
gtccaggggc gtggaggccg cccgggagcg gatgttcaat ggtgagaaga tcaactacac 300
cgagggtcga gccgtgctgc acgtggctct gcggaaccgg tcaaacacac ccatcctggt 360
agacggcaag gatgtgatgc cagaggtcaa caaggttctg gacaagatga 410

<210> 187
<211> 506
<212> DNA
<213> Homo sapien

<400> 187
ctttcgtggc tcactccctt tcctctgctg ccgctcggtc acgcttgtgc ccgaaggagg 60
aaacagtgac agacctggag actgcagttc tctatccttc acacagctct ttcaccatgc 120
ctggatcact tcctttgaat gcagaagctt gctggccaaa agatgtggga attgttgccc 180
ttgagatcta ttttccttct caatatgttg atcaagcaga gttggaaaaa tatgatggtg 240
tagatgctgg aaagtatacc attggcttgg gccaggccaa gatgggcttc tgcacagata 300
gagaagatat taactctctt tgcatgactg tggttcagaa tcttatggag agaaataacc 360
tttcctatga ttgcattggg cggctggaag ttggaacaga gacaatcatc gacaaatcaa 420
agtctgtgaa gactaatttg atgcagctgt ttgaagagtc tgggaataca gatatagaag 480
gaatcgacac aactaatgca tgctat 506

<210> 188
<211> 506
<212> DNA
<213> Homo sapien

<400> 188
gccacagagg cggcggagag atggccttca gcggttccca ggctccctac ctgagtccag 60
ctgtcccctt ttctgggact attcaaggag gtctccagga cggacttcag atcactgtca 120
atgggaccgt tctcagctcc agtggaacca ggtttgctgt gaactttcag actggcttca 180
gtggaaatga cattgccttc cacttcaacc ctcggtttga agatggaggg tacgtggtgt 240
gcaacacgag gcagaacgga agctgggggc ccgaggagag gaagacacac atgcctttcc 300
agaaggggat gccctttgac ctctgcttcc tggtgcagag ctcagatttc aaggtgatgg 360
tgaacgggat cctcttcgtg cagtacttcc accgcgtgcc cttccaccgt gtggacacca 420
tctccgtcaa tggctctgtg cagctgtcct acatcagctt ccagcctccc ggcgtgtggc 480
ctgccaaccc ggctcccatt acccag 506

<210> 189
<211> 399
<212> DNA
<213> Homo sapien

<400> 189
ctggacagga gaagagcctg gctgctgaag gcagggctga cacgaccacg ggcagcattg 60
ctggagcccc agaggatgaa agatcgcaga gcacagcccc ccaggcacca gagtgcttcg 120
accctgccgg accggctggg ctcgtgaggc cgacatctgg cctttcccag ggcccaggaa 180

aggaaacctt ggaaagtgct ctaatcgctc tagactctga aaaacccaag aaacttcgct 240
tccacccaaa gcagctgtac ttctctgcca ggcagggtga gctgcagaag gtgcttctca 300
tgctggttga tggaattgat cccaacttca aaatggagca ccaaagtaag cgttccccat 360
tacatgctgc tgcggaggct ggccacgtgg acatctgcc 399

<210> 190
<211> 401
<212> DNA
<213> Homo sapien

<400> 190
cggcgacggt ggtggtgact gagcggagcc cggtgacagg atgttggtgt tggtattagg 60
agatctgcac atcccacacc ggtgcaacag tttgccagct aaattcaaaa aactcctggt 120
gccaggaaaa attcagcaca ttctctgcac aggaaacctt tgcaccaaag agagttatga 180
ctatctcaag actctggctg gtgatgttca tattgtgaga ggagacttcg atgagaatct 240
gaattatcca gaacagaaag ttgtgactgt tggacagttc aaaattggtc tgatccatgg 300
acatcaagtt attccatggg gagatatggc cagcttagcc ctgttgcaga ggcaatttga 360
tgtggacatt cttatctcgg gacacacaca caaatttgaa g 401

<210> 191
<211> 406
<212> DNA
<213> Homo sapien

<400> 191
tggcagccta agccgtggga gggttccagt cgagaatggg aagatgaaag acttcagatg 60
gaacagaaat aaatgccttt tttgacaaac gcagcagtgc gtgcctctag cttgcaagag 120
cgttactccc cttcatagct ttaaaggttt ttcgcactgc gtgcagttag agtagctaaa 180
tcttgtgtga cgctccacaa acacttgtaa gaattttgca gagaaagata accgttgcca 240
cccaatgccc cccacaggca ttctactccc cagtacctct tagggtggga gaaatggtga 300
agagttgttc ctacaacttg ctaacctagt ggacagggta gtagattagc atcatccgga 360
tagatgtgaa gaggacggct gtttggataa taattaagga taaaat 406

<210> 192
<211> 316
<212> DNA
<213> Homo sapien

<400> 192
cccggggagg ccctggtcat aaaactttaa attttactag tgttacttaa tgtatattct 60
aaaaagagaa tgcagtaact aatgccctaa atgtttgatc tctgtttgtc attacttttt 120
caaaattatt tttttctgta aagtataata tataaaactt cttgcttaaa ttgaatttct 180
atattagtgg ttaattgcag tttattaaag ggatcattat cagtaatttc atagcaactg 240
ttctagtgtt ttgtgttttt aaaacagaat taggaatttg agatatctga ttatattttt 300
catatgaatc acagac 316

<210> 193
<211> 146
<212> DNA
<213> Homo sapien

<400> 193
gaaacatgga ctgcccctta aattttgact gtcctaaaaa cctatttctg atttataata 60
tgctgcctga taaagtgaca ctagatgtac cagctgagtg tttaatcttc ccatcacaga 120
tcagatttga gcattaacag gtattt 146

<210> 194
<211> 405
<212> DNA
<213> Homo sapien

<400> 194
cggatgtgct cactgacatt ctactccaag tcggagatgc agatccactc caagtcacac 60
accgagacca agccccacaa gtgcccacat tgctccaaga ccttcgccaa cagctcctac 120
ctggcccagc acatccgtat acactcaggg gctaagccct acagttgtaa cttctgtgag 180
aaatccttcc gccagctctc ccaccttcag cagcacaccc gaatccacac tggtgataga 240
ccatacaaat gtgcacaccc aggctgtgag aaagccttca cacaactctc caatctgcag 300
tcccacagac ggcaacacaa caaagataaa cccttcaagt gccacaactg tcatcgggcg 360
tacacggatg cagcctcact agaggtgcac ctgtctacgc acaca 405

<210> 195
<211> 421
<212> DNA
<213> Homo sapien

<400> 195
agaattcggc acgagctact ccttgcgcgc tggcactccg cagcctttaa ggttcgcgcg 60
ggggccaggc aagagttagc catgaagagc ctcaagtccc gcctgaggag gcaggacgtg 120
cccggccccg cgtcgtctgg cgccgccgcc gccagcgcgc atgcagcaga ttggaataaa 180
tatgatgacc gattgatgaa agcagcagaa aggggggatg tagaaaaagt gacgtcaatc 240
cttgctaaaa agggggtcaa tccaggcaaa ctagatgtgg aaggcagatc tgtcttccat 300
gttgtgacct caaaggggaa tcttgagtgt ttgaatgcca tccttataca tggagttgat 360
attacaacca gtgacactgc agggagaaat gctcttcacc tggctgctaa gtatggacat 420
g 421

<210> 196
<211> 476
<212> DNA
<213> Homo sapien

<400> 196
agaattgatc tatagattta atgcaatgcc tactaaaatc ccagtacgat tttttacagg 60
catagacaat agacatagcc aaaacttatt ctaaaataca tatgaagatg cacaggccct 120
agttatacaa tcttgacaaa gaagaataaa gtgggaagaa tctatttgat tttaaggctt 180
accatgtaac tacagtcatc aagagagtgt ggtatcggca gacggtcaga catacagatc 240
aatggaatgt aacagaggac ccagaaatag gcccacacag atatgctcaa tggatatttg 300
acaagcgtgc aaaacaattc aatggaagaa taagctttca aaaaaatggc gttggagcaa 360
ccggacatcc ataggaaaaa atgaacccat acctaaacca taaaccttat ataaaaataa 420
acacaaaatg aatcataggc ttaaatgtaa gctataaaac ttttagagaa aaacac 476

<210> 197
<211> 503
<212> DNA
<213> Homo sapien

<400> 197
tagccctcgg tgaagcccca gaccacagct atgagtccct tcgtgtgacg tctgcgcaga 60
aacatgttct gcatgtccag ctcaaccggc ccaacaagag gaatgccatg aacaaggtct 120
tctggagaga gatggtagag tgcttcaaca agatttcgag agacgctgac tgtcgggcgg 180
tggtgatctc tggtgcagga aaaatgttca ctgcaggtat tgacctgatg gacatggctt 240

cggacatcct gcagcccaaa ggagatgatg tggcccggat cagctggtac ctccgtgaca 300
tcatcactcg ataccaggag accttcaacg tcatcgagag gtgccccaag cccgtgattg 360
ctgccgtcca tgggggctgc attggcggag gtgtggacct tgtcaccgcc tgtgacatcc 420
ggtactgtgc ccaggatgct ttcttccagg tgaaggaggt ggacgtgggt ttggctgccc 480
atgtaggaac actgcagcgc ctg 503

<210> 198
<211> 168
<212> PRT
<213> Homo sapien

<400> 198
Phe Val Ala His Ser Leu Ser Ser Ala Ala Ala Arg Ser Arg Leu Cys
1               5                   10                  15
Pro Lys Glu Glu Thr Val Thr Asp Leu Glu Thr Ala Val Leu Tyr Pro
            20                  25                  30
Ser His Ser Ser Phe Thr Met Pro Gly Ser Leu Pro Leu Asn Ala Glu
        35                  40                  45
Ala Cys Trp Pro Lys Asp Val Gly Ile Val Ala Leu Glu Ile Tyr Phe
    50                  55                  60
Pro Ser Gln Tyr Val Asp Gln Ala Glu Leu Glu Lys Tyr Asp Gly Val
65                  70                  75                  80
Asp Ala Gly Lys Tyr Thr Ile Gly Leu Gly Gln Ala Lys Met Gly Phe
                85                  90                  95
Cys Thr Asp Arg Glu Asp Ile Asn Ser Leu Cys Met Thr Val Val Gln
            100                 105                 110
Asn Leu Met Glu Arg Asn Asn Leu Ser Tyr Asp Cys Ile Gly Arg Leu
        115                 120                 125
Glu Val Gly Thr Glu Thr Ile Ile Asp Lys Ser Lys Ser Val Lys Thr
    130                 135                 140
Asn Leu Met Gln Leu Phe Glu Glu Ser Gly Asn Thr Asp Ile Glu Gly
145                 150                 155                 160
Ile Asp Thr Thr Asn Ala Cys Tyr
                165

<210> 199
<211> 168
<212> PRT
<213> Homo sapien

<400> 199
His Arg Gly Gly Gly Glu Met Ala Phe Ser Gly Ser Gln Ala Pro Tyr
1               5                   10                  15
Leu Ser Pro Ala Val Pro Phe Ser Gly Thr Ile Gln Gly Gly Leu Gln
            20                  25                  30
Asp Gly Leu Gln Ile Thr Val Asn Gly Thr Val Leu Ser Ser Ser Gly
        35                  40                  45
Thr Arg Phe Ala Val Asn Phe Gln Thr Gly Phe Ser Gly Asn Asp Ile
    50                  55                  60
Ala Phe His Phe Asn Pro Arg Phe Glu Asp Gly Gly Tyr Val Val Cys
65                  70                  75                  80
Asn Thr Arg Gln Asn Gly Ser Trp Gly Pro Glu Glu Arg Lys Thr His
                85                  90                  95
Met Pro Phe Gln Lys Gly Met Pro Phe Asp Leu Cys Phe Leu Val Gln
            100                 105                 110

Ser Ser Asp Phe Lys Val Met Val Asn Gly Ile Leu Phe Val Gln Tyr
        115                 120                 125
Phe His Arg Val Pro Phe His Arg Val Asp Thr Ile Ser Val Asn Gly
    130                 135                 140
Ser Val Gln Leu Ser Tyr Ile Ser Phe Gln Pro Pro Gly Val Trp Pro
145                 150                 155                 160
Ala Asn Pro Ala Pro Ile Thr Gln
                165

<210> 200
<211> 132
<212> PRT
<213> Homo sapien

<400> 200
Gly Gln Glu Lys Ser Leu Ala Ala Glu Gly Arg Ala Asp Thr Thr Thr
 1               5                  10                  15
Gly Ser Ile Ala Gly Ala Pro Glu Asp Glu Arg Ser Gln Ser Thr Ala
            20                  25                  30
Pro Gln Ala Pro Glu Cys Phe Asp Pro Ala Gly Pro Ala Gly Leu Val
        35                  40                  45
Arg Pro Thr Ser Gly Leu Ser Gln Gly Pro Gly Lys Glu Thr Leu Glu
    50                  55                  60
Ser Ala Leu Ile Ala Leu Asp Ser Glu Lys Pro Lys Lys Leu Arg Phe
65                  70                  75                  80
His Pro Lys Gln Leu Tyr Phe Ser Ala Arg Gln Gly Glu Leu Gln Lys
                85                  90                  95
Val Leu Leu Met Leu Val Asp Gly Ile Asp Pro Asn Phe Lys Met Glu
            100                 105                 110
His Gln Ser Lys Arg Ser Pro Leu His Ala Ala Ala Glu Ala Gly His
        115                 120                 125
Val Asp Ile Cys
    130

<210> 201
<211> 120
<212> PRT
<213> Homo sapien

<400> 201
Met Leu Val Leu Val Leu Gly Asp Leu His Ile Pro His Arg Cys Asn
 1               5                  10                  15
Ser Leu Pro Ala Lys Phe Lys Lys Leu Leu Val Pro Gly Lys Ile Gln
            20                  25                  30
His Ile Leu Cys Thr Gly Asn Leu Cys Thr Lys Glu Ser Tyr Asp Tyr
        35                  40                  45
Leu Lys Thr Leu Ala Gly Asp Val His Ile Val Arg Gly Asp Phe Asp
    50                  55                  60
Glu Asn Leu Asn Tyr Pro Glu Gln Lys Val Val Thr Val Gly Gln Phe
65                  70                  75                  80
Lys Ile Gly Leu Ile His Gly His Gln Val Ile Pro Trp Gly Asp Met
                85                  90                  95
Ala Ser Leu Ala Leu Leu Gln Arg Gln Phe Asp Val Asp Ile Leu Ile
            100                 105                 110
Ser Gly His Thr His Lys Phe Glu

115                 120

<210> 202
<211> 135
<212> PRT
<213> Homo sapien

<400> 202
Arg Met Cys Ser Leu Thr Phe Tyr Ser Lys Ser Glu Met Gln Ile His
1               5                   10                  15
Ser Lys Ser His Thr Glu Thr Lys Pro His Lys Cys Pro His Cys Ser
            20                  25                  30
Lys Thr Phe Ala Asn Ser Ser Tyr Leu Ala Gln His Ile Arg Ile His
        35                  40                  45
Ser Gly Ala Lys Pro Tyr Ser Cys Asn Phe Cys Glu Lys Ser Phe Arg
    50                  55                  60
Gln Leu Ser His Leu Gln Gln His Thr Arg Ile His Thr Gly Asp Arg
65                  70                  75                  80
Pro Tyr Lys Cys Ala His Pro Gly Cys Glu Lys Ala Phe Thr Gln Leu
                85                  90                  95
Ser Asn Leu Gln Ser His Arg Arg Gln His Asn Lys Asp Lys Pro Phe
            100                 105                 110
Lys Cys His Asn Cys His Arg Ala Tyr Thr Asp Ala Ala Ser Leu Glu
        115                 120                 125
Val His Leu Ser Thr His Thr
    130                 135

<210> 203
<211> 135
<212> PRT
<213> Homo sapien

<400> 203
   Leu Leu Leu Ala Arg Trp His Ser Ala Ala Phe Lys Val Arg Ala Gly
   1               5                   10                  15
   Ala Arg Gln Glu Leu Ala Met Lys Ser Leu Lys Ser Arg Leu Arg Arg
               20                  25                  30
   Gln Asp Val Pro Gly Pro Ala Ser Ser Gly Ala Ala Ala Ala Ser Ala
           35                  40                  45
   His Ala Ala Asp Trp Asn Lys Tyr Asp Asp Arg Leu Met Lys Ala Ala
       50                  55                  60
   Glu Arg Gly Asp Val Glu Lys Val Thr Ser Ile Leu Ala Lys Lys Gly
   65                  70                  75                  80
   Val Asn Pro Gly Lys Leu Asp Val Glu Gly Arg Ser Val Phe His Val
                   85                  90                  95
   Val Thr Ser Lys Gly Asn Leu Glu Cys Leu Asn Ala Ile Leu Ile His
               100                 105                 110
   Gly Val Asp Ile Thr Thr Ser Asp Thr Ala Gly Arg Asn Ala Leu His
           115                 120                 125
   Leu Ala Ala Lys Tyr Gly His
       130                 135

<210> 204
<211> 167
<212> PRT

<213> Homo sapien

<400> 204
Ala Leu Gly Glu Ala Pro Asp His Ser Tyr Glu Ser Leu Arg Val Thr
1               5                   10                  15
Ser Ala Gln Lys His Val Leu His Val Gln Leu Asn Arg Pro Asn Lys
            20                  25                  30
Arg Asn Ala Met Asn Lys Val Phe Trp Arg Glu Met Val Glu Cys Phe
        35                  40                  45
Asn Lys Ile Ser Arg Asp Ala Asp Cys Arg Ala Val Val Ile Ser Gly
    50                  55                  60
Ala Gly Lys Met Phe Thr Ala Gly Ile Asp Leu Met Asp Met Ala Ser
65                  70                  75                  80
Asp Ile Leu Gln Pro Lys Gly Asp Asp Val Ala Arg Ile Ser Trp Tyr
                85                  90                  95
Leu Arg Asp Ile Ile Thr Arg Tyr Gln Glu Thr Phe Asn Val Ile Glu
            100                 105                 110
Arg Cys Pro Lys Pro Val Ile Ala Ala Val His Gly Gly Cys Ile Gly
        115                 120                 125
Gly Gly Val Asp Leu Val Thr Ala Cys Asp Ile Arg Tyr Cys Ala Gln
    130                 135                 140
Asp Ala Phe Phe Gln Val Lys Glu Val Asp Val Gly Leu Ala Ala His
145                 150                 155                 160
Val Gly Thr Leu Gln Arg Leu
                165

<210> 205
<211> 381
<212> DNA
<213> Homo sapien

<400> 205
aaatttggga tcatcgcctg ttctgaaaac tagatgcacc aaccgtatca ttatttgttt      60
gaggaaaaaa agaaatctgc attttaattc atgttggtca aagtcgaatt actatctatt     120
tatcttatat cgtagatctg ataaccctat ctaaaagaaa gtcacacgct aaatgtattc     180
ttacatagtg cttgtatcgt tgcatttgtt ttaatttgtg gaaaagtatt gtatctaact     240
tgtattactt tggtagtttc atctttatgt attattgata tttgtaattt tctcaactat     300
aacaatgtag ttacgctaca acttgcctaa aacattcaaa cttgttttct tttttctgtt     360
gttttctttg ttaattcatt t                                               381

<210> 206
<211> 514
<212> DNA
<213> Homo sapien

<400> 206
aaaagtaaat tgcataaaat tacatccaat ttctttctct aaaccaacat attcttcacc      60
ttcacaaagc aaacacatgg tgcactgaaa ccgaggtgtt accagcttta catactgttc     120
tgccatttgt ggggggtgca accacaacat aagtcagaaa aaaagctatc cagcttttcg     180
tggaatctgg tgaagtttac acttagcgat aagcctctaa gcctgaactt agcagggcta     240
gcaaaacttt atttatttcc taactcctat tattttagaa tggttttcaa aataatactg     300
caagttccta attgaaatac aaaacagaac aaaaagctgt gagaaatctt tttttttctt     360
tggctcctta aagacttgga ataatttata ttagtgttgc atacatttta ccttctacat     420
tttgatgtac ttgctcttga aagcactaga acaaattaat tgaaataaaa cctctctgaa     480
accatttgaa tctttgatcc taccatagag tttt                                 514

<210> 207
<211> 522
<212> DNA
<213> Homo sapien

<220>
<221> misc_feature
<222> (1)...(522)
<223> n = A,T,C or G

<400> 207
caagcttttg gtgcatagca gccngcctgg aagcattctg agtgctctgt ctgccctggt 60
gggtttcatt atcctgtctg tcaaacaggc caccttaaat cctgcctcac tgcagtgtga 120
gttggacaaa aataatatac caacaagaag ttatgtttct tacttttatc atgattcact 180
ttataccacg gactgctata cagccaaagc cagtctggct ggaactctct ctctgatgct 240
gatttgcact ctgctggaat tctgcctagc tgtgctcact gctgtgctgc ggtggaaaca 300
ggcttactct gacttccctg ggagtgtact tttcctgcct cacagttaca ttggtaattc 360
tggcatgtcc tcaaaaatga ctcatgactg tggatatgaa gaactattga cttcttaaga 420
aaaaagggag aaatattaat cagaaagttg attcttatga taatatggaa aagttaacca 480
ttatagaaaa gcaaagcttg agtttcctaa atgtaagctt tt 522

<210> 208
<211> 278
<212> DNA
<213> Homo sapien

<400> 208
   aaaatgcact accccttttt tccaacacgg agcttaaaac aaattaatga aagagtggaa 60
   aattcaaaat aagggcaaga gataaggttt tttttttttt tcctttaaga tagactcagg 120
   ataggtagat agctttcact gatgtagatg tggaataaat tattacttca ggaaaaaaat 180
   tcccaaacat cttatgaaaa agtatacaac tctacttcaa aatatgctat ttactcactg 240
   ccaaagacag ttttatttga aatcttgttt ctgtattt 278

<210> 209
<211> 234
<212> DNA
<213> Homo sapien

<220>
<221> misc_feature
<222> (1)...(234)
<223> n = A,T,C or G

<400> 209
cctcccaaat ttagcaggtg ctgggnagga ccctagggag tggtttatgg gggctagctg 60
gtgaaactgc cctttccttt ctgttctatg agtgtgatgg tgtttgagaa aatgtggggc 120
tatggttcag gcgcacttca catgtgcaaa gatggagaaa gcactcacct acacgtttag 180
gctcagaatg ttgattgaaa cattttgaat gatcaaaaat aaaatgttat tttt 234

<210> 210
<211> 186
<212> DNA
<213> Homo sapien

<220>
<221> misc_feature
<222> (1)...(186)
<223> n = A,T,C or G

<400> 210
aaaataactg atggcaaaat aaaanattta catcacatca tactgtgtaa acatgtaagg 60
tctctgtaca aagaaatata catgcaaaat aatgtaaaaa tttaactgaa ataataaaag 120
aaacaataca caaataaaaa ttatgaggtt acgaatacac atccagtttc gaatccaatt 180
tctttt 186

<210> 211
<211> 403
<212> DNA
<213> Homo sapien

<400> 211
aaaaattggt aaaatattta agtacaaaat aagtagcttc cagcgaggtt tttataccat 60
agtaagagca cacaatagat attactagca cacatgggtt atctgggagc gctatagcta 120
caataaacct aattatggaa cagaaatttg cattctgttt ccagtgctac tacactccta 180
ctttctcaaa agtctgctct attaatatca gctcagtgca gtttactatg aatagtttat 240
gtctgtgatg caaagcatta attgttctct ttttacaaac atacattttt ttcataagga 300
agactggggg aaaacccaga aacatacaga gaaaaggaaa gcatcatcaa atatatgtta 360
aaaattaaga tgatgtttac tactagtcat cctacaacaa ttt 403

<210> 212
<211> 345
<212> DNA
<213> Homo sapien

<400> 212
cctctttatg agttcattac tgctgttcag tctcggcaca cagacacccc tgtgcaccgg 60
ggtgtacttt ctactctgat cgctgggcct gtggttgaga taagtcacca gctacggaag 120
gtttctgacg tagaagagct tacccctcca gagcatcttt ctgatcttcc accattttca 180
aggtgtttaa taggaataat aataaagtct tcgaatgtgg tcaggtcatt tttggatgaa 240
ttaaaggcat gtgtggcttc taatgatatt gaaggcattg tgtgcctcac ggctgctgtg 300
catattatcc tggttattaa tgcaggtaaa cataaaagct caaaa 345

<210> 213
<211> 318
<212> DNA
<213> Homo sapien

<400> 213
aaaatgtttt attattttga aaataatgtt gtaattcatg ccagggactg acaaaagact 60
tgagacagga tggttattct tgtcagctaa ggtcacattg tgcctttttg accttttctt 120
cctggactat tgaaatcaag cttattggat taagtgatat ttctatagcg attgaaaggg 180
caatagttaa agtaatgagc atgatgagag tttctgttaa tcatgtatta aaactgattt 240
ttagctttac aaatatgtca gtttgcagtt atgcagaatc caaagtaaat gtcctgctag 300
ctagttaagg attgtttt 318

<210> 214
<211> 462
<212> DNA
<213> Homo sapien

<400> 214
aaacacatct ggttctggca gcaagttata ttatgcattt agagcaatag gtgccctgaa 60
agttattgtt gctttttttg tttttttttt cagtttgtgc gtgtcacttg aatcagaaac 120
caaacacatg taaaaaaata tcatcctcaa tgccccccat taactctctc tccagaaggt 180
gacaatgtta gtgaactcaa gactctcact gatgatggta ttttacaatg aaaacacaag 240
gaaacccttt gaggtccaat tttcacatca tattctccaa atagtaaaat agcagctcta 300
catgttgatg aaaagaaatt tcaatttctt cctatttgtt tttactcata tcaacattaa 360
tatgtatctg gatttattaa tttccaaaaa gaaaatttta gttaccaaat atttcagaaa 420
tttaataaag cattatatat atgtaattag cacttatcta cc 462

<210> 215
<211> 280
<212> DNA
<213> Homo sapien

<400> 215
   aaacttttct gaaacgatta gctgtagcca aattatgtgg ttacgttttg ctacattaga 60
   atttgaaaat gcaatatgtg tggtaaatct actgtttgaa atttataatg gtctctgata 120
   tgattcgaat tttggtaact tttgaaagtt attttccccc tttagtcatg gatttctatt 180
   tgttttttaa tgttaatttt tctagaaagc atctgaattg actaggcttt tcctatataa 240
   aaaactcaaa acttgttaac tctgtacttt aataaaattt 280

<210> 216
<211> 210
<212> DNA
<213> Homo sapien

<400> 216
aaaatctctg gcttcaaagt ttcttgggga aaggtcggtt tacctcacat tttttgtttc 60
cattagtaat attctaggta cctcacaaaa tgtattatgg tgccatggct gttagttttt 120
agtgagtgct gtaggattaa ttcgaaaata ggcagaattc cattcctccc aaggtggcaa 180
aaattagcta tactgatgta attgtcattt 210

<210> 217
<211> 398
<212> DNA
<213> Homo sapien

<400> 217
ctggagctgc tagaacttga gatgagggca agagcgatta aagccctaat gaaagctggt 60
gatataaaaa agccagccta ggtatttaac ttgattttga attttaggta tgtttgaaca 120
aagccacatc atttaatttt gtatctaaaa tttatttggg gtcttatatg ttatttctca 180
tgtaaccctt attaggactc attttagccc taaattacct gtggctgttt ctttttattt 240
ttttgactac ttttatatta taaatgtgtg ttactgtctt atgaattcat ggcaatatag 300
ttggatagcc tggatacttt gttagatgag tatttagctg tgtctgcaaa tcttaaaagc 360
cattagcaaa gagtcgtggt atttttttct ttattttt 398

<210> 218
<211> 487
<212> DNA
<213> Homo sapien

<400> 218
ctgccgccgg tcaggctggt taaagatcag gtcccccagg accttgcgat ttatgtcgcc 60

attctccagc aagacctcag tgccgaagac ctctacgatg cgccggtggg cagggtatcc 120
tggctgcacg acgtgccggg ccatcacgtc cacgtcaatc accgcacagc ccagtttcag 180
tgtttttaca cattatattg ttataatctc acaataacta taaattaggt agaacaggaa 240
atgaggtttg gagaagatac ttgacttatc cgaccatctg tacttgtccc atagtaagga 300
gcctcaagca gagacaaagg aggaagttgc ctatgttgta tggtttacag gccataaatg 360
aatgtcatct ttttcctccc ctggggaaaa atgtctcaaa aatcccacca taggacatga 420
catctccaga acctctatta caaaatacac atttcctgta gaggggtaac aaatttgggt 480
taacctg 487

<210> 219
<211> 390
<212> DNA
<213> Homo sapien

<400> 219
aaaaaataca ccacacgata caactcaata caggagtatt tcttctcaaa ttcttctagc 60
accatcaaca ttcttcaagt atctgaaata ctattaatta gcacctttgt attatgaaca 120
aaacaaaaca aggacctcag ttcatctctg tctaggtcag cacctaacaa tgtggatcac 180
actcatggga aagtgttttg aggtagttta aacctttgga agtttgggtt ttaaacttcc 240
ctctgtggaa gatattcaaa agccacaagt ggtgcaaatg tttatggttt ttatttttca 300
atttttattt tggttttctt acaaaggttg acattttcca taacaggtgt aagagtgttg 360
aaaaaaaagt tcaaattttt gggggagcgg 390

<210> 220
<211> 341
<212> DNA
<213> Homo sapien

<220>
<221> misc_feature
<222> (1)...(341)
<223> n = A,T,C or G

<400> 220
aaaacaggca aagttttaca gagaggatac atttaataaa actgcgagga catcaaagtg 60
gtaaatactg tgaaatacct tttctnnnca aaaggcaaat attgaagttg tttatcaact 120
tcgctagaaa aaaaaaaaca cttggcatac aaaatattta agtgaaggag aagtctaacg 180
ctgaactnnn aatgaaggga aattgtttat gtgttatgaa catccaagtc tttcttcttt 240
tttaagttgt caaagaagct tccacaaaat tagaaaggac aacagttctg agctgtaatt 300
tcgccttaaa ctctggacac tctatatgta gtgcattttt a 341

<210> 221
<211> 234
<212> DNA
<213> Homo sapien

<400> 221
ccagggggaa ttgagggagg ctctaagcta ggggcactgc atggtgggac aggatggccc 60
cttgaggact gaaccctggg gagaagacaa acagtaataa taaaaacaaa taacaagtac 120
tttaagaatg gattgtatga cctatagtga cagatgacat cactaatact gaaagcttct 180
tatattaata attttggcaa aatgtcattt tgtaatatag tatatgcttt ccag 234

<210> 222
<211> 186
<212> DNA

<213> Homo sapien

<400> 222
aaattttcat tgagttgtcc atctccagca tatagggctt caggagcaga gcagaccttg 60
tttttagtgg ttccatggga taaaatggga ttggaggagc tagaagaatt cagggtctgg 120
tccaatctgc cagtcttcct gaaatatcga aaatacacca gggctgctat atcagagcca 180
ccctgg 186

<210> 223
<211> 486
<212> DNA
<213> Homo sapien

<400> 223
ccataagcag ataagtagca gttcaactgg atgtctctct tctccaaatg ctacagtaca 60
aagccctaag catgagtgga aaatcgttgc ttcagaaaag acttcaaata acacttactt 120
gtgcctggct gtgctggatg gtatattctg tgtcattttt cttcatggga gaaacagccc 180
acagagctca ccaacaagta ctccaaaact aagtaagagt ttaagctttg agatgcaaca 240
agatgagcta atcgaaaagc ccatgtctcc tatgcagtac gcacgatctg gtctgggaac 300
agcagagatg aatggcaaac tcatagctgc aggtggctat aacagagagg aatgtcttcg 360
aacagtcgaa tgctataatc cacatacaga tcactggtcc tttcttgctc ccatgagaac 420
accaagagcc cgatttcaaa tggctgtact catgggccag ctctatgtgg taggtggatc 480
aaatgg 486

<210> 224
<211> 322
<212> DNA
<213> Homo sapien

<400> 224
aaatgttcac tatgtcattt agtgtccaac tttacggata ggttgactat ctaaataggc 60
atttttagtc attaaaaaaa aatctagtca ccaggaggat ccctataact caaaataact 120
tgtttgtaaa agaaaatttg tttacttacc cattagtaag ttcctgcata ttcattataa 180
gatggcaaat caaacttttc taggatgaag acagcttatt tttaagttgt atagtcttag 240
ttggtttagg gtctcaattt taattaataa aatacttggt ttttatttgc ttgtcctttt 300
gaattcctgt tttaataatt tt 322

<210> 225
<211> 489
<212> DNA
<213> Homo sapien

<400> 225
aaatgtagga ataaaatggc tggcatctaa gcactttagt aaaagaggtt tttacaaata 60
actaaggatt gtagagcttc cttctctttt tttttctttt tctttctttt gttttacatg 120
aactcaactt attcctaaca tttgtctacc tcaaagaaat ttcaagatta tttagataac 180
atggatatgt gccaaatcct ttgagctgtt aagatgataa tttcctgctt tcctcctaca 240
tcttctcctc ccactccctc ctttggtgtg aatattggct tcccaattaa gacctttttt 300
ttttttttcc agtttgtttt agcttattat aggttttgga ggaactttgc cattttgtaa 360
tctttcaaat cattcttcac ccttcctcac atcagcttcc tgcttttccc agtgttttac 420
tgtaaattgt gtagcatatg acaaatcttg agctgacttt cctcttcact gatgtcatct 480
tgagctctt 489

<210> 226
<211> 398

<212> DNA
<213> Homo sapien

<400> 226
caagggccca ccgcagagca cacctatgct atggggagcc ctgctggcag ccccgagagc 60
catgccatgg cctgcaggag ccaggctcct gtgtggatga agtccctctt cctctgtgcc 120
ttgatccctt gggggtgcct ttggtcatct cttctgtcct ttcctgtctc tgaaatagtc 180
atcactcccc ttgactctct ctgttcacgt cttctcagtc tgcagagtta acttctgtaa 240
ggagtttaat ctggggttcc aagaaaacaa gttccttgtt aacatagcac tgactttgca 300
acaatagaaa actaacaaat gagcaacaat ataaagagta gaggtagttc tcattgggtg 360
taacttcaac ccattctgct tgtggttaga atttataa 398

<210> 227
<211> 535
<212> DNA
<213> Homo sapien

<400> 227
ctgctgcata gaaaatatgc taacatacaa cagtcaagtt taagcctgtg catagagaag 60
ataaagcact tatggtaact gcaaatggta acgagtcctt aaggtttgta caacctagta 120
tgggtccata aggaaaaact gtagtagaaa tggttaggac aaacaataaa gtagaaacag 180
gggggaaact tgagaagaga agaaagaagc aagaaaaaaa gactttcaat tgtataaaat 240
tcacaaacca gtaaagtata aagacaccat ggagaaatgg ttaactctgc cccaaacacc 300
caacagcaaa caaaaccaga atgaataagc ctttggcaga caattttaga aatttgaatg 360
ttacatttct caataattca caaacaatat attatatggt atatttatat taaatattgg 420
gaaaccaatg ttgtaaattt gatgcttata atgctttagc caatgagagc acaatgatat 480
caatcaagct aaatgaatgc tggtgttatc acaacagtgc tcatttatga aacaa 535

<210> 228
<211> 301
<212> DNA
<213> Homo sapien

<400> 228
aaacaataaa caccatcaac cttattgact ttattgtccc ttaaattata ttgactgttg 60
tgattccatc aagtttgtac actcttttct ctccctgttt tgcagcaaca aattgcgaag 120
tgcttttgtt tgtttgtttt cgtttggtta aagcttattg ccatgctggt gcggctatgg 180
agactgtctg gaaggcttgg aatggtttat tgcttatggt aaaatttgcc tgatttctta 240
caggcagcgt ttggaaacct tttattatat agttgtttac atacttataa gtctatcatt 300
t 301

<210> 229
<211> 420
<212> DNA
<213> Homo sapien

<400> 229
aaagttgctt tgctggaagt ttttataagg aatctcagat taaaccttta gaagtttaat 60
tgacactagg aagccaaacc aaggctgact tcagactttg tttgtagtac ctgtgggttt 120
attacctatg ggtttatatc ctcaaatacg acattctagt caaagtcttg gtaatataac 180
caatgttttc aaatgtattc tgtcatacaa agagcagatt tttattgaac ttgtgcaata 240
actatattac catacaatat aaatattcat gaatagtttc ccaagtctgg agcgaccaca 300
tagggagaaa atgcaaatgt ctcaattttt gttcacaaaa gtatatttta tcaaattgct 360
gtaagctgtg gatagcttaa aagaaaaaaa gtttcctgaa atctgggaaa caagacattt 420

<210> 230
<211> 419
<212> DNA
<213> Homo sapien

<400> 230
gtgaagtcct aaagcttgca ttccaccagc ttctacaata gccggcttat tactagagca 60
gacagatagc accttcagca ctctgcttgt ggtccacagt agtttttcgt aagtataggt 120
cctcattata tttactaaag cttggggtcc accactagcc agtatgatga gcttgctttc 180
ttggttgcca taagctaaaa tttgaaggca gtctgtcgta atagccaaga atttaacatt 240
tgttttgttg agcaaggcaa ccattttctg cagcccacca gctaaacgca ctgccatttt 300
agctccttct tgatgtaata aaaggttgtg gagagttgta atggcataaa acaacacaga 360
atccactggt gaaccaagca ttttcaccag ggcaggaatg cctccagact taaagatgg 419

<210> 231
<211> 389
<212> DNA
<213> Homo sapien

<400> 231
ttgttcagag ccctggtgga tcttgcaatc cagtgcccta caaaggctag aacactacag 60
gggatgaatt cttcaaatag gagccgatgg atctgtggtc ctttgggact catcaaagcc 120
ttggtttagc attttgtcag ttttatcttc agaaattctc tgcgattaag aagataattt 180
attaaaggtg gtccttccta cctctgtggt gtgtgtcgcg cacacagctt agaagtgcta 240
taaaaaagga aagagctcca aattgaatca cctttataat ttacccattt ctatacaaca 300
ggcagtggaa gcagtttcag agaacttttt gcatgcttat ggttgatcag ttaaaaaaga 360
atgttacagt aacaaataaa gtgcagttt 389

<210> 232
<211> 397
<212> DNA
<213> Homo sapien

<400> 232
ccaggataat atacacaggt ttgcagctaa aactgtgcac agtgggtcat tgatgctagt 60
cacagtggaa ctgaaggaag gctctacagc ccagcttatc ataaacactg agaaaactgt 120
gattggctct gttctgctgc gggaactgaa gcctgtcctg tctcaggggt aacctgctta 180
catctggact ttagaatctg gcacacaaca aaagtgcctg gcatccacta ctgctgcctt 240
tcatttataa taatagccct tccatctggc agtgggggaa gaatacactc ttgacattct 300
tgtctcctgc tttagaatgc tagtgtgtat ctatcatgta tgcaatactt tccccctttt 360
tgctttgcta accaaagagc atatatttta ctgtcag 397

<210> 233
<211> 508
<212> DNA
<213> Homo sapien

<400> 233
cgaggagtcg cttaagtgcg aggacctcaa agtgggacaa tatatttgta aagatccaaa 60
aataaatgac gctacgcaag aaccagttaa ctgtacaaac tacacagctc atgtttcctg 120
ttttccagca cccaacataa cttgtaagga ttccagtggc aatgaaacac attttactgg 180
gaacgaagtt ggttttttca agcccatatc ttgccgaaat gtaaatggct attcctacaa 240
agtggcagtc gcattgtctc tttttcttgg atggttggga gcagatcgat tttaccttgg 300
ataccctgct ttgggtttgt taaagttttg cactgtaggg ttttgtggaa ttgggagcct 360
aattgatttc attcttattt caatgcagat tgttggacct tcagatggaa gtagttacat 420

tatagattac tatggaacca gacttacaag actgagtatt actaatgaaa catttagaaa 480
aacgcaatta tatccataaa tatttttt 508

<210> 234
<211> 358
<212> DNA
<213> Homo sapien

<400> 234
aaatgttggt attcaaaacc aaagatataa ccgaaaggaa aaacagatga gacataaaat 60
gatttgcaag atgggaaata tagtagttta tgaatgtaaa ttaaattcca gttataatag 120
tggctacaca ctctcactac acacacagac cccacagtcc tatatgccac aaacacattt 180
ccataacttg aaaatgagta ttttgcatat ctcagttcag gatatgtttt ttacaagtta 240
atcctaaagt cataaagcaa gaagctattc atagtacaag attttatttg ctaagcttta 300
caaattaaac tctaaaaaat tattacaatg atactgaaag atattttatt ggcctttt 358

<210> 235
<211> 482
<212> DNA
<213> Homo sapien

<400> 235
gaagaaagtt agatttacgc cgatgaatat gatagtgaaa tggattttgg cgtaggtttg 60
gtctagggtg tagcctgaga ataggggaaa tcagtgaatg aagcctccta tgatggcaaa 120
tacagctcct attgatagga catagtggaa gtgagctaca acgtagtacg tgtcgtgtag 180
tacgatgtct agtgatgagt ttgctaatac aatgccagtc aggccaccta cggtgaaaag 240
aaagatgaat cctagggctc agagcactgc agcagatcat ttcatattgc ttccgtggag 300
tgtggcgagt cagctaaata ctttgacgcc ggtggggata gcgatgatta tggtagcgga 360
ggtgaaatat gctcgtgtgt ctacgtctat tcctactgta aatatatggt gtgctcacac 420
gataaaccct aggaagccaa ttgatatcat agctcagacc atacctatgt atccaaatgg 480
tt 482

<210> 236
<211> 149
<212> DNA
<213> Homo sapien

<400> 236
cctcttcatt gttcacatgt cacaggagga ggctctgagc aaaggccact ggcaagttag 60
ggcaacacca agaaggctct gcggagagac tccctgtggg ttggggcctg gcaggaacgg 120
tgcctgtgga ctgtttatgg tctgtccag 149

<210> 237
<211> 391
<212> DNA
<213> Homo sapien

<400> 237
gaagctaaat ccaaagaaat atgaaggtgg ccgtgaatta agtgatttta ttagctatct 60
acaaagagaa gctacaaacc cccctgtaat tcaagaagaa aaacccaaga agaagaagaa 120
ggcacaggag gatctctaaa gcagtagcca aacaccactt tgtaaaagga ctcttccatc 180
agagatggga aaaccattgg ggaggactag gacccatatg ggaattatta cctctcaggg 240
ccgagaggac agaatggata taatctgaat cctgttaaat tttctctaaa ctgtttctta 300
gctgcactgt ttatggaaat accaggacca gtttatgttt gtggttttgg gaaaaattat 360
ttgtgttggg ggaaatgttg tgggggtggg g 391

<210> 238
<211> 374
<212> DNA
<213> Homo sapien

<400> 238
aaaaaacaaa acaatgtaag taaaggatat ttctgaatct taaaattcat cccatgtgtg 60
atcataaact cataaaaata attttaagat gccggaaaag gatactttga ttaaataaaa 120
acactcatgg atatgtaaaa actgtcaaga ttaaaattta atagtttcat ttatttgtta 180
ttttatttgt aagaaatagt gatgaacaaa gatccttttt catactgata cctggttgta 240
tattatttga tgcaacagtt ttctgaaatg atatttcaaa ttgcatcaag aaattaaaat 300
catctatctg agtagtcaaa atacaagtaa aggagagcaa ataaacaaca tttggaaaaa 360
aaaaaaaaaa aaaa 374

<210> 239
<211> 200
<212> DNA
<213> Homo sapien

<400> 239
aaagatgtct ttgaccgcat atgtactgga aatttcaaac gtggatcttc ccaggttgta 60
gtctttgtgt tatgatcaat gaagaagggc cggccgtttg gcgctatcct catttcccag 120
ccgggtggca agaagctctg tgtgactttg tgttgtggtt tgggggagtt gtaaggtgat 180
ggctgtgggg actgtgggtt 200

<210> 240
<211> 314
<212> DNA
<213> Homo sapien

<220>
<221> misc_feature
<222> (1)...(314)
<223> n = A,T.C or G

<400> 240
ctggtaaact gtccaaaaca aggttccaaa taacacctct tactgattta ccctacccat 60
acatatncca natagntttt gatcaaaaac atgaaatana tccacctgct tattttaagc 120
atattaaaaa ggaaactaat tggaccattt tctatttgtc tattttatac aaaaaggcta 180
cacaattgat acactctatt cagataacaa tcaattagag tgantatgaa ttactggcga 240
caccatcact caattcttaa aaattagaaa ttgctgtagc agtattcact ataacttaac 300
actaccgaga gact 314

<210> 241
<211> 375
<212> DNA
<213> Homo sapien

<220>
<221> misc_feature
<222> (1)...(375)
<223> n = A,T,C or G

<400> 241

ccaagtcctt ggagttatag gatattcatt acttcctctc attgtaatag cccctgtact 60
tttggtggtt ggatcatttg aagtggtgtc tacacttata aaactgtttg gtgtgttttg 120
ggctgcctac agtgctgctt cattgttagt gggtgaagaa ttcaagacca aaaagcctct 180
tctgatttat ccaatctttt tattatacat ttatcttttg tcgttatata ctggtgtgtg 240
atccaagtta tacatgaata gaaaaagatg gtgttaaatt tgtgtgtagg ctgggaattc 300
tngctaaagg aatggnaaaa aacctgtnnt tgnaaaattn acntgtccca aagnnaagga 360
anctaaacgc ttttt 375

<210> 242
<211> 387
<212> DNA
<213> Homo sapien

<400> 242
aaaggcattc tctgatttac atgagaattg agaaactgag atgtatgatt tgtctgttag 60
tcaatttcac accctttcat tctcataagc cccaaatttt gctcagttaa ggagcttgct 120
ttaggcccac ctatgtaagt ctgttatact agctaatgtg cccatttgaa tagttcaagg 180
gtcagctaat gctctgagct tcatggctcc agtataaaga acaaatttaa caaaattaag 240
ctgttactgt agccgagtta cccttctgct ccacacatat gtagtgggat cttgcaggat 300
ttccatagtg ccaattatca aaggccttga ctacttagca ttgctgtatt acagatgtgc 360
aaactgaggc actgaaaagt caaattt 387

<210> 243
<211> 536
<212> DNA
<213> Homo sapien

<220>
<221> misc_feature
<222> (1)...(536)
<223> n = A,T,C or G

<400> 243
aaaccaaaag gacgaagaaa aaacactttn aaaaaaaaaa aaaaaaaaga aaaaccaaac 60
catattttgc cacatgtgag agtacggtca agcagtattt acaaaaaggt taacggaaca 120
acactctgac acatgctctg agaatactgg gactgctgtt tcaaaaaaaa aggttcaaac 180
ttattgtcac agcatcatca caaaatagag gatcaccatt ggtttgcttg gcttttcttt 240
ttttttttcc cccaagtgag gacctaactc caaataatac aatagaatat gcaaattatc 300
ttcacatcaa gagtacccca agaaaaacga aatccatggc acanacactg tacaagggtg 360
cagggcaggg ctctgagggg cccaaacccc attttgccaa ctcgattttc tagcattgaa 420
gggagcaagg ggtcaggcat atgatggaga tgatactgaa atgatttatc caaaatccat 480
gcaaatcaag ttctttggat agaggtgaan aacttggaca tggctgtttc aggcag 536

<210> 244
<211> 397
<212> DNA
<213> Homo sapien

<400> 244
ccaggataat atacacaggt ttgcagctaa aactgtgcac agtgggtcat tgatgctagt 60
cacagtggaa ctgaaggaag gctctacagc ccagcttatc ataaacactg agaaaactgt 120
gattggctct gttctgctgc gggaactgaa gcctgtcctg tctcaggggt aacctgctta 180
catctggact ttagaatctg gcacacaaca aaagtgcctg gcatccacta ctgctgcctt 240
tcatttataa taatagccct tccatctggc agtgggggaa gaatacactc ttgacattct 300
tgtctcctgc tttagaatgc tagtgtgtat ctatcatgta tgcaatactt tccccctttt 360

tgctttgcta accaaagagc atatatttta ctgtcag 397

<210> 245
<211> 508
<212> DNA
<213> Homo sapien

<400> 245
   cgaggagtcg cttaagtgcg aggacctcaa agtgggacaa tatatttgta aagatccaaa 60
   aataaatgac gctacgcaag aaccagttaa ctgtacaaac tacacagctc atgtttcctg 120
   ttttccagca cccaacataa cttgtaagga ttccagtggc aatgaaacac attttactgg 180
   gaacgaagtt ggttttttca agcccatatc ttgccgaaat gtaaatggct attcctacaa 240
   agtggcagtc gcattgtctc tttttcttgg atggttggga gcagatcgat tttaccttgg 300
   ataccctgct ttgggtttgt taaagttttg cactgtaggg ttttgtggaa ttgggagcct 360
   aattgatttc attcttattt caatgcagat tgttggacct tcagatggaa gtagttacat 420
   tatagattac tatggaacca gacttacaag actgagtatt actaatgaaa catttagaaa 480
   aacgcaatta tatccataaa tatttttt 508

<210> 246
<211> 358
<212> DNA
<213> Homo sapien

<400> 246
aaatgttggt attcaaaacc aaagatataa ccgaaaggaa aaacagatga gacataaaat 60
gatttgcaag atgggaaata tagtagttta tgaatgtaaa ttaaattcca gttataatag 120
tggctacaca ctctcactac acacacagac cccacagtcc tatatgccac aaacacattt 180
ccataacttg aaaatgagta ttttgcatat ctcagttcag gatatgtttt ttacaagtta 240
atcctaaagt cataaagcaa gaagctattc atagtacaag attttatttg ctaagcttta 300
caaattaaac tctaaaaaat tattacaatg atactgaaag atattttatt ggcctttt 358

<210> 247
<211> 673
<212> DNA
<213> Homo sapien

<220>
<221> misc_feature
<222> (1)...(673)
<223> n = A,T,C or G

<400> 247
gaagaaagtt agatttacgc cgatgaatat gatagtgaaa tggattttgg cgtaggtttg 60
gtctagggtg tagcctgaga ataggggaaa tcagtgaatg aagcctccta tgatggcaaa 120
tacagctcct attgatagga catagtggaa gtgagctaca acgtagtacg tgtcgtgtag 180
tacgatgtct agtgatgagt ttgctaatac aatgccagtc aggccaccta cggtgaaaag 240
aaagatgaat cctagggctc agagcactgc agcagatcat ttcatattgc ttccgtggag 300
tgtggcgagt cagctaaata ctttgacgcc ggtggggata gcgatgatta tggtagcgga 360
ggtgaaatat gctcgtgtgt ctacgtctat tcctactgta aatatatggt gtgctcacac 420
gataaaccct aggaagccaa ttgatatcat agctcagacc atacctatgt atccaaatgg 480
ttcttttttt ccggagtagt aagttacaat atgggagatt attccgaagc ctggtaggat 540
aagaatataa acttcagggt gaccgaaaaa tcagaatagg tgttggtata gaatggggtc 600
tcctnctccg cggggtcnaa gaaggtggtg ttgangttgc cggnctgtta ntagtatagn 660
gatgccanca gct 673

<210> 248
<211> 149
<212> DNA
<213> Homo sapien

<400> 248
cctcttcatt gttcacatgt cacaggagga ggctctgagc aaaggccact ggcaagttag 60
ggcaacacca agaaggctct gcggagagac tccctgtggg ttggggcctg gcaggaacgg 120
tgcctgtgga ctgtttatgg tctgtccag 149

<210> 249
<211> 458
<212> DNA
<213> Homo sapien

<220>
<221> misc_feature
<222> (1)...(458)
<223> n = A,T,C or G

<400> 249
   gaagctaaat ccaaagaaat atgaaggtgg ccgtgaatta agtgatttta ttagctatct 60
   acaaagagaa gctacaaacc cccctgtaat tcaagaagaa aaacccaaga agaagaagaa 120
   ggcacaggag gatctctaaa gcagtagcca aacaccactt tgtaaaggga ctcttccatc 180
   agagatggga aaaccattgg ggaggactag gacccatatg ggaattatta cctctcaggg 240
   ccgagaggac agaatggata taatctgaat cctgttaaat tttctctaaa ctgtttctta 300
   gctgcactgt ttatggaaat accaggacca gtttatgttt gtggttttgg gaaaaattat 360
   ttgtgttggg ggaaatgttg tgggggtggg gttgagttgg gggtattttc taattttttt 420
   tgtacatttg gaacagtgac aataaatgan accccttt 458

<210> 250
<211> 374
<212> DNA
<213> Homo sapien

<400> 250
aaaaaacaaa acaatgtaag taaaggatat ttctgaatct taaaattcat cccatgtgtg 60
atcataaact cataaaaata attttaagat gccggaaaag gatactttga ttaaataaaa 120
acactcatgg atatgtaaaa actgtcaaga ttaaaattta atagtttcat ttatttgtta 180
ttttatttgt aagaaatagt gatgaacaaa gatccttttt catactgata cctggttgta 240
tattatttga tgcaacagtt ttctgaaatg atatttcaaa ttgcatcaag aaattaaaat 300
catctatctg agtagtcaaa atacaagtaa aggagagcaa ataaacaaca tttggaaaaa 360
aaaaaaaaaa aaaa 374

<210> 251
<211> 356
<212> DNA
<213> Homo sapien

<400> 251
   aaagatcttc tctaacaagc tatgggaatt tggcttcata ctctttcttt gcaacagcag 60
   tgttctgggt gataattttg aattgatacc tgttcctttt tctgggtttt gttggctttt 120
   tgaaaaattg tctttcctta tcattggtgg gaggcttggt agcaaagtaa cattttttgg 180
   aaaagaggac agaaaaattg aactacagct tgagaacgta ttcttttttt cctactttgt 240
   tattgcaaat tgaggaatca cttttaactg ttttaggtgt gtgtgtccag agtgagcaag 300
   gattatgttt ttggattgtc aaagaggatg cttagtctta aaataaaaat aaattt 356

<210> 252
<211> 484
<212> DNA
<213> Homo sapien

<400> 252
ctggtaaact gtccaaaaca aggttccaaa taacacctct tactgattta ccctacccat 60
acatatccca aatagttttt gatcaaaaac atgaaataga tccacctgct tattttaagc 120
atattaaaaa ggaaactaat tggaccattt tctatttgtc tattttatac aaaaaggcta 180
cacaattgtt acactttatt cagattacaa ttaattagag tgattatgaa ttagtgttct 240
acaccattac tcaattctta aaaattagaa attgctgtag cagtattcac tataacttaa 300
cactacgaga gacttaaaaa acagttactg caaaaaaaaa aaagagctac ttcaaagcaa 360
gcaaagtcag taccattaca gatattctta aaaaaaaaaa aaaatttaac aagcaaggct 420
agggtttgat aaattccatc ttgtgatcca ttcttgtgca ttcttcactt cttgagtcac 480
tccc 484

<210> 253
<211> 379
<212> DNA
<213> Homo sapien

<400> 253
aaaaagcgct tagacttccc tttccatctg gaacatgtaa aattttgcag caacaggttt 60
tctccaattc cttcagcaag aattcccagc ctacacacaa atttaacacc atctttttct 120
attcatgtat aacttggatc acacaccagt atataacgac aaaagataaa tgtataataa 180
aaagattgga taaatcagaa gaggcttttt ggtcttgaat tcttcaccca ctaacaatga 240
agcagcactg taggcagccc aaaacacacc aaacagtttt ataagtgtag acaccacttc 300
aaatgatcca accaccaaaa gtacaggggc tattacaatg agaggaagta atgaatatcc 360
tataactcca aggacttgg 379

<210> 254
<211> 387
<212> DNA
<213> Homo sapien

<220>
<221> misc_feature
<222> (1)...(387)
<223> n = A,T,C or G

<400> 254
aaatttgact tttcagtgcc tcagtttgca catctgtaat acagcaatgc taagtagtca 60
aggccnttga taattggcac tatggaaatc ctgcaagatc ccactacata tgtgtggagc 120
agaagggtaa ctcggctaca gtaacagctt aattttgtta aatttgttct ttatactgga 180
gccatgaagc tcagagcatt agctgaccct tgaactattc aaatgggcac attagctagt 240
ataacagact tacataggtg ggcctaaagc aagctcctta actgagcaaa atttggggct 300
tatgagaatg aaagggtgtg aaattgacta acagacaaat catacatctc agtttctcaa 360
ttctcatgta aatcagagaa tgccttt 387

<210> 255
<211> 225
<212> DNA
<213> Homo sapien

<220>
<221> misc_feature
<222> (1)...(225)
<223> n = A,T,C or G

<400> 255
aaatgtcttg tttcccagat ttcaggaaan tttttttctt ttaagctatc cacagcttac 60
agcacctttg ataaaatata cttttgtgaa caaaaattga gacatttaca ttttctccct 120
atgtggtcgc tccagacttg ggaaactatt catgaatatt tatattgtat ggtaatatag 180
ttattgcaca agttcaataa aaatctgctc tttgtatgac agaat 225

<210> 256
<211> 544
<212> DNA
<213> Homo sapien

<220>
<221> misc_feature
<222> (1)...(544)
<223> n = A,T,C or G

<400> 256
ccttgcttaa agcccagaag tggtttaggc ntttggaaaa tctggttcac atcataaaga 60
acttgatttg aaatgttttc tatagaaaca agtgctaagt gtaccgtatt atacttgatg 120
ttggtcattt ctcagtccta tttctcagtt ctattatttt agaacctagt cagttcttta 180
agattataac tggtcctaca ttaaaataat gcttctcgat gtcagatttt acctgtttgc 240
tgctgagaac atctctgcct aatttaccaa agccagacct tcagttcaac atgcttcctt 300
agcttttcat agttgtctga catttccatg aaaacaaagg aaccaacttt gttttaacca 360
aactttgttt ggttacagtt ttcaggggag cgtttcttcc atgacacaca gcaacatccc 420
aaagaaataa acaagtgtga caaanaaaaa aacaaaccta aatgctactg ttccaaagag 480
caacttgatg gtttttttta atactgagtg caaaaggnca cccaaattcc tatgatgaaa 540
tttt 544

<210> 257
<211> 420
<212> DNA
<213> Homo sapien

<400> 257
aaatgtcttg tttcccagat ttcaggaaac tttttttctt ttaagctatc cacagcttac 60
agcaatttga taaaatatac ttttgtgaac aaaaattgag acatttacat tttctcccta 120
tgtggtcgct ccagacttgg gaaactattc atgaatattt atattgtatg gtaatatagt 180
tattgcacaa gttcaataaa aatctgctct ttgtatgaca gaatacattt gaaaacattg 240
gttatattac caagactttg actagaatgt cgtatttgag gatataaacc cataggtaat 300
aaacccacag gtactacaaa caaagtctga agtcagcctt ggtttggctt cctagtgtca 360
attaaacttc taaaagttta atctgagatt ccttataaaa acttccagca aagcaacttt 420

<210> 258
<211> 736
<212> DNA
<213> Homo sapien

<400> 258
aaacaaaatg ctaaacctaa aaacattgtt ctgtcagttc ccaaattaaa tctacttaga 60

acaaaaacaa aaatttatag ctcggtcaca tactacttaa ataatattgt tcaggcatct 120
ctaaaatcct ccatgttttc aagtatggaa atagaactca aatattccac aatacagtac 180
taaacagatg gagtatttag gaaagacttt gttgtcatat ggcacaatat taatattttg 240
ttgcttcaat acgttttgaa ataaatatca gatttttgtt tttttttcct aaaagaccaa 300
aattataatc tacattaaga taattctgac tgtggttaag acttaagagt gtaaaataca 360
acatcaatat tttatcacaa aagtaaagct ggtaacaaat tataaaagga gccagtactc 420
tactgagaca ggctcggaga ttaaagctca tcatgataga aatagtcatc atggagctgt 480
ctgccataat ctgtggcttc actggtgaga aacaagtccg ggttttccag aatctcttct 540
tcagagagct ttttgtcacc attcaaatcc atttcatcaa ttagatgaag cgcctcctct 600
tgtgcaatgc cctgattatt aggtctaccc aaggtaacag ctcttgggga tcaagcctgc 660
catcgttatc tttgtcataa tcattcaccg aatctgtctt tctcacaagt atcccattct 720
ggatcttcat ttgcag 736

<210> 259
<211> 437
<212> DNA
<213> Homo sapien

<220>
<221> misc_feature
<222> (1)...(437)
<223> n = A,T,C or G

<400> 259
aaaaccatac tgaaatcatt taccaaataa cnaagatctt aatctaaaag atagtgaata 60
catcatcatc atgaaatctg gttttatgtg ctctatgaag tacttggaga attgcttttt 120
tatttttctt ttgctttatt aggtcacaca aaacagaatg aattagcaga aaaatgtatg 180
ttataaaaca gcatttacta cttcaattta atttttttta ctaacaattg tggacctttt 240
tgatgacact tatgtatgtt tttaataaat tatgtactta ttagtactta atgagccctt 300
cctgcctcaa tataaaatta ctaaacttgg agaattacag attttattgt aggccctgat 360
gttagtcact ttggagaagc taaaaatttg gaaatgatgt aattcccact gtaatagcat 420
agggattttg gaagcag 437

<210> 260
<211> 592
<212> DNA
<213> Homo sapien

<400> 260
tttttttttt gaaaaatata aaattttaat aaaggctaca tctcttaatt acaataatta 60
ttgtaccaag taattttcct taaatgaact ctttataatg cataatttac agtataagta 120
gaacaaaatg tcatgacaaa agtcattgag tacaagactt gtaataaaaa ggcataaaat 180
atatttatac ataaacccct ttcaaaaaac aagggaaagc ttgagccctc aatatagggc 240
gacacacgga gcgggtgacc gtgcaggtac aggtactgta ctgatttaaa gtcaagcact 300
agagatagtg gattaatact cttttgccgt acactatata cagatgtata gtacaagtaa 360
caatggcaaa cagaatgtac agattaactt aacacaaaaa cccgaacatc aaaatgaagg 420
tgtgtggagg aaaggtgctg ctgggtctcc ctacaactgt tcatttcttt gtggggcagg 480
gggtagttcc tgaatggctg tggtccaatg actaatgtaa aacaaaaaca gaaacaaaaa 540
aaacaaggaa ctgtcatttc cacgaaagca cagcggcagt gattctagca gg 592

<210> 261
<211> 450
<212> DNA
<213> Homo sapien

<400> 261
gtggcagggc ccagccccga accagacaag ggacccctca aggagcttca ttctagcatg 60
agaaaattga gaagtaaacc agaaagttac agaatgtctg aaggggacag tgtgggagaa 120
tccgtccatg ggaaaccttc ggtggtgtac agatttttca caagacttgg acagatttat 180
cagtcctggc tagacaagtc cacaccctac acggctgtgc gatgggtcgt gacactgggc 240
ctgagctttg tctacatgat tcgagtttac ctgctgcagg gttggtacat tgtgacctat 300
gccttgggga tctaccatct aaatcttttc atagcttttc tttctcccaa agtggatcct 360
tccttaatgg aagactcaga tgacggtcct tcgctaccca ccaaacagaa cgaggaattc 420
cgccccttca ttcgaaggct cccagagttt 450

<210> 262
<211> 239
<212> DNA
<213> Homo sapien

<220>
<221> misc_feature
<222> (1)...(239)
<223> n = A,T,C or G

<400> 262
taactttgat gacaaaatct aaaattaaag anttagtctt aaaagcctat agtgacttgt 60
ttacttgcat aaataatatt ttcacttagt acaggctatt aatataagta atgagaattt 120
aagtattaac tcaaaaaaag atagaggctc caaacttttc taagaaatta atgcattttc 180
aaagtaataa tataatcaat ctgtaagtca aaagtaattt catattcatt gccaaattt 239

<210> 263
<211> 376
<212> DNA
<213> Homo sapien

<220>
<221> misc_feature
<222> (1)...(376)
<223> n = A,T,C or G

<400> 263
aaaaaaaaaa aaaaaaaatt ccttgtngtt tnttagagga aaaaaagaaa aaccccaact 60
tttancactg atactacata ttgctctgtt aaagaatttt ctctgccaaa aaaaagaaaa 120
aacaaaaaaa cgcttaaagc tggagtttga cattctgctt tcagatgctg tctttttatt 180
agtgagtgat gatggtttgc taataatcaa taggtaataa ttttttgtaa tcccatcaag 240
tggctccata tgtttctgct ctctcgtgac tgtgttaatg tttaactgtt gtaccttaaa 300
gccgaaatca gtaactatgc atactgtaac caaggtattg ggcttacaga gttgtttgtt 360
gnataaagaa aatttt 376

<210> 264
<211> 207
<212> DNA
<213> Homo sapien

<400> 264
aaattagcat tccacaaata tacaggtaat ttaataatta ttgtgcatga atacatacac 60
aatgcttata tatacaaatt ccagtttgtt ttcatgtgct ggcaagggat ttgtatacaa 120
tcataagctg tgttcatatt ggtcccattg aatattcaca atacaaaagc acaaaagaac 180
cattgattta caaaaggaaa tctattt 207

<210> 265
<211> 388
<212> DNA
<213> Homo sapien

<220>
<221> misc_feature
<222> (1)...(388)
<223> n = A,T,C or G

<400> 265
naactgcact ttatttgtta ctgtaacatt nttttttaac tgatcaacca taagcatgca 60
aaagnccnct gaaactgctt ccactgcctg ttgtatagaa atgggtaaat tataaaggtg 120
attcaatttg gagctccttc cttttttata gcacttctaa gctgtgtgcg cgacacacac 180
cacagaggta ggaaggacca cctttaataa attatcttct taatcgcaga gaatttctga 240
agataaaact gacaaaatgc taaaccaagg ctttgatgag tcccaaagga ccacagatcc 300
atcggctcct atttgaagaa ttcatcccct gtagtgttct agcctttgta gggcactgga 360
ttacaagatc caccagggct ctgaacaa 388

<210> 266
<211> 616
<212> DNA
<213> Homo sapien

<220>
<221> misc_feature
<222> (1)...(616)
<223> n = A,T,C or G

<400> 266
aaatacagag tcaaaagatg atttataaaa tntaaaacat tttctgcttg gccgtatttg 60
aagacaagct gaatacatat ctatgttctg aataagtcca ctatggatat atataggaag 120
agatatacat atatccatcc acagatacac acacacatat atatttctgc atgtatatat 180
acataattct ttctatagtt acaggaaata cttcttctat aattctgatt ttgactccca 240
tcctccacca tttactcatc cactcattac ctaaatcttg gctttctttc ctatattgta 300
aataatccat ccaaacttct agccagtact gtcaggaggg ttcttgctcg agtgagctgt 360
taatactatt ttccactgac aacttctgca catcgaggac acagtgtatc tgaagactcc 420
gctgtatact tccaacaacg ggggcatttt tctttcgtag tcggcatgac aattacttta 480
taggaagact cttcacgaat atcaccacct tctaagttga tgaggaattt ccctttaagc 540
tcgattacat ctgcagtcat ctctcgtggt tcctgaccag taaagttgac tcagaagcca 600
tcattaattc attcaa 616

<210> 267
<211> 341
<212> DNA
<213> Homo sapien

<400> 267
ccattatgta tgtattttct tgaaaaatac ttatttcagc tacttatttt taatagttac 60
ttattcttgt tgtattgtca tttgagtttt gtatatattt ttgatattaa ccccttgtca 120
catgtataat ttgcaaatat tttctccctt tttttagttg tcacattctg ttcattgtat 180
cagattctgt gcagcagctt tttaatttga agtgatctga ctgacttgtt cttccttttg 240
tgtcctggga tatttaggtt aaatcaaaaa acttgctgcc cagaccaatg ttatggggct 300
ttcactctat tttttggtag tagtagttta agagttttag g 341

<210> 268
<211> 367
<212> DNA
<213> Homo sapien

<220>
<221> misc_feature
<222> (1)...(367)
<223> n = A,T,C or G

<400> 268
ttgtagattg gaatagcaaa agtgaatgct ntgaccaaaa tttttgccct cctaaataaa 60
gacgtntcct tctagagagc aaatctatca taaaatgtca aaactagaag agaataaaat 120
gaaaggaaaa aacctagaaa aatatcctaa aatatcaaat gcagtcattt ctaaatataa 180
gccataatta tagctttacc tattgttctt attgttccta tgctgcttct acaatgttac 240
atcaactata cttagcttta ctctcccaaa atcttggtga tgaagccttc tgagtgtgct 300
ttccaatgtg ccagaaccag aagggcattc caaggcttcc ccacatttcc tccatttacg 360
gagacag 367

<210> 269
<211> 270
<212> DNA
<213> Homo sapien

<220>
<221> misc_feature
<222> (1)...(270)
<223> n = A,T,C or G

<400> 269
caaatctctc cctcactaga cgtaagccnt ttnctcactc tctcaatctt atgcatcata 60
gnaangcngn tgaggtggat taaaccaaac ccagctacgc aaaatcttag catactcctc 120
aattacccac ataggatgaa taatagcagt tctaccgtac aaccctaaca taaccattct 180
taatttaact atttatatta tcctaactac taccgcatcc ctactactca acttaaactc 240
cagcaccacg accctactac tatntcgcac 270

<210> 270
<211> 368
<212> DNA
<213> Homo sapien

<220>
<221> misc_feature
<222> (1)...(368)
<223> n = A,T,C or G

<400> 270
ctgaatcatg aataacacta tataatagag tntaaggaac acaagcatta gatgtgatcc 60
ttgccccata cccttagatt atgtcagact aaagctgaca attctgccag gctctgaacc 120
cctagtgccc ccaacccaaa tcttggaagc aaagaatatg ccctgtcata caactttgta 180
caagttgtag taaaacaaag cttaagtttt ctcatctttc tacagcaaat ggtcagttat 240
ttaataaaca ctaaaatgct cctaagaatc cattttgagt ttgtttacca aacacattgt 300
gcaagaactg actacacaaa aagttccttt gaaatttggt ccacaaattc acttaaggtt 360
ggaaattt 368

<210> 271
<211> 313
<212> DNA
<213> Homo sapien

<220>
<221> misc_feature
<222> (1)...(313)
<223> n = A,T,C or G

<400> 271
   aaatttatat aaaactctgt acatgttcac tttattattg cataaacagc ataatcttca 60
   agacaanngt ttgcaaacac atgtccaatt caggaaaaaa aatttcacgt ttctcgtctg 120
   gctttttctt tctttttatt ttgtttggga gattcccagc tagtttcaga cttggtctgt 180
   gaaggaggca cactattttg cttggtattt gacttggatt tatctgtctc ttgtagtatt 240
   ggcggcactt gggaagagct cttgtcagaa tcactttttg ataagattac agatggctcg 300
   gtagaagtag cag 313

<210> 272
<211> 462
<212> DNA
<213> Homo sapien

<220>
<221> misc_feature
<222> (1)...(462)
<223> n = A,T,C or G

<400> 272
aaaaaacatt tattttaata agactattgc naacacatta aaaaaactaa atagtaatat 60
tacaaaatct atatacttgc acatttagta tttgtcaatg tgccagaggt tttcttcatg 120
aaatttgact tctttgaagt gaaggctttt ttctatcatc tcttatagct ctgactgaat 180
aagtcttaat gctttcttca tgttttctat caataggggt aaatcccgag gctcatatgt 240
gtacaatctg ttagagtatc ttccagctat gtcagctcta actgttaaag aagggtctac 300
aaacatgatt ctaggcacat attgcccatc aggtgataaa ttcttatcag tggtttcatg 360
cataaggttt agcatgatga acttattctg agccatttct tgtatttctt cattttgggc 420
aaatactttc tttagtgctt gagagtattg acaatcctcc ag 462

<210> 273
<211> 282
<212> DNA
<213> Homo sapien

<220>
<221> misc_feature
<222> (1)...(282)
<223> n = A,T,C or G

<400> 273
ctgatcaaag catgggatat tttaatagtn ttatacataa tatttttaca tagaaaactt 60
tacatnncat ttcatattat ataattctgc ttattctttc aaaaatttat acatccattg 120
ggcaaggaat ggttttcatt aaattaccaa tattaaatgc acttaatcat tgtgtatagg 180
ttaaaccaaa gtaactatta actaactttt aggcatttta aggaggtaaa acatacattt 240
tacacataag tatttgatgc aaatatgcag ataaaatttt tt 282

<210> 274
<211> 125
<212> DNA
<213> Homo sapien

<220>
<221> misc_feature
<222> (1)...(125)
<223> n = A,T,C or G

<400> 274
cagccctaga cctcaactac ctaaccaacn ttncttaaaa taaaatcccc actatgcaca 60
ttnaatcnct ccaacatact cggattctac cctagcatca cacaccgcac aatcccctat 120
ctagg 125

<210> 275
<211> 528
<212> DNA
<213> Homo sapien

<220>
<221> misc_feature
<222> (1)...(528)
<223> n = A,T,C or G

<400> 275
aaagctgtgg aaaagcttta ttatagattt ttntacagaa ttaaaaaagt tcaaacaata 60
ataagccngg aaccacaaat aattaaaagg aaacacagca atcccataaa caagcattct 120
ggcatctgtt agaaattttc cctcaaatta tgaaatgtag ctctccatgc tttccaatga 180
ttgttataat acccacaaat atctgtgatt tcagtggaat actttaacaa aagttttctt 240
tttaaggcat gatcctgatt cattttttct tcaatatctc agtcatttca ggaactacct 300
taaataaatc tgcaactatt ccataatctg ccacttggaa aattggagct tctgggtctt 360
tattaattgc cacaattgtc ttgctgtctt tcatcccagc taaatgttgg atggctccag 420
atattccaac agcaatataa agttctggtg ctactatttt tcccgtctgn ccaacttgca 480
tgtcattggg aacaaagcca gcatcaacag cagcacggga agcaccaa 528

<210> 276
<211> 420
<212> DNA
<213> Homo sapien

<220>
<221> misc_feature
<222> (1)...(420)
<223> n = A,T,C or G

<400> 276
aaatgtcttg tttcccagat ttcaggaaan tttttttctt ttaagctatc cacagcttac 60
agaaacctga taaaatatac ttttgtgaac aaaaattgag acatttacat tttctcccta 120
tgtggtcgct ccagacttgg gaaactattc atgaatattt atattgtatg gtaatatagt 180
tattgcacaa gttcaataaa aatctgctct ttgtatgaca gaatacattt gaaaacattg 240
gttatattac caagactttg actagaatgt cgtatttgag gatataaacc cataggtaat 300
aaacccacag gtactacaaa caaagtctga agtcagcctt ggtttggctt cctagtgtca 360
attaaacttc taaaagttta atctgagatt ccttataaaa acttccagca aagcaacttt 420

<210> 277
<211> 668
<212> DNA
<213> Homo sapien

<220>
<221> misc_feature
<222> (1)...(668)
<223> n = A,T,C or G

<400> 277
ccagggtggc tctgatatag cagccctggt ntattttcga tatttcagga agactggcag 60
atngcaccag accctgaatt cttctagctc ctccaatccc attttatccc atggaaccac 120
taaaaacaag gtctgctctg ctcctgaagc cctatatgct ggagatggac aactcaatga 180
aaatttaaag ggaaaaccct caggcctgag gtgtgtgcca ctcagagact tcacctaact 240
agagacaggc aaactgcaaa ccatggtgag aaattgacga cttcacacta tggacagctt 300
ttcccaagat gtcaaaacaa gactcctcat catgataagg ctcttacccc cttttaattt 360
gtccttgctt atgcctgcct ctttcgcttg gcaggatgat gctgtcatta gtatttcaca 420
agaagtagct tcagagggta acttaacaga gtatcagatc tatcttgtca atcccaacgt 480
tttacataaa ataagagatc ctttagtgca cccagtgact gacattagca gcatctttaa 540
cacagccgtg tgttcaaatg tacagnggtc cttttcagag ttggacttct agactcacct 600
gttctcactc cctgttttaa ttcaacccag ccatgcaatg ccaaataata gaaattgctc 660
cctaccag 668

<210> 278
<211> 202
<212> DNA
<213> Homo sapien

<220>
<221> misc_feature
<222> (1)...(202)
<223> n = A,T,C or G

<400> 278
   aaattggtat cgacggcaac caggggaagn tnctaaactc ctaatctatt ctggatccaa 60
   ttngcnaagt ggggtcccat caaggttcag tggcagtgga tctgggacag atttcactct 120
   cacgatcagc agtctgcaac ccgaagattt tgcaacttac tactgtcaac agagttacat 180
   gtccccgtac acttttggac cc 202

<210> 279
<211> 694
<212> DNA
<213> Homo sapien

<220>
<221> misc_feature
<222> (1)...(694)
<223> n = A,T,C or G

<400> 279
ctgtacttgg acaaaataag ttaattctat ttggttgtcc attaaagttt tatgtggcta 60
tgnacccact ggagctaaaa attggctttt aactgtttcc aaatcagaac tagcagagga 120
gagaagtaaa taaagccaat ggcactccct tcagaggctc aaaatggtta gattttgatg 180

cagatttaac cttagcgagt ttcagtcagt ccatttagat gatcctgtag gttcatacaa 240
atacactgaa ccgttggttt aacttctctt ccttcctcaa agtttatgat aaagagactc 300
atccctgtat tgggagtgac tgacataagt tcagatctgc tcagagtggc tggtaaggaa 360
cacttaaggt cagtcagaaa ataatcaaac agacttctca tgtaagcacc gtgactcaca 420
actaagacac tggctgctaa tcctggaata ccgctgtctg aattaacttt agagctgtga 480
ttttttccta aaggaaatat ctctgccaaa gaagtttcca gacagntgct tgggagatcc 540
ttggggaaaa ctggtctttt tgatccggtt ctttcangan taggtngaca aaagaaatnc 600
aaaaaagnct atcccacgcn tttntcacct gggcccagcg gnnctcctcc nggggggggn 660
aaacacangg gactcttccc ngggctngct tnng 694

<210> 280
<211> 441
<212> DNA
<213> Homo sapien

<400> 280
aaaaaacttc catgcaactt ctggtttatt gtttggcaac tccacatgat aaaaaaataa 60
aaacagccca accgagtttc ggaattaagt actcttctag taagtgattc aaacttgtaa 120
tatttgccac aggactgact tatttattta ctagctagaa gctcttaagt tcacttgttt 180
atcagggcat atacagaagg gtttgttaaa actcgatgtt aactttacaa ctttctgacc 240
tggtgcatga attctcaagt actgtatttc actgtgttgg tgtgtctgat ggaaatttcg 300
aggtggtccc acaaaaatat tttatgtagt gtgccttcaa agagaaccat ttatttctct 360
tcacttatcg tcccacaaag tcacatttgg tggtggtcag ccaagtcgca tctggtctag 420
ttttactctt gtcccaattt t 441

<210> 281
<211> 398
<212> DNA
<213> Homo sapien

<400> 281
aaatttgtta ggtctgaaga atctaaaact gttaatttaa cccttaactt gtgcctagaa 60
actacagcac atataaaata tgtaaacacc agcctgttgc tgtacttttc tgcttatttt 120
acagcctcaa atatttctca ttatcttgtc acttagttct tcatgtttct ccttctgact 180
tttaataatg gtaataggaa aacaaaaccc aaagcttttc agaacttcag tgtgaggttt 240
cctattttga caagttaact tgtaaatact caggttttac gatgtataat ttacctaata 300
gaccaaacta actcatggag atattttgaa ctattattta ggtacaaact ttataaagaa 360
tgttagtatg tcataaaata taacattaca gcttattt 398

<210> 282
<211> 226
<212> DNA
<213> Homo sapien

<220>
<221> misc_feature
<222> (1)...(226)
<223> n = A,T,C or G

<400> 282
aaaacaatat tctctttttg aaaatagtat naacaggcca tgcatataat gtacagtgta 60
ttacnccaat atgtaaagat tcttcaaggt aacaagggtt tgggttttga aataaacatc 120
tggatcttat agaccgttca tacaatggtt ttagcaagtt catagtaaga caaacaagtc 180
ctatcttttt ttttggctgg ggtgggggcg cccaggccga ggctgg 226

<210> 283
<211> 358
<212> DNA
<213> Homo sapien

<400> 283
aaacaaaaat actcaagatc atttatattt ttttggagag aaaactgtcc taatttagaa 60
tttccctcaa atctgaggga cttttaagaa atgctaacag atttttctgg aggaaattta 120
gacaaaacaa tgtcatttag tagaatattt cagtatttaa gtggaatttc agtatactgt 180
actatccttt ataagtcatt aaaataatgt ttcatcaaat ggttaaatgg accactggtt 240
tcttagagaa atgtttttag gcttaattca ttcaattgtc aagtacactt agtcttaata 300
cactcaggtt tgaacagatt attctgaata ttaaaattta atccattctt aatatttt 358

<210> 284
<211> 288
<212> DNA
<213> Homo sapien

<400> 284
aaaacttttg ttaagaaaaa ctgccagttt gtgcttttga aatgtctgtt ttgacatcat 60
agtctagtaa aattttgaca gtgcatatgt actgttacta aaagctttat atgaaattat 120
taatgtgaag tttttcattt ataattcaag gaaggatttc ctgaaaacat ttcaagggat 180
ttatgtctac atatttgtgt gtgtgtgtgt gtatatatat gtaatatgca tacacagatg 240
catatgtgta tatataatga aatttatgtt gctggtattt tgcatttt 288

<210> 285
<211> 629
<212> DNA
<213> Homo sapien

<220>
<221> misc_feature
<222> (1)...(629)
<223> n = A,T,C or G

<400> 285
cctaaaagca gccaccaatt aacaaagcgt ncannctcaa cacccactac ctaaaaaatc 60
ccaaacatat aactgaactc ctcacaccca attggaccaa tctatcaccc tatanaagaa 120
ctaatgttag tataagtaac atgaaaacat tctcctctgc ataagcctgc gtcagattaa 180
aacactgaac tgacaattaa cagcccaata tctacaatca accaacaagt cattattacc 240
ctcactgtca acccaacaca ggcatgctca taaggaaagg ttaaaaaaag taaaaggaac 300
tcggcaaatc ttaccccgcc tgtttaccaa aaacatcacc tctagcatca ccagtattag 360
aggcaccgcc tgcccagtga cacatgttta acggccgcgg taccctaacc gtgcaaaggt 420
agcataatca cttgntcctt aattagggac ctgtatgaat ggcttcacga gggttcagct 480
gtctcttact tttaaccagt gaaattgacc tgcccgtgaa gaggcnggca tgacacagca 540
agacgagaag accctatgga gctttaattt attaatgcaa acagnaccta acaaacccca 600
caggtcctaa acttacccaa accctggca 629

<210> 286
<211> 485
<212> DNA
<213> Homo sapien

<400> 286
aaatgtactt gctcagctca actgcatttc agttgtatta tagtccagtt cttatcaaca 60

ttaaaaccta tagcaatcat ttcaaatcta ttctgcaaat tgtataagaa taaagttaga 120
attaacaatt ttattttgta caacagtgga attttctgtc atggataatg tgcttgagtc 180
cctataatct atagacatgt gatagcaaaa gaaacaaaca aaagccagga aaacactcat 240
tttcgccttg aatatgtaaa tgggattaat tttgtcctgt gccttatgtg gaaaggaact 300
tctttggttt tccttttttg ttctggtgga agcatgtgca ggagacatat catccaaaca 360
taaaccatta aaatgtttgt ggtttgcttg gctgtaattt tcaaagtagt taattgagga 420
caaagggtaa tgcagaagtg atagctttgg tttgctgagt cttgttttaa gtggccttga 480
tattt 485

<210> 287
<211> 340
<212> DNA
<213> Homo sapien

<400> 287
cctggagtcc aataaccacc ccctcatacc acaccctgtg catacaccag ccaagccttt 60
cctggtctgg gaagggaaga gaaaaaagac gcaggccacc tgggggttct gcagtctttg 120
gtcagtccag ccttctatct tagctgcctt tggcttccgc agtgtaaacc ttgcctgccc 180
ggaggcagga ggcccagctg gacctccgag ggccatgagc aggcagcagc catcttggcc 240
tcaagcttgc ctttcccttg agtccctctc tcccctcggc tctagccaga ggtgtagcct 300
gcagatctag gaagagaaga gctggggagg aggatgaagg 340

<210> 288
<211> 290
<212> DNA
<213> Homo sapien

<400> 288
aaacagtctc tcctcggtgt tctccttgtc aaactgttca tcccagtttc ctctgaaata 60
gacagcattc accagaacca gccttgtcaa tggatccact gagcccggag agagcaactc 120
cgcaatttta ccttctgtct tttcagctac ccaggtgttt atgtgttttc tggacttctc 180
tacggcgctg ataaagtcaa gctcctccat ctctgcttgg tagaattttt ggcaggaatc 240
tctaaaagat gagaggaaat cacaagactt ttccccaaag agcctgttgg 290

<210> 289
<211> 404
<212> DNA
<213> Homo sapien

<400> 289
ccacccacgc ttaggttccc atcacactga tgactccggg tttggcgagc acaggagcgc 60
aaaccttttc acattctttc tgtgatccaa atttgttttc gtttccacca caacctccat 120
accagaatct tgcacagctt ttggtgtttg gatcatagta ccattttaat atgaaatccc 180
tgcaagttcc ttcgtctttc ggcaacttgc atatatctgt ttcagtgaga gccaatggtt 240
ctgtgctcac cattagattg atggttgaac tagaagctga ccttgctggc tgtggaggtg 300
ggggctgaga tttctttgta ctgaaacttc cgtggtaggt ggctctgacc tgagacctca 360
ggtagcagac cacagccaca tggtatgtct gcccagcgag cagg 404

<210> 290
<211> 384
<212> DNA
<213> Homo sapien

<220>
<221> misc_feature

<222> (1)...(384)
<223> n = A,T,C or G

<400> 290
ccaggcgctc cttgtcggca tcagggaggg tggccttgaa ctgctcatgg gctgtggtca 60
gtccctggat ctcctcaatg gtgtgcacaa tgaaggtgtc ctgcaggtcc tccatggccc 120
cctccatcca gttgttgaag ggtgcagccc gcttggcata ctccaagtac agctggtcaa 180
tggtctccag cagtttctcg gtccgctcca gagcttccct tcgcttctga gttagggccc 240
ccagattgtc ccactggtca cagatctttt ggcaacgggc gttgacactg ggtgagtcat 300
aatantccag ctcattgagc tcctgtgcga tggcggcaat ctgctccaca cggtcctggt 360
gggcagccag gccactctcg aagg 384

<210> 291
<211> 278
<212> DNA
<213> Homo sapien

<400> 291
aaagtttatt tttactattt ctttatcact ttattgtatc atcaccattg gtttcataat 60
gtaaatacta tatgttgaac aaattaaatg tcaaaatttt ttattaccat agtccatgtt 120
aatagtgggg ctttcaggtg tttagagatt ttttttgttg ttgttaacat tcattgcaaa 180
agtactagat ggtgtataac tctagagttg aattttaagg gattccctaa tatgtatact 240
atctttttat ctgaagtaat aaataaacaa tgatcttg 278

<210> 292
<211> 177
<212> DNA
<213> Homo sapien

<400> 292
ccttggcccg gtcattcttg tccagtttga taggttcagg aaattcgttg tacagctcca 60
cctccgtttc ctgcttaagt gcattccgtg caatcgtctg gaacgcctgc tccacgttga 120
tggcctcctt ggcactggtc tcaaagtagg gaatgttgtt tttgctgtag caccagg 177

<210> 293
<211> 403
<212> DNA
<213> Homo sapien

<400> 293
aaaaagaagg acttagggtg tcgttttcac atatgacaat gttgcattta tgatgcagtt 60
tcaagtacca aaacgttgaa ttgatgatgc agttttcata tatcgagatg ttcgctcgtg 120
cagtactgtt ggttaaatga caatttatgt ggattttgca tgtaatacac agtgagacac 180
agtaatttta tctaaattac agtgcagttt agttaatcta ttaatactga ctcagtgtct 240
gcctttaaat ataaatgata tgttgaaaac ttaaggaagc aaatgctaca tatatgcaat 300
ataaaatagt aatgtgatgc tgatgctgtt aaccaaaggg cagaataaat aagcaaaatg 360
ccaaaagggg tcttaattga aatgaaaatt taattttgtt ttt 403

<210> 294
<211> 305
<212> DNA
<213> Homo sapien

<220>
<221> misc_feature

<222> (1)...(305)
<223> n = A,T,C or G

<400> 294
aaagcaatct ggcatggtgt cctgtagtga agcagaggat cataacataa gtaaactctc 60
tatgggtgga agttggagag aaggacattt tggctttgta catgaaaaga ctctccagat 120
agaaacagat tctgcccata agtgaaataa aatgctttgt gggggtaatg agtgacttat 180
agtattcagg cagatgttac ataactgcta attaagtttc cctggattga ntttanncaa 240
anaattgaaa gtngattttg gtcangtgtc agnaaactac tgcctataaa cccatatcnt 300
accca 305

<210> 295
<211> 397
<212> DNA
<213> Homo sapien

<220>
<221> misc_feature
<222> (1)...(397)
<223> n = A,T,C or G

<400> 295
   cctatctggt tggccttttt gaagacacca acctgtgtgc tatccatgcc aaacgtgtaa 60
   caattatgcc aaaagacatc cagctagcac gccgcatacg tggagaacgt gcttaagaat 120
   ccactatgat gggaaacatt tcattcccaa aaaaaaaaaa aaaaaaaaat ttctcttctt 180
   cctgttattg gtagttctga acgttagata ttttttttcc atggggtcaa aaggtaccta 240
   agtatatgat tgccgagtgg aaaaataggg gacagaaatc aggtattggc agtttttcca 300
   tttncatttg tgggngaatt tttaatataa atgcggagac gtaaagcatt aatgcnagtt 360
   aaaatgtttc agtgaacaag tttcagcggt tcaactt 397

<210> 296
<211> 447
<212> DNA
<213> Homo sapien

<400> 296
ccatcctcga tgttgaagtt gtcgtggggc ccgaagacgt tggtggggat gacagcggtg 60
aaggtgcagc cgtactgctg gaagtaggcc ctgttctgca cgtcgatcat cctcttggca 120
tacgagtacc caaaattgct gttgtgggga ggcccattgt ggatcatggt ctcatctatc 180
gggtaggtcg tcttgtcagg gaagatacag gtggacaggc aggacaccac cttgcgggcg 240
cccacctcga aggccgagtg caggacgttg tcgttcatgt gcacgttttt cctccagaag 300
tccaaattgt atttgatatt ccggaacagg ccccccacca ttgcagcaag atggatgacg 360
tgtgtgagtt ggaccttctc aaacagggcg cgggtctgtg ctgtatccgt gagatcggcg 420
tctttagagg agacaaacac ccagtcc 447

<210> 297
<211> 681
<212> DNA
<213> Homo sapien

<220>
<221> misc_feature
<222> (1)...(681)
<223> n = A,T,C or G

<400> 297
   aaataacagc atgtaaaata ttaaaataca agctttcaaa aataaataca taaataagta 60
   gaaccctcgt aagaaatagt caaacacatt aagtcctttc cagctgtccc tagaaagctg 120
   ctgttctctt tttcattttc agctctggta agggcaggga ccaccctgca ggaagtgtca 180
   atgatacgct gataagcttc ttacttctct cctgtcagtt ggtgctcccc ctgtgatgag 240
   aaaagggtta ctgttgcagg tgctaaggaa ggctgctctt ctgtcactct gaagttgctt 300
   ggagggatgt ccccatgcag actctctccc agccctccac tcagggaagg tctgtctgta 360
   cccactgcct tctatagcag aaaacttgca ctcctgaatg cttttttttt ttttcaagaa 420
   agaagnggct gnggactcaa ctagattctt ggtttgaaaa agccaaaaca tattggtcac 480
   tgattgtcac attgggttag aaatgtccat tcatgatctc ccttaagctg cacacaaccc 540
   tatgaaataa ctaccattat ctaccctatt ttgctaaagc tcaaagagat taaataatgt 600
   tgacagggat cttagccttg aactcactga aggngttact gcaaagttct gctcttcacc 660
   aagaaggntt acaggccaaa g 681

<210> 298
<211> 353
<212> DNA
<213> Homo sapien

<220>
<221> misc_feature
<222> (1)...(353)
<223> n = A,T,C or G

<400> 298
cctggcttaa gaccagacat ttgaagaagg ctccaggcag ggaaaggaaa ggagaggcca 60
gccccacnct gncccctccc tgcccccacg tctccagcaa cacaaggcgg ccagtggacc 120
gtgaaccatt tatttccaaa ctataaagaa acctgctctc tgagaaaana cactgcccag 180
gngatgaagc tccagcccct ggaggtccaa aacccagtcc aaactcagtc cctttagaaa 240
gctgctgtgc cttggaaatg annntcggnt gtcanagcct gggaagtggt gggaagaacc 300
agcccactcc cctctcctgc tgcgattcca gcgcncgttg ggnccagatc tgg 353

<210> 299
<211> 560
<212> DNA
<213> Homo sapien

<400> 299
aaagttcaag gactaacctt atttatttgg gaaaggggag gaggaaggaa atgatatggt 60
acccagacac tgggctaggc tgcaacttta tctcatttaa tactcccagc tgtcatgtga 120
gaaagaaagc aggctaggca tgtgaaatca ctttcatgga ttattaatgg atttaagagg 180
gcatcaatca gctcaactca agatttcata atcattttta gtatttagat tgtgcctcaa 240
agttgtagta cctcacaata cctccactgg tttcctgttg taaaaacctt cagtgagttt 300
gaccattgtg ctcttggctc ttgggctgga gtaccgtggt gagggagtaa acactagaag 360
tctttagtac aaaactgctc tagggacacc tggtgattcc tacacaagtg atgtttatat 420
ttctcataaa gagtcttccc tatcccaagg tcttcatgat gccagtagcc atatatgata 480
aattatgttc agtgataact tagttatcag aaatcagctc agtggtcttc cccgccatga 540
ttcacatttg atgagttttt 560

<210> 300
<211> 165
<212> DNA
<213> Homo sapien

<220>

<221> misc_feature
<222> (1)...(165)
<223> n = A,T,C or G

<400> 300
aaaaactaca taggggtgtg tgtgtgtgtg tatgtttatt ttatacacac atatttgtat 60
attctaatat attactaagg caattttaat gaattaccat gtatataaaa aaatatctgn 120
cacttggcac acaggtttgt atgtatgtgt atatatatat gtatg 165

<210> 301
<211> 438
<212> DNA
<213> Homo sapien

<400> 301
aaaatatatg tatttaaaaa caaaaagcaa cagtaatcta tgtgtttctg taacaaattg 60
ggatctgtct tggcattaaa ccacatcatg gaccaaatgt gccatactaa tgatgagcat 120
ttagcacaat ttgagactga aatttagtac actatgttct aggtcagtct aacagtttgc 180
ctgctgtatt tatagtaacc attttccttt ggactgttca agcaaaaaag gtaactaact 240
gcttcatctc cttttgcgct tatttggaaa ttttagttat agtgtttaac tggcatggat 300
taatagagtt ggagttttat ttttaagaaa aattcacaag ctaacttcca ctaatccatt 360
atcctttatt ttattgaaat gtataattaa cttaactgaa gaaaaggttc ttcttgggag 420
tatgttgtca taacattt 438

<210> 302
<211> 172
<212> DNA
<213> Homo sapien

<400> 302
ccaaaacagg agtcctgggt gatatcatca tgagacccag ctgtgctcct ggatggtttt 60
accacaagtc caattgctat ggttacttca ggaagctgag gaactggtct gatgccgagc 120
tcgagtgtca gtcttacgga aacggagccc acctggcatc tatcctgagt tt 172

<210> 303
<211> 552
<212> DNA
<213> Homo sapien

<400> 303
   ccagcctgtt gcaggctgct tcgtagcggg cgtcggctgc ggacttccct tcccgggtct 60
   ggatcttttc atcctaccag atgagaaagg gaatgagtga atggagtgac cccgcaccct 120
   gtcactttcc tgagacatga ctgccaggaa gaagagctgc tctggtctcc atcagggctg 180
   gcaggacaaa ctgaccagtg agtcagtagg cagagttcac actgaaaaag ggcacaaggg 240
   ctgtcccaca atgggaggaa atggggtctc agaacttcta cttctctgaa aactaagaca 300
   caattgggac aaccaccacc cccgtgtgag atttctcacc tcgagacagg acaagatgaa 360
   gttcacggct tcttctgggg taaagacctt gaagagccca tcacaggcca acaaaatgaa 420
   cctacaacac cagggagaaa tataaacggg ttttaggccc aaccaaaaaa taaaaaataa 480
   aaaaagggcc tggagatgga gataaaataa atatttgtcc aactattcaa aggctaaggt 540
   ttttttttct tt 552

<210> 304
<211> 601
<212> DNA
<213> Homo sapien

<400> 304
cctttgattc ttggtagtac attgcatgta aaatgtttat aagaagctac ttttccttca 60
tgggaagaaa ttcccacatg agattcataa attcttagac tccgtggctt ctttggtccg 120
gaatgcttaa actcatatga gtgttctgga tcccagtgta tccaatcata attcacatta 180
tcaccttcac gaaccacata ctttgcccac ggtgaaatac gatacaagat ctctccgctt 240
ttactagtaa taactacctt taatttggat ccatgaggca cgagtacaga tttattctgc 300
tttggtggga tatacagctc ccattttcca taatccagtt ttttgtatgg gtacgaaaat 360
ggattccaac cattaaaatc tccagtaaga aaaactcctt ctgctcccgg ggcccattct 420
ttgcagtata aaccaccatc agcacatctg tggacgccaa atgattcata gcctctggaa 480
aacttatcaa taccaccttc attttctcca atgttcttca aaatttggct aaactgctta 540
tacctgcgct ggaagtccac ggcgtagggc ttcaagtacc ggtcgatctc caggagtctg 600
g 601

<210> 305
<211> 401
<212> DNA
<213> Homo sapien

<400> 305
aaataacagc atgtaaaata ttaaaataca agctttcaaa aataaataca taaataagta 60
gaaccctcgt aagaaatagt caaacacatt aagtcctttc cagctgtccc tagaaagctg 120
ctgttctctt tttcattttc agctctggta agggcaggga ccaccctgca ggaagtgtca 180
atgatacgct gataagcttc ttacttctct cctgtcagtt ggtgctcccc ctgtgatgag 240
aaaagggtta ctgttgcagg tgctaaggaa ggctgctctt ctgtcactct gaagttgctt 300
ggagggatgt ccccatgcag actctctccc agccctccac tcagggaagg tctgtctgta 360
cccactgcct tctatagcag aaaacttgca ctcctgaatg c 401

<210> 306
<211> 313
<212> DNA
<213> Homo sapien

<400> 306
aaactgacta tggattcctt gaaggtctgg cagttgttga tgatggcgat catgtactga 60
acgtagcagt gagggtgctg ccgattcctc aggtgctctt ctttatacag ctgcgcttca 120
tctttatatc tgaggacaga caggcttcgg tcagacagca ctaagggcaa catggagctg 180
tttcaaatgc cacgctgacg tcacgcctgg cctgaaattt cacatcacta acatctgacc 240
ggatgagcct ctaaaaataa aacaatcttt agacgatcca gactaatgga aggacagaga 300
ggttgattac ttt 313

<210> 307
<211> 366
<212> DNA
<213> Homo sapien

<220>
<221> misc_feature
<222> (1)...(366)
<223> n = A,T,C or G

<400> 307
aaagatgctg ntaatgaaca ttacggacaa ttcatggtgt ggctagttgg taacacttca 60
gctgattttt cttatgagat ggaaaaaaaa aatcagccaa gtaagggcac atcttcactt 120
catttataag tcagcatcca aggtaaaaga attctctgtt ggacttgaca tcactcccat 180

cctctgatac tcgcctactc tcttctcaaa gaagttagnt ctttccttcc antgaaatat 240
tctcataaaa gtcaaatggg ttctctactc tgaaaacctt gctaaaaccc aattccagca 300
taagtttgtc tgncacaaac ncaatgnatt gcttcattaa antgcaattc atcccaatga 360
gcttcc 366

<210> 308
<211> 534
<212> DNA
<213> Homo sapien

<220>
<221> misc_feature
<222> (1)...(534)
<223> n = A,T,C or G

<400> 308
ccagctatca gctgatcgtc ttctgtctgg acgctcgtcc tgcttctgac atcaaaatct 60
tctgtctcaa agtcagagtc atccaactcc tcaggggtcc ttatcatcag cactgctttc 120
ctgatgtccc ggatgccatc atataccagg cgggaagcat cgataaactc attctcatcc 180
atgggctggg cagggtccga gctgagggct tccacggctg cttctacttg ctcagtaaaa 240
cgtggcatga ctgtgttgga gagcagctta gtggcttcca gaaccttctc tgtgtagact 300
cctggctcat agtcgtccat ctctgaggtg actacgtgaa tgacccgggc tgcccggcct 360
cgaattgcac cagctgtgcg gccaggccat ccacatcctt ctcttggaga gcaatgacac 420
atttggtcac atcttccaaa atgtgattct ctgagacagc caagaagtca tcaatggaag 480
taatgncatc gacagcatct gtgagaacac cgacttgttt ttccattgnt cttt 534

<210> 309
<211> 164
<212> DNA
<213> Homo sapien

<400> 309
catactcctt acactattcc tcatcaccca actaaaaata ttaaacacaa actaccacct 60
acctccctca ccaaagccca taaaaataaa aaattataac aaaccctgag aaccaaaatg 120
aacgaaaatc tgttcgcttc attcattgcc cccacaatcc tagg 164

<210> 310
<211> 131
<212> DNA
<213> Homo sapien

<400> 310
aaaaatcatt tatctttcgg tgcttcaaca tgatgccaaa caaaaatcta ctgaataaaa 60
atagcaagga agggaatcaa acatttataa gatatattta ttatttttct gaccaaagtg 120
caatgatttt t 131

<210> 311
<211> 626
<212> DNA
<213> Homo sapien

<400> 311
cctatgtgcg ccagtttcag gtcatcgaca accagaacct cctcttcgag ctctcctaca 60
agctggaggc aaacagtcag tgagagtgga ggctccagtc agacccgcca gatccttggg 120
cacctggcac tcaagcactt tgcacgatgt ctcaaccaac atctgacatc tttcccgtgg 180

agcaacttcc tgctccacgg gaaagaggtc gatggattta cccctggacc cataagtctg 240
ttcatcctgc tgaagtcccc tccccattgc tccttcaagc caaaactaca ctttgctggt 300
tcctgtcccc tctgagaaag gggatagaaa gctccttcct ctatgtcctc ccatcgagat 360
ctgttctggg gatggagctt ccaacttcct cttgcagcag gaaagaatgc tgctcaccct 420
tctgtcttgc agagtgggat tgtgggaggg attggcagcc ttcttctcca ccacctgtcc 480
agcttcctcc tggtcagggc tgggaccccc aggaatatta tgttgccgtg tgtgtgtgtg 540
tgtgtgtgtg tcttctttta gggagcagga gtgcatctgg taattgaggg tagatgttgt 600
gtgtgctggg gaggggtcct tctgtt 626

<210> 312
<211> 616
<212> DNA
<213> Homo sapien

<400> 312
aaaccaaaga aattaagaaa aaagacttca ttgcttgaat gacgcgaaca gctgtctgag 60
tcacctagac tttaacacca cctggggccc tgggaatgac gctgacgaga gatctgcaca 120
tagtaggcgt gggctccaaa tgtgctcatc agctgacttc acatcctcac aagtcagcct 180
cagatatgac ccaagggata cgtaccatct cttcttgaaa cagcgtgtca aattatatat 240
atgtatgcaa aaaagagtaa tgtactaagc aaaccaagtt tcgtcttttt cttctgaatc 300
tggttttaat gtgacctgtc atccccatct ttcgaattta tgagctccat cttctctaga 360
ctgttaactt cttgaggaaa acatgctatt ttaccacctt tcactgctga atccctagcc 420
cttaagcaca gtctctggca cagaataaat acgaaatgaa tgagtgaatg aatggatgga 480
tgggtgaaga gaaaaggcaa tgcacaagat ttacctatca aaatccacca atggtcctta 540
aaaatggttt tgtcagtaga gatgctgaat atattcatat aatacattta tttcaatact 600
attaagaatt ctagtg 616

<210> 313
<211> 553
<212> DNA
<213> Homo sapien

<400> 313
aaaaaatggc agcattgtac ttgaatcaga aagcttactg ggatttcctc atcgaaagta 60
gagattgcag ctaatcctag taccttttgt tagtaattac ttaaggcaca gtgcaaagtt 120
gaaggactgt tttggtacaa actcaagcca gctacatgta tgcttgcctt ggtatccttg 180
ctagagcaca tgcgggtata ataccgtatt atacacaaca aggccaccct gttgtatctg 240
tgttacaatt aaacatcagt cccagaaagt gaaccctagt catttattat aggtgcccac 300
ctctgacttg gaacaaaatg ccactccatt catgttcatt tttgtcctgg agaggattta 360
tttcctaaaa gattctgaaa gccaacaaat caatgtagtt cttcatagag aacttaagag 420
taaggctcaa aatggcctca aaatgggctt cttggatgac ttccaacagt gactggcctt 480
ctcaacactg cagatgtctg agcactacca taacctaacg aagtgaggaa ggaggaggca 540
aattggtatt ttt 553

<210> 314
<211> 330
<212> DNA
<213> Homo sapien

<400> 314
ccagcgactc cagcggtggc agcaggcagt gcacgtactc tgggcctccc accagggtag 60
tgaaggttcc cagctgttct gccagggcca ggaggacctc atcttcatca tagatggtat 120
ctgtaaggaa aggcagaagc tcacttcggg tcctttcaac cccaagggcc aaggcgatgg 180
tggacagctt cttgatgctg ttgaggcgaa gctgaacgtc ctcattgcgg agttcgtcta 240
tgagcaccgc gatggggtac agcgagtcgt cgccgtcggc cgccgccatc ttggctccgt 300

ccctttcctg tcagactgcg gccagcgctg                                    330

<210> 315
<211> 380
<212> DNA
<213> Homo sapien

<400> 315
aaaaatgaca ttgcgtttag cttattgtaa gaggttgaac ttttgtattt tgtaactatc      60
tttaagccct tcagtttata attcatataa aatgcctttt gtatttaaaa taatcctatt     120
ttaatcagtg catgaaattt gcttttttaa agttcatttg aatgattatt ccttccctct     180
aaagaaatga ttttggtaat gttgagaggt accttaccac aaatcctaac tgtaagtgta     240
ttcatggtta ttttcaaaag aattatgact cttccccaaa agaatcctaa aaaacttgta     300
ataaacctat aaagctgatt tgcatattta caaaattttg aatagcaaat ataggcaact     360
catatatgta tataattttt                                                 380

<210> 316
<211> 222
<212> DNA
<213> Homo sapien

<400> 316
aaactacaga gggttttcca gctattattt cctttagttt ctaaaagtaa cgacttatat      60
taatgtttta taaaagatag tgatgaaaaa aaggtaatgc tgaaataaag gcgcttttag     120
aaatatttaa ggacaacata aggtattaat attggaaaaa aactgtacat attttcaagc     180
acaacactga aatattgcag cagtgtttaa ctgaattgtt tt                        222

<210> 317
<211> 490
<212> DNA
<213> Homo sapien

<400> 317
ccttgaatga gcgtggagag cgattaggcc gagcagagga gaagacagaa gacctgaaga      60
acagcgccca gcagtttgca gaaactgcgc acaagcttgc catgaagcac aaatgttgag     120
aaactgccta tcctggtgac tcttcttaag agaaactgaa gagtttgttc agcagttttt     180
acaagaattc gggacctccg cttgcttctt tttttccaat atttggacac ttagagtggt     240
ttttgttttt tcttttcaga tgttaatgtg aaagaaaggg tgttgcattt ttacatttcc     300
ctaatgatct tgctaataaa tgctacaata gcatcggctt cattttgggt ttttgcctcc     360
tcccactgtg tgtatgtgtg tatatgtatg ttttgaatat gttttcttta ttaaaaaata     420
ttttttgtag tttgaatatg aaatttggac caaatgataa actgcgctga gtctaaactg     480
gcaacatgta                                                            490

<210> 318
<211> 340
<212> DNA
<213> Homo sapien

<400> 318
cctggagtcc aataaccacc ccctcatacc acaccctgtg catacaccag ccaagccttt      60
cctggtctgg gaagggaaga gaaaaaagac gcaggccacc tgggggttct gcagtctttg     120
gtcagtccag ctttctatct tagctgcctt tggcttccgc agtgtaaacc ttgcctgccc     180
ggaggcagga ggcccagctg gacctccgag ggccatgagc aggcagcagc catcttggcc     240
tcaagcttgc ctttcccttg agtccctctc tcccctcggc tctagccaga ggtgtagcct     300
gcagatctag gaagagaaga gctggggagg aggatgaagg                           340

<210> 319
<211> 373
<212> DNA
<213> Homo sapien

<400> 319
aaagatgctg ttaatgaaca ttacggacaa ttcatggtgt ggctagttgg taacacttca 60
gctgattttt cttatgagat ggaaaaaaaa atcagccaag taagggcaca tcttcagttc 120
atttagaagt cagcatccaa ggtaaaagaa ttctctgttg gacttgacat cactcccatc 180
ctctgatact cgcctactct cttctcaaag aagttagtct ttccttccag tgaaatattc 240
tccataaagt caaatgggtt ctctactctg aaaaccttgc taaaacccag ttccagcata 300
agtctgtctg ccacaaactc aatgtattgc ttcattagag tgcaattcat gccaatgagc 360
ttcacaggca agg 373

<210> 320
<211> 509
<212> DNA
<213> Homo sapien

<400> 320
aaaaacaaaa ttaaattttc atttcaatta agaccccttt tggcattttg cttacttatt 60
ctgccctttg gttaacagca tcagcatcac attactattt tatattgcat atatgtagca 120
tttgcttcct taagttttca acatatcatt tatatttaaa ggcagacact gagtcagtat 180
taatagatta actaaactgc actgtaattt agataaaatt actgtgtctc actgtgtatt 240
acatgcaaaa tccacataaa ttgtcattta accaacagta ctgcacgagc gaacatctcg 300
atatatgaaa actgcatcat caattcaacg ttttggtact tgaaactgca tcataaatgc 360
aacattgtca tatgtgaaaa cgacacccta agtccttctt tttaaaaatg acattgcgtt 420
tagcttattg taagaggttg aacttttgta ttttgtaact atctttaagc tcttcagttt 480
ataattcata taaaatgcct tttgtattt 509

<210> 321
<211> 617
<212> DNA
<213> Homo sapien

<400> 321
   ccaaggcccc ttttgcagcc cacggctatg gtgccttcct gactctcagt atcctcgacc 60
   gatactacac accgactatc tcacgtgaga gggcagtgga actccttagg aaatgtctgg 120
   aggagctcca gaaacgcttc atcctgaatc tgccaacctt cagtgttcga atcattgaca 180
   aaaatggcat ccatgacctg gataacattt ccttccccaa acagggctcc taacatcatg 240
   tcctccctcc cacttgccag ggaacttttt tttgatgggc tcctttattt ttttctactc 300
   ttttcaggcg cactcttgat aaatggttaa ttcagaataa aggtgactat ggatataatt 360
   gagccctctg gtccaggtct cagtttacct aatattacct cagaaaggat atggagggaa 420
   gatgatcttt ttgccaggtc tgacttttct tcctgctccg ccctccatta acgctcagta 480
   ccctttagca gctgacggcc ccacgttcta ctccatgctt ggcttccttt ccaactagct 540
   ctttcatata ttttacttgc tagtatctcc attctctcta aagtagtggt tctttttgcc 600
   cttaaactta aattttt 617

<210> 322
<211> 403
<212> DNA
<213> Homo sapien

<400> 322

aaaaagaagg acttagggtg tcgttttcac atatgacaat gttgcattta tgatgcagtt 60
tcaagtacca aaacgttgaa ttgatgatgc agttttcata tatcgagatg ttcgctcgtg 120
cagtactgtt ggttaaatga caatttatgt ggattttgca tgtaatacac agtgagacac 180
agtaatttta tctaaattac agtgcagttt agttaatcta ttaatactga ctcagtgtct 240
gcctttaaat ataaatgata tgttgaaaac ttaaggaagc aaatgctaca tatatgcaat 300
ataaaatagt aatgtgatgc tgatgctgtt aaccaaaggg cagaataaat aagcaaaatg 360
ccaaaagggg tcttaattga aatgaaaatt taattttgtt ttt 403

<210> 323
<211> 298
<212> DNA
<213> Homo sapien

<400> 323
ccagaattag ggaatcagaa tcaaaccagt gtaaggcagt gctggctgcc attgcctggt 60
cacattgaaa ttggtggctt cattctagat gtagcttgtg cagatgtagc aggaaaatag 120
gaaaacctac catctcagtg agcaccagct gcctcccaaa ggaggggcag ccgtgcttat 180
atttttatgg ttacaatggc acaaaattat tatcaaccta actaaaacat tccttttctc 240
ttttttcctg aattatcatg gagttttcta attctctctt ttggaatgta gatttttt 298

<210> 324
<211> 78
<212> DNA
<213> Homo sapien

<400> 324
ccatgggaag gtttaccagt agaatccttg ctaggttgat gtgggccata cattccttta 60
ataaaccatt gtgtacat 78

<210> 325
<211> 174
<212> DNA
<213> Homo sapien

<400> 325
ccatcatggt caggaactcc gggaagtcaa tggtcccgtt cccatctgca tccacctcat 60
tgatcatatc ctgcagctct gcttcagtgg ggttctgtcc cagggatctc atcactgtcc 120
ccaactcctt ggtggtgata gtgccatctc catccttgtc aaagagggag aagg 174

<210> 326
<211> 679
<212> DNA
<213> Homo sapien

<220>
<221> misc_feature
<222> (1)...(679)
<223> n = A,T,C or G

<400> 326
aaaactgaaa tacctcttaa aataatttga tccccagcgt ttgctctttt tgaagtaacc 60
aacttactct taaaaaggat ggntgccaag atggaaagtc ttactgggtt ttcatgttaa 120
cctattcttt ggacataact atgaattttg tatacaatgc acttcatgaa aagttgtggc 180
tcccccagat tgcccacaag tgtgatcttg aagtcctaaa catttgtcca tgtaagcttc 240
aaaacagcgt taactgagtt attcaagtag cagtacttaa agatacaatt cttgaagcag 300

tttcaatggt ttctgatcca aataatcagt ttctgaacat tactacttca cataatagag 360
tccatcttca gtttcttctc actttctctt tcccttttgg gtttcctttt tgtggcctga 420
ggccaccagt tctttgggta ctatcaagat acttccatca tgggtacact ggagagcata 480
gtggttggga ttgactggcc taccttggtc atctcttaat ctactaaaaa tatcatgata 540
aaggtcatgc agtttctgtt tcattatgtt aatagctttg gtacattgtg cttgctctct 600
cttaanagtt tccttctttg cttgcaagtt acatacatca tcttctaaat tcaaaattat 660
gtccattttg gcgtttacc 679

<210> 327
<211> 619
<212> DNA
<213> Homo sapien

<220>
<221> misc_feature
<222> (1)...(619)
<223> n = A,T,C or G

<400> 327
aaaataagtt actggtaaat ggagttgcat tctatagtca cttaataaat attaacaaaa 60
tatttataac tggaacctta atgaaatgta tcatcaaatc aggtaaaagc aacttgtccg 120
cagttaccaa agcctanata cgcgttagat gcgccttttc cggcctgtgc gtctgctctg 180
gttcctctca ggcagcaaag ctggggaagg aagctcaggc aggagcctcc ccgacgccac 240
aacggcacaa gcagcagcta aagcaccgca ctttgctcta ctaacctttt acttaaatga 300
ggttttgcca aatccacatc tggaaccgcg tcacacccat ttgcaaggat gtttgttctt 360
tgatgaaact gcatctctac tgcacatgag ggctttcatt gtaggacaag aggagagttc 420
gtttattttt gtaactgttt tacatgttcc gattagttaa tcggtagctt atgtcatttg 480
ctatgcctgn agncttctaa tctctcctta ctaaaacatt acttcaaatt tgaattgacc 540
cttggttata atttatttag ccgggatttg tgtgtcattg tagagcaact ctaattcaag 600
aatagtgaca acttttaag 619

<210> 328
<211> 132
<212> DNA
<213> Homo sapien

<400> 328
aaatccaaat acaaaagcat agtctctgca agattttgtt ctttgaattt cttgatattg 60
taattgatta ttgataactg tcatcatgaa attatctctc aataataaga taaataaact 120
agcatatgaa tc 132

<210> 329
<211> 854
<212> DNA
<213> Homo sapien

<220>
<221> misc_feature
<222> (1)...(854)
<223> n = A,T,C or G

<400> 329
ccttgaggta actattgcaa aatatacagt gtaagttcag tctgatggaa accccagatt 60
catcaaggat acaaatctac agtagcccaa tggcggtttc atagtgtata atttattatc 120
aataaaatta actccgttac aatcagcatt catttcctcc aattaaaatt aagcataaac 180

cctaggtagt aaccttctgc acatatgtat agctccgaat ttcctcactg ttcgtctggt 240
gcaaaaacaa tattcaagct tgtctgatta tgcatatttt ctttaatcat atagattata 300
tatacaatag acaagacagg actatataga taatggacag acttaaatgc ccgcattttt 360
aaggtggaga aaatgatgaa tctatgcatc cccgagaaca cttaaaattt ttttttattt 420
cactgggaaa ttcttacagc tactttacaa tcataggtta acagcctagt tatacagaag 480
acatattcca ctacagagct atactctatg caactgtttt ttcccctcat aaacaacctg 540
agttcaaatt gaattctatc ttccacaatc acaatgggtg catcacccag tacacagaag 600
tttgaatcac aaaacataat taccacaata aaacacagtg ttcaagtatc ttggcagagc 660
aatctgccgc acaaactgca aattaaatta actacacaga ctaaaaacta tacagcctac 720
catcacagtt gtgcattata aaaaagggag tttctttcct ttggttttaa gtcaggaaca 780
gggtaggatt ttttaccctc nggccgggga ccacgctaaa ggggcgaaat ttcttgccan 840
natattccnt tcac 854

<210> 330
<211> 299
<212> DNA
<213> Homo sapien

<400> 330
ccaatgaata actgacttta taatcctggg caatcagctt ttggcgggtt gtaagtgctt 60
ctcgacactt ttcactcatg gattcttcaa atttatggtt aaagaggcac ttatacactc 120
tgccctcacc agcttgtgta ttttcacaaa aacgctcccg atcatctcgg caagcaaaat 180
ataaatgccg gtctaagtga aagtcatccg atgacagctc agccacccgg agaatggctt 240
tcttgcagag ttcagaaact tgaatcttgg gttctctttc ttctgcttct ttcaccagg 299

<210> 331
<211> 573
<212> DNA
<213> Homo sapien

<400> 331
aaagatatga acagcttaat tttccgtgtg attatctaat taaaaaagaa aaacaaaaca 60
agcaaaatgt tcaagttaaa aaaaaaacat accgggtgag caatgcacta aaattatcca 120
catgaaaaca aatggtctgt aatcttataa accaacatag catttcactg tcaacaatgt 180
gaaaatttaa tatcttctca aacaggcata agatgaagaa gtgctatttt ttaattgtaa 240
aaggaactta tgtaatgtaa aattacatta taatttttca ttccgaattg acaaatgatt 300
tcaaaaacaa ggatcaaagt ttgactgcaa atagtaatgc aatataattt cataaaaatc 360
cttcaatttc tatttttttc cttttctgta gttgacatat gaagaccact tcaatttcta 420
aaaaagggaa ccattccaat tttccctccc caagaaaatg tctcacaatt acaaagtaga 480
aaaacagccg ttcataaatg caaaaaaatt ctgatttata tatgaaataa tttctagatc 540
aattcaacat atttgatgac atttgttgag ttt 573

<210> 332
<211> 555
<212> DNA
<213> Homo sapien

<400> 332
aaatttgaaa gttgtaagca ctgatgttaa tgtgattgat cagcatgggc atatgtaaaa 60
tgtccttttc tggttgcctc tctatgctat tgtgttcaga tacttacacc ataattaaac 120
agtaagttat agacttgctg agtttggcat agatagtgcg ctcatttaat ctgtgcctct 180
caaaacttca gaatattagc atattaccac aaataatttt tggtgaaact attgagatat 240
taaaattttt gaaatcacta ctgttacctg ttatagaaaa tagtgttggc ttagtctagt 300
ctctgtgtaa ctggttacat tttgatggtt gtctatactc aactggatat gtgtatgtaa 360
attagaaaat acatacctat ccagacataa atgctaagta acattttttt cttcctccaa 420

ctacataatt tgtagctcat catttttcct taatcctttc ctaacttgtc gcagcagttt 480
gaatttccca gatatttatg tttgaacata atggctcaga atacatattt gaacatcata 540
gttgtatata ttttt 555

<210> 333
<211> 460
<212> DNA
<213> Homo sapien

<400> 333
aaatttcttt caacagtcta ttggggtcca aaaagcatat atcaaaacaa aaataacaaa 60
agcaaaacaa aatgctacat gtaaaagcta aagaaagaaa atgcagcata ttcaggttct 120
ttttcttgag gtacctatat aaatttaatc acctgcccca aagtcctctc gttaggttaa 180
aaacacaatg cgtcctgggg agccaattgc ccggcacgtc ttattactga gaaagtgcaa 240
gaatgctgat catcttatgc agcatactaa aggatgattt actctttaca aaatagagct 300
taagtatcaa cctgatggaa gttagaaaat taaaaacatt taagtagaat catctctctc 360
tctatttttg agatcctgca gcaaaaagcc tcccaaatca actttcaaag ttctgccatt 420
aaggaatgtt ggttctcttg taaaattcag agatctcttt 460

<210> 334
<211> 190
<212> DNA
<213> Homo sapien

<400> 334
ccaaggaagg ctgtgctcta gcccatctga ccctgtctgc aaaccacctg ggggacaagg 60
ctgatagaga cctgtgcaga tgtctctctc tgtgcccctc actcatctca ctggatctgt 120
ctgccaaccc tgagatcagc tgtgccagct tggaagagct cctgtccacc ctccaaaagc 180
ggccccaagg 190

<210> 335
<211> 394
<212> DNA
<213> Homo sapien

<400> 335
aaatttggac agacttctag cggacagtta cttctcaaga attttctata caaaagctgt 60
gccaggcata tattttctca ccaggacaca tggggcagcg gacccctggt gtcagtaaga 120
acacacccag aatgatataa ccagatattt ttcagtttct aaattaaggc atattcaaaa 180
aattccatgt acaagtttac accacttttc taagttactc accaggtaat taaagcagat 240
tcacagatga attactctca gtttaactat atgcaacaac catgccaata acttttcttc 300
taaattttgc ataataatgg ttaaaaaaag tggtagttta actatcatgt tcacaattgt 360
catttttcaa ggcagtagaa gaccaagaca tttt 394

<210> 336
<211> 429
<212> DNA
<213> Homo sapien

<400> 336
aaaagctatc accattgtag tagaatcatc cttctttttt gaaatttgaa gcatcccagg 60
cttaaaatct tgtgtttcag aaagacagtt tataccatga ctgcttaatt atccccccaa 120
agaccttctg attgaagtca tgtacagttc agtggcctaa attctctgcc tttttaactt 180
gctttgcaag cctactctga aaataagtta tttagtcaag ttattctcaa agatgtccca 240
gttgcctaga aaggatcaaa tggaacattt gacacacata ctcaaaaaaa tgtaactgac 300

tataaacact ttaacctaat catctgtatc aaactttcta aaaatcaaat ctcaggattg 360
ttccacttta gagattctat gtaaagttta tataactata cttgtcaaat agcacctatc 420
tatgcattt 429

<210> 337
<211> 373
<212> DNA
<213> Homo sapien

<400> 337
aaagatgctg ttaatgaaca ttacggacaa ttcatggtgt ggctagttgg taacacttca 60
gctgattttt cttatgagat ggaaaaaaaa atcagccaag taagggcaca tcttcagttc 120
atttagaagt cagcatccaa ggtaaaagaa ttctctgttg gacttgacat cactcccatc 180
ctctgatact cgcctactct cttctcaaag aagttagtct ttccttccag tgaaatattc 240
tccataaagt caaatgggtt ctctactctg aaaaccttgc taaaacccag ttccagcata 300
agtctgtctg ccacaaactc aatgtattgc ttcatcagag tgcaattcat cccaatgagt 360
ttcacaggca agg 373

<210> 338
<211> 366
<212> DNA
<213> Homo sapien

<400> 338
ccatcccctt atgagcgggc gcagtgatta taggctttcg ctctaagatt aaaaatgccc 60
tagcccactt cttaccacaa ggcacaccta caccccttat ccccatacta gttattatcg 120
aaaccatcag cctactcatt caaccaatag ccctggccgt acgcctaacc gctaacatta 180
ctgcaggcca cctactcatg cacctaattg gaagcgccac cctagcaata tcaaccatta 240
accttccctc tacacttatc atcttcacaa ttctaattct actgactatc ctagaaatcg 300
ctgtcgcctt aatccaagcc tacgttttca cacttctagt aagcctctac ctgcacgaca 360
acacat 366

<210> 339
<211> 319
<212> DNA
<213> Homo sapien

<400> 339
   ccttccctcc ccaccaccat caacctcttc aaaacctact ccctccctct aagtatctct 60
   caacacagta tgtctggggc tagatttcaa aacccacgta atgaaaaagt cagttttaca 120
   agcctaattt tgttgttttt ttttttatat caattaacgt taaaaattgc atcaactatt 180
   taattcatga ggatctttca tattaaaatt taaccttaag attcaaccgc catgtgcttt 240
   tataaaggaa acatttttta gagacgtctg agctcacttt tacatggtgg tgcctactgc 300
   cgttaatgtt tgtgatttt 319

<210> 340
<211> 278
<212> DNA
<213> Homo sapien

<220>
<221> misc_feature
<222> (1)...(278)
<223> n = A,T,C or G

<400> 340
ctaataaaat gaattaacca ctcattcatn natctaccca cccnatccaa catctccnca 60
tgatgaaacn ncggctcact ccttggcgcc tgcctgatcc tccaantcac cacaggacta 120
ttcctagcca tgcactactn accagacncc tcaacngcct tttnatcaat nggncacatn 180
actcganacn taaatnatgg ctgaatcatc cgctacctnc acgccaatgg cagcctcaat 240
attctttatg ctgcctcttc ctacacatgc gggcgagg 278

<210> 341
<211> 400
<212> DNA
<213> Homo sapien

<400> 341
ccagcatggg gctgcagctg aacctcacct atgagaggaa ggacaacacg acggtgacaa 60
ggcttctcaa catcaacccc aacaagacct cggccagcgg gagctgcggc gcccacctgg 120
tgactctgga gctgcacagc gagggcacca ccgtcctgct cttccagttc gggatgaatg 180
caagttctag ccggtttttc ctacaaggaa ttcagttgaa tacaattctt cctgacgcca 240
gagaccctgc ctttaaagct gccaacggct ccctgcgagc gctgcaggcc acagtcggca 300
attcctacaa gtgcaacgcg gaggagcacg tccgtgtcac gaaggcgttt tcagtcaata 360
tattcaaagt gtgggtccag gctttcaagg tggaaggtgg 400

<210> 342
<211> 536
<212> DNA
<213> Homo sapien

<400> 342
aaagaacaat gggaaaaaca agtccgtgtt ctcacagatg ctgtcgatga cattacttcc 60
attgatgact tcttggctgt ctcagagaat cacattttgg aagatgtgaa caaatgtgtc 120
attgctctcc aagagaagga tgtggatggc ctggaccgca cagctggtgc aattcgaggc 180
cgggcagccc gggtcattca cgtagtcacc tcagagatgg acaactatga gccaggagtc 240
tacacagaga aggttctgga agccactaag ctgctctcca acacagtcat gccacgtttt 300
actgagcaag tagaagcagc cgtggaagcc ctcagctcgg accctgccca gcccatggat 360
gagaatgagt ttatcgatgc ttcccgcctg gtatatgatg gcatccggga catcaggaaa 420
gcagtgctga tgataaggac ccctgaggag ttggatgact ctgactttga gacagaagat 480
tttgatgtca gaagcaggac gagcgtccag acagaagacg atcagctgat agctgg 536

<210> 343
<211> 646
<212> DNA
<213> Homo sapien

<400> 343
aaaacttcta ttcatcaaaa gacataaaga aaacagtcaa gccacagact aggtgtaata 60
tctcaataca tatatccgac aagagaattg catctagaat gtataaagaa tttctatgac 120
ccaattatag ctatcaggga tatacaaatt aaaaccaaaa tgaaacatca ctacacaccg 180
attggaatgg ttaaaaagga aaaatactga caacaccaat atttgtaaag acaggaggta 240
ccagaactct cattcattat attcataaat tgacaaatat aaaaactgct atagtagggc 300
agtcttcctt agaaagggat tgtgggcatg acagagaaca atattaatct gtccattata 360
ttccttaact gtaaaatgga gaccatatgt tccaccagct tcacttggta attatgatac 420
atggctatta agagactcaa atgactccat ttcatcaact aatatgccct gtcaattcta 480
cttctaaagt atcccatgtt ctatccaatg tcataccact atcataattt aagtgttcat 540
aactctctat aatatttcaa taatctaact ggtctcaatg cctgtagtag aaattgcaga 600
ttgggctccc caatttctgt tccctaggaa ggctgagaaa gctttt 646

<210> 344
<211> 383
<212> DNA
<213> Homo sapien

<400> 344
cctgcacccc agtataaggg cctccccagc tgagtaagaa gctgcttccc ctcctctcat 60
aggccaagcc tattgtgtga aaccatctca tggtcttggt gacgtagacc atttttgaaa 120
ccgtctcatg gtcttggtga cgtagaccgt ttgcttcttt aactccagcc gcggaatgac 180
attagtggaa ccgggctagg gaactgctgg aagttcagga tgccaccacc ttgaacacct 240
aggccaggga tccccaccat gtcccgggtt tctttcttcg agagtataga accgttcatt 300
cttgctttgt gtcccattcc atctcttgaa aaaatgtagt ctttgaatgt gtgaaaatct 360
agggacattc aatctagtct ttt 383

<210> 345
<211> 263
<212> DNA
<213> Homo sapien

<400> 345
   cctccccttc ccctttgctg gtgggaggag ctcgtgtgct ccttggccgc ttactggaag 60
   ggcgtttttc agagctgcag ggacagggtg agcagctgaa gggctaggag ggaagccggc 120
   ccccgctctg cagaagctgc atttcagctg aatctgtgtt tcagcctcag ttggttgcac 180
   cgttagcccc tctcctcccg gatggtcatg tttttgtcac attagagaat aaacagccac 240
   acacacattt ttttttttcc ttt 263

<210> 346
<211> 132
<212> DNA
<213> Homo sapien

<400> 346
aaatccaaat acaaaagcat agtctctgca agattttgtt ctttgaattt cttgatattg 60
taattgatta ttgataactg tcatcatgaa attatctctc aataataaga taaataaact 120
agcatatgaa tc 132

<210> 347
<211> 564
<212> DNA
<213> Homo sapien

<220>
<221> misc_feature
<222> (1)...(564)
<223> n = A,T,C or G

<400> 347
cctgggtatc cagggaggct ctgcagccct gctgaagggc cctaactaga gttctagagt 60
ttctgattct gtttctcagt agtcctttta gaggcttgct atacttggtc tgcttcaagg 120
aggtcgacct tctaatgtat gaagaatggg atgcatttga tctcaagacc aaagacagat 180
gtcagtgggc tgctctggcc ctggtgtgca cggctgtggc agctgttgat gccagtgtcc 240
tctaactcat gctgtccttg tgattaaaca cctctatctc ccttgggaat aagcacatac 300
aggcttaagc tctaagatag ataggtgttt gtccttttac catcgagcta cttcccataa 360
taaccacttt gcatccaaca ctcttcaccc acctcccata cgcaagggga tgtggatact 420
tggcccaaag taactggtgg taggaatctt agaaacaaga ccacttatac tgtctgtctg 480

aggnagaaga taacagcagc atctcgacca gcctctgcct taaaggaaat ctttattaat 540
cacgtatggt tcacaagata attc 564

<210> 348
<211> 321
<212> DNA
<213> Homo sapien

<220>
<221> misc_feature
<222> (1)...(321)
<223> n = A,T,C or G

<400> 348
gcncatgaac anggagcaac ganaagagat gtcgggctaa gggcccggga cgggcggcac 60
ccatcctgcn acggaacacn ttcgggttnt ggttttgatt ngttcacctc tgtttatatg 120
canctatttg ntcctcctcc cccaccccag nccccaactt catgcttntc ttccgcnctc 180
agccnccctg ccctgtcctc gcggtgagtc antgaccacn gnttcccctg cangagccgc 240
cgggcgtgag acncngaccc tcnntgcata caccaggccg ggcccnngct ggctcccccn 300
gnggccctgt gaaanagctg g 321

<210> 349
<211> 255
<212> DNA
<213> Homo sapien

<400> 349
ccatgacagt gaaggggctg ttaggaatat caacaccacc gaagcgcaca tagatcacat 60
atgtgcccgg cttggcagct gtgtagaaga tgtcataggt tccatcttca ttctcaatga 120
catcggcctc ggcctcagtg ccatctgggg tcagaaccgt gcaggtcact ttacccttcc 180
cggcagtctt ggcatcaacc acaaagccta cttcttcgcc agttttcaca gtggaggcga 240
ttccaggacc cgtag 255

<210> 350
<211> 496
<212> DNA
<213> Homo sapien

<220>
<221> misc_feature
<222> (1)...(496)
<223> n = A,T,C or G

<400> 350
gggcttattn gctcacaaaa tcattcnctt ttggaactat ggccaattga agctacacac 60
tgaatttatt aatacagcat taagtttctt tgtgtnaaaa aatctttgtn cncagtaata 120
aaaaaagata aggcaagatg cattaaacat gaaaccttct ggctcttttc ctctgcgttt 180
ttacagagcc actgatgact atctgcaaca aaagagttaa gtttctgatt ttccgtatca 240
agcatcttat gcctttgctg tggtaagaat tctggccaag caccctgaag gacagatgct 300
ggtgatggnc tttggcactt atgctggcaa actgagcttc tttcccttga gtacttttgn 360
aatgtacaag tagaagaagt cacaagtata ggatggtctg gactacgccg gccaccacag 420
caatgaggtc aaagaagccc tcaaagnaga agcgnccaga tccagttgac aagatacaaa 480
gcacgataga ggccca 496

<210> 351

<211> 109
<212> DNA
<213> Homo sapien

<220>
<221> misc_feature
<222> (1)...(109)
<223> n = A,T,C or G

<400> 351
ccatagtgaa gcctgggaat gagtgttact gcagcatctg ggctgccanc cacagggaag 60
ggccaagccc catgtagccc cagtcatcct gcccagcccc gcctcctgg 109

<210> 352
<211> 384
<212> DNA
<213> Homo sapien

<400> 352
ccttcgagag tgacctggct gcccaccagg accgtgtgga gcagattgcc gccatcgcac 60
aggagctcaa tgagctggac tattatgact cacccagtgt caacgcccgt tgccaaaaga 120
tctgtgacca gtgggacaat ctgggggccc taactcagaa gcgaagggaa gctctggagc 180
ggaccgagaa actgctggag accattgacc agctgtactt ggagtatgcc aagcgggctg 240
cacccttcaa caactggatg gagggggcca tggaggacct gcaggacacc ttcattgtgc 300
acaccattga ggagatccag ggactgacca cagcccatga gcagttcaag gccaccctcc 360
ctgatgccga caaggagcgc ctgg 384

<210> 353
<211> 345
<212> DNA
<213> Homo sapien

<220>
<221> misc_feature
<222> (1)...(345)
<223> n = A,T,C or G

<400> 353
ccttggtcag gatgaagtng gctgacacac cttagcttgg ntttgcttat tcaaaagana 60
aaataactac acatggaaat gaaactagct gaagcctttt cttgttttan caactgaaaa 120
ttgnacttgg ncacttttgt gcttgaggag gcccattttc tgcctggcag ggggcaggta 180
tgtgccctcc cgctgactcc tgctgtgtcc tgaggtgcat ttcctgttgn ncacacaang 240
gccangntcc attctccctc ccttttcacc agngccacan cctnntctgg aaaaangacc 300
agnggtcccg gaggaaccca tttgngctct gcttggacag canag 345

<210> 354
<211> 712
<212> DNA
<213> Homo sapien

<400> 354
ccatctacaa tagcatcaat ggtgccatca cccagttctc ttgcaacatc tcccacctca 60
gcagcctgat cgctcagcta gaagagaagc agcagcagcc caccagggag ctcctgcagg 120
acattgggga cacattgagc agggctgaaa gaatcaggat tcctgaacct tggatcacac 180
ctccagattt gcaagagaaa atccacattt ttgcccaaaa atgtctattt ttgacggaga 240

gtctaaagca gttcacagaa aaaatgcagt cagatatgga gaaaatccaa gaattaagag 300
aggctcagtt atactcagtg gacgtgactc tggacccaga cacggcctac cccagcctga 360
tcctctctga taatctgcgg caagtgcggt acagttacct ccaacaggac ctgcctgaca 420
accccgagag gttcaatctg tttccctgtg tcttgggctc tccatgcttc atcgccggga 480
gacattattg ggaggtagag gtgggagata aagccaagtg gaccataggt gtctgtgaag 540
actcagtgtg cagaaaaggt ggagtaacct cagcccccca gaatggattc tgggcagtgt 600
ctttgtggta tgggaaagaa tattgggctc ttacctccca atgactgccc tacccctgcg 660
gaccccgctc cagcgggtgg gggattttct tggactatga tgctggggga gg 712

<210> 355
<211> 385
<212> DNA
<213> Homo sapien

<400> 355
cctcatagcc gcttagcaca gttacagaat gtctgaaggg gacagtgtgg gagaatccgt 60
ccatgggaaa ccttcggtgg tgtacagatt tttcacaaga cttggacaga tttatcagtc 120
ctggctagac aagtccacac cctacacggc tgtgcgatgg gtcgtgacac tgggcctgag 180
ctttgtctac atgattcgag tttacctgct gcagggttgg tacattgtga cctatgcctt 240
ggggatctac catctaaatc ttttcatagc ttttctttct cccaaagtgg atccttcctt 300
aatggaagac tcagatgacg gtccttcgct acccaccaaa cagaacgagg aattccgccc 360
cttcattcga aggctcccag agttt 385

<210> 356
<211> 347
<212> DNA
<213> Homo sapien

<400> 356
aaatgagata aagaaagtct ccttttgttt ttagatggaa aagaaagcac aagtttttc 60
tacctgtgaa tgaactttgg tgacctatat gtgccattca tgcagcattt ttgttcatat 120
tggcttagaa ttcagtgcat gaatatcatt acattcttat atctaacatt cctagttagc 180
tttgattcaa aatatacaaa atctgataca tgaatacttt gctagattaa tgacttgatc 240
atctttggaa tgagtaggca agacgatttt tacctattat ttctatgttg tgggtaatgt 300
taaaactaaa tacagatgat aataattgct atttcacagt gatgttt 347

<210> 357
<211> 313
<212> DNA
<213> Homo sapien

<400> 357
aaagtaatca acctctctgt ccttccatta gtctggatcg tctaaagatt gttttatttt 60
tagaggctca tccggtcaga tgttagtgat gtgaaatttc aggccaggcg tgacgtcagc 120
gtggcatttg aaacagctcc atgttgccct tagtgctgtc tgaccgaagc ctgtctgtcc 180
tcagatataa agatgaagcg cagctgtata aagaagagca cctgaggaat cggcagcacc 240
ctcactgcta cgttcagtac atgatcgcca tcatcaacaa ctgccagacc ttcaaggaat 300
ccatagtcag ttt 313

<210> 358
<211> 403
<212> DNA
<213> Homo sapien

<400> 358

aaaaagaagg acttagggtg tcgttttcac atatgacaat gttgcattta tgatgcagtt 60
tcaagtacca aaacgttgaa ttgatgatgc agttttcata tatcgagatg ttcgctcgtg 120
cagtactgtt ggttaaatga caatttatgt ggattttgca tgtaatacac agtgagacac 180
agtaatttta tctaaattac agtgcagttt agttaatcta ttaatactga ctcagtgtct 240
gcctttaaat ataaatgata tgttgaaaac ttaaggaagc aaatgctaca tatatgcaat 300
ataaaatagt aatgtgatgc tgatgctgtt aaccaaaggg cagaataaat aagcaaaatg 360
ccaaaagggg tcttaattga aatgaaaatt taattttgtt ttt 403

<210> 359
<211> 411
<212> DNA
<213> Homo sapien

<400> 359
aaataaatac ttagaacacg acttggctcc tacaagcatc tggactctag gtctcagtac 60
tggagtgtct cacccatggg ccccacgcag ggacgccacg gttccctccc accccgtgat 120
caagacacgg aatcggctgc cgatggttgg atcgcaatgc gccccttttc tagagccttc 180
cccggccatc tacaggcagg atgcggctgg gaaaaagaca actggaattt ctcgaaggtt 240
gatggtccgc acggttgagg attctacgtg gttctcttgg ttcccctggt gtgtgtgtgt 300
gtggaggagg ccgcggccct tagatcacct tcttgagctc gtcgtacagg accagcacga 360
aggcgccccc catgccccgc aggacgttgg accacgcacc cttgaagaag g 411

<210> 360
<211> 378
<212> DNA
<213> Homo sapien

<220>
<221> misc_feature
<222> (1)...(378)
<223> n = A,T,C or G

<400> 360
cctcttcagg ggcccgagcc agggacaggg ccttggtttc cttctccctg gcttctgcct 60
cagctctgtc cctctcatcc gcgtatttgg aagagatgtt tttctcctcg gctaacaact 120
gatcaaattt cctctgcttc ttttccaggt tggacacgag ttgccgctgg ttgtccaaat
180caacaaccag gtcgtccagc tcctgctgaa gcctgttctt ggtcttttcc agtttatcat
240
aagcggccgc cttctcctcg tactgctggg tgaggntctc gatctccttc tggaacctct 300
tcttcccctc ttccagagct tccacggngc tggcaaagtc ctgcagcttc ttcttcgagt 360
cggagagctg gatgttga 378

<210> 361
<211> 372
<212> DNA
<213> Homo sapien

<400> 361
aaatactggg ggccattaag agtggatgta gctaagagct tagctaacat tgccttttca 60
ctctattttt ctcagatatt gtaagcattc tgtttttcaa tattgtagtt aattttttgg 120
ctttcaacag cagccctagt aatggtggag ttgttaatta atgtgtatat tgtactgaat 180
ttctgtcagt taaggggttc actgctttgg tggaaattgg tggaaattgc tagcaggttc 240
cacgatgttt atttttttct ccatgttgta tatcattacc atttcacata cgcgtttcta 300
tttttcttcc tctcctcctg atctccttaa aaatgaatct agagttggtg gctttttccc 360
cctcctcttt gg 372

<210> 362
<211> 544
<212> DNA
<213> Homo sapien

<400> 362
cctgagtcac ctagcatagg gttgcagcaa gccctggatt cagagtgtta aacagaggct 60
tgccctcttc aggacaacag ttccaattcc aaggagccta cctgaggtcc ctactctcac 120
tggggtcccc aggatgaaaa cgacaatgtg cctttttatt attatttatt tggtggtcct 180
gtgttattta agagatcaaa tgtataacca cctagctctt ttcacctgac ttagtaataa 240
ctcatactaa ctggtttgga tgcctgggtt gtgacttcta ctgaccgcta gataaacgtg 300
tgcctgtccc ccaggtggtg ggaataattt acaatctgtc caaccagaaa agaatgtgtg 360
tgtttgagca gcattgacac atatctactt tgataagaga cttcctgatt ctctaggtcg 420
gttcgtggtt atcccattgt ggaaattcat cttgaatccc attgtcctat agtcctagca 480
ataagagaaa tttcctcaag tttccatgtg cggttctcct agctgcagca atactttgac 540
attt 544

<210> 363
<211> 328
<212> DNA
<213> Homo sapien

<400> 363
aaactggtta tgacaaaagc ctttagttgt gtttcttgaa ctataaagaa aacaaatttt 60
ggcagtcttt aagtatatat agcttaaaat ataattttta gcatttggca ccatatgtat 120
gccattatat ttgattttgc attactgttt cacaatgaag ctttctttaa ggctttgatt 180
tttatgatta tgaaagaaat aaggcacaac cacagttttt ctttcttaaa tttcatcact 240
gttgatgtgg ttcttttgtg ttaaaaaaaa aaagtgcaac tatcaaaact aaaaaattat 300
agagtaatat tgccgttctg ctgatttt 328

<210> 364
<211> 569
<212> DNA
<213> Homo sapien

<400> 364
cctgggcacc tctttgcttg aaatatggca agacttggaa aaatgtttgc ccttagaatc 60
tatctcacta ctttagttag ttgtctcctt tgggcctggg cacagttctg gccctgatct 120
ggaacagact cccttttcta aaactgaact tgaccacatc aaaagtttgt aaaacaatct 180
ccatggtaat taaacttgca ttcaacacca tatggtaaca gaagatggca aaggataaga 240
ttcagatctt agatctttcc aagtagggca tgttagatga tagaaggatt agttgcaagc 300
tggatctgag ctcaggcttg ggcatgaagg aaactgtctc ccatgtggtt tggaagagtt 360
aggggctccc tgagctctat tgtgaactat acgggtttca tccaaggaat ggtatgatgt 420
gggcataaaa ccattcttca gacaactgaa gatggtcccc ttctgtagcc agaaacacta 480
gctgtcctgc attgtccatt tcctttagcc ccaggcggtc ctgtgtgtac agggaggtct 540
cctgtaaggg aatggtttcc ttggcttgg 569

<210> 365
<211> 151
<212> DNA
<213> Homo sapien

<400> 365
aaaaaaaaaa atccttttat tatggaattt gtcaaacaca cacacaagca taacaaaccc 60

ctaggtaccc atctccaagt tttgacccct attataattt catcttcagt gttttattat 120
ccacttcctc tctctctatc tttagtattt t 151

<210> 366
<211> 508
<212> DNA
<213> Homo sapien

<220>
<221> misc_feature
<222> (1)...(508)
<223> n = A,T,C or G

<400> 366
agtataaaga tatattccat aaaagagttt ggcagtcaaa ganaagcatc gcacttccga 60
aaaacacaag cattcttctc ctagtctaca gagaattgng taaaaaaaaa aaaaaatcat 120
catcaacagc cnccantnta cnccacacta gaatgtacac tccggcaagt aaattaaggn 180
tgcagtccat ccctgaacga tganaagngg tctgagctat ggcaaagngt tanaaagtag 240
cccagctana caaatgcccc agctatcccc aggggagtta ttcagtactt aanacttcat 300
ttccaananc agccccggaa aagccctgac aggaaggggg gaccagngat caccgatntc 360
ccattagggg cggncaccaa aaacaaaatg cctggagctt ntgagcagct gcagcctggg 420
gttgtggcta ggcncngggn gnggttgcaa aaaaacggct gtntccgggg agaggcaaat 480
ggcaggccag ccagccctgg gtacatgg 508

<210> 367
<211> 382
<212> DNA
<213> Homo sapien

<400> 367
cctgagcggc tagtctttaa gatgcgcttc tatcgtttgc tgcaaatccg agcagaagcc 60
ctcctggcgg caggcagcca tgtgatcatt ctgggtgacc tgaatacagc ccaccgcccc 120
attgaccact gggatgcagt caacctggaa tgctttgaag aggacccagg gcgcaagtgg 180
atggacagct tgctcagtaa cttggggtgc cagtctgcct ctcatgtagg gcccttcatc 240
gatagctacc gctgcttcca accaaagcag gagggggcct tcacctgctg gtcagcagtc 300
actggcgccc gccatctcaa ctatggctcc cggcttgact atgtgctggg ggacaggacc 360
ctggtcatag acacctttca gg 382

<210> 368
<211> 174
<212> DNA
<213> Homo sapien

<400> 368
ccttctccct ctttgacaag gatggagatg gcactatcac caccaaggag ttggggacag 60
tgatgagatc cctgggacag aaccccactg aagcagagct gcaggatatg atcaatgagg 120
tggatgcaga tgggaacggg accattgact tcccggagtt cctgaccatg atgg 174

<210> 369
<211> 216
<212> DNA
<213> Homo sapien

<400> 369
aaatctcatg ggttctatta aaaaaatata tatatagggc cccaatccat tgccatcaaa 60

ttgcccttgg acttttccaa ggtatattat ggggttttat gcaaaattcc aagctaccat 120
gtaacttttt ttaaccattt aacaaggagg gggaactgtt tcctaccttc tttacatgtt 180
gtgcattgtt gtggtccaga aatgccaaac cttttt 216

<210> 370
<211> 344
<212> DNA
<213> Homo sapien

<400> 370
ccttggtcag gatgaagttg gctgacacag cttagcttgg ttttgcttat tcaaaagaga 60
aaataactac acatggaaat gaaactagct gaagcctttt cttgttttag caactgaaaa 120
ttgtacttgg tcacttttgt gcttgaggag gcccattttc tgcctggcag ggggcaggtc 180
tgtgccctcc cgctgactcc tgctgtgtcc tgaggtgcat ttcctgttgt acacacaagg 240
gccaggctcc attctccctc cctttccacc agtgccacag cctcgtctgg aaaaaggacc 300
aggggtcccg gaggaaccca tttgtgctct gcttggacag cagg 344

<210> 371
<211> 741
<212> DNA
<213> Homo sapien

<220>
<221> misc_feature
<222> (1)...(741)
<223> n = A,T,C or G

<400> 371
aaattacata tctaattgtg tgatttgtta aatgcccatt tcttcatcta agtgctaagt 60
gctaagtgta gcagtttgtt ccctgctaca ctccaaggca caaaggagtt caaggaatgt 120
gcaatggaaa tcagttagat gaatgtgtta ggaaccttcc ctttaataaa gctggatccc 180
acactagccc ctacaccctc tcatcaccaa atattcctgc ttcctctcac ctgcacttgc 240
tgttctctcc tctgccacac aaatctacct ctcaagccta ggtcccacct gcttcatgac 300
aactttccag actattccag aacctttaac catctctgac ctctcatcag atctatgttg 360
tacataacac caattaatga gatcattact gctttatgct ctaattgctt cctgtattca 420
aaatcttctc tccaaccaca taatgactcc ctaaacttct cttgtatttt ccaatgcctt 480
gtacaagcac agaactggtc aatcaataaa tactcactgg ttatttgagg aaaaaatgtt 540
gccaagcacc atctttatca gaaaataaat caattcttct aaacttggag aaatcaccct 600
attcctagta tgtgatctta attagaacaa ttcagattga gaangngaca gcatgctggc 660
agtcctcaga gccctcgctt gctctcggna cctccctgcc tgggctccca ctttggtggc 720
atttgaggag cccttcagcc t 741

<210> 372
<211> 218
<212> DNA
<213> Homo sapien

<220>
<221> misc_feature
<222> (1)...(218)
<223> n = A,T,C or G

<400> 372
ccgccagtgt gctggaattc gcccttggcc gcccgggcag gtaccacaac agcaggnctg 60

agtgagaaat ctaccacctt ctacagtagc cccagatcac cggacacaac actctcacct 120
gccagcacga caagctcagg cgtcagtgaa gaatccacca cctcccacag ccgaccaggc 180
tcaacgcaca caacagcatt ccctggcagt accttggn 218

<210> 373
<211> 168
<212> DNA
<213> Homo sapien

<400> 373
actgctaggg aatgctgttg tgtgcattga gcctggtcgg ctgtgggagg tggtggattc 60
ttcactgacg cctgagcttg tcgtgctggc aggtgagagt gttgtgtccg gtgatctggg 120
gctactgtag aaggtggtag atttctcact caggcctgct gttgtggt 168

<210> 374
<211> 154
<212> DNA
<213> Homo sapien

<220>
<221> misc_feature
<222> (1)...(154)
<223> n = A,T,C or G

<400> 374
tgagaaatct accaccttct acagngagcc ccanatcacc ggacacaaca ctctcacctg 60
ccagcacgac aagctcaggc gtcagtgaag aatccaccac ctcccacagc cgaccaggct 120
caacgcacac aacagcattc cctggcagta cctc 154

<210> 375
<211> 275
<212> DNA
<213> Homo sapien

<400> 375
actgccaggg gacagtgctg tgtcagttga acctgggctg ctgtgggaag ttgttgattc 60
ctgactgggg cctgaggtgg tggtgctggc aggtaacagt gttgtatccg ttgagcctgg 120
gctgctgtgg gaagttgtag aatgccgact gaggcctggc gtggtggtgc tgtcagggaa 180
tgctgttgtg tgcgttgagc ctggtcggct gtgggaggtg gtggattctt cactgacgcc 240
tgagcttgtc gtgctggcag gtgagagtgt tgtgg 275

<210> 376
<211> 191
<212> DNA
<213> Homo sapien

<220>
<221> misc_feature
<222> (1)...(191)
<223> n = A,T,C or G

<400> 376
actgccaggg gacagtgctg tgtcagttga acctgagctg ctgtgggaag ttgttgattc 60
ctgactggag cctgaggtgg tggtgctggc aggtaacagt gttgtatccg ttgagcctgg 120
gctgctgtgg gaagttgtag aatgccgact gaggcctgcc gtggtggtgc tgntagggaa 180

tgctgctagc g                                                          191

<210> 377
<211> 476
<212> DNA
<213> Homo sapien

<400> 377
ccgccagtgt gctggaattc gcccttggcc gcccgggcag gtacatttcc ttgtagactc      60
tgttaatttc ctgcagctcc tggttggttc tggagcagat gatctcaatg agagagtcct     120
cgtcggttcc cagccccttc atggaagctt ttagctcaga agcgtcatac tgagcaggtg     180
tcttcaatag gcccaaaatc accgtctcca ggtggccaga taaggctgac ttcagtgctg     240
atgcaagttc ctttttggtc cttctctggt aggcgaaggc aatatcctgt ctctgtgcat      300
tgctgcggtt ggtcaaaatg ttgacaatgg tgacctcatc cacacctttg gtcttgatgg     360
ctgtttcaat gttcaaagca tcccgctcag catcaaagtt agtataggct ttgacagacc     420
catatgcact tgggggtgta gagtgatcac cctccaagcc gagcttgcac aggatt         476

<210> 378
<211> 455
<212> DNA
<213> Homo sapien

<220>
<221> misc_feature
<222> (1)...(455)
<223> n = A,T,C or G

<400> 378
agtgtgctgg aattcgccct tggccgcccg ggcaggtaca catcccatct tcaaatttaa      60
aatcatattg tcagttgtcc aaagcagctt gaatttaaag tttgtgctat aaaattgtgc     120
aaatatgtta aggattgaga cccaccaatg cactactgta atatttcgct tcctaaattt     180
cttccaccta cagataatag acaacaagtc tgagaaacta aggctaacca aacttagata     240
taaatcctac caataaaatt tttcagtttt aagttttaca gtttgattta aaaacaaaac     300
agaaacaaat ttcaaaataa atcacatctt ctcttaaaac ttggcaaacc cttccctaac     360
tgtccaagtn tgagcataca ctgccactgg ctttagatac tccaattaaa tgcactactc     420
tttcactggt ctgaatgaag tatggtgaaa caagc                                455

<210> 379
<211> 297
<212> DNA
<213> Homo sapien

<220>
<221> misc_feature
<222> (1)...(297)
<223> n = A,T,C or G

<400> 379
agctcggatc cctagnacgg ccgccagtgt gctggaattc gcccttagcg gcggcccggg      60
caggtacaaa gaatccttag acgccatact gagttttaag ttccttaatt cctaatttaa     120
ggcttctagt gaagcctcct cacagtaggc ttcactaggc ccacagtgcc cctagacctc     180
tgacaatccc accctagaca gactttattg caaaatgcgc ctgaagaggc agatgattcc     240
caagagaact caccaaatca agacaaatgt cctagatctc tagtgtggna gaactat         297

<210> 380

<211> 144
<212> DNA
<213> Homo sapien

<220>
<221> misc_feature
<222> (1)...(144)
<223> n = A,T,C or G

<400> 380
actttgctga aaattctttt tcccagggtc tataaaacat taatttgttt ttatatttta 60
ctattttttt gngttttttt gtttttaaat caataagtaa tctaggacta gcattatgtt 120
tgctagacct ggcatttgct cggc 144

<210> 381
<211> 424
<212> DNA
<213> Homo sapien

<400> 381
actcttgaat acaagtttct gataccactg cactgtctga gaatttccaa aactttaatg 60
aactaactga cagcttcatg aaactgtcca ccaagatcaa gcagagaaaa taattaattt 120
catgggacta aatgaactaa tgaggataat attttcataa ttttttattt gaaattttgc 180
tgattcttta aatgtcttgt ttcccagatt tcaggaaact ttttttcttt taagctatcc 240
acagcttaca gcaatttgat aaaatatact tttgtgaaca aaaattgaga catttacatt 300
ttctccctat gtggtcgctc cagacttggg aaactattca tgaatattta tattgtatgg 360
taatatagtt attgcacaag ttcaataaaa atctgctctt tgtataacag aatacatttg 420
aaaa 424

<210> 382
<211> 408
<212> DNA
<213> Homo sapien

<400> 382
actcttgaat acaagtttct gataccactg cactgtctga gaatttccaa aactttaatg 60
aactaactga cagcttcatg aaactgtcca ccaagatcaa gcagagaaaa taattaattt 120
catgggacta aatgaactaa tgaggataat attttcataa ttttttattt gaaattttgc 180
tgattcttta aatgtcttgt ttcccagatt tcaggaaact ttttttcttt taagctatcc 240
acagcttaca gcaatttgat aaaatatact tttgtgaaca aaaattgaga catttacatt 300
ttctccctat gtggtcgctc cagacttggg aaactattca tgaatattta tattgtatgg 360
taatatagtt attgcacaag ttcaataaaa atctgctctt tgtatgac 408

<210> 383
<211> 455
<212> DNA
<213> Homo sapien

<220>
<221> misc_feature
<222> (1)...(455)
<223> n = A,T,C or G

<400> 383
actcttgaat acaagtttct gataccactg cactgtctga gaatttccaa aactttaatg 60

aactaactgn cnncttcatg aaactgtcca ccaagatcaa gcagagaaaa taattaattt 120
catgggacta aatgaactaa tgaggataat attttcataa ttttttattt gaaattttgc 180
tganncttta aatgtcttgt ttcccagatt tcaggaaact tttttccttt taagctatcc 240
acagcttata gcaatttgat aaaatatact tttgtgaaca aaaattgaga catttacatt 300
ttctccctat gtggtcgctc cagacttggn aaactattca tgaatattta tattgtatgg 360
taatatagtt attgcacaag ttcaataaaa atctgctctt tgtataacag aatacatttg 420
aaaacattgg ttatattacc aagactttga ctaga 455

<210> 384
<211> 376
<212> DNA
<213> Homo sapien

<220>
<221> misc_feature
<222> (1)...(376)
<223> n = A,T,C or G

<400> 384
actcttgaat acaaggttct gatatcactg cactgtctga gaatttccaa aactttaatg 60
aactaactga cagcttcatg aaactgtcca ccaagatcaa gcagagaaaa taattaattt 120
catgggacta aatgaactaa tgaggataat attttcataa ttttttattt gaaattttgc 180
tgattcttta aatgtcttgt ttcccagatt tcaggaaact tttttttctt ttaagctatc 240
cacagcttac agcaatttga taaaatatac ttttgngaac aaaaattgag acatttacat 300
tttctcccta tgtgggcgct ccagacttgg gaaactattc atgaatattt atattgnatg 360
ggaatatagc attgcc 376

<210> 385
<211> 422
<212> DNA
<213> Homo sapien

<400> 385
acctgtgggt ttattaccta tgggtttata tcctcaaata cgacattcta gtcaaagtct 60
tggtaatata accaatgttt tcaaatgtat tctgtcatac aaagagcaga tttttattga 120
acttgtgcaa taactatatt accatacaat ataaatattc atgaatagtt tcccaagtct 180
ggagcgacca catagggaga aaatgtaaat gtctcaattt ttgttcacaa aagtatattt 240
tatcaaattg ctgtaagctg tggatagctt aaaagaaaaa aagtttcctg aaatctggga 300
aacaagacat ttaaagaatc agcaaaattt caaataaaaa attatgaaaa tattatcctc 360
attagttcat ttagtcccat gaaattaatt attttctctg cttgatcttg gtggacagtt 420
tc 422

<210> 386
<211> 313
<212> DNA
<213> Homo sapien

<400> 386
caagtaggtc tacaagacgc tacttcccct atcatagaag agcttatcac ctttcatgat 60
cacgccctca taatcatttt ccttatctgc ttcctagtcc tgtatgccct tttcctaaca 120
ctcacaacaa aactaactaa tactaacatc tcagacgctc aggaaataga aaccgtctga 180
actatcctgc ccgccatcat cctagtcctc atcgccctcc catccctacg catcctttac 240
ataacagacg aggtcaacga tccctccctt accatcaaat caattggcca ccaatggtac 300
tgaacctacg agt 313

<210> 387
<211> 236
<212> DNA
<213> Homo sapien

<400> 387
cgccctcata atcattttcc ttatctgctt cctagtcctg tatgcccttt tcctaacact 60
cacaacaaaa ctaactaata ctaacatctc agacgctcag gaaatagaaa ccgtctgaac 120
tatcctgccc gccatcatcc tagtcctcat cgccctccca tccctacgca tcctttacat 180
aacagacgag gtcaacgatc cctcccttac catcaaatca attggccacc aatggt 236

<210> 388
<211> 195
<212> DNA
<213> Homo sapien

<400> 388
acgccctttt cctaacactc acaacaaaac taactaatac taacatctca gacgctcagg 60
aaatagaaac cgtctgaact atcctgcccg ccatcatcct agtcctcatc gccctcccat 120
ccctacgcat cctttacata acagacgagg tcaacgatcc ctcccttacc atcaaatcaa 180
ttggccacca atggt 195

<210> 389
<211> 183
<212> DNA
<213> Homo sapien

<220>
<221> misc_feature
<222> (1)...(183)
<223> n = A,T,C or G

<400> 389
taacactcac aacaaaacta actaatacta nnatctcaga cgctcaggaa atagaaaccn 60
cctgaactat cctgcccgcc atcatcctag tcctcatcgc cctcccatcc ctacncatcc 120
tttacataac agacgaggtc aacgatccct cccttaccat caaatcaatt ggccaccaat 180
ggt 183

<210> 390
<211> 473
<212> DNA
<213> Homo sapien

<400> 390
acaaagcagc aactgcaata ctcaaggtta aaacattaga aaagcatttg tgtgacaggt 60
atattacagt attatcaaaa tattacattt tcagacttac ttagcagata atcatccacc 120
agagcttaaa tctttaaatt atttccatag tcttaaaaaa tatgtaatgt cagaatgcat 180
ataaaaagaa tgtaaaagga aacctaaaat acaaatggaa taatgtaaca aataaatatt 240
tgatttcagt aactgttaat aatcagctca acaccaccat tctctctaaa ctcaatttaa 300
ttcttatagg aataatgaac tgtcaaatgc catggcataa ttatttattt ccaagctatc 360
atcaatgatt agaactaaaa aaaatttggc ataaaaaaat cacaattcag cataaataaa 420
gctattttta gcttcaacac tagctagcat ctctaagaat tgttgaaata agt 473

<210> 391
<211> 216

<212> DNA
<213> Homo sapien

<220>
<221> misc_feature
<222> (1)...(216)
<223> n = A,T,C or G

<400> 391
atttgtattt taggtttcct tttacattct ttttatatgc nntctgacat tacatatttt 60
ttaagactat ggaaataatt taaagattta agctctggtg gatgattatc tgctaagtaa 120
gtctgaaaat gtaatatttt gataatactg taatatacct gtcacacaaa tgcttttcta 180
atgttttaac cttgagtatt gcagttgctg ctttgt 216

<210> 392
<211> 98
<212> DNA
<213> Homo sapien

<400> 392
acttatttca acaattctta gagatgctag ctagtgttga agctaaaaat agctttattt 60
atgctgaatt gtgatttttt tatgccaaat tttttttaa 98

<210> 393
<211> 397
<212> DNA
<213> Homo sapien

<400> 393
tgccgatata ctctagatga agttttacat tgttgagcta ttgctgttct cttgggaact 60
gaactcactt tcctcctgag gctttggatt tgacattgca tttgaccttt tatgtagtaa 120
ttgacatgtg ccagggcaat gatgaatgag aatctacccc cagatccaag catcctgagc 180
aactcttgat tatccatatt gagtcaaatg gtaggcattt cctatcacct gtttccattc 240
aacaagagca ctacattcat ttagctaaac ggattccaaa gagtagaatt gcattgaccg 300
cgactaattt caaaatgctt tttattatta ttatttttta gacagtctca ctttgtcgcc 360
caggccggag tgcagtggtg cgatctcaga tcagtgt 397

<210> 394
<211> 373
<212> DNA
<213> Homo sapien

<220>
<221> misc_feature
<222> (1)...(373)
<223> n = A,T,C or G

<400> 394
ttacattgtt gagctattgc tgttctcttg ggaactgaac tcactttcct cctgaggctt 60
tggatttgac attgcatttg accttttatg tagtaattga catgtgccag ggcaatgatg 120
aatgagaatc tacccccaga tccaagcatc ctgagcaact cttgattatc catattgagt 180
caaatggtag gcatttccta tcacctgttt ccattcaaca agagcactac attcatttag 240
ctaaacggat tccaaagagt agaattgcat tgaccacgac tantttcaaa atgcttttta 300
ttattattat tttttagaca gtctcacttt gtcgcccagg ccggagtgca gtggtgcgat 360
ctcagatcag tgt 373

<210> 395
<211> 411
<212> DNA
<213> Homo sapien

<220>
<221> misc_feature
<222> (1)...(411)
<223> n = A,T,C or G

<400> 395
actgatcatt ctatttcccc ctctattgat ccccacctcc aaatatctca tcaacaaccg 60
actaatcacc acccaacaat gactaatcaa actaacctca aaacaaatga taaccataca 120
caacactaaa ggacgaacct gatctcttat actagtatcc ttaatcattt ttattgccac 180
aactaacctc ctcggactcc tgcctcactc atttacacca accacccaat tatctataaa 240
cctagccatg gccatcccct tatgagcggg cgcagtgatt ataggctttc gctctaagat 300
taaaaatgcc ctagcccact tcttacngca aggcacacct acacccctta tccccatact 360
agttattatc gaaaccatca gcctactcat tcaaccaata gccctggccg t 411

<210> 396
<211> 411
<212> DNA
<213> Homo sapien

<400> 396
actgatcatt ctatttcccc ctctattgat ccccacctcc aaatatctca tcaacaaccg 60
actaattacc acccaacaat gactaatcaa actaacctca aaacaaatga tagccataca 120
caacactaaa ggacgaacct gatctcttat actagtatcc ttaatcattt ttattgccac 180
aactaacctc ctcggactcc tgcctcactc atttacacca accacccaac tatctataaa 240
cctagccatg gccatcccct tatgagcggg cgcagtgatt ataggctttc gctctaagat 300
taaaaatgcc ctagcccact tcttaccaca aggcacacct acacccctta tccccatact 360
agttattatc gaaaccatca gcctactcat tcaaccaata gccctggccg t 411

<210> 397
<211> 351
<212> DNA
<213> Homo sapien

<220>
<221> misc_feature
<222> (1)...(351)
<223> n = A,T,C or G

<400> 397
ngccgangta caaaaaaaag cacattccta gaaaaaggta ttggcaaata gtaaaaatgg 60
gaggtcaaaa ncaaaaaaaa aaaaaacaaa acnaaaaaaa gaaaaaacca acaattcttc 120
aattcagtgt gcaaacatta tataaaaata gaaatactaa ctctacaggc agtatttcct 180
gataaattat ttaaatagca tatctacnca atctgagata tctattccaa tggcaatgag 240
aaaataattt ataaaaataa agcaatggta taccanatga tagaaaaaaa cataactttc 300
agaaattgta tttaacattt caatgctatt tccttattgn gaatncttct c 351

<210> 398
<211> 363
<212> DNA

<213> Homo sapien

<400> 398
acaaaaaaaa gcacattcct agaaaaaggt attggcaaat agtaaaaatg ggaggtcaaa 60
agcaaaaaa aaaaaacaa aacaaaaaa agaaaaacc aacaattctt caattcagtg 120
tgcaaacatt atataaaaat agaaatacta actctacagg cagtatttcc tgataaatta 180
tttaaatagc atatctacac aatctgagat atctattcca atggcaatga gaaaataatt 240
tataaaaata aagcaatggt ataccagatg atagaaaaaa acataacttt cagaaattgt 300
atttaacatt tcaatgctat ttccttattg ggaatacttc tctgcagagt ttttatgcta 360
tgt 363

<210> 399
<211> 360
<212> DNA
<213> Homo sapien

<400> 399
actgtttcct cgtggttcag gggtgtgcat gaaggctctt aggagagcaa acacctgttc 60
ctattctgta tgtccctccc tcatttcaaa tgagagtaac caattgagta aaataaccaa 120
ataaccattg ccccaccatg aacatggggc ttgggaagac agtcctacaa tcttcatcat 180
atatttaggt ttttaggcca gccagctctt tttttccaaa gctttctttt gaatacccgc 240
ccgggcggcc cctaagggcg aattctgcag atatccatca cactggcggc cgctcgagca 300
tgcatctaga gggcccaatt cgccctatag tgagtcgtat tacaattcac tggccgtcgt 360

<210> 400
<211> 87
<212> DNA
<213> Homo sapien

<220>
<221> misc_feature
<222> (1)...(87)
<223> n = A,T,C or G

<400> 400
ctgcacatat cnattacact ggcggccgct cgagcatgca tgnagagggc ccaattctcc 60
ctatattgag tggaattaca atncnct 87

<210> 401
<211> 328
<212> DNA
<213> Homo sapien

<220>
<221> misc_feature
<222> (1)...(328)
<223> n = A,T,C or G

<400> 401
acccagggac acaaacactc tgcctaggaa aaccagagac ctttgttcac ttgtttatct 60
gctgaccttc cttccactat tgtcctatga ccctgccaaa tccccctctg cgagaaacac 120
ccaagaatga tcaataaaaa ataaaataaa attaaattaa aaaaaaaaaa agagaggaac 180
ccacaaaaaa aaaaaaaaag aaagtntata aaataaaata ttgaagtcct ttcccattaa 240
aaaaaaaaaa aagaaaaagc acggactctt tcatccagtt ctgatgtgat tatctctgga 300
aggcattttc tcctcctctt ccctcccc 328

<210> 402
<211> 268
<212> DNA
<213> Homo sapien

<220>
<221> misc_feature
<222> (1)...(268)
<223> n = A,T,C or G

<400> 402
nacataatga caacatcttc actagactga gtgttcaagg atttgagatg attcgctatt 60
catcacaccc cgaagattga gatccactgt atttacacaa agcaaagcca tgtcagcaag 120
ggactgtcaa cctgattctg agaacataaa cattcaaaat ttattttcca gtgttccttt 180
ttggaaacca acaacacatc tttaatacct acacacacac acatctntac ctttaaaaaa 240
aaaaaaaaag tgnaacttca cagatagt 268

<210> 403
<211> 538
<212> DNA
<213> Homo sapien

<400> 403
acagtgatag ctccccctgg gcaatacaat acaagaacag tgggttttgt caaattggaa 60
caaggaaaca gaaccacaga aataaataca ttggttaaca tcagattagt tcaggttact 120
tttttgtaaa agttaaagta gaggggactt ctgtattatg ctaactcaag tagactggaa 180
tctcctgtgt tctttttttt tttaaattgg ttttaatttt ttttaattgg atctatcttc 240
ttccttaaca tttcagttgg agtatgtagc atttagcacc actggctcaa tgcgctcacc 300
taggtgagag tgtgaccaaa tcttaaagca ttagtgctat tatcagttac caccatttgg 360
ggcttttatc cttcatgggt tatgatgttc tcctgatgac acatttctct gagttttgta 420
attccagcca aagagagacc attcactatt tgatggctgg ctgcatgcag acatttaaag 480
cttttagaga atacactaca ccagggagta tgactactag tatgactatt aggagggt 538

<210> 404
<211> 310
<212> DNA
<213> Homo sapien

<400> 404
   tttttttata gatacaattg gcttttattt gtgattcatg agtcagggca gtttccattc 60
   tgcaaaatat agtgatagct cctactgggc aatacaacag tagaacagtg ggttttgtaa 120
   aatgggaatc caggaacaga agaatataaa taaattgatt taaataaact gattggttaa 180
   tttcagaata cttcatatta cttttttcta agagttaaag cagaaaggac tttcttactg 240
   tgctgactca gacagcctgg actctcatgt ttttaggaaa attttgtctg ttctgggatc 300
   tacctgcttc 310

<210> 405
<211> 559
<212> DNA
<213> Homo sapien

<400> 405
acaaatcaca attattaact cactggtagg gcagtgatga tcaaaccaat tgcattcatc 60
catgctgtaa tgttctctct tggcactaaa ggctgactgc agccggcaaa aaagaatgta 120

agtatgaatt tataaaaaca ttttagatgg ctgacaacgg atcttatttt taaagaatat 180
gtctaattca gaggatcgac aactaatcca tttcaataaa acaatgggga attttttatt 240
gaataaaaat gtaatatgca taaaaactca agaaggcttt ttaaaaatac ttcctcccca 300
atcattatcc catacttcat gctaattttt aaaagaatct tgaaatcttg aaaacaagat 360
gaagagaatc ttgttttaag tgacaagtta acattattcc tatattaaat gtcaaactgc 420
tattaatgag tagaagtagg aacaaacccg gatcttagga tcctgtccag ggctcattcc 480
ataactccta tatcacaaag acaagatctg gaaccagaaa acagtcatca tccaatgtgc 540
atcagccttg cggcaacag 559

<210> 406
<211> 427
<212> DNA
<213> Homo sapien

<400> 406
acaacagaat atctcgggaa tggactcaga agtatgccat gtgatgctac cttaaagtca 60
gaataacctg cattatagct ggaataaact ttaaattact gttccttttt tgatttcttt 120
atccggctgc tcccctatca gacctcatct tttttaattt tattttttgt ttacctccct 180
ccattcattc acatgctcat ctgagaagac ttaagttctt ccagctttgg acaataactg 240
cttttagaaa ctgtaaagta gttacaagag aacagttgcc caagactcag aatttttaaa 300
aaaaaaaatg gagcatgtgt attatgtggc caatgtcttc actctaactt ggttatgaga 360
ctaaaaccat tcctcactgc tctaacatgc tgaagaaatc atctgagggg gagggagatg 420
gatgctc 427

<210> 407
<211> 419
<212> DNA
<213> Homo sapien

<400> 407
acaatttgta gttgtttcca ggtttggcta ataatcattc cttaacctag aattcagatg 60
atcctggaat taaggcaggt cagaggactg taatgataga attaaattag tgtcactaaa 120
aactgtccca aagtgctgct tcctaatagg aattcattaa cctaaaacaa gatgttacta 180
ttatatcgat agactatgaa tgctatttct agaaaaagtc tagtgccaaa tttgtcttat 240
taaataaaaa caatgtagga gcagcttttc ttctagtttg atgtcattta agaattacta 300
acacagtggc agtgttaaat gaagatgctg tctacaaggt agataatata ctgtttgata 360
ctcaaaacat ttttcatttt gtttaaagta gaagttacat aattctatat tttaagtct 419

<210> 408
<211> 523
<212> DNA
<213> Homo sapien

<220>
<221> misc_feature
<222> (1)...(523)
<223> n = A,T,C or G

<400> 408
acatttgatg ttatgtgaat gttgagtttt tttcttctaa ttttcacttc agcagtgttt 60
agggctttca gatgccttat tccagtgtga acagaaaaag ttcatatttt atgtggttaa 120
tgctttgatg tgtcacataa agagtagttt gtagaaaatg ttggcacaat tttaacttct 180
tagtggcttg tgacattata tattatatat atatgtatat atatctttat aacattcctg 240
tgtttagtag tgtaaatgtt ctgggcaagt tttaatattt tgaatgcctt tggatattcc 300
agcaataaag gcatcatgtt ctgcaatagg atttcttact catttaccta ttttaacact 360

aaaatagacc acaactgagc acaaattcct tttataaatg ttatagaagc agggaagaat 420
aataaacaca tttgtgaatt gtggttcagt ttatttatct ttagggaagg ctgatcattt 480
atcttatagc acataacccc agcctcttat tcattatggn taa 523

<210> 409
<211> 191
<212> DNA
<213> Homo sapien

<220>
<221> misc_feature
<222> (1)...(191)
<223> n = A,T,C or G

<400> 409
accccgtagt gatgagcact gactggttca ctggccacat tttagttctt cataataata 60
ggccacaaaa gggctctgtg gtttgcctcc atgtgcactg gcccctcccc acccctaggg 120
ggcactcagt agctgctgag aaggcctgtc cacgangctg ttggaacccc ttcaataaat 180
acttagaagn a 191

<210> 410
<211> 403
<212> DNA
<213> Homo sapien

<220>
<221> misc_feature
<222> (1)...(403)
<223> n = A,T,C or G

<400> 410
acactggcca gtgtgttttt ggcgattaaa cataatcctg tgaatcagat taattcactt 60
gctgagtgtt catttgcggc atccctctgt tgggtcttgg gggccctcca cgacctcgtg 120
gggctccccg tggtccactc tgcccagagc ctcgcttgaa attctgctga tatccatccc 180
gttgatagcc agagtaatcc cggggagcac tgaactgaga ctgtgtataa ccactgtttg 240
gagtgttaga gaatgaaggg cggtaaccat catatcctcc tctgaatcca ttggcagggc 300
cccggtatcc attcatcaag cctctagcac cacgggagcc tccacgagac acaccacgac 360
tattgtaata gggctgattg ctacgtggaa atccagtgnt ctg 403

<210> 411
<211> 384
<212> DNA
<213> Homo sapien

<400> 411
acgtgaaatc ataacaacat gttctcttgt gtttggcttc tcttgctcag catgatattt 60
ttacggttca cccatattgc atgtatcagg aatataatcc tttttattat tgagtagtgt 120
tctattgtat gtatatacca cagtttattt ctcccttcat cctttgctag attttggggt 180
tttttcacat tgcgctattc aagtataaac ctgctctcaa cattcatgtg caagtctttg 240
agtggacata tatttgccgt ttctcttgag tgaatgcacc ttgttgggtc acgtggctta 300
atttaaaaaa attttaatca ctgtggtgca tatgtagtga ttattagtga ttatctcata 360
attttatttt cttgatgact aatg 384

<210> 412
<211> 315

<212> DNA
<213> Homo sapien

<220>
<221> misc_feature
<222> (1)...(315)
<223> n = A,T,C or G

<400> 412
acaatatttc tcctttgaga agataggata tatgattttc ccaaaaatca caactttgaa 60
ggaagactta nttgctgact tcaattatat cctggaactg gcaacttgtg cccttccttt 120
gcttcaaaaa aagtgtaaga aagagtgata agatcaactt taatcattct tggatcttca 180
gcaaattcag gatcaatgta gaaaaacact ggcatatcta cttcctcttg gggattaagc 240
ctttgttctt caaaacagaa gcactgtatt ttattgaaat actgtccacc ttcaaatgga 300
acaatattgt atgna 315

<210> 413
<211> 554
<212> DNA
<213> Homo sapien

<220>
<221> misc_feature
<222> (1)...(554)
<223> n = A,T,C or G

<400> 413
acaggtttca ctattacaaa tatatgatgt taaactaaca aactcatgac cttcaaagat 60
gtcttcgtcc cacgcacaca catttgtaat ttgtgtccat ttgctatttc ccttcttcta 120
taatcttcaa attatatagt tatgcattga gttccctatg catctcaccc atctccttta 180
tctcagcctt ctcatacttt gccattctct tctttctgga aataaccagc acaacaattc 240
cagcaacaac tgctatcacc acaaccacaa taacagcaat aacaccagct tttagaccct 300
gcattgagaa ttcaggtgct ttttcatcaa cataataaat taaagtttga ccaggatcca 360
gatccagttg ttccccattt actgtcaggt gccattttct tagaatgaaa caaggattca 420
cctttaacat ctttttcaaa ataataagcc acatcagcta tgtccacatc attctgagnt 480
ttttgagaag aattttgaac cagatcaata gtgataacat tattctcata caaaatactc 540
gngataaatt ntgg 554

<210> 414
<211> 267
<212> DNA
<213> Homo sapien

<400> 414
accagaaagg cacacgattt tacaatattt gttggaatta ccttactttt taacctcctc 60
atagcagttt tggtttgagt atattgatga aagccaaagt ctggtatcta aaacttgggc 120
caatgtttcc caactggtat atgtcaggct ttcccaatag cttaactgtg accctatacg 180
gatggctttt tagatagttc tatactgctg tattgtgtta gcacttttct ttgtcattaa 240
caacacactt taaatgacat ttggtga 267

<210> 415
<211> 454
<212> DNA
<213> Homo sapien

<400> 415
accggaacct gcagaaacag tgtgagaaat taagtcctgg ttcactgcgc agtagcaaag 60
atggtcaagg ccatggaaaa agcagaaatt taccaagaaa gctgataccc atgtatagtt 120
cccactcatc tcaaatacat ctgctatctt tttaagctaa gtcctagaca tatcggggat 180
aacatggggg ttgattagtg accacagtta tcagaagcag agaaatgtaa ttccatattt 240
tatttgaaac ttattccata ttttaattgg atattgagtg attgggttat caaacaccca 300
caaactttaa ttttgttaaa tttatatggc tttgaaatag aagtataagt tgctaccatt 360
ttttgataac attgaaagat agtattttac catctttaat catcttggaa aatacaagtc 420
ctgtgaacaa ccactctttc acctagcagt atga 454

<210> 416
<211> 370
<212> DNA
<213> Homo sapien

<400> 416
ccgacacggt gccagcgccc tgctgcgtgc ccgccagcta caatcccatg gtgctcattc 60
aaaagaccga taccggggtg tcgctccaga cctatgatga cttgttagcc aaagactgcc 120
actgcatatg agcagtcctg gtccttccac tgtgcacctg cgcggaggac gcgacctcag 180
ttgtcctgcc ctgtggaatg ggctcaaggt tcctgagaca cccgattcct gcccaaacag 240
ctgtatttat ataagtctgt tatttattat taatttattg gggtgacctt cttggggact 300
cgggggctgg tctgatggaa ctgtgtattt atttaaaact ctggtgataa aaataaagct 360
gtctgaactg 370

<210> 417
<211> 463
<212> DNA
<213> Homo sapien

<400> 417
acactttata tattccaaat tgatcagata tatggtttgc aaattcatct caatctgtag 60
cttatctttt cctcttctta aatcacaagt ttttaaattt tgaagaagtc caatatatca 120
gattttgtct tttatggatg tgctttcggg gcaaagtcca agaacttgtc acctagccca 180
agatcctgaa gatttttctc ctgtggcttt tttcaaagtt atctagtttt atgtatcaca 240
tttaagtccg ttatacattt tgagttaaat tttatataag atgtgaggtt taagtagagg 300
ttcttttttc tcctcgccat gggtgtctaa ttgctctagc ataatttgtc agaaaggcta 360
ttcttcctcc attgaattgc tttttcactt tttcaaaatc agctgagcat atttatatgg 420
gtttatttct gggttctctc atctgttcca ttgacgtatg tgt 463

<210> 418
<211> 334
<212> DNA
<213> Homo sapien

<400> 418
   ttagcatttg cttttatttt tttactttga tgccttttca aattggcatg tctttaaagt 60
   atttttcttc ctgattaaaa atgtgtgtgt atgtgtgtgt gtgtgtgtat atatatattt 120
   ttttaaatca cattaatttt accaagtgaa accaagccat actgtttttg agccaattaa 180
   gaaaattgcc atttttaaag tgtagcattt cagggtaaag acccatgaaa tggcttgatg 240
   tattctagac tactgaaaga aaaccacttc aaagattttg ttgaaagttt tagtgttgtc 300
   tgaaatgcaa gagggaaggt gattggtagt gagt 334

<210> 419
<211> 297
<212> DNA

<213> Homo sapien

<400> 419
acttctttga ccaaggaata ccacagacac cctaccgata gaacagtggc tcagatctta 60
cttgctcctg cttacgaagt attcccaatc actggtcatc tgaccctact tgaacactcc 120
tgaacagtca tgttttttaa aatcttcctt tatatcaagt cagagagtat acttctataa 180
atttcactca tggatgttag gaaatctagt catcttccct gtgattgccc tgttaagtat 240
ttaaccatag ctatcatgtg tttcccaaat cttctctaga ttaaatatct tcagtta 297

<210> 420
<211> 418
<212> DNA
<213> Homo sapien

<400> 420
acgagaggaa ccgcaggttc agacatttgg tgtatgtcct atcaatagga gctgtatttg 60
ccatcatagg aggcttcatt cactgatttc ccctattctc aggctacacc ctagaccaaa 120
cctacgccaa aatccatttc gctatcatat tcatcggcgt aaatctaact ttcttcccac 180
aacactttct cggcctatcc ggaatgcccc gacgttactc ggactacccc gatacataca 240
ccacatgaaa tatcctatca tctgtaggct cattcatttc tctaacagca gtaatattaa 300
taattttcat gatttgagaa gccttcgctt cgaagcgaaa agtcctaata gtagaagaac 360
cctccataaa cctggagtga ctatatggat gcccccaccc ctaccacaca ttcgaaga 418

<210> 421
<211> 304
<212> DNA
<213> Homo sapien

<400> 421
   acgcctggac ccctgtgact tgcagcctat ctttgatgac atgctccact ttctaaatcc 60
   tgaggagctg cgggtgattg aagagattcc ccaggctgag gacaaactag accggctatt 120
   cgaaattatt ggagtcaaga gccaggaagc cagccagacc ctcctggact ctgtttatag 180
   ccatcttcct gacctgctgt agaacatagg gatactgcat tctggaaatt actcaattta 240
   gtggcagggt ggttttttaa ttttcttctg tttctgattt ttgttgtttg gggtgtgtgt 300
   gtgt 304

<210> 422
<211> 578
<212> DNA
<213> Homo sapien

<400> 422
actgtgcagg cagattcaca gggtggtggt aaagcatcca caatggctct ggcagcatca 60
ggatcacact tgaaggggct ctcagacaaa gttgtattca tgcaactgat tccttttcca 120
ttcgttttct tagtcactaa tgctttccaa tggtcatgag tgcttttaat aatatcaatg 180
gcaaagtcct tatctttaaa ttctgcatta aacgcaaact cattttctgg ttttccatca 240
ggaaccttat accttctaaa ccagtccaca gtagcttcta agtagccagg tttcagccgt 300
ttgacatcat tgatatcatt ataattggct gcatcaggat catccacatt aatggcaatg 360
actttccagt cggtttcccc ttcgtcaatc atagccaata tgcctagaac tttcaattat 420
ttatttcacc tcttgcacat accttgcttc caatttcaca cacatcaatt gggtcattgt 480
caccacaaca gccagtatgt ttatcattgt gccctgggtc ttcccaagtc tgagggatgg 540
caccatagtt ccagatatat cctttatacg ggaacaaa 578

<210> 423
<211> 327

<212> DNA
<213> Homo sapien

<220>
<221> misc_feature
<222> (1)...(327)
<223> n = A,T,C or G

<400> 423
acagtatatt tttagaaact catttttcta ctaaaacaaa cacagtttac tttagagaga 60
ctgcaataga atcaaaattt gaaactgaaa tctttgttta aaagggttaa gttgaggcaa 120
gaggaaagcc ctttctctct cttataaaaa ggcacaacct cattggggag ctaagctagg 180
tcattgtcat ggtgaagaag agaagcatcg tttttatatt taggaaattt taaaagatga 240
tggaaagcac atttagcttg gtctgaggca ggttctgttg gggcagtgtt aatggaaagg 300
gctcactgnt gntactacta gaaaaat 327

<210> 424
<211> 384
<212> DNA
<213> Homo sapien

<400> 424
acgaaaaata aatctcctta aaaactaaat aaaatgcact gtattcttac agttaatgtt 60
tataactata gtaaaaaatt aatatatatc ctattacata aatgttattt cttaggtgtt 120
ccattaagaa gagcaataga ataatgctaa aaaataatgc ctataaatct tcagagtata 180
aagacatcca ttcagaaaca aaaattagca ctaaattttt tataaaatag accagatgac 240
aaaatttatt ttatttttaa acagtggttt tgacacaaat tatgttattg aaaagcatta 300
ttaatgttta atttatttaa aattttggaa tttgccattt ctcagagaat gatcaggcct 360
taggaaatta atacagtagt agta 384

<210> 425
<211> 255
<212> DNA
<213> Homo sapien

<400> 425
actatcaggc tttgtgctga tttcctgaac aaactgcatt atattatgaa aacaaaagga 60
aaagaagaaa taataaaaac tatactccca tatttcactt acagtgtttg agttcctgga 120
aggacctata taatggaggc agcattcaaa caagaaatta tgccaatcaa ctgtcaaatt 180
ttcactataa ttttcctaaa aaggcgtttt tcccccaata tctattaatc tcaaagaaac 240
ataagttgtg aatgt 255

<210> 426
<211> 196
<212> DNA
<213> Homo sapien

<220>
<221> misc_feature
<222> (1)...(196)
<223> n = A,T,C or G

<400> 426
acatgaantn nccaggccca cacagccaga cagcaacaga accaagacct agggctcttc 60
actcctgtta catcacacca tggcaatgat tttacattct ccaactgatt caaatcatat 120

ggcagctagg gatttggggg ctccatgttt tatttcaatt gcaagttcaa gatttctttt 180
tatctttgtg ggctga 196

<210> 427
<211> 163
<212> DNA
<213> Homo sapien

<400> 427
acagaagatc catggaggca agtgctgtca ggaaggacac tgcctccctc caccctccca 60
aatgtcacca ccaagttcct tcaggtgaga cctcacacaa tgtcaagtgc tttctaggaa 120
atactaagat caggttgaga gattctgctt ggtctagtca atc 163

<210> 428
<211> 315
<212> DNA
<213> Homo sapien

<220>
<221> misc_feature
<222> (1)...(315)
<223> n = A,T,C or G

<400> 428
nactgagtan agatgctggg gaatgtgcaa tatgccttga agaattgcag cagggagata 60
ctatagcacg actgccttgt ctatgcatat atcataaagg ctgcatagat gaatggtttg 120
aagtaaatag atcttgccct gagcaccctt cagattaagc gtcagcttcc tgttttatag 180
gttttcttgt cttgacaaga tgcttgaaaa accaagagga tatgaaaatc tgtctctgga 240
gaaacaaaga cgcaggcata ctcagccaga aatctgagtt ttgtgagact tggtaataca 300
gagatggaca atcgt 315

<210> 429
<211> 131
<212> DNA
<213> Homo sapien

<220>
<221> misc_feature
<222> (1)...(131)
<223> n = A,T,C or G

<400> 429
acagttaggn actagaacat ttgttaagcc tcccaaagta gngtgcatgg aagattctag 60
agtgtccagc tcttgcacta caaatgtaat aataacagaa taaatacact taccctgatg 120
atattgaggg t 131

<210> 430
<211> 503
<212> DNA
<213> Homo sapien

<400> 430
actgattttt aataaaagaa ataaggttca aagtttagca caacaacaca gcaataagaa 60
gctgacaact tggataaaaa tacaagaaag taacacagag cccaggctac ccattattta 120
ctgtgtgcat acaggaatgc tatacttcag atgtataaat tagagactga ttttaagtta 180

ttaatttaac tactttttgt ccactgtgct aaactaaatt ttatactaat gtgctactgc 240
gtaaacactt caaagcaatc ttcattaaaa tgctgcaaag aaaaacaaga atacacatca 300
tccaaaacta aggatgtcat tgcagttcac agtttgtata ataaataccc tccctttcaa 360
tcactactaa gatcactaca tcctatctac tcatcagcac aaccttgaag caacttatac 420
ttacaaatat tagcaatgca gccaaacatt tgttttttgc aaagcaacta gtaaaaatca 480
agaattttaa ttaagacggt gca 503

<210> 431
<211> 207
<212> DNA
<213> Homo sapien

<400> 431
acaagtgtgg cctcatcaag ccctgcccag ccaactactt tgcgtttaaa atctgcagtg 60
gggccgccaa cgtcgtgggc cctactatgt gctttgaaga ccgcatgatc atgagtcctg 120
tgaaaaacaa tgtgggcaga ggcctaaaca tcgccctggt gaatggaacc acgggagctg 180
tgctgggaca gaaggcattt gacatgt 207

<210> 432
<211> 485
<212> DNA
<213> Homo sapien

<220>
<221> misc_feature
<222> (1)...(485)
<223> n = A,T,C or G

<400> 432
aaaaaaagta atggaaaaat ggttgcaggt ttaatcncaa aangaactta attttngtng 60
attttgtttt atctgctaaa acactaatat ctataaatat gaactgacag catcgttcta 120
aatttacttc tgaagagctg tcgagacttc aataaaatat aagcaagtta ctggatcata 180
tttatggact gctgaattaa ctacccgaaa agtatcagtt actttcaaag aacacaaaac 240
aaagtgaacg tggaaaaaag ccttctttgc aaaagtcctt ttattagtcc tatcctctaa 300
aattccaagc cacagagcct tgatattcct ggattctgtt ttaagtaacc ttagttttaa 360
atatgacact tgggatatgc acaatgggaa agggtaggat atgtgaacaa aatttaattt 420
cttttttcca aaggnagnca ttttctttaa atncatccta tccacttttg cccacttccc 480
catgt 485

<210> 433
<211> 280
<212> DNA
<213> Homo sapien

<400> 433
actgtcacta caatattaca ttctgcaaat gttattctgt tgtatcagat acaaaatttt 60
agtgaggtat ctctaaggca catagtagaa aacaaaattg gttaattact caagttcctt 120
tcactgtgat ttggaaatga tttaatcttt atagaatgag aacctttttt ggactagctt 180
ttttattaaa atggctcaat ttgtgttgat aaggattgca ttaatattta atagtgcttg 240
cttttcctct gggcacacca ttttgatcat taaccagagt 280

<210> 434
<211> 234
<212> DNA
<213> Homo sapien

<400> 434
ctttgctgcg catcaggtgc tttaagcttc ggaacaactg tgcaggattc tattttagta 60
ttctggaagc atcattgagg aagtagtcca gtgaagttag ctctaaaaaa actctttact 120
ctaacaatta aaagaaatat gccaaaggat ccataaggga tgaataaatt attaaactat 180
taagaagttg ctataaatat gcagtgttaa ttcaataatt cataacggac tggt 234

<210> 435
<211> 330
<212> DNA
<213> Homo sapien

<400> 435
acctcccgtg tcaccagttc ccacagaagc actgcaaaac tccacatgtc tgctgagcgt 60
ctgtttgtgt cttcaggctt cttctgcaga gcttcggggg ctacccaggc aggtgcatac 120
atgcgaccag gacattggaa agagaacttg acatcagcca tgctaattcg ggcagtcatg 180
tcctcatcaa tcattacact acggctattg agtgcatgtc gtgggatgag gggctctagt 240
gtgtgtagga aagccatgcc ccttgccatg tccaaagcaa acttcacagc ctggctctgg 300
tccacgacga aattggtgcc ttcatgtagt 330

<210> 436
<211> 311
<212> DNA
<213> Homo sapien

<400> 436
acaactttac aatggaattg tatttcaatg attattttga tatcagatta aaccttccaa 60
aaagttacac ataattcagg tctatttttt ctaccagtaa gagttctgct aaattacaaa 120
accccataat cacagtgttc agtttttaaa aaattaaaca cacagtaatc ctgtcaatgt 180
taatcaaaat caaaacttcg gaatgccgtg gcatttatgt gaccaatctg agttttagat 240
acaaatacca gctgtttatc ccatgaacca tttttcctag gctgaggctg tgaaaaatcg 300
aaagtcggcg t 311

<210> 437
<211> 355
<212> DNA
<213> Homo sapien

<400> 437
actagtggat gggggtcagg gtgtcactcc aaggccctct acagacccag agaagaggaa 60
agtcaaaaaa gccagatatg agactgctga agtggtgtta agaaatatag gcaaggtaaa 120
gggaacaaga tctgggctcc ctcctacttg tgtccctcac tggacctcag acaccctacc 180
tctaagactg gttcttagaa ggctgaacag taaggagcat tccaatagct tctgaaactc 240
ccaaggctgt ttcaagtagt cgaaagccat ccctggactg ttcaggtgcc ttttctattt 300
cccacctgag ctctctgccc tttctttgag cctcacaggt ttccagaatt acagt 355

<210> 438
<211> 431
<212> DNA
<213> Homo sapien

<400> 438
acagtaactt taactttaca tagagctgag ataaaaataa agctttctta caaattacat 60
tttttttcca gtgaattact tttgcagtaa aaatagctgc tacataaatc cctcctgatc 120
tctgaaaagg agttgcatat ttccaaaaat aatattctta ttttaatcac acagaagaac 180

gtggagcaca ggaaggaaat ggctgggtgg tcagagagag gtgagctgtc ggagaaacac 240
agttaaacta aaaaataaaa tccattttgt gtataaactg acttaaacgc atgcaaagaa 300
gtggaaaaca tatgccattt gtcaagaaaa atactgcttt atagctttta ctttacaatt 360
aaaggagaaa gcagaggcca gatataagcc cagataataa catttaagtt tctcataaaa 420
ctcccaaatg t 431

<210> 439
<211> 170
<212> DNA
<213> Homo sapien

<400> 439
actgtcataa aaaacagtgg agctctgtat tagaaagccc ctcagaactg ggaaggccag 60
gtaactctag ttacacagaa actgtgacta aagtctatga aactgattac aacagactgt 120
aagaatcaaa gtcaactgac atctatgcta catattatta tatagtttgt 170

<210> 440
<211> 400
<212> DNA
<213> Homo sapien

<400> 440
acgtaaaaag aacatccttc ccatcttcaa ggtcaagatt gaacgctgac tcctgcagga 60
agtcttccag gattcccagg caggaatgat ggctccctgt ccctgtagct ccaggagttc 120
ttgcttcacg cacgcctcac ataccagact gaatgttggc aggaggagtg accaggtcgg 180
tcatctgtgt ccctaccacc tacaacaggc cagcaatcta cccgtgtgtg tttgttggac 240
agaattaacc atgatgggcg gccgagggcg cctggagcta tttgggggct tggagagaac 300
ctcttaggag agtgtcaggc tctaggccag tgtcaccaga ggaggtcagt ctcagtcctt 360
ggagtggtgg gatggaaacc agacgggact ggcatggtcc 400

<210> 441
<211> 204
<212> DNA
<213> Homo sapien

<400> 441
acctagttac ttcttaagat caggtgtata aaactgtgga gtggagcggt atggtatgga 60
atgacttgga atgtaagctg tcagggagaa aatgttgtta cacttttgct aagatctggg 120
ggtttcttca tattcctgct gttggaagca gttgaccaga aatgcttgcc agtactgcca 180
aagcactgct gtgaaatgtg aagt 204

<210> 442
<211> 649
<212> DNA
<213> Homo sapien

<400> 442
acatttaatt ttttacaaca ttttctccct agagatataa tttagatatt cctatcttca 60
aagtaaaaat caaaatagga aataagcata gaaacagcct attggcagtg gttacacctg 120
catggtattt atgagtctcc aaactattgg aaatttattt caaccaaggt tctcttaagt 180
cttcattact tgggtgtaac tcgagagaaa actaatttat atcaatttac agtttagtgg 240
tcatgatcag gggaaagtga tactcttcca ctgactacaa gtcattgcag aggcagttta 300
gaacttttcc tttattccta atatacagga caaaccttgc cgacatctca ctacctcaaa 360
aatcaaattt aaatgaagta tccaggagta gcctaaagaa tgagtgtaat ctggatggat 420
tttagtctaa atttatgcct tgctcttcag taaagtatag taactccaga tatatgttcc 480

acagatgcaa taatttctgt tccttgttcg gtgcagaata taatttatac ttcctgaaat 540
caactttgtc tattcatgaa aatagctgct ttttatttgc ctttgtctca ctttgaatat 600
atatgatcca caggttacag acttttccaa taactacatt tcaacttgt 649

<210> 443
<211> 346
<212> DNA
<213> Homo sapien

<400> 443
acgtgggatt gaaatgcaca tacatgtttt tgctaagagc acatacattt cattctcctc 60
actttgttca taacctcagc attgtcagat aacctcagtg agttaactca aagcctttta 120
ttatggaaag aactggcaca gttacatttg ccagtggcaa catccttaaa aattaataac 180
tgatgggtca cggacagatt tttgacctag ttcctttttc ttttagagca aaaagaactt 240
ttacctcggc atccagccca acccctaaag actgacaata tccttcaagc tcctttgaaa 300
gcaccctaaa cagccatttc cattttaata gttggatgcg gattgt 346

<210> 444
<211> 425
<212> DNA
<213> Homo sapien

<400> 444
accaatttcc ttttacagta aaggggcttt tcctgttgct tgttgaaccg gttcccagct 60
gcccattacc accaagccca aaagagtaaa ttcgtcctga tgaaggaaca aaagcagaag 120
tgtgctgccg tccacaagca atctcagtga caatgcttcc cataagttca aaaactttcc 180
ttgggtttat ttcatgactg gtagaattat ggcccaactg accataccct ccagctccaa 240
aagtaaacac tccaccttcc ttggttagag cagcagtatg atcttctcca caacaaatat 300
aaactatttt ctgagatctt agtgacttta gtaaattagg aacataccta tcattttcat 360
cattaagacc tagctgacca aacttgttgc gtccccatcc aaagatagct ccagaaaggg 420
tgagt 425

<210> 445
<211> 210
<212> DNA
<213> Homo sapien

<220>
<221> misc_feature
<222> (1)...(210)
<223> n = A,T,C or G

<400> 445
nactgtccca atataaaaca gtaattattt gacctttgca ctgtttgtct ggtccttttc 60
agtttgattg catataaatg tggaacttga tagatctcta tatttttaat gcacttgtga 120
taaactggca gcagggttag acattacttt caaagcttga ggtagaccga gtcagcatgc 180
tagacaggct tctctctcta accaaaactg 210

<210> 446
<211> 326
<212> DNA
<213> Homo sapien

<400> 446
tcgaaagacc cctgtaaaag agcccaacag tgaaaatgta gatatcagca gtggaggagg 60

cgtgacaggc tggaagagca aatgctgctg agcattctcc tgttccatca gttgccatcc 120
actaccccgt tttctcttct tgctgcaaaa taaaccactc tgcccatttt taactctaaa 180
cagatatttt tgtttctcat cttaactatc caagccacct attttatttg ttctttcatc 240
tgtgactgct tgctgacttt atcataattt tcttcaaaca aaaaaatgta tagaaaaatc 300
atgtctgtga gttcattttt aaatgt 326

<210> 447
<211> 304
<212> DNA
<213> Homo sapien

<220>
<221> misc_feature
<222> (1)...(304)
<223> n = A,T,C or G

<400> 447
ncntcnaggt acatgctaga agtctgatgt ngtnngtaac acagaaacat acacagtctt 60
catattcaaa gtcttcacng ggatgtcgtt ctgtaatttc ctgcgtttgg gtctcttcca 120
gaaacagctt tagcttcctg ctccgaaggc caaacacctt ggctgcttca tacagaagac 180
cttggtgggt gagtccattc tgcccaagtg ggttttcaag caggagagtg cccactgtcc 240
ccattaaaca ctcttgtggc tttgcattca ggagctgtag gttgatatac tgacaaggaa 300
gagt 304

<210> 448
<211> 203
<212> DNA
<213> Homo sapien

<400> 448
acatgaaagc ggcaatgcgg taaaaagcga attcttaccc aaggtcagaa ttttttatta 60
agcgcatttt cattagttgg acaaacaacc ttataaaccc ttatgtcaaa ccatataatg 120
tgaagaatct ccatgggaga gatttttttt cacccttcag aattatcttt ttcccctaag 180
accttcatat gaatcttcct tgt 203

<210> 449
<211> 481
<212> DNA
<213> Homo sapien

<220>
<221> misc_feature
<222> (1)...(481)
<223> n = A,T,C or G

<400> 449
acttgttcta taatactctg atgtttcctt aaattcctga acaacattct gtttactaaa 60
tttcttttct tcctttattc acaccaaatt ccaccctata atagaagcta attatttcag 120
aaagcttttt agtgatcatt tattactttg tgtttactag atattaattc taagatgaat 180
tcctttagaa ttttagaaaa aattattcta gacaacaatc aaagtaaagg atacatccag 240
cattgaaacc ataagccggc aagtctccag gttaaaaggt ttgtatcctc cagcaatgcc 300
agactgtgtc agacatctct gcaattcatc agcatctatc tgcccatcct gtccagctac 360
agcagcaaag taaccataca gcggatcctg agtttgtccg ggaaacgcag gccctccggg 420
agcccctcca tactgcatct tgagttgaag tcttatangt agaagctggt gatccttaga 480
g 481

<210> 450
<211> 296
<212> DNA
<213> Homo sapien

<400> 450
acatggttta atacaacaac aaaaaaattt aatcaagtga aacgtaataa actgaacaat 60
aaacactcaa aacattttcc attggaaaca tgtaaagaca atatgaggtt ttgttaccat 120
cttactgcaa ttttcttatg tgttactagt ctacataccc catgttttct gtaatcatgc 180
agatgtgaat ggaagtttga atgattaaat aaatgaaaag tccgtttact gcagggaatc 240
atttcacaag gcagccaaac cgggtttaga gaacaaaact attcaagaaa ttctcc 296

<210> 451
<211> 294
<212> DNA
<213> Homo sapien

<220>
<221> misc_feature
<222> (1)...(294)
<223> n = A,T,C or G

<400> 451
acatgntcca aggcacgcgn ctgtgaactt cctctgagtg aaggcatccc ctccagcacc 60
tttcagcctg ctagttagga cgacccgccg ccaccctcca ggacctccag ccctgcactg 120
cctttcctct cttttaaata attcttcatt gagttctaat atgtaaaaaa aaagtttact 180
gtaaagtttg caaataanga aatttttttt aaaagtcctc agtaatctta ccagtaacaa 240
ttgttatggg cacatttgct tttggaagat ttcttttgta tgcatgggat aagt 294

<210> 452
<211> 129
<212> DNA
<213> Homo sapien

<400> 452
acttttagat cacaaatttg cctttaagta acacataata cacttaaggc agatttgcct 60
tacaggtggc ctcagcttct aaacaccact acactgcttt atataaaaaa caaaaatcac 120
atagaagag 129

<210> 453
<211> 151
<212> DNA
<213> Homo sapien

<220>
<221> misc_feature
<222> (1)...(151)
<223> n = A,T,C or G

<400> 453
   actctcaann tgtatttagg tgccaacaca tttaggatca ttgngnnttc tcagtgaatt 60
   gaccttttta tgagaataaa atgtctattt ctgaaatgtc cctatttctg gaaatgttcc 120
   ttatactaaa gtccaacttg tgtggattan t 151

<210> 454
<211> 119
<212> DNA
<213> Homo sapien

<220>
<221> misc_feature
<222> (1)...(119)
<223> n = A,T,C or G

<400> 454
tgctgatgna gcatgctttt taaatccttt aaaaacactc accatataaa cttgcatttg 60
agcttgtgtg ttcttttgtt aatgtgtaga gttctccttt ctcgaaattg ccagtgtgt 119

<210> 455
<211> 515
<212> DNA
<213> Homo sapien

<400> 455
accttataaa gttccttttc atccttctct gtcttcaact gacattcaag ttgttctctt 60
tcatgttgtg ccttcttgag tttggccttt aaactgtcta attcggtttc tttttcaatt 120
gctttatgtg ttactgacac aatatcttcc tcaagctgat gggctttgga tgtagcatca 180
ctgaacctct tcttaaactc ttcattttcc atttttaagc tttgtgttac ttcagtaaga 240
cccttttgtt ctgcttgcag ttggtcacat ctttctttct catggttaag ttctctttcc 300
attctcccaa cttgttctcg aagttgtgct gtttcttttt ccagaacggc aattaacttt 360
aacagttctt cttttctttt catggttttc tcaattttca actcaagaag gcctgctttt 420
gtggtcacca ctaacatgtc agaatttcct tcatcttcca tagtaagcag ctcttcaact 480
ggagaagaag ctcgaaactg gaaaggtgta cctgc 515

<210> 456
<211> 350
<212> DNA
<213> Homo sapien

<220>
<221> misc_feature
<222> (1)...(350)
<223> n = A,T,C or G

<400> 456
actcccctcc ccaaatagaa acctcaaaga ctgatccatt tcccctaggg cctgggccag 60
gagtagctca ctgctcactg ctgaggagaa aggcacaaga tataatgtca taagagcagg 120
acagtggctc agcctacaga gttccctata ggggaaagaa ggcaggaaat aggcgcaggg 180
tctggtcctg tccctgcacc accctgagca gctagtcttg ggaagggatt acaggccctg 240
ggccataggc tgctcgccat tctgctttcc tatcctgttt ctctccctgt gctgctccct 300
tttagccagn gctgagaaat gttcancacc tgaggcaaaa ctgccatagt 350

<210> 457
<211> 293
<212> DNA
<213> Homo sapien

<400> 457
gcagggccaa cagtcacagc agccctgacc agagcattcc tggagctcaa gctcctctac 60

aaagaggtgg acagagaaga cagcagagac catgggaccc ccctcagccc ctccctgcag 120
attgcatgtc ccctggaagg aggtcctgct cacagcctca cttctaacct tctggaaccc 180
acccaccact gccaagctca ctattgaatc cacgccattc aatgtcgcag aggggaagga 240
ggttcttcta ctcgcccaca acctgcccca gaatcgtatt ggttacagct ggt 293

<210> 458
<211> 500
<212> DNA
<213> Homo sapien

<400> 458
actagactcc agattaccct ttcttaataa atatctcagg gtaaggaaag aaagaaactg 60
tatagatata tttaaaatag agaatacttt ccaagcaata catgatgcct ttcctaaaag 120
actctaaaag aaaaagattc tgtaactctc ttttagcacc aaattattgt ttatcttgct 180
ggatatttta tatgaacagt gttaatttag atgcactaaa gcaaaggtag gcaaactaca 240
accatgagtc aaacatggcc acacccattc atttgctatt gtctaagctg gttttgcact 300
acaactgcag agttgaatag atgcagcaga tcctttacag aaaaagtttt ctgacctcaa 360
ttctaaagta attgtagtag ggagctggag gactttcttt ccctttatgg taattttttg 420
agctacaaaa agagccttgc agaaatgggt gaagggatta atcttttaaa aataaatgct 480
atatattagg aaaataaaaa 500

<210> 459
<211> 394
<212> DNA
<213> Homo sapien

<400> 459
ggtgaaaaga cttgattttt tgaaaggatt gtttatcaaa cacaattcta atctcttctc 60
ttatgtattt ttgtgcacta ggcgcagttg tgtagcagtt gagtaatgct ggttagctgt 120
taaggtggcg tgttgcagtg cagagtgctt ggctgtttcc tgttttctcc cgattgctcc 180
tgtgtaaaga tgccttgtcg tgcagaaaca aatggctgtc cagtttatta aaatgcctga 240
caactgcact tccagtcacc cgggccttgc atataaataa cggagcatac agtgagcaca 300
tctagctgat gataaataca cctttttttc cctcttcccc ctaaaaatgg taaatctgat 360
catatctaca tgtatgaact taacatggaa aatg 394

<210> 460
<211> 279
<212> DNA
<213> Homo sapien

<220>
<221> misc_feature
<222> (1)...(279)
<223> n = A,T,C or G

<400> 460
actnccgatt gaagccccca ttcgtataat aattacatca caagacgtct tgcactcatg 60
agctgtcccc acattaggct taaaaacaga tgcaattccc ggacgtctaa accaaaccac 120
tttcaccgct acacgaccgg gggtatacta cggtcaatgc tctgaaatct gtggagcaaa 180
ccacagtttc atgcccatcg tcctagaatt aattccccta aaaatctttg aaatagggcc 240
cgtatttacc ctatagcacc ccctctagag caaaaaaaa 279

<210> 461
<211> 278
<212> DNA

<213> Homo sapien

<400> 461
tttggacact aggaaaaaac cttgtagaga gagtaaaaaa tttaacaccc atagtaggcc 60
taaaagcagc caccaattaa gaaagcgttc aagctcaaca cccactacct aaaaaatccc 120
aaacatataa ctgaactcct cacacccaat tggaccaatc tatcacccta tagaagaact 180
aatgttagta taaagtaaca tgaaaacatt ctcctccgca taagcctgcg tcagattaaa 240
acactggact gacaattaac agccaatatc tacaatca 278

<210> 462
<211> 556
<212> DNA
<213> Homo sapiens

<400> 462
aacgtccaag ggggccacat cgatgatggg caggcgggag gtcttggtgg ttttgtattc 60
aatcactgtc ttgccccagg ctccggtgtg actcgtgcag ccatcgacag tgacgctgta 120
ggtgaagcgg ctgttgccct cggcgcggat ctcgatctcg ttggagccct ggaggagcag 180
ggccttcttg aggttgccag tctgctggtc catgtaggcc acgctgttct tgcagtggta 240
ggtgatgttc tgggaggcct cggtggacat caggcgcagg aaggtcagct ggatggccac 300
atcggcaggg tcggagccct ggccgccata ctcgaactgg aatccatcgg tcatgctctc 360
gccgaacccg acatgcctct tgtccttggg gttcttgctg atgtaccagt tcttctgggc 420
cacactgggc tgagtggggt acacgcaggt ctcaccagtc tccatgttgc agaagacttt 480
gatggcatcc aggttgcagc cttggttggg gtcaatccag tactctccac tcttccagtc 540
agagtggcac atcttg 556

<210> 463
<211> 659
<212> DNA
<213> Homo sapiens

<400> 463
cacactgtgc ccttccagtt gctggcccgg tacaaaggcc tgaacctcac cgaggatacc 60
tacaagcccc ggatttacac ctcgcccacc tggagtgcct ttgtgacaga cagttcctgg 120
agtgcacgga agtcacaact ggtctatcag tccagacggg ggcctttggt caaatattct 180
tctgattact tccaagcccc ctctgactac agatactacc cctaccagtc cttccagact 240
ccacaacacc ccagcttcct cttccaggac aagagggtgt cctggtccct ggtctacctc 300
cccaccatcc agagctgctg gaactacggc ttctcctgct cctcggacga gctccctgtc 360
ctgggcctca ccaagtctgg cggctcagat cgcaccattg cctacgaaaa caaagccctg 420
atgctctgcg aagggctctt cgtggcagac gtcaccgatt tcgagggctg gaaggctgcg 480
attcccagtg ccctggacac caacagctcg aagagcacct cctccttccc ctgcccggca 540
gggcacttca acggcttccg cacggtcatc cgccccttct acctgaccaa ctcctcaggt 600
gtggactaga cggcgtggcc caagggtggt gagaaccgga gaaccccagg acgccctca 659

<210> 464
<211> 695
<212> DNA
<213> Homo sapiens

<400> 464
accttcattt gaccccatca gcttcagggc cttctttaca tttccactgg cctgatccat 60
gtatgcaatg ctatttttgc agtgatatgt gatgttctgg gaagctcggc tggagagaag 120
tcgaaggaat gccagctgca catcaaggac atcttcagga agttcaggat tgccgtagct 180
aaactgaaaa ccaccatcca tggactctcc aaaccaaacg tgtttcttct cagcactaga 240
atctgtccac cagtgtttcc gtggaacatt caaaggattg gcacttatgc atgtttcccc 300

agtttccata ttacagaata ccttgatagc atccaatttg catccttggt tagggtcaac 360
ccagtattct ccactcttga gttcaggatg gcagaatttc aggtctctgc agtttctagc 420
ggggttttta cgagaaccat caggactaat gaggctttct atttgtccat taacagactt 480
gagtgaagtc ataatctcat cggtgttgat tttgaaatcc attggttcat ctccataata 540
cggggcaaaa ccgccagctt tttcacctcc aatcccagca atggcagcgg ctccaacacc 600
accacagcaa ggaccagggg caccaggagg tccaggaggg cctggttgcc ctgggtggcc 660
tggggagccc tcagatcctc tttcacctct gttac 695

<210> 465
<211> 73
<212> DNA
<213> Homo sapiens

<400> 465
caggtccaga gctcccaggt ttccaggttg cagtccctcc agtcccagag ctcccagggt 60
ttcggtttcc agt 73

<210> 466
<211> 507
<212> DNA
<213> Homo sapiens

<220>
<221> misc_feature
<222> (1)...(507)
<223> n = A,T,C or G

<400> 466
agcactggca gaggnagcca aatatagtga tgtgcgccag agataagtat tctcctctcc 60
aagcatattg ctatacaaga ctttaaagac ttcataaaag ccaaacttgc agagtccctg 120
catggagtag ccaaggaaag tcggagccca tcctttagcc aaaccacgaa caccatcctc 180
tttaagtgta actgagaatc cgttaaatat gcccttgtac ttttgggggt ccacctgcat 240
acggcatttc actaaatcca ggggaaccac agcagtgtgt gtcagaccac aacttaagac 300
cccaccaaag ccacacagtg cataatactt cgcggagcca aattcacaac tgtactcttc 360
cacggcggcg gctgccaggt tgcgagggcg gcggggctgg cccgtgggcc ctggggagct 420
gctgcggagg tccccgagac catcgtgcac canctgcaga tgtggcgtgt tgaaggggtt 480
cgcccgcgcc aggtgcgcca cggacga 507

<210> 467
<211> 183
<212> DNA
<213> Homo sapiens

<400> 467
   cctcatgagc taccgggcca gctctgtact gaggctcacc gtctttgtag gggcctacac 60
   cttctgagga gcaggaggga gccaccctcc ctgcagctac cctagctgag gagcctgttg 120
   tgaggggcag aatgagaaag gcaataaagg gagaaagaaa aaaaaaaaaa aaaagggcgg 180
   ccg 183

<210> 468
<211> 129
<212> DNA
<213> Homo sapiens

<220>

<221> misc_feature
<222> (1)...(129)
<223> n = A,T,C or G

<400> 468
gcggccgcgt cgaccggcgc cgtcgggcnc cgggccgggc catggagctg tggacgtgtc 60
tggccgcggc gctgctgttg ntgntgctgn tggtgcagtt gagccgcncn gccgagttct 120
acnccaang 129

<210> 469
<211> 243
<212> DNA
<213> Homo sapiens

<220>
<221> misc_feature
<222> (1)...(243)
<223> n = A,T,C or G

<400> 469
gcggccgcgt cgacnggcca tggagactgt ggcacagtag actgtagtgt gaggctcgcg 60
ggggcagtgg ccatggaggc cgtgctgaac gagctggtgt ctgtggagga cctgctgaag 120
tttgaaaaga aatttcagtc tgagaaggca gcaggctcgg tgtccaagag cacgcagttt 180
gagtacgcct ggtgcctggt gcggagcaag tacaatgatg acatccgtaa aggcatcgtg 240
ctg 243

<210> 470
<211> 452
<212> DNA
<213> Homo sapiens

<400> 470
cctcaagtac gtccggcctg gtggtgggtt cgagcccaac ttcatgctct tcgagaagtg 60
cgaggtgaac ggtgcggggg cgcaccctct cttcgccttc ctgcgggagg ccctgccagc 120
tcccagcgac gacgccaccg cgcttatgac cgaccccaag ctcatcacct ggtctccggt 180
gtgtcgcaac gatgttgcct ggaactttga gaagttcctg gtgggccctg acggtgtgcc 240
cctacgcagg tacagccgcc gcttccagac cattgacatc gagcctgaca tcgaagccct 300
gctgtctcaa gggctcagct gtgcctaggg cgcccctcct accccggctg cttggcagtt 360
gcagtgctgc tgtctcgggg gggttttcat ctatgagggt gtttcctcta aacctacgag 420
ggaggaacac ctgatcttac agaaaatacc ac 452

<210> 471
<211> 168
<212> DNA
<213> Homo sapiens

<220>
<221> misc_feature
<222> (1)...(168)
<223> n = A,T,C or G

<400> 471
cttctccgct ccttctanga tctccgcctg gttcggnccg cctgcctcca ctcctgcctc 60
taccatgtcc atcaggtgga cccagaagtc ctacaaggtg tccacctctg gcccccgggc 120
cttcagcagc cgctcctaca cgagtgggcc cggttcccgc atcagctc 168

<210> 472
<211> 479
<212> DNA
<213> Homo sapiens

<220>
<221> misc_feature
<222> (1)...(479)
<223> n = A,T,C or G

<400> 472
gccaggcgtc cctctgtctg cccactcagt ggcaacaccc gggagctggt ttgtcctttg 60
tggagcctca ncagttccct ctttcanaac tcactgccaa gagccctgaa caggagccac 120
catgcagtgc ttcagcttca ttaagaccat gatgatcctc ttcaatttgc tcatctttct 180
gngtggcgca gccctgttgg cagcgggcat ctgggtgnca atcgatgggg catcctttct 240
gaagatcttc gggccactgt cgtccactgc catgcagttt gtcaacgngg gctacttcct 300
catcgcagcc ggcgttgtgg tntttgctct tggtttcctg ggctgctatg gtgctaanac 360
tgagagcaag tgtgccctcg tgacgntctt cttcatcctc ctcctcntct tcattgctga 420
ggntgcagnt gctgaggtcc gccttggtgt acaccacaat ggctgagccc ttnctgacn 479

<210> 473
<211> 69
<212> DNA
<213> Homo sapiens

<400> 473
gagcgatgga gcgtgggtag ggagggtcca cagtgtccac tcgccgtgtg cgaaggttga 60
ctcggtagt 69

<210> 474
<211> 155
<212> DNA
<213> Homo sapiens

<400> 474
gccgccactg ccgggagagc tcgatgggct tctcctgcgc gccgcccggt gtctggccga 60
gtccagagag ccgcggcgcc tcgttccgag gagccatcgc cgaagcccga ggccgggtcc 120
cgggttgggg actgcagggg aaggcagcgg tggcg 155

<210> 475
<211> 282
<212> DNA
<213> Homo sapiens

<400> 475
ggcttcgacg ttggccctgt ctgcttcctg taaactccct ccatcccaac ctggctccct 60
cccacccaac caactttccc cccaacccgg aaacagacaa gcaacccaaa ctgaaccccc 120
tcaaaagcca aaaaatggga gacaatttca catggacttt ggaaaatatt tttttccttt 180
gcattcatct ctcaaactta gtttttatct ttgaccaacc gaacatgacc aaaaaccaaa 240
agtgcattca accttaccaa aaaaaaaaaa aaagggcggc cg 282

<210> 476
<211> 434
<212> DNA

<213> Homo sapiens

<400> 476
ctccaggaca gcgtccagct tggtgtcgtt gaagacgaag tggagcggat ggttgtagaa 60
acgagtgatg gtgctgagcg gcgtgcagtc ttcgggatcc acgaaggcca agtccttgag 120
gtagagcatg tccacgatgt tggagcgctc ctcctcgtac accgggatgc gcgtgtggcc 180
gctctgcatg atgctggcca ggacgccgaa gtccagcacg gtgctggcgt ccagcatgaa 240
gcagtcttcg aggggcgtga gcacgtcctc cacggtccgg cagcgcagca cgcccttgct 300
gagatcgctg taggggtcgc cgccgccgcg cgccagctcc agcacccgct cccgcagccg 360
cccgggccgc gccgccagct ccagcagctg ccccacgggc agcgcgacgg gcagagtgag 420
caggacggcc aggc 434

<210> 477
<211> 314
<212> DNA
<213> Homo sapiens

<400> 477
ggcgggcgct agctggctcc gggcagctcg gccttggggg cttcggggcc ccgagacgcg 60
gggcgtatga gtggggcgtg cgctccacgc ggaagtcgga gcctcctccc ctggataggg 120
tgtacgagat ccctggactg gagcccatca cctttgcggg gaagatgcac ttcgtgccct 180
ggctggcgcg gccgatcttt ccgccctggg accgcggcta caaggaccca aggttctacc 240
gctcgccccc tcttcacgag catccgctgt acaaagacca ggcctgctat atctttcacc 300
accgttgccg cctt 314

<210> 478
<211> 317
<212> DNA
<213> Homo sapiens

<400> 478
aacagagtga tcattccagt taagcggggc gaagagaata cagactatgt gaacgcatcc 60
tttattgatg gctaccggca gaaggactcc tatatcgcca gccagggccc tcttctccac 120
acaattgagg acttctggcg aatgatctgg gagtggaaat cctgctctat cgtgatgcta 180
acagaactgg aggagagagg ccaggagaag tgtgcccagt actggccatc tgatggactg 240
gtgtcctatg gagatattac agtggaactg aagaaggagg aggaatgtga gagctacacc 300
gtccgagacc tcctggt 317

<210> 479
<211> 171
<212> DNA
<213> Homo sapiens

<400> 479
aggtgctttg ctagatgctg tgacaggtat gccaccaaca ctgctcacag cctttctgag 60
gacaccagtg aaagaagcca cagctcttct tggcgtattt atactcactg agtcttaact 120
tttcaccagg ggtgctcacc tctgccccta ttgggagagg tcataaaatg t 171

<210> 480
<211> 65
<212> DNA
<213> Homo sapiens

<400> 480
cccccagtgg aaggctccca ccctggtaga tgaacagccc ctggagaact acctggatat 60

ggagt                                                              65

<210> 481
<211> 207
<212> DNA
<213> Homo sapiens

<400> 481
cacagcgtgc tctgcggggt cactcccact ttgttagtga tgtggttatc tcctcagatg 60
gccagtttgc cctctcaggc tcctgggatg gaaccctgcg cctctgggat ctcacaacgg 120
gcaccaccac gaggcgattt gtgggccata ccaaggatgt gctgagtgtg gccttctcct 180
ctgacaaccg gcagattgtc tctggat                                      207

<210> 482
<211> 319
<212> DNA
<213> Homo sapiens

<220>
<221> misc_feature
<222> (1)...(319)
<223> n = A,T,C or G

<400> 482
cacactgtgc ccttccagtt gctggcccgg tacaaaggcc tgaacctcac cgaggatacc 60
tacaagcccc ggatttacac ctcgcccacc tggagtgcct ttgtgacaga cagttcctgg 120
agtgcacgga agtcacaact ggtctatcag tccagacggg ggcctttggt caaatattct 180
tctgattact tccaagcccc ctctgactac agatactacc cctaccagtg cttccaaact 240
gcacaacacc cnagcttnct cttccagnac aagagggtgt cctggtccct ggcctacctc 300
cccaccatcc agagctgct                                               319

<210> 483
<211> 233
<212> DNA
<213> Homo sapiens

<220>
<221> misc_feature
<222> (1)...(279)
<223> n = A,T,C or G

<400> 483
acaggcccag tggcgcctag ccttcagctg ctgggctctc ccgagcctgc cttagcccat 60
acaaccactt gatcacgcgg gcattgcgct ccaccaccga cacgccatag ggaacgcgct 120
cccgggcccg ctcctcaaca gtcaccgagc tgcggcggga gcagccccct tcagagctgc 180
ccggcccagc actgggccct gccagggaca cnatatccga gctggcccgt gcc         233

<210> 484
<211> 194
<212> DNA
<213> Homo sapiens

<400> 484
agagcccttg ctggggggtg cctgggagat ggggtaagaa gagctttcat ttgtctggta 60
gatagatagc atgtaagggg gtggttgtcc caggaggcag ctgctgacag gtttgctaca 120

cacagccccg gactgtgttg cctgggtgct cattcagaga ggggctatca tctgggagcc 180
tgtgcccctg ggtc 194

<210> 485
<211> 67
<212> DNA
<213> Homo sapiens

<400> 485
tccatatcca ggtagttctc caggggctgt tcatctacca gggtgggagc ctcccactgg 60
gggaagt 67

<210> 486
<211> 70
<212> DNA
<213> Homo sapiens

<400> 486
taccgagtca accttcgcac acggcgagtg gacactgtgg accctcccta cccacgctcc 60
atcgctcagt 70

## Description

### TECHNICAL FIELD

The present invention relates generally to therapy and diagnosis of cancer, such as colon cancer. The invention is more specifically related to polypeptides comprising at least a portion of a colon tumor protein, and to polynucleotides encoding such polypeptides. Such polypeptides and polynucleotides may be used in vaccines and pharmaceutical compositions for prevention and treatment of colon cancer, and for the diagnosis and monitoring of such cancers.

### BACKGROUND OF THE INVENTION

Cancer is a significant health problem throughout the world. Although advances have been made in detection and therapy of cancer, no vaccine or other universally successful method for prevention or treatment is currently available. Current therapies, which are generally based on a combination of chemotherapy or surgery and radiation, continue to prove inadequate in many patients.

Colon cancer is the second most frequently diagnosed malignancy in the United States as well as the second most common cause of cancer death. An estimated 95,600 new cases of colon cancer will be diagnosed in 1998, with an estimated 47,700 deaths. The five-year survival rate for patients with colorectal cancer detected in an early localized stage is 92%; unfortunately, only 37% of colorectal cancer is diagnosed at this stage. The survival rate drops to 64% if the cancer is allowed to spread to adjacent organs or lymph nodes, and to 7% in patients with distant metastases.

The prognosis of colon cancer is directly related to the degree of penetration of the tumor through the bowel wall and the presence or absence of nodal involvement, consequently, early detection and treatment are especially important. Currently, diagnosis is aided by the use of screening assays for fecal occult blood, sigmoidoscopy, colonoscopy and double contrast barium enemas. Treatment regimens are determined by the type and stage of the cancer, and include surgery, radiation therapy and/or chemotherapy. Recurrence following surgery (the most common form of therapy) is a major problem and is often the ultimate cause of death. In spite of considerable research into therapies for the disease, colon cancer remains difficult to diagnose and treat. In spite of considerable research into therapies for these and other cancers, colon cancer remains difficult to diagnose and treat effectively. Accordingly, there is a need in the art for improved methods for detecting and treating such cancers. The present invention fulfills these needs and further provides other related advantages.

### SUMMARY OF THE INVENTION

Briefly stated, the present invention provides compositions and methods for the diagnosis and therapy of cancer, such as colon cancer. In one aspect, the present invention provides polypeptides comprising at least a portion of a colon tumor protein, or a variant thereof. Certain portions and other variants are immunogenic, such that the ability of the variant to react with antigen-specific antisera is not substantially diminished. Within certain embodiments, the polypeptide comprises a sequence that is encoded by a polynucleotide sequence selected from the group consisting of: (a) sequences recited in SEQ ID NO: 1-121, 123-197 and 205-486; (b) variants of a sequence recited in SEQ ID NO: 1-121, 123-197 and 205-486; and (c) complements of a sequence of (a) or (b).

The present invention further provides polynucleotides that encode a polypeptide as described above, or a portion thereof (such as a portion encoding at least 15 amino acid residues of a colon tumor protein), expression vectors comprising such polynucleotides and host cells transformed or transfected with such expression vectors.

Within other aspects, the present invention provides pharmaceutical compositions comprising a polypeptide or polynucleotide as described above and a physiologically acceptable carrier.

Within a related aspect of the present invention, vaccines are provided. Such vaccines comprise a polypeptide or polynucleotide as described above and an immunostimulant.

The present invention further provides pharmaceutical compositions that comprise: (a) an antibody or antigen-binding fragment thereof that specifically binds to a colon tumor protein; and (b) a physiologically acceptable carrier.

Within further aspects, the present invention provides pharmaceutical compositions comprising: (a) an antigen presenting cell that expresses a polypeptide as described above and (b) a pharmaceutically acceptable carrier or excipient. Antigen presenting cells include dendritic cells, macrophages, monocytes, fibroblasts and B cells.

Within related aspects, vaccines are provided that comprise: (a) an antigen presenting cell that expresses a polypeptide as described above and (b) an immunostimulant.

The present invention further provides, in other aspects, fusion proteins that comprise at least one polypeptide as described above, as well as polynucleotides encoding such fusion proteins.

Within related aspects, pharmaceutical compositions comprising a fusion protein, or a polynucleotide encoding a fusion protein, in combination with a physiologically acceptable carrier are provided.

Vaccines are further provided, within other aspects, that comprise a fusion protein, or a polynucleotide encoding a fusion protein, in combination with an immunostimulant.

Within further aspects, the present invention provides methods for inhibiting the development of a cancer in a patient, comprising administering to a patient a pharmaceutical composition or vaccine as recited above.

The present invention further provides, within other aspects, methods for removing tumor cells from a biological sample, comprising contacting a biological sample with T cells that specifically react with a colon tumor protein, wherein the step of contacting is performed under conditions and for a time sufficient to permit the removal of cells expressing the protein from the sample.

Within related aspects, methods are provided for inhibiting the development of a cancer in a patient, comprising administering to a patient a biological sample treated as described above.

Methods are further provided, within other aspects, for stimulating and/or expanding T cells specific for a colon tumor protein, comprising contacting T cells with one or more of: (i) a polypeptide as described above; (ii) a polynucleotide encoding such a polypeptide; and/or (iii) an antigen presenting cell that expresses such a polypeptide; under conditions and for a time sufficient to permit the stimulation and/or expansion of T cells. Isolated T cell populations comprising T cells prepared as described above are also provided.

Within further aspects, the present invention provides methods for inhibiting the development of a cancer in a patient, comprising administering to a patient an effective amount of a T cell population as described above.

The present invention further provides methods for inhibiting the development of a cancer in a patient, comprising the steps of: (a) incubating CD4⁺ and/or CD8⁺ T cells isolated from a patient with one or more of: (i) a polypeptide comprising at least an immunogenic portion of a colon tumor protein; (ii) a polynucleotide encoding such a polypeptide; and (iii) an antigen-presenting cell that expresses such a polypeptide; and (b) administering to the patient an effective amount of the proliferated T cells, and thereby inhibiting the development of a cancer in the patient. Proliferated cells may, but need not, be cloned prior to administration to the patient.

Within further aspects, the present invention provides methods for determining the presence or absence of a cancer in a patient, comprising: (a) contacting a biological sample obtained from a patient with a binding agent that binds to a polypeptide as recited above; (b) detecting in the sample an amount of polypeptide that binds to the binding agent; and (c) comparing the amount of polypeptide with a predetermined cut-off value, and therefrom determining the presence or absence of a cancer in the patient. Within preferred embodiments, the binding agent is an antibody, more preferably a monoclonal antibody. The cancer may be colon cancer.

The present invention also provides, within other aspects, methods for monitoring the progression of a cancer in a patient. Such methods comprise the steps of: (a) contacting a biological sample obtained from a patient at a first point in time with a binding agent that binds to a polypeptide as recited above; (b) detecting in the sample an amount of polypeptide that binds to the binding agent; (c) repeating steps (a) and (b) using a biological sample obtained from the patient at a subsequent point in time; and (d) comparing the amount of polypeptide detected in step (c) with the amount detected in step (b) and therefrom monitoring the progression of the cancer in the patient.

The present invention further provides, within other aspects, methods for determining the presence or absence of a cancer in a patient, comprising the steps of: (a) contacting a biological sample obtained from a patient with an oligonucleotide that hybridizes to a polynucleotide that encodes a colon tumor protein; (b) detecting in the sample a level of a polynucleotide, preferably mRNA, that hybridizes to the oligonucleotide; and (c) comparing the level of polynucleotide that hybridizes to the oligonucleotide with a predetermined cut-off value, and therefrom determining the presence or absence of a cancer in the patient. Within certain embodiments, the amount of mRNA is detected via polymerase chain reaction using, for example, at least one oligonucleotide primer that hybridizes to a polynucleotide encoding a polypeptide as recited above, or a complement of such a polynucleotide. Within other embodiments, the amount of mRNA is detected using a hybridization technique, employing an oligonucleotide probe that hybridizes to a polynucleotide that encodes a polypeptide as recited above, or a complement of such a polynucleotide.

In related aspects, methods are provided for monitoring the progression of a cancer in a patient, comprising the steps of: (a) contacting a biological sample obtained from a patient with an oligonucleotide that hybridizes to a polynucleotide that encodes a colon tumor protein; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b) and therefrom monitoring the progression of the cancer in the patient.

Within further aspects, the present invention provides antibodies, such as monoclonal antibodies, that bind to a polypeptide as described above, as well as diagnostic kits comprising such antibodies. Diagnostic kits comprising one or more oligonucleotide probes or primers as described above are also provided.

These and other aspects of the present invention will become apparent upon reference to the following detailed description and attached figures. All references disclosed herein are hereby incorporated by reference in their entirety as if each was incorporated individually.

### SEQUENCE IDENTIFIERS

SEQ ID NO: 1 is a first determined cDNA sequence for Contig 1, showing homology to Neutrophil Gelatinase Associated Lipocalin.
SEQ ID NO: 2 is the determined cDNA sequence for Contig 2, showing no significant homology to any known genes.
SEQ ID NO: 3 is the determined cDNA sequence for Contig 4, showing homology to Carcinoembryonic antigen.
SEQ ID NO: 4 is the determined cDNA sequence for Contig 5, showing homology to Carcinoembryonic antigen.
SEQ ID NO: 5 is the determined cDNA sequence for Contig 9, showing homology to Carcinoembryonic antigen.
SEQ ID NO: 6 is the determined cDNA sequence for Contig 52, showing homology to Carcinoembryonic antigen.
SEQ ID NO: 7 is the determined cDNA sequence for Contig 6, showing homology toVillin.
SEQ ID NO: 8 is the determined cDNA sequence for Contig 8, showing no significant homology to any known genes.
SEQ ID NO: 9 is the determined cDNA sequence for Contig 10, showing homology to Transforming Growth Factor (BIGH3).
SEQ ID NO: 10 is the determined cDNA sequence for Contig 19, showing homology to Transforming Growth Factor (BIGH3).
SEQ ID NO: 11 is the determined cDNA sequence for Contig 21, showing homology to Transforming Growth Factor (BIGH3).
SEQ ID NO: 12 is the determined cDNA sequence for Contig 11, showing homology to CO-029.
SEQ ID NO: 13 is the determined cDNA sequence for Contig 55, showing homology to CO-029.
SEQ ID NO: 14 is the determined cDNA sequence for Contig 12, showing homology to Chromosome 17, clone hRPC.1 171_I_10, also referred to as C798P.
SEQ ID NO: 15 is the determined cDNA sequence for Contig 13, showing no significant homology to any known gene.
SEQ ID NO: 16 is the determined cDNA sequence for Contig 14, also referred to as 14261, showing no significant homology to any known gene.
SEQ ID NO: 17 is the determined cDNA sequence for Contig 15, showing homology to Ets-Related Transcription Factor (ERT).
SEQ ID NO: 18 is the determined cDNA sequence for Contig 16, showing homology to Chromosome 5, PAC clone 228g9 (LBNL H142).
SEQ ID NO: 19 is the determined cDNA sequence for Contig 24, showing homology to Chromosome 5, PAC clone 228g9 (LBNL H142).
SEQ ID NO: 20 is the determined cDNA sequence for Contig 17, showing homology to Cytokeratin.
SEQ ID NO: 21 is the determined cDNA sequence for Contig 18, showing homology to L1-Cadherin.
SEQ ID NO: 22 is the determined cDNA sequence for Contig 20, showing no significant homology to any known gene.
SEQ ID NO: 23 is the determined cDNA sequence for Contig 22, showing homology to Bumetanide-sensitive Na-K-Cl cotransporter (NKCC1).
SEQ ID NO: 24 is the determined cDNA sequence for Contig 23, showing no significant homology to any known gene.
SEQ ID NO: 25 is the determined cDNA sequence for Contig 25, showing homology to Macrophage Inflammatory Protein 3 alpha.
SEQ ID NO: 26 is the determined cDNA sequence for Contig 26, showing homology to Laminin.
SEQ ID NO: 27 is the determined cDNA sequence for Contig 48, showing homology to Laminin.
SEQ ID NO: 28 is the determined cDNA sequence for Contig 27, showing homology to Mytobularin (MTM1).
SEQ ID NO: 29 is the determined cDNA sequence for Contig 28, showing homology to Chromosome 16 BAC clone CIT987SK-A-363E6.
SEQ ID NO: 30 is the determined cDNA sequence for Contig 29, also referred to as C751P and 14247, showing no significant homology to any known gene, but partial homology to Rat GSK-3β-interacting protein Axil homolog.
SEQ ID NO: 31 is the determined cDNA sequence for Contig 30, showing homology to Zinc Finger Transcription Factor (ZNF207).
SEQ ID NO: 32 is the determined cDNA sequence for Contig 31, showing no significant homology to any known gene, but partial homology to Mus musculus GOB-4 homolog.
SEQ ID NO: 33 is the determined cDNA sequence for Contig 35, showing no significant homology to any known gene, but partial homology to Mus musculus GOB-4 homolog.
SEQ ID NO: 34 is the determined cDNA sequence for Contig 32, showing no significant homology to any known gene.
SEQ ID NO: 35 is the determined cDNA sequence for Contig 34, showing homology to Desmoglein 2.
SEQ ID NO: 36 is the determined cDNA sequence for Contig 36, showing no significant homology to any known gene.
SEQ ID NO: 37 is the determined cDNA sequence for Contig 37, showing homology to Putative Transmembrane Protein.
SEQ ID NO: 38 is the determined cDNA sequence for Contig 38, also referred to as C796P and 14219, showing no significant homology to any known gene.
SEQ ID NO: 39 is the determined cDNA sequence for Contig 40, showing homology to Nonspecific Cross-reacting Antigen.
SEQ ID NO: 40 is the determined cDNA sequence for Contig 41, also referred to as C799P and 14308, showing no significant homology to any known gene.
SEQ ID NO: 41 is the determined cDNA sequence for Contig 42, also referred to as C794P and 14309, showing no significant homology to any known gene.
SEQ ID NO: 42 is the determined cDNA sequence for Contig 43, showing homology to Chromosome 1 specific transcript KIAA0487.
SEQ ID NO: 43 is the determined cDNA sequence for Contig 45, showing homology to hMCM2.
SEQ ID NO: 44 is the determined cDNA sequence for Contig 46, showing homology to ETS2.
SEQ ID NO: 45 is the determined cDNA sequence for Contig 49, showing homology to Pump-1.
SEQ ID NO: 46 is the determined cDNA sequence for Contig 50, also referred to as C792P and 18323, showing no significant homology to any known gene.
SEQ ID NO: 47 is the determined cDNA sequence for Contig 51, also referred to as C795P and 14317, showing no significant homology to any known gene.
SEQ ID NO: 48 is the determined cDNA sequence for 11092, showing no significant homology to any known gene.
SEQ ID NO: 49 is the determined cDNA sequence for 11093, showing no significant homology to any known gene.
SEQ ID NO: 50 is the determined cDNA sequence for 11094, showing homology Human Putative Enterocyte Differentiation Protein.
SEQ ID NO: 51 is the determined cDNA sequence for 11095, showing homology to Human Transcriptional Corepressor hKAP1/TIF1B mRNA.
SEQ ID NO: 52 is the determined cDNA sequence for 11096, showing no significant homology to any known gene.
SEQ ID NO: 53 is the determined cDNA sequence for 11097, showing homology to Human Nonspecific Antigen.
SEQ ID NO: 54 is the determined cDNA sequence for 11098, showing no significant homology to any known gene.
SEQ ID NO: 55 is the determined cDNA sequence for 11099, showing homology to Human Pancreatic Secretory Inhibitor (PST) mRNA.
SEQ ID NO: 56 is the determined cDNA sequence for 11186, showing homology to Human Pancreatic Secretory Inhibitor (PST) mRNA.
SEQ ID NO: 57 is the determined cDNA sequence for 11101, showing homology to Human Chromosome X.
SEQ ID NO: 58 is the determined cDNA sequence for 11102, showing homology to Human Chromosome X.
SEQ ID NO: 59 is the determined cDNA sequence for 11103, showing no significant homology to any known gene.
SEQ ID NO: 60 is the determined cDNA sequence for 11174, showing no significant homology to any known gene.
SEQ ID NO: 61 is the determined cDNA sequence for 11104, showing homology to Human mRNA for KIAA0154.
SEQ ID NO: 62 is the determined cDNA sequence for 11105, showing homology to Human Apurinic/Apyrimidinic Endonuclease (hap1)mRNA.
SEQ ID NO: 63 is the determined cDNA sequence for 11106, showing homology toHuman Chromosome 12p13.
SEQ ID NO: 64 is the determined cDNA sequence for 11107, showing homology to Human 90 kDa Heat Shock Protein.
SEQ ID NO: 65 is the determined cDNA sequence for 11108, showing no significant homology to any known gene.
SEQ ID NO: 66 is the determined cDNA sequence for 11112, showing no significant homology to any known gene.
SEQ ID NO: 67 is the determined cDNA sequence for 11115, showing no significant homology to any known gene.
SEQ ID NO: 68 is the determined cDNA sequence for 11117, showing no significant homology to any known gene.
SEQ ID NO: 69 is the determined cDNA sequence for 11118, showing no significant homology to any known gene.
SEQ ID NO: 70 is the determined cDNA sequence for 11119, showing homology to Human Elongation Factor 1-alpha.
SEQ ID NO: 71 is the determined cDNA sequence for 11121, showing homology to Human Lamin B Receptor (LBR) mRNA.
SEQ ID NO: 72 is the determined cDNA sequence for 11122, showing homology to H. sapiens mRNA for Novel Glucocorticoid.
SEQ ID NO: 73 is the determined cDNA sequence for 11123, showing homology to H. sapiens mRNA for snRNP protein B.
SEQ ID NO: 74 is the determined cDNA sequence for 11124, showing homology to Human Cisplatin Resistance Associated Beta-protein.
SEQ ID NO: 75 is the determined cDNA sequence for 11127, showing homology to M. musculus Calumenin mRNA.
SEQ ID NO: 76 is the determined cDNA sequence for 11128, showing homology to Human ras-related small GTP binding protein.
SEQ ID NO: 77 is the determined cDNA sequence for 11130, showing homology to Human Cosmid U 169d2.
SEQ ID NO: 78 is the determined cDNA sequence for 11131, showing homology to H. sapiens mRNA for protein homologous to Elongation 1-g.
SEQ ID NO: 79 is the determined cDNA sequence for 11134, showing no significant homology to any known gene.
SEQ ID NO: 80 is the determined cDNA sequence for 11135, showing homology to H. sapiens Nieman-Pick (NPC1) mRNA.
SEQ ID NO: 81 is the determined cDNA sequence for 11137, showing homology to H. sapiens mRNA for Niecin b-chain.
SEQ ID NO: 82 is the determined cDNA sequence for 11138, showing homology to Human Endogenous Retroviral Protease mRNA.
SEQ ID NO: 83 is the determined cDNA sequence for 11139, showing homology to H. sapiens mRNA for DMBT1 protein.
SEQ ID NO: 84 is the determined cDNA sequence for 11140, showing homology to H. sapiens ras GTPase activating-like protein.
SEQ ID NO: 85 is the determined cDNA sequence for 11143, showing homology to Human Acidic Ribosomal Phosphoprotein PO mRNA.
SEQ ID NO: 86 is the determined cDNA sequence for 11144, showing homology to H. sapiens U21 mRNA.
SEQ ID NO: 87 is the determined cDNA sequence for 11145, showing homology to Human GTP-binding protein.
SEQ ID NO: 88 is the determined cDNA sequence for 11148, showing homology to H. sapiens U21 mRNA.
SEQ ID NO: 89 is the determined cDNA sequence for 11151, showing no significant homology to any known gene.
SEQ ID NO: 90 is the determined cDNA sequence for 11154, showing no significant homology to any known gene.
SEQ ID NO: 91 is the determined cDNA sequence for 11156, showing homology to H. sapiens Ribosomal Protein L27.
SEQ ID NO: 92 is the determined cDNA sequence for 11157, showing homology to H. sapiens Ribosomal Protein L27.
SEQ ID NO: 93 is the determined cDNA sequence for 11158, showing no significant homology to any known gene.
SEQ ID NO: 94 is the determined cDNA sequence for 11162, showing homology to Ag-X antigen.
SEQ ID NO: 95 is the determined cDNA sequence for 11164, showing homology to H. sapiens mRNA for Signal Recognition Protein sub14.
SEQ ID NO: 96 is the determined cDNA sequence for 11165, showing homology to Human PAC 204e5/127h14.
SEQ ID NO: 97 is the determined cDNA sequence for 11166, showing homology to Human mRNA for KIAA0108.
SEQ ID NO: 98 is the determined cDNA sequence for 11167, showing homology to H. sapiens mRNA for Neutrophil Gelatinase assct. Lipocalin.
SEQ ID NO: 99 is the determined cDNA sequence for 11168, showing no significant homology to any known gene.
SEQ ID NO: 100 is the determined cDNA sequence for 11172, showing no significant homology to any known gene.
SEQ ID NO: 101 is the determined cDNA sequence for 11175, showing no significant homology to any known gene.
SEQ ID NO: 102 is the determined cDNA sequence for 11176, showing homology to Human maspin mRNA.
SEQ ID NO: 103 is the determined cDNA sequence for 11177, showing homology to Human Carcinoembryonic Antigen.
SEQ ID NO: 104 is the determined cDNA sequence for 11178, showing homology to Human A-Tubulin mRNA.
SEQ ID NO: 105 is the determined cDNA sequence for 11179, showing homology to Human mRNA for proton-ATPase-like protein.
SEQ ID NO: 106 is the determined cDNA sequence for 11180, showing homology to Human HepG2 3' region cDNA clone hmd.
SEQ ID NO: 107 is the determined cDNA sequence for 11182, showing homology to Human MHC homologous to Chicken B-Complex Protein.
SEQ ID NO: 108 is the determined cDNA sequence for 11183, showing homology to Human High Mobility Group Box (SSRP1) mRNA.
SEQ ID NO: 109 is the determined cDNA sequence for 11184, showing no significant homology to any known gene.
SEQ ID NO: 110 is the determined cDNA sequence for 11185, showing no significant homology to any known gene.
SEQ ID NO: 111 is the determined cDNA sequence for 11187, showing no significant homology to any known gene.
SEQ ID NO: 112 is the determined cDNA sequence for 11190, showing homology to Human Replication Protein A 70kDa.
SEQ ID NO: 113 is the determined cDNA sequence for Contig 47, also referred to as C797P, showing homology to Human Chromosome X clone bWXD342.
SEQ ID NO: 114 is the determined cDNA sequence for Contig 7, showing homology to Equilibrative Nucleoside Transporter 2 (ent2).
SEQ ID NO: 115 is the determined cDNA sequence for 14235.1, also referred to as C791P, showing homology to H. sapiens chromosome 21 derived BAC containing ets-2
gene.
SEQ ID NO: 116 is the determined cDNA sequence for 14287.2, showing no significant homology to any known gene, but some degree of homology to Putative Transmembrane Protein.
SEQ ID NO: 117 is the determined cDNA sequence for 14233.1, also referred to as Contig 48, showing no significant homology to any known gene.
SEQ ID NO: 118 is the determined cDNA sequence for 14298.2, also referred to as C793P, showing no significant homology to any known gene.
SEQ ID NO: 119 is the determined cDNA sequence for 14372, also referred to as Contig 44, showing no significant homology to any known gene.
SEQ ID NO: 120 is the determined cDNA sequence for 14295, showing homology to secreted cement gland protein XAG-2 homolog.
SEQ ID NO: 121 is the determined full-length cDNA sequence for a clone showing homology to Beta IG-H3.
SEQ ID NO: 122 is the predicted amino acid sequence for the clone of SEQ ID NO: 121.
SEQ ID NO: 123 is a longer determined cDNA sequence for C751P.
SEQ ID NO: 124 is a longer determined cDNA sequence for C791P.
SEQ ID NO: 125 is a longer determined cDNA sequence for C792P.
SEQ ID NO: 126 is a longer determined cDNA sequence for C793P.
SEQ ID NO: 127 is a longer determined cDNA sequence for C794P.
SEQ ID NO: 128 is a longer determined cDNA sequence for C795P.
SEQ ID NO: 129 is a longer determined cDNA sequence for C796P.
SEQ ID NO: 130 is a longer determined cDNA sequence for C797P.
SEQ ID NO: 131 is a longer determined cDNA sequence for C798P.
SEQ ID NO: 132 is a longer determined cDNA sequence for C799P.
SEQ ID NO: 133 is a first partial determined cDNA sequence for CoSub-3 (also known as 23569).
SEQ ID NO: 134 is a second partial determined cDNA sequence for CoSub-3 (also known as 23569).
SEQ ID NO: 135 is a first partial determined cDNA sequence for CoSub-13 (also known as 23579).
SEQ ID NO: 136 is a second partial determined cDNA sequence for CoSub-13 (also known as 23579).
SEQ ID NO: 137 is the determined cDNA sequence for CoSub-17 (also known as 23583).
SEQ ID NO: 138 is the determined cDNA sequence for CoSub-19 (also known as 23585).
SEQ ID NO: 139 is the determined cDNA sequence for CoSub-22 (also known as 23714).
SEQ ID NO: 140 is the determined cDNA sequence for CoSub-23 (also known as 23715).
SEQ ID NO: 141 is the determined cDNA sequence for CoSub-26 (also known as 23717).
SEQ ID NO: 142 is the determined cDNA sequence for CoSub-33 (also known as 23724).
SEQ ID NO: 143 is the determined cDNA sequence for CoSub-34 (also known as 23725).
SEQ ID NO: 144 is the determined cDNA sequence for CoSub-35 (also known as 23726).
SEQ ID NO: 145 is the determined cDNA sequence for CoSub-37 (also known as 23728).
SEQ ID NO: 146 is the determined cDNA sequence for CoSub-39 (also known as 23730).
SEQ ID NO: 147 is the determined cDNA sequence for CoSub-42 (also known as 23766).
SEQ ID NO: 148 is the determined cDNA sequence for CoSub-44 (also known as 23768).
SEQ ID NO: 149 is the determined cDNA sequence for CoSub-47 (also known as 23771).
SEQ ID NO: 150 is the determined cDNA sequence for CoSub-54 (also known as 23778).
SEQ ID NO: 151 is the determined cDNA sequence for CoSub-55 (also known as 23779).
SEQ ID NO: 152 is the determined cDNA sequence for CT1 (also known as 24099).
SEQ ID NO: 153 is the determined cDNA sequence for CT2 (also known as 24100).
SEQ ID NO: 154 is the determined cDNA sequence for CT3 (also known as 24101).
SEQ ID NO: 155 is the determined cDNA sequence for CT6 (also known as 24104).
SEQ ID NO: 156 is the determined cDNA sequence for CT7 (also known as 24105).
SEQ ID NO: 157 is the determined cDNA sequence for CT12 (also known as 24110).
SEQ ID NO: 158 is the determined cDNA sequence for CT13 (also known as 24111).
SEQ ID NO: 159 is the determined cDNA sequence for CT14 (also known as 24112).
SEQ ID NO: 160 is the determined cDNA sequence for CT15 (also known as 24113).
SEQ ID NO: 161 is the determined cDNA sequence for CT17 (also known as 24115).
SEQ ID NO: 162 is the determined cDNA sequence for CT18 (also known as 24116).
SEQ ID NO: 163 is the determined cDNA sequence for CT22 (also known as 23848).
SEQ ID NO: 164 is the determined cDNA sequence for CT24 (also known as 23849).
SEQ ID NO: 165 is the determined cDNA sequence for CT31 (also known as 23854).
SEQ ID NO: 166 is the determined cDNA sequence for CT34 (also known as 23856).
SEQ ID NO: 167 is the determined cDNA sequence for CT37 (also known as 23859).
SEQ ID NO: 168 is the determined cDNA sequence for CT39 (also known as 23860).
SEQ ID NO: 169 is the determined cDNA sequence for CT40 (also known as 23861).
SEQ ID NO: 170 is the determined cDNA sequence for CT51 (also known as 24130).
SEQ ID NO: 171 is the determined cDNA sequence for CT53 (also known as 24132).
SEQ ID NO: 172 is the determined cDNA sequence for CT63 (also known as 24595).
SEQ ID NO: 173 is the determined cDNA sequence for CT88 (also known as 24608).
SEQ ID NO: 174 is the determined cDNA sequence for CT92 (also known as 24800).
SEQ ID NO: 175 is the determined cDNA sequence for CT94 (also known as 24802).
SEQ ID NO: 176 is the determined cDNA sequence for CT102 (also known as 24805).
SEQ ID NO: 177 is the determined cDNA sequence for CT103 (also known as 24806).
SEQ ID NO: 178 is the determined cDNA sequence for CT111 (also known as 25520).
SEQ ID NO: 179 is the determined cDNA sequence for CT118 (also known as 25522).
SEQ ID NO: 180 is the determined cDNA sequence for CT121 (also known as 25523).
SEQ ID NO: 181 is the determined cDNA sequence for CT126 (also known as 25527).
SEQ ID NO: 182 is the determined cDNA sequence for CT135 (also known as 25534).
SEQ ID NO: 183 is the determined cDNA sequence for CT140 (also known as 25537).
SEQ ID NO: 184 is the determined cDNA sequence for CT145 (also known as 25542).
SEQ ID NO: 185 is the determined cDNA sequence for CT147 (also known as 25543).
SEQ ID NO: 186 is the determined cDNA sequence for CT148 (also known as 25544).
SEQ ID NO: 187 is the determined cDNA sequence for CT502 (also known as 26420).
SEQ ID NO: 188 is the determined cDNA sequence for CT507 (also known as 26425).
SEQ ID NO: 189 is the determined cDNA sequence for CT521 (also known as 27366).
SEQ ID NO: 190 is the determined cDNA sequence for CT544 (also known as 27375).
SEQ ID NO: 191 is the determined cDNA sequence for CT577 (also known as 27385).
SEQ ID NO: 192 is the determined cDNA sequence for CT580 (also known as 27387).
SEQ ID NO: 193 is the determined cDNA sequence for CT594 (also known as 27540).
SEQ ID NO: 194 is the determined cDNA sequence for CT606 (also known as 27547).
SEQ ID NO: 195 is the determined cDNA sequence for CT607 (also known as 27548).
SEQ ID NO: 196 is the determined cDNA sequence for CT599 (also known as 27903).
SEQ ID NO: 197 is the determined cDNA sequence for CT632 (also known as 27922).
SEQ ID NO: 198 is the predicted amino acid sequence for CT502 (SEQ ID NO: 187).
SEQ ID NO: 199 is the predicted amino acid sequence for CT507 (SEQ ID NO: 188).
SEQ ID NO: 200 is the predicted amino acid sequence for CT521 (SEQ ID NO: 189).
SEQ ID NO: 201 is the predicted amino acid sequence for CT544 (SEQ ID NO: 190).
SEQ ID NO: 202 is the predicted amino acid sequence for CT606 (SEQ ID NO: 194).
SEQ ID NO: 203 is the predicted amino acid sequence for CT607 (SEQ ID NO: 195).
SEQ ID NO: 204 is the predicted amino acid sequence for CT632 (SEQ ID NO: 197).
SEQ ID NO: 205 is the determined cDNA sequence for clone 25244.
SEQ ID NO: 206 is the determined cDNA sequence for clone 25245.
SEQ ID NO: 207 is the determined cDNA sequence for clone 25246.
SEQ ID NO: 208 is the determined cDNA sequence for clone 25248.
SEQ ID NO: 209 is the determined cDNA sequence for clone 25249.
SEQ ID NO: 210 is the determined cDNA sequence for clone 25250.
SEQ ID NO: 211 is the determined cDNA sequence for clone 25251.
SEQ ID NO: 212 is the determined cDNA sequence for clone 25252.
SEQ ID NO: 213 is the determined cDNA sequence for clone 25253.
SEQ ID NO: 214 is the determined cDNA sequence for clone 25254.
SEQ ID NO: 215 is the determined cDNA sequence for clone 25255.
SEQ ID NO: 216 is the determined cDNA sequence for clone 25256.
SEQ ID NO: 217 is the determined cDNA sequence for clone 25257.
SEQ ID NO: 218 is the determined cDNA sequence for clone 25259.
SEQ ID NO: 219 is the determined cDNA sequence for clone 25260.
SEQ ID NO: 220 is the determined cDNA sequence for clone 25261.
SEQ ID NO: 221 is the determined cDNA sequence for clone 25262.
SEQ ID NO: 222 is the determined cDNA sequence for clone 25263.
SEQ ID NO: 223 is the determined cDNA sequence for clone 25264.
SEQ ID NO: 224 is the determined cDNA sequence for clone 25265.
SEQ ID NO: 225 is the determined cDNA sequence for clone 25266.
SEQ ID NO: 226 is the determined cDNA sequence for clone 25267.
SEQ ID NO: 227 is the determined cDNA sequence for clone 25268.
SEQ ID NO: 228 is the determined cDNA sequence for clone 25269.
SEQ ID NO: 229 is the determined cDNA sequence for clone 25271.
SEQ ID NO: 230 is the determined cDNA sequence for clone 25272.
SEQ ID NO: 231 is the determined cDNA sequence for clone 25273.
SEQ ID NO: 232 is the determined cDNA sequence for clone 25274.
SEQ ID NO: 233 is the determined cDNA sequence for clone 25275.
SEQ ID NO: 234 is the determined cDNA sequence for clone 25276.
SEQ ID NO: 235 is the determined cDNA sequence for clone 25277.
SEQ ID NO: 236 is the determined cDNA sequence for clone 25278.
SEQ ID NO: 237 is the determined cDNA sequence for clone 25280.
SEQ ID NO: 238 is the determined cDNA sequence for clone 25281.
SEQ ID NO: 239 is the determined cDNA sequence for clone 25282.
SEQ ID NO: 240 is the determined cDNA sequence for clone 25283.
SEQ ID NO: 241 is the determined cDNA sequence for clone 25284.
SEQ ID NO: 242 is the determined cDNA sequence for clone 25285.
SEQ ID NO: 243 is the determined cDNA sequence for clone 25286.
SEQ ID NO: 244 is the determined cDNA sequence for clone 25287.
SEQ ID NO: 245 is the determined cDNA sequence for clone 25288.
SEQ ID NO: 246 is the determined cDNA sequence for clone 25289.
SEQ ID NO: 247 is the determined cDNA sequence for clone 25290.
SEQ ID NO: 248 is the determined cDNA sequence for clone 25291.
SEQ ID NO: 249 is the determined cDNA sequence for clone 25292.
SEQ ID NO: 250 is the determined cDNA sequence for clone 25293.
SEQ ID NO: 251 is the determined cDNA sequence for clone 25294.
SEQ ID NO: 252 is the determined cDNA sequence for clone 25295.
SEQ ID NO: 253 is the determined cDNA sequence for clone 25296.
SEQ ID NO: 254 is the determined cDNA sequence for clone 25297.
SEQ ID NO: 255 is the determined cDNA sequence for clone 25418.
SEQ ID NO: 256 is the determined cDNA sequence for clone 25419.
SEQ ID NO: 257 is the determined cDNA sequence for clone 25420.
SEQ ID NO: 258 is the determined cDNA sequence for clone 25421.
SEQ ID NO: 259 is the determined cDNA sequence for clone 25422.
SEQ ID NO: 260 is the determined cDNA sequence for clone 25423.
SEQ ID NO: 261 is the determined cDNA sequence for clone 25424.
SEQ ID NO: 262 is the determined cDNA sequence for clone 25426.
SEQ ID NO: 263 is the determined cDNA sequence for clone 25427.
SEQ ID NO: 264 is the determined cDNA sequence for clone 25428.
SEQ ID NO: 265 is the determined cDNA sequence for clone 25429.
SEQ ID NO: 266 is the determined cDNA sequence for clone 25430.
SEQ ID NO: 267 is the determined cDNA sequence for clone 25431.
SEQ ID NO: 268 is the determined cDNA sequence for clone 25432.
SEQ ID NO: 269 is the determined cDNA sequence for clone 25433.
SEQ ID NO: 270 is the determined cDNA sequence for clone 25434.
SEQ ID NO: 271 is the determined cDNA sequence for clone 25435.
SEQ ID NO: 272 is the determined cDNA sequence for clone 25436.
SEQ ID NO: 273 is the determined cDNA sequence for clone 25437.
SEQ ID NO: 274 is the determined cDNA sequence for clone 25438.
SEQ ID NO: 275 is the determined cDNA sequence for clone 25439.
SEQ ID NO: 276 is the determined cDNA sequence for clone 25440.
SEQ ID NO: 277 is the determined cDNA sequence for clone 25441.
SEQ ID NO: 278 is the determined cDNA sequence for clone 25442.
SEQ ID NO: 279 is the determined cDNA sequence for clone 25443.
SEQ ID NO: 280 is the determined cDNA sequence for clone 25444.
SEQ ID NO: 281 is the determined cDNA sequence for clone 25445.
SEQ ID NO: 282 is the determined cDNA sequence for clone 25446.
SEQ ID NO: 283 is the determined cDNA sequence for clone 25447.
SEQ ID NO: 284 is the determined cDNA sequence for clone 25448.
SEQ ID NO: 285 is the determined cDNA sequence for clone 25844.
SEQ ID NO: 286 is the determined cDNA sequence for clone 25845.
SEQ ID NO: 287 is the determined cDNA sequence for clone 25846.
SEQ ID NO: 288 is the determined cDNA sequence for clone 25847.
SEQ ID NO: 289 is the determined cDNA sequence for clone 25848.
SEQ ID NO: 290 is the determined cDNA sequence for clone 25850.
SEQ ID NO: 291 is the determined cDNA sequence for clone 25851.
SEQ ID NO: 292 is the determined cDNA sequence for clone 25852.
SEQ ID NO: 293 is the determined cDNA sequence for clone 25853.
SEQ ID NO: 294 is the determined cDNA sequence for clone 25854.
SEQ ID NO: 295 is the determined cDNA sequence for clone 25855.
SEQ ID NO: 296 is the determined cDNA sequence for clone 25856.
SEQ ID NO: 297 is the determined cDNA sequence for clone 25857.
SEQ ID NO: 298 is the determined cDNA sequence for clone 25858.
SEQ ID NO: 299 is the determined cDNA sequence for clone 25859.
SEQ ID NO: 300 is the determined cDNA sequence for clone 25860.
SEQ ID NO: 301 is the determined cDNA sequence for clone 25861.
SEQ ID NO: 302 is the determined cDNA sequence for clone 25862.
SEQ ID NO: 303 is the determined cDNA sequence for clone 25863.
SEQ ID NO: 304 is the determined cDNA sequence for clone 25864.
SEQ ID NO: 305 is the determined cDNA sequence for clone 25865.
SEQ ID NO: 306 is the determined cDNA sequence for clone 25866.
SEQ ID NO: 307 is the determined cDNA sequence for clone 25867.
SEQ ID NO: 308 is the determined cDNA sequence for clone 25868.
SEQ ID NO: 309 is the determined cDNA sequence for clone 25869.
SEQ ID NO: 310 is the determined cDNA sequence for clone 25870.
SEQ ID NO: 311 is the determined cDNA sequence for clone 25871.
SEQ ID NO: 312 is the determined cDNA sequence for clone 25872.
SEQ ID NO: 313 is the determined cDNA sequence for clone 25873.
SEQ ID NO: 314 is the determined cDNA sequence for clone 25875.
SEQ ID NO: 315 is the determined cDNA sequence for clone 25876.
SEQ ID NO: 316 is the determined cDNA sequence for clone 25877.
SEQ ID NO: 317 is the determined cDNA sequence for clone 25878.
SEQ ID NO: 318 is the determined cDNA sequence for clone 25879.
SEQ ID NO: 319 is the determined cDNA sequence for clone 25880.
SEQ ID NO: 320 is the determined cDNA sequence for clone 25881.
SEQ ID NO: 321 is the determined cDNA sequence for clone 25882.
SEQ ID NO: 322 is the determined cDNA sequence for clone 25883.
SEQ ID NO: 323 is the determined cDNA sequence for clone 25884.
SEQ ID NO: 324 is the determined cDNA sequence for clone 25885.
SEQ ID NO: 325 is the determined cDNA sequence for clone 25886.
SEQ ID NO: 326 is the determined cDNA sequence for clone 25887.
SEQ ID NO: 327 is the determined cDNA sequence for clone 25888.
SEQ ID NO: 328 is the determined cDNA sequence for clone 25889.
SEQ ID NO: 329 is the determined cDNA sequence for clone 25890.
SEQ ID NO: 330 is the determined cDNA sequence for clone 25892.
SEQ ID NO: 331 is the determined cDNA sequence for clone 25894.
SEQ ID NO: 332 is the determined cDNA sequence for clone 25895.
SEQ ID NO: 333 is the determined cDNA sequence for clone 25896.
SEQ ID NO: 334 is the determined cDNA sequence for clone 25897.
SEQ ID NO: 335 is the determined cDNA sequence for clone 25899.
SEQ ID NO: 336 is the determined cDNA sequence for clone 25900.
SEQ ID NO: 337 is the determined cDNA sequence for clone 25901.
SEQ ID NO: 338 is the determined cDNA sequence for clone 25902.
SEQ ID NO: 339 is the determined cDNA sequence for clone 25903.
SEQ ID NO: 340 is the determined cDNA sequence for clone 25904.
SEQ ID NO: 341 is the determined cDNA sequence for clone 25906.
SEQ ID NO: 342 is the determined cDNA sequence for clone 25907.
SEQ ID NO: 343 is the determined cDNA sequence for clone 25908.
SEQ ID NO: 344 is the determined cDNA sequence for clone 25909.
SEQ ID NO: 345 is the determined cDNA sequence for clone 25910.
SEQ ID NO: 346 is the determined cDNA sequence for clone 25911.
SEQ ID NO: 347 is the determined cDNA sequence for clone 25912.
SEQ ID NO: 348 is the determined cDNA sequence for clone 25913.
SEQ ID NO: 349 is the determined cDNA sequence for clone 25914.
SEQ ID NO: 350 is the determined cDNA sequence for clone 25915.
SEQ ID NO: 351 is the determined cDNA sequence for clone 25916.
SEQ ID NO: 352 is the determined cDNA sequence for clone 25917.
SEQ ID NO: 353 is the determined cDNA sequence for clone 25918.
SEQ ID NO: 354 is the determined cDNA sequence for clone 25919.
SEQ ID NO: 355 is the determined cDNA sequence for clone 25920.
SEQ ID NO: 356 is the determined cDNA sequence for clone 25921.
SEQ ID NO: 357 is the determined cDNA sequence for clone 25922.
SEQ ID NO: 358 is the determined cDNA sequence for clone 25924.
SEQ ID NO: 359 is the determined cDNA sequence for clone 25925.
SEQ ID NO: 360 is the determined cDNA sequence for clone 25926.
SEQ ID NO: 361 is the determined cDNA sequence for clone 25927.
SEQ ID NO: 362 is the determined cDNA sequence for clone 25928.
SEQ ID NO: 363 is the determined cDNA sequence for clone 25929.
SEQ ID NO: 364 is the determined cDNA sequence for clone 25930.
SEQ ID NO: 365 is the determined cDNA sequence for clone 25931.
SEQ ID NO: 366 is the determined cDNA sequence for clone 25932.
SEQ ID NO: 367 is the determined cDNA sequence for clone 25933.
SEQ ID NO: 368 is the determined cDNA sequence for clone 25934.
SEQ ID NO: 369 is the determined cDNA sequence for clone 25935.
SEQ ID NO: 370 is the determined cDNA sequence for clone 25936.
SEQ ID NO: 371 is the determined cDNA sequence for clone 25939.
SEQ ID NO: 372 is the determined cDNA sequence for clone 32016.
SEQ ID NO: 373 is the determined cDNA sequence for clone 32021.
SEQ ID NO: 374 is the determined cDNA sequence for clone 31993.
SEQ ID NO: 375 is the determined cDNA sequence for clone 31997.
SEQ ID NO: 376 is the determined cDNA sequence for clone 31942.
SEQ ID NO: 377 is the determined cDNA sequence for clone 31937.
SEQ ID NO: 378 is the determined cDNA sequence for clone 31952.
SEQ ID NO: 379 is the determined cDNA sequence for clone 31992.
SEQ ID NO: 380 is the determined cDNA sequence for clone 31961.
SEQ ID NO: 381 is the determined cDNA sequence for clone 31964.
SEQ ID NO: 382 is the determined cDNA sequence for clone 32005.
SEQ ID NO: 383 is the determined cDNA sequence for clone 31980.
SEQ ID NO: 384 is the determined cDNA sequence for clone 31940.
SEQ ID NO: 385 is the determined cDNA sequence for clone 32004.
SEQ ID NO: 386 is the determined cDNA sequence for clone 31956.
SEQ ID NO: 387 is the determined cDNA sequence for clone 31934.
SEQ ID NO: 388 is the determined cDNA sequence for clone 31998.
SEQ ID NO: 389 is the determined cDNA sequence for clone 31973.
SEQ ID NO: 390 is the determined cDNA sequence for clone 31976.
SEQ ID NO: 391 is the determined cDNA sequence for clone 31988.
SEQ ID NO: 392 is the determined cDNA sequence for clone 31948.
SEQ ID NO: 393 is the determined cDNA sequence for clone 32013.
SEQ ID NO: 394 is the determined cDNA sequence for clone 31986.
SEQ ID NO: 395 is the determined cDNA sequence for clone 31954.
SEQ ID NO: 396 is the determined cDNA sequence for clone 31987.
SEQ ID NO: 397 is the determined cDNA sequence for clone 32029.
SEQ ID NO: 398 is the determined cDNA sequence for clone 32028.
SEQ ID NO: 399 is the determined cDNA sequence for clone 32012.
SEQ ID NO: 400 is the determined cDNA sequence for clone 31959.
SEQ ID NO: 401 is the determined cDNA sequence for clone 32027.
SEQ ID NO: 402 is the determined cDNA sequence for clone 31957.
SEQ ID NO: 403 is the determined cDNA sequence for clone 31950.
SEQ ID NO: 404 is the determined cDNA sequence for clone 32011.
SEQ ID NO: 405 is the determined cDNA sequence for clone 32022.
SEQ ID NO: 406 is the determined cDNA sequence for clone 32014.
SEQ ID NO: 407 is the determined cDNA sequence for clone 31963.
SEQ ID NO: 408 is the determined cDNA sequence for clone 31989.
SEQ ID NO: 409 is the determined cDNA sequence for clone 32015.
SEQ ID NO: 410 is the determined cDNA sequence for clone 32002.
SEQ ID NO: 411 is the determined cDNA sequence for clone 31939.
SEQ ID NO: 412 is the determined cDNA sequence for clone 32003.
SEQ ID NO: 413 is the determined cDNA sequence for clone 31936.
SEQ ID NO: 414 is the determined cDNA sequence for clone 32007.
SEQ ID NO: 415 is the determined cDNA sequence for clone 31965.
SEQ ID NO: 416 is the determined cDNA sequence for clone 31935.
SEQ ID NO: 417 is the determined cDNA sequence for clone 32008.
SEQ ID NO: 418 is the determined cDNA sequence for clone 31966.
SEQ ID NO: 419 is the determined cDNA sequence for clone 32020.
SEQ ID NO: 420 is the determined cDNA sequence for clone 31971.
SEQ ID NO: 421 is the determined cDNA sequence for clone 31977.
SEQ ID NO: 422 is the determined cDNA sequence for clone 31985.
SEQ ID NO: 423 is the determined cDNA sequence for clone 32023.
SEQ ID NO: 424 is the determined cDNA sequence for clone 31981.
SEQ ID NO: 425 is the determined cDNA sequence for clone 32006.
SEQ ID NO: 426 is the determined cDNA sequence for clone 31991.
SEQ ID NO: 427 is the determined cDNA sequence for clone 31995.
SEQ ID NO: 428 is the determined cDNA sequence for clone 32000.
SEQ ID NO: 429 is the determined cDNA sequence for clone 31990.
SEQ ID NO: 430 is the determined cDNA sequence for clone 31946.
SEQ ID NO: 431 is the determined cDNA sequence for clone 31938.
SEQ ID NO: 432 is the determined cDNA sequence for clone 31941.
SEQ ID NO: 433 is the determined cDNA sequence for clone 31982.
SEQ ID NO: 434 is the determined cDNA sequence for clone 31996.
SEQ ID NO: 435 is the determined cDNA sequence for clone 32010.
SEQ ID NO: 436 is the determined cDNA sequence for clone 31974.
SEQ ID NO: 437 is the determined cDNA sequence for clone 31983.
SEQ ID NO: 438 is the determined cDNA sequence for clone 31999.
SEQ ID NO: 439 is the determined cDNA sequence for clone 31949.
SEQ ID NO: 440 is the determined cDNA sequence for clone 31947.
SEQ ID NO: 441 is the determined cDNA sequence for clone 31994.
SEQ ID NO: 442 is the determined cDNA sequence for clone 31958.
SEQ ID NO: 443 is the determined cDNA sequence for clone 31975.
SEQ ID NO: 444 is the determined cDNA sequence for clone 31984.
SEQ ID NO: 445 is the determined cDNA sequence for clone 32024.
SEQ ID NO: 446 is the determined cDNA sequence for clone 31972.
SEQ ID NO: 447 is the determined cDNA sequence for clone 31943.
SEQ ID NO: 448 is the determined cDNA sequence for clone 32018.
SEQ ID NO: 449 is the determined cDNA sequence for clone 32026.
SEQ ID NO: 450 is the determined cDNA sequence for clone 32009.
SEQ ID NO: 451 is the determined cDNA sequence for clone 32019.
SEQ ID NO: 452 is the determined cDNA sequence for clone 32025.
SEQ ID NO: 453 is the determined cDNA sequence for clone 31967.
SEQ ID NO: 454 is the determined cDNA sequence for clone 31968.
SEQ ID NO: 455 is the determined cDNA sequence for clone 31955.
SEQ ID NO: 456 is the determined cDNA sequence for clone 31951.
SEQ ID NO: 457 is the determined cDNA sequence for clone 31970.
SEQ ID NO: 458 is the determined cDNA sequence for clone 31962.
SEQ ID NO: 459 is the determined cDNA sequence for clone 32001.
SEQ ID NO: 460 is the determined cDNA sequence for clone 31953.
SEQ ID NO: 461 is the determined cDNA sequence for clone 31944.
SEQ ID NO: 462 is the determined cDNA sequence for clone 31825.
SEQ ID NO: 463 is the determined cDNA sequence for clone 31828.
SEQ ID NO: 464 is the determined cDNA sequence for clone 31830.
SEQ ID NO: 465 is the determined cDNA sequence for clone 31841.
SEQ ID NO: 466 is the determined cDNA sequence for clone 31847.
SEQ ID NO: 467 is the determined cDNA sequence for clone 31850.
SEQ ID NO: 468 is the determined cDNA sequence for clone 31852.
SEQ ID NO: 469 is the determined cDNA sequence for clone 31855.
SEQ ID NO: 470 is the determined cDNA sequence for clone 31858.
SEQ ID NO: 471 is the determined cDNA sequence for clone 31861.
SEQ ID NO: 472 is the determined cDNA sequence for clone 31868.
SEQ ID NO: 473 is the determined cDNA sequence for clone 31870.
SEQ ID NO: 474 is the determined cDNA sequence for clone 31872.
SEQ ID NO: 475 is the determined cDNA sequence for clone 31873.
SEQ ID NO: 476 is the determined cDNA sequence for clone 31877.
SEQ ID NO: 477 is the determined cDNA sequence for clone 31878.
SEQ ID NO: 478 is the determined cDNA sequence for clone 31885.
SEQ ID NO: 479 is the determined cDNA sequence for clone 31888.
SEQ ID NO: 480 is the determined cDNA sequence for clone 31890.
SEQ ID NO: 481 is the determined cDNA sequence for clone 31893.
SEQ ID NO: 482 is the determined cDNA sequence for clone 31898.
SEQ ID NO: 483 is the determined cDNA sequence for clone 31901.
SEQ ID NO: 484 is the determined cDNA sequence for clone 31909.
SEQ ID NO: 485 is the determined cDNA sequence for clone 31910.
SEQ ID NO: 486 is the determined cDNA sequence for clone 31914.

### DETAILED DESCRIPTION OF THE INVENTION

As noted above, the present invention is generally directed to compositions and methods for the therapy and diagnosis of cancer, such as colon cancer. The compositions described herein may include colon tumor polypeptides, polynucleotides encoding such polypeptides, binding agents such as antibodies, antigen presenting cells (APCs) and/or immune system cells (*e.g*., T cells). Polypeptides of the present invention generally comprise at least a portion (such as an immunogenic portion) of a colon tumor protein or a variant thereof. A "colon tumor protein" is a protein that is expressed in colon tumor cells at a level that is at least two fold, and preferably at least five fold, greater than the level of expression in a normal tissue, as determined using a representative assay provided herein. Certain colon tumor proteins are tumor proteins that react detectably (within an immunoassay, such as an ELISA or Western blot) with antisera of a patient afflicted with colon cancer. Polynucleotides of the subject invention generally comprise a DNA or RNA sequence that encodes all or a portion of such a polypeptide, or that is complementary to such a sequence. Antibodies are generally immune system proteins, or antigen-binding fragments thereof, that are capable of binding to a polypeptide as described above. Antigen presenting cells include dendritic cells, macrophages, monocytes, fibroblasts and B-cells that express a polypeptide as described above. T cells that may be employed within such compositions are generally T cells that are specific for a polypeptide as described above.

The present invention is based on the discovery of human colon tumor proteins. Sequences of polynucleotides encoding specific tumor proteins are provided in SEQ ID NO: 1-121, 123-197 and 205-486.

### COLON TUMOR PROTEIN POLYNUCLEOTIDES

Any polynucleotide that encodes a colon tumor protein or a portion or other variant thereof as described herein is encompassed by the present invention. Preferred polynucleotides comprise at least 15 consecutive nucleotides, preferably at least 30 consecutive nucleotides and more preferably at least 45 consecutive nucleotides, that encode a portion of a colon tumor protein. More preferably, a polynucleotide encodes an immunogenic portion of a colon tumor protein. Polynucleotides complementary to any such sequences are also encompassed by the present invention. Polynucleotides may be single-stranded (coding or antisense) or double-stranded, and may be DNA (genomic, cDNA or synthetic) or RNA molecules. RNA molecules include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i.e*., an endogenous sequence that encodes a colon tumor protein or a portion thereof) or may comprise a variant of such a sequence. Polynucleotide variants may contain one or more substitutions, additions, deletions and/or insertions such that the immunogenicity of the encoded polypeptide is not diminished, relative to a native tumor protein. The effect on the immunogenicity of the encoded polypeptide may generally be assessed as described herein. Variants preferably exhibit at least about 70% identity, more preferably at least about 80% identity and most preferably at least about 90% identity to a polynucleotide sequence that encodes a native colon tumor protein or a portion thereof.

Two polynucleotide or polypeptide sequences are said to be "identical" if the sequence of nucleotides or amino acids in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 *Methods in Enzymology* vol. 183, Academic Press, Inc., San Diego, CA; Higgins, D.G. and Sharp, P.M. (1989) *CABIOS* 5:151-153; Myers, E.W. and Muller W. (1988) CABIOS 4:11-17; Robinson, E.D. (1971) *Comb. Theor 11*:105; Santou, N. Nes, M. (1987) *Mol. Biol. Evol.* 4:406-425; Sneath, P.H.A. and Sokal, R.R. (1973) *Numerical Taxonomy - the Principles and Practice of Numerical Taxonomy,* Freeman Press, San Francisco, CA; Wilbur, W.J. and Lipman, D.J. (1983) *Proc. Natl. Acad., Sci. USA 80:726-*730.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (i.e. gaps) of 20 percent or less, usually 5 to 15 percent, or 10 to 12 percent, as compared to the reference sequence (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Variants may also, or alternatively, be substantially homologous to a native gene, or a portion or complement thereof. Such polynucleotide variants are capable of hybridizing under moderately stringent conditions to a naturally occurring DNA sequence encoding a native colon tumor protein (or a complementary sequence). Suitable moderately stringent conditions include prewashing in a solution of 5X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1 % SDS.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Polynucleotides may be prepared using any of a variety of techniques. For example, a polynucleotide may be identified, as described in more detail below, by screening a microarray of cDNAs for tumor-associated expression (i.e., expression that is at least two fold greater in a colon tumor than in normal tissue, as determined using a representative assay provided herein). Such screens may be performed using a Synteni microarray (Palo Alto, CA) according to the manufacturer's instructions (and essentially as described by Schena et al., *Proc. Natl. Acad. Sci. USA* 93:10614-10619, 1996 and Heller et al., *Proc. Natl. Acad Sci. USA 94*:2150-2155, 1997). Alternatively, polypeptides may be amplified from cDNA prepared from cells expressing the proteins described herein, such as colon tumor cells. Such polynucleotides may be amplified via polymerase chain reaction (PCR). For this approach, sequence-specific primers may be designed based on the sequences provided herein, and may be purchased or synthesized.

An amplified portion may be used to isolate a full length gene from a suitable library (e.g., a colon tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e.g*., by nick-translation or end-labeling with ³²P) using well known techniques. A bacterial or bacteriophage library is then screened by hybridizing filters containing denatured bacterial colonies (or lawns containing phage plaques) with the labeled probe *(see* Sambrook et al., *Molecular Cloning: A Laboratory Manual,* Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR *(see* Triglia et al., *Nucl. Acids Res. 16*:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom et al., *PCR Methods Applic. 1:* 111 - 19, 1991) and walking PCR (Parker et al., *Nucl. Acids Res. 19*:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e.g*., NCBI BLAST searches), and such ESTs may be used to generate a contiguous full length sequence.

Certain nucleic acid sequences of cDNA molecules encoding portions of colon tumor proteins are provided in SEQ ID NO: 1-121, 123-197 and 205-486. These polynucleotides were isolated from colon tumor cDNA libraries using conventional and/or PCR-based subtraction techniques, as described below.

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis *(see* Adelman et al., *DNA* 2:183, 1983). Alternatively, RNA molecules may be generated by *in vitro* or *in vivo* transcription of DNA sequences encoding a colon tumor protein, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo (e.g.,* by transfecting antigen-presenting cells, such as dendritic cells, with a cDNA construct encoding a colon tumor polypeptide, and administering the transfected cells to the patient).

A portion of a sequence complementary to a coding sequence *(i.e.,* an antisense polynucleotide) may also be used as a probe or to modulate gene expression. cDNA constructs that can be transcribed into antisense RNA may also be introduced into cells of tissues to facilitate the production of antisense RNA. An antisense polynucleotide may be used, as described herein, to inhibit expression of a tumor protein. Antisense technology can be used to control gene expression through triple-helix formation, which compromises the ability of the double helix to open sufficiently for the binding of polymerases, transcription factors or regulatory molecules *(see* Gee et al., *In* Huber and Carr, *Molecular and Immunologic Approaches,* Futura Publishing Co. (Mt. Kisco, NY; 1994)). Alternatively, an antisense molecule may be designed to hybridize with a control region of a gene (*e.g*., promoter, enhancer or transcription initiation site), and block transcription of the gene; or to block translation by inhibiting binding of a transcript to ribosomes.

A portion of a coding sequence, or of a complementary sequence, may also be designed as a probe or primer to detect gene expression. Probes may be labeled with a variety of reporter groups, such as radionuclides and enzymes, and are preferably at least 10 nucleotides in length, more preferably at least 20 nucleotides in length and still more preferably at least 30 nucleotides in length. Primers, as noted above, are preferably 22-30 nucleotides in length.

Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Within certain embodiments, polynucleotides may be formulated so as to permit entry into a cell of a mammal, and expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (*e.g*., avian pox virus). Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery *vehicle in vitro* and *in vivo* is a liposome *(i.e.,* an artificial membrane vesicle). The preparation and use of such systems is well known in the art.

### COLON TUMOR POLYPEPTIDES

Within the context of the present invention, polypeptides may comprise at least an immunogenic portion of a colon tumor protein or a variant thereof, as described herein. As noted above, a "colon tumor protein" is a protein that is expressed by colon tumor cells. Proteins that are colon tumor proteins also react detectably within an immunoassay (such as an ELISA) with antisera from a patient with colon cancer. Polypeptides as described herein may be of any length. Additional sequences derived from the native protein and/or heterologous sequences may be present, and such sequences may (but need not) possess further immunogenic or antigenic properties.

An "immunogenic portion," as used herein is a portion of a protein that is recognized (*i.e*., specifically bound) by a B-cell and/or T-cell surface antigen receptor. Such immunogenic portions generally comprise at least 5 amino acid residues, more preferably at least 10, and still more preferably at least 20 amino acid residues of a colon tumor protein or a variant thereof. Certain preferred immunogenic portions include peptides in which an N-terminal leader sequence and/or transmembrane domain have been deleted. Other preferred immunogenic portions may contain a small N- and/or C-terminal deletion (*e.g*., 1-30 amino acids, preferably 5-15 amino acids), relative to the mature protein.

Immunogenic portions may generally be identified using well known techniques, such as those summarized in Paul, *Fundamental Immunology,* 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e*., they react with the protein in an ELISA or other immunoassay, and do not react detectably with unrelated proteins). Such antisera and antibodies may be prepared as described herein, and using well known techniques. An immunogenic portion of a native colon tumor protein is a portion that reacts with such antisera and/or T-cells at a level that is not substantially less than the reactivity of the full length polypeptide (*e.g*., in an ELISA and/or T-cell reactivity assay). Such immunogenic portions may react within such assays at a level that is similar to or greater than the reactivity of the full length polypeptide. Such screens may generally be performed using methods well known to those of ordinary skill in the art, such as those described in Harlow and Lane, *Antibodies: A Laboratory Manual,* Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As noted above, a composition may comprise a variant of a native colon tumor protein. A polypeptide "variant," as used herein, is a polypeptide that differs from a native colon tumor protein in one or more substitutions, deletions, additions and/or insertions, such that the immunogenicity of the polypeptide is not substantially diminished. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and evaluating the reactivity of the modified polypeptide with antigen-specific antibodies or antisera as described herein. Preferred variants include those in which one or more portions, such as an N-terminal leader sequence or transmembrane domain, have been removed. Other preferred variants include variants in which a small portion (*e.g*., 1-30 amino acids, preferably 5-15 amino acids) has been removed from the N- and/or C-terminal of the mature protein.

Polypeptide variants preferably exhibit at least about 70%, more preferably at least about 90% and most preferably at least about 95% identity (determined as described above) to the identified polypeptides.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity. values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain non-conservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g.*, poly-His), or to enhance binding of the polypeptide to a solid support. For example, a polypeptide may be conjugated to an immunoglobulin Fc region.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, J. *Am. Chem. Soc. 85*:2149-2146, 1963. Equipment for automated synthesis of polypeptides is commercially available from suppliers such as Perkin Elmer/Applied BioSystems Division (Foster City, CA), and may be operated according to the manufacturer's instructions.

Within certain specific embodiments, a polypeptide may be a fusion protein that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and the second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., *Gene 40*:39-46, 1985; Murphy et al., *Proc. Natl. Acad. Sci. USA 83:*8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins *(see,* for example, Stoute et al. *New Engl. J. Med.,* 336:86-91, 1997).

Within preferred embodiments, an immunological fusion partner is derived from protein D, a surface protein of the gram-negative bacterium Haemophilus influenza B (WO 91/18926). Preferably, a protein D derivative comprises approximately the first third of the protein (*e.g.*, the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E. coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; *Gene 43*:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E*. *coli* C-LYTA expressing plasmids useful for expression of fusion proteins. Purification of hybrid proteins containing the C-LYTA fragment at the amino terminus has been described *(see Biotechnology 10*:795-798, 1992). Within a preferred embodiment, a repeat portion of LYTA may be incorporated into a fusion protein. A repeat portion is found in the C-terminal region starting at residue 178. A particularly preferred repeat portion incorporates residues 188-305.

In general, polypeptides (including fusion proteins) and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

### BINDING AGENTS

The present invention further provides agents, such as antibodies and antigen-binding fragments thereof, that specifically bind to a colon tumor protein. As used herein, an antibody, or antigen-binding fragment thereof, is said to "specifically bind" to a colon tumor protein if it reacts at a detectable level (within, for example, an ELISA) with a colon tumor protein, and does not react detectably with unrelated proteins under similar conditions. As used herein, "binding" refers to a noncovalent association between two separate molecules such that a complex is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about 10³ L/mol. The binding constant may be determined using methods well known in the art.

Binding agents may be further capable of differentiating between patients with and without a cancer, such as colon cancer, using the representative assays provided herein. In other words, antibodies or other binding agents that bind to a colon tumor protein will generate a signal indicating the presence of a cancer in at least about 20% of patients with the disease, and will generate a negative signal indicating the absence of the disease in at least about 90% of individuals without the cancer. To determine whether a binding agent satisfies this requirement, biological samples (*e.g*., blood, sera, sputum, urine and/or tumor biopsies) from patients with and without a cancer (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.,* Harlow and Lane, *Antibodies: A Laboratory Manual,* Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e.g*., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, *Eur. J. Immunol.* 6:511-519, 1976, and improvements thereto. Briefly, these methods involve the preparation of immortal cell lines capable of producing antibodies having the desired specificity (*i.e.*, reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein. A bead columns (Harlow and Lane, *Antibodies: A Laboratory Manual,* Cold Spring Harbor Laboratory, 1988) and digested by papain to yield Fab and Fc fragments. The Fab and Fc fragments may be separated by affinity chromatography on protein A bead columns.

Monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. *Preferred* toxins include ricin, abrin, diptheria toxin, cholera toxin, gelonin, Pseudomonas exotoxin, Shigella toxin, and pokeweed antiviral protein.

A therapeutic agent may be coupled *(e.g.,* covalently bonded) to a suitable monoclonal antibody either directly or indirectly *(e.g.,* via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group *(e.g.,* a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g*., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond *(e.g.,* U.S. Patent No. 4,489,710, to Spitler), by irradiation of a photolabile bond *(e.g.,* U.S. Patent No. 4,625,014, to Senter et al.), by hydrolysis of derivatized amino acid side chains *(e.g.,* U.S. Patent No. 4,638,045, to Kohn et al.), by serum complement-mediated hydrolysis *(e.g.,* U.S. Patent No. 4,671,958, to Rodwell et al.), and acid-catalyzed hydrolysis *(e.g.,* U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins *(e.g.,* U.S. Patent No. 4,507,234, to Kato et al.), peptides and polysaccharides such as aminodextran *(e.g.,* U.S. Patent No. 4,699,784, to Shih et al.). A carrier may also bear an agent by noncovalent bonding or by encapsulation, such as within a liposome vesicle *(e.g.,* U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

### T CELLS

Immunotherapeutic compositions may also, or alternatively, comprise T cells specific for a colon tumor protein. Such cells may generally be prepared *in vitro* or *ex vivo, using* standard procedures. For example, T cells may be isolated from bone marrow, peripheral blood, or a fraction of bone marrow or peripheral blood of a patient, using a commercially available cell separation system, such as the ISOLEX™ system, available from Nexell Therapeutics Inc., Irvine, CA. Alternatively, T cells may be derived from related or unrelated humans, non-human mammals, cell lines or cultures.

T cells may be stimulated with a colon tumor polypeptide, polynucleotide encoding a colon tumor polypeptide and/or an antigen presenting cell (APC) that expresses such a polypeptide. Such stimulation is performed under conditions and for a time sufficient to permit the generation of T cells that are specific for the polypeptide. Preferably, a colon tumor polypeptide or polynucleotide is present within a delivery vehicle, such as a microsphere, to facilitate the generation of specific T cells.

T cells are considered to be specific for a colon tumor polypeptide if the T cells kill target cells coated with the polypeptide or expressing a gene encoding the polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., *Cancer Res. 54*:1065-1070, 1994. Alternatively, detection of the proliferation of T cells may be accomplished by a variety of known techniques. For example, T cell proliferation can be detected by measuring an increased rate of DNA synthesis (e.g., by pulse-labeling cultures of T cells with tritiated thymidine and measuring the amount of tritiated thymidine incorporated into DNA). Contact with a colon tumor polypeptide (100 ng/ml - 100 µg/ml, preferably 200 ng/ml - 25 µg/ml) for 3 - 7 days should result in at least a two fold increase in proliferation of the T cells. Contact as described above for 2-3 hours should result in activation of the T cells, as measured using standard cytokine assays in which a two fold increase in the level of cytokine release (*e.g*., TNF or IFN-γ) is indicative of T cell activation *(see* Coligan et al., Current Protocols in Immunology, vol. 1, Wiley Interscience (Greene 1998)). T cells that have been activated in response to a colon tumor polypeptide, polynucleotide or polypeptide-expressing APC may be CD4⁺ and/or CD8⁺. Colon tumor protein-specific T cells may be expanded using standard techniques. Within preferred embodiments, the T cells are derived from either a patient or a related, or unrelated, donor and are administered to the patient following stimulation and expansion.

For therapeutic purposes, CD4⁺ or CD8⁺ T cells that proliferate in response to a colon tumor polypeptide, polynucleotide or APC can be expanded in number either *in vitro* or *in vivo.* Proliferation of such T cells *in vitro* may be accomplished in a variety of ways. For example, the T cells can be re-exposed to a colon tumor polypeptide, or a short peptide corresponding to an immunogenic portion of such a polypeptide, with or without the addition of T cell growth factors, such as interleukin-2, and/or stimulator cells that synthesize a colon tumor polypeptide. Alternatively, one or more T cells that proliferate in the presence of a colon tumor protein can be expanded in number by cloning. Methods for cloning cells are well known in the art, and include limiting dilution.

### PHARMACEUTICAL COMPOSITIONS AND VACCINES

### Within certain aspects, polypeptides, polynucleotides, T cells and/or binding agents disclosed herein may be incorporated into pharmaceutical compositions or immunogenic compositions (i.e., vaccines). Pharmaceutical compositions comprise one or more such compounds and a physiologically acceptable carrier. Vaccines may comprise one or more such compounds and an immunostimulant. An immunostimulant may be any substance that enhances or potentiates an immune response to an exogenous antigen. Examples of immunostimulants include adjuvants, biodegradable microspheres (e.g., polylactic galactide) and liposomes (into which the compound is incorporated; see e.g., Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive. For example, one or more immunogenic portions of other tumor antigens may be present, either incorporated into a fusion polypeptide or as a separate compound, within the composition or vaccine.

A pharmaceutical composition or vaccine may contain DNA encoding one or more of the polypeptides as described above, such that the polypeptide is generated *in situ.* As noted above, the DNA may be present within any of a variety of delivery systems known to those of ordinary skill in the art, including nucleic acid expression systems, bacteria and viral expression systems. Numerous gene delivery techniques are well known in the art, such as those described by Rolland, *Crit. Rev. Therap. Drug Carrier Systems 15*:143-198, 1998, and references cited therein. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin)* that expresses an immunogenic portion of the polypeptide on its cell surface or secretes such an epitope. In a preferred embodiment, the DNA may be introduced using a viral expression system *(e.g.,* vaccinia or other pox virus, retrovirus, or adenovirus), which may involve the use of a non-pathogenic (defective), replication competent virus. Suitable systems are disclosed, for example, in Fisher-Hoch et al., *Proc. Natl. Acad. Sci. USA 86*:317-321, 1989; Flexner et al., *Ann. N.Y. Acad. Sci. 569*:86-103, 1989; Flexner et al., *Vaccine 8*:17-21, 1990; U.S. Patent Nos.4,603,112, 4,769,330, and 5,017,487; WO 89/01973; U.S. Patent No. 4,777,127; GB 2,200,651; EP 0,345,242; WO 91/02805; Berkner, *Biotechniques 6*:616-627, 1988; Rosenfeld et al., *Science 252*:431-434, 1991; Kolls et al., *Proc. Natl. Acad. Sci. USA 91*:215-219, 1994; Kass-Eisler et al., *Proc. Natl. Acad. Sci. USA 90*:11498-11502, 1993; Guzman et al., *Circulation 88*:2838-2848, 1993; and Guzman et al., *Cir. Res. 73*:1202-1207, 1993. Techniques for incorporating DNA into such expression systems are well known to those of ordinary skill in the art. The DNA may also be "naked," as described, for example, in Ulmer et al., *Science 259*:1745-1749, 1993 and reviewed by Cohen, *Science 259*:1691-1692, 1993. The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres *(e.g.,* polylactate polyglycolate) may also be employed as carriers for the pharmaceutical compositions of this invention. Suitable biodegradable microspheres are disclosed, for example, in U.S. Patent Nos. 4,897,268 and 5,075,109.

Such compositions may also comprise buffers (*e.g*., neutral buffered saline or phosphate buffered saline), carbohydrates (*e.g*., glucose, mannose, sucrose or dextrans), mannitol, proteins, polypeptides or amino acids such as glycine, antioxidants, chelating agents such as EDTA or glutathione, adjuvants (e.g., aluminum hydroxide) and/or preservatives. Alternatively, compositions of the present invention may be formulated as a lyophilizate. Compounds may also be encapsulated within liposomes using well known technology.

Any of a variety of immunostimulants may be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

Within the vaccines provided herein, the adjuvant composition is preferably designed to induce an immune response predominantly of the Th1 type. High levels of Th1-type cytokines (*e.g.*, IFN-γ, TNFα, IL-2 and IL-12) tend to favor the induction of cell mediated immune responses to an administered antigen. In contrast, high levels of Th2-type cytokines (*e.g*., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, *Ann. Rev. Immunol. 7*:145-173, 1989.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Ribi ImmunoChem Research Inc. (Hamilton, MT) *(see* US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555. Another preferred adjuvant is a saponin, preferably QS21, which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210. Any vaccine provided herein may be prepared using well known methods that result in a combination of antigen, immune response enhancer and a suitable carrier or excipient.

The compositions described herein may be administered as part of a sustained release formulation *(i.e.,* a formulation such as a capsule, sponge or gel (composed of polysaccharides, for example) that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets tumor cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver *(i.e.,* matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, *Nature 392*:245-251, 1998) and have been shown to be effective as a physiological adjuvant for eliciting prophylactic or therapeutic antitumor immunity *(see* Timmerman and Levy, *Ann. Rev. Med. 50*:507-529, 1999). In general, dendritic cells may be identified based on their typical shape (stellate *in situ,* with marked cytoplasmic processes (dendrites) visible *in vitro),* their ability to take up, process and present antigens with high efficiency, and their ability to activate naïve T cell responses. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine *(see* Zitvogel et al., *Nature Med. 4*:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g*., CD54 and CD11) and costimulatory molecules (*e.g.*, CD40, CD80, CD86 and 4-1BB).

APCs may generally be transfected with a polynucleotide encoding a colon tumor protein (or portion or other variant thereof) such that the colon tumor polypeptide, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., *Immunology and cell Biology 75*:456-460, 1997. Antigen loading of dendritic cells may be achieved by incubating dendritic cells or progenitor cells with the colon tumor polypeptide, DNA (naked or within a plasmid vector) or RNA; or with antigen-expressing recombinant bacterium or viruses (e.g., vaccinia, fowlpox, adenovirus or lentivirus vectors). Prior to loading, the polypeptide may be covalently conjugated to an immunological partner that provides T cell help (e.g., a carrier molecule). Alternatively, a dendritic cell may be pulsed with a nonconjugated immunological partner, separately or in the presence of the polypeptide.

### CANCER THERAPY

In further aspects of the present invention, the compositions described herein may be used for immunotherapy of cancer, such as colon cancer. Within such methods, pharmaceutical compositions and vaccines are typically administered to a patient. As used herein, a "patient" refers to any warm-blooded animal, preferably a human. A patient may or may not be afflicted with cancer. Accordingly, the above pharmaceutical compositions and vaccines may be used to prevent the development of a cancer or to treat a patient afflicted with a cancer. A cancer may be diagnosed using criteria generally accepted in the art, including the presence of a malignant tumor. Pharmaceutical compositions and vaccines may be administered either prior to or following surgical removal of primary tumors and/or treatment such as administration of radiotherapy or conventional chemotherapeutic drugs.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides disclosed herein).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T cells as discussed above, T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage, monocyte, fibroblast and/or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow in vivo and to survive long term in substantial numbers by repeated stimulation with antigen supplemented with IL-2 *(see,* for example, Cheever et al., *Immunological Reviews 157*:177, 1997).

Alternatively, a vector expressing a polypeptide recited herein may be introduced into antigen presenting cells taken from a patient and clonally propagated *ex vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions disclosed herein, as well as dosage, will vary from individual to individual, and may be readily established using standard techniques. In general, the pharmaceutical compositions and vaccines may be administered by injection (*e.g*., intracutaneous, intramuscular, intravenous or subcutaneous), intranasally (*e.g*., by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (i.e., untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g*., more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 25 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (e.g., more frequent remissions, complete or partial, or longer disease-free survival) in treated patients as compared to non-treated patients. Increases in preexisting immune responses to a colon tumor protein generally correlate with an improved clinical outcome. Such immune responses may generally be evaluated using standard proliferation, cytotoxicity or cytokine assays, which may be performed using samples obtained from a patient before and after treatment.

### METHODS FOR DETECTING CANCER

In general, a cancer may be detected in a patient based on the presence of one or more colon tumor proteins and/or polynucleotides encoding such proteins in a biological sample (for example, blood, sera, sputum, urine and/or tumor biopsies) obtained from the patient. In other words, such proteins may be used as markers to indicate the presence or absence of a cancer such as colon cancer. In addition, such proteins may be useful for the detection of other cancers. The binding agents provided herein generally permit detection of the level of antigen that binds to the agent in the biological sample. Polynucleotide primers and probes may be used to detect the level of mRNA encoding a tumor protein, which is also indicative of the presence or absence of a cancer. In general, a colon tumor sequence should be present at a level that is at least three fold higher in tumor tissue than in normal tissue

There are a variety of assay formats known to those of ordinary skill in the art for using a binding agent to detect polypeptide markers in a sample. *See, e.g.,* Harlow and Lane, *Antibodies: A Laboratory Manual,* Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length colon tumor proteins and portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner *(see, e.g.,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (i.e., incubation time) is a period of time that is sufficient to detect the presence of polypeptide within a sample obtained from an individual with colon cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as colon cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., *Clinical Epidemiology: A Basic Science for Clinical Medicine,* Little Brown and Co., 1985, p. 106-7. Briefly, in this embodiment, the cut-off value may be determined from a plot of pairs of true positive rates *(i.e.,* sensitivity) and false positive rates (100%-specificity) that correspond to each possible cut-off value for the diagnostic test result. The cut-off value on the plot that is the closest to the upper left-hand corner *(i.e.,* the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1 µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use colon tumor polypeptides to detect antibodies that bind to such polypeptides in a biological sample. The detection of such colon tumor protein specific antibodies may correlate with the presence of a cancer.

A cancer may also, or alternatively, be detected based on the presence of T cells that specifically react with a colon tumor protein in a biological sample. Within certain methods, a biological sample comprising CD4⁺ and/or CD8⁺ T cells isolated from a patient is incubated with a colon tumor polypeptide, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with one or more representative polypeptides *(e.g.*, 5-25 µg/ml). It may be desirable to incubate another aliquot of a T cell sample in the absence of colon tumor polypeptide to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a colon tumor protein in a biological sample. For example, at least two oligonucleotide primers may be employed in a polymerase chain reaction (PCR) based assay to amplify a portion of a colon tumor cDNA derived from a biological sample, wherein at least one of the oligonucleotide primers is specific for *(i.e.,* hybridizes to) a polynucleotide encoding the colon tumor protein. The amplified cDNA is then separated and detected using techniques well known in the art, such as gel electrophoresis. Similarly, oligonucleotide probes that specifically hybridize to a polynucleotide encoding a colon tumor protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a colon tumor protein that is at least 10 nucleotides, and preferably at least 20 nucleotides, in length. Preferably, oligonucleotide primers and/or probes will hybridize to a polynucleotide encoding a polypeptide disclosed herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence recited in SEQ ID NO: 1-121, 123-197 and 205-486. Techniques for both PCR based assays and hybridization assays are well known in the art *(see,* for example, Mullis et al., *Cold Spring Harbor Symp. Quant. Biol., 51*:263, 1987; Erlich ed., *PCR Technology,* Stockton Press, NY, 1989).

One preferred assay employs RT-PCR, in which PCR is applied in conjunction with reverse transcription. Typically, RNA is extracted from a biological sample, such as biopsy tissue, and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another embodiment, the disclosed compositions may be used as markers for the progression of cancer. In this embodiment, assays as described above for the diagnosis of a cancer may be performed over time, and the change in the level of reactive polypeptide(s) or polynucleotide evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple colon tumor protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

### DIAGNOSTIC KITS

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a colon tumor protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding a colon tumor protein in a biological sample. Such kits generally comprise at least one oligonucleotide probe or primer, as described above, that hybridizes to a polynucleotide encoding a colon tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a colon tumor protein.

The following Examples are offered by way of illustration and not by way of limitation.

### EXAMPLES

### Example 1

### ISOLATION AND CHARACTERIZATION OF COLON TUMOR POLYPEPTIDES BY PCR-BASED SUBTRACTION AND MICROARRAY ANALYSIS

A cDNA library was constructed in the PCR2.1 vector (Invitrogen, Carlsbad, CA) by subtracting a pool of three colon tumors with a pool of normal colon, spleen, brain, liver, kidney, lung, stomach and small intestine using PCR subtraction methodologies (Clontech, Palo Alto, CA). The subtraction was performed using a PCR-based protocol, which was modified to generate larger fragments. Within this protocol, tester and driver double stranded cDNA were separately digested with five restriction enzymes that recognize six-nucleotide restriction sites (MluI, MscI, PvuII, SalI and StuI). This digestion resulted in an average cDNA size of 600 bp, rather than the average size of 300 bp that results from digestion with RsaI according to the Clontech protocol. This modification did not affect the subtraction efficiency. Two tester populations were then created with different adapters, and the driver library remained without adapters.

The tester and driver libraries were then hybridized using excess driver cDNA. In the first hybridization step, driver was separately hybridized with each of the two tester cDNA populations. This resulted in populations of (a) unhybridized tester cDNAs, (b) tester cDNAs hybridized to other tester cDNAs, (c) tester cDNAs hybridized to driver cDNAs, and (d) unhybridized driver cDNAs. The two separate hybridization reactions were then combined, and rehybridized in the presence of additional denatured driver cDNA. Following this second hybridization, in addition to populations (a) through (d), a fifth population (e) was generated in which tester cDNA with one adapter hybridized to tester cDNA with the second adapter. Accordingly, the second hybridization step resulted in enrichment of differentially expressed sequences which could be used as templates for PCR amplification with adaptor-specific primers.

The ends were then filled in, and PCR amplification was performed using adaptor-specific primers. Only population (e), which contained tester cDNA that did not hybridize to driver cDNA, was amplified exponentially. A second PCR amplification step was then performed, to reduce background and further enrich differentially expressed sequences.

This PCR-based subtraction technique normalizes differentially expressed cDNAs so that rare transcripts that are over-expressed in colon tumor tissue may be recoverable. Such transcripts would be difficult to recover by traditional subtraction methods.

To characterize the complexity and redundancy of the subtracted library, 96 clones were randomly picked and 65 were sequenced, as previously described. These sequences were further characterized by comparison with the most recent Genbank database (April, 1998) to determine their degree of novelty. No significant homologies were found to 21 of these clones, hereinafter referred to as 11092, 11093, 11096, 11098, 11103, 11174, 11108, 11112, 11115, 11117, 11118, 11134, 11151, 11154, 11158, 11168, 11172, 11175, 11184, 11185 and 11187. The determined cDNA sequences for these clones are provided in SEQ ID NO: 48, 49, 52, 54, 59, 60, 65-69, 79, 89, 90, 93, 99-101 and 109-111, respectively.

Two-thousand clones from the above mentioned cDNA subtraction library were randomly picked and submitted to a round of PCR amplification. Briefly, 0.5 µl of glycerol stock solution was added to 99.5 µl of pcr MIX (80 µl H₂O, 10 µl 10X PCR Buffer, 6 µl 25 mM MgCl₂, 1 µl 10 mM dNTPs, 1 µl 100 mM M13 forward primer (CACGACGTTGTAAAACGACGG), 1 µl 100 mM M13 reverse primer (CACAGGAAACAGCTATGACC)), and 0.5 µl 5 u/ml Taq polymerase (primers provided by (Operon Technologies, Alameda, CA). The PCR amplification was run for thirty cycles under the following conditions: 95°C for 5 min., 92°C for 30 sec., 57°C for 40 sec., 75°C for 2 min. and 75°C for 5 minutes.

mRNA expression levels for representative clones were determined using microarray technology (Synteni, Palo Alto, CA) in colon tumor tissues (n=25), normal colon tissues (n=6), kidney, lung, liver, brain, heart, esophagus, small intestine, stomach, pancreas, adrenal gland, salivary gland, resting PBMC, activated PBMC, bone marrow, dendritic cells, spinal cord, blood vessels, skeletal muscle, skin, breast and fetal tissues. The number of tissue samples tested in each case was one (n=1), except where specifically noted above; additionally, all the above-mentioned tissues were derived from humans. The PCR amplification products were dotted onto slides in an array format, with each product occupying a unique location in the array. mRNA was extracted from the tissue sample to be tested, and fluorescent-labeled cDNA probes were generated by reverse transcription according to the protocol provided by Synteni. The microarrays were probed with the labeled cDNA probes, the slides scanned, and fluorescence intensity was measured. This intensity correlates with the hybridization intensity.

One hundred and forty nine clones showed two or more fold over-expression in the colon tumor probe group as compared to the normal tissue probe group. These cDNA clones were further characterized by DNA sequencing with a Perkin Elmer/Applied Biosystems Division Automated Sequencer Model 373A and/or Model 377 (Foster City, CA). These sequences were compared to known sequences in the most recent GenBank database. No significant homologies to human gene sequences were found in forty nine of these clones, represented by the following sixteen cDNA consensus sequences: SEQ ID NO: 2, 8, 15, 16, 22, 24, 30, 32-34, 36, 38, 40, 41, 46 and 47, hereinafter referred to as Contig 2, 8, 13, 14, 20, 23, 29, 31, 35, 32, 36, 38, 41, 42, 50 and 51, respectively). Contig 29 (SEQ ID NO: 30) was found to be a Rat GSK-3-β-interacting protein Axil homolog. Also, Contigs 31 and 35 (SEQ ID NO: 32 and 33, respectively) were found to be a Mus musculus GOB-4 homolog. The determined cDNA sequences of SEQ ID NO: 1, 3-7, 9-14, 17-21, 23, 25-29, 31, 35, 37, 39, 42-45, 50, 51, 53, 55-58, 61-64, 70-78, 80-88, 91, 92, 94-98, 102-108 and 112 were found to show some homology to previously identified genes sequences.

Microarray analysis demonstrated Contig 2 (SEQ ID NO: 2) showed over-expression in 34% of colon tumors tested, as well as increased expression in normal pancreatic tissue, with no over-expression in normal colon tissues. Upon further analysis, Contigs 2, 8 and 23 were found to share homology to the known gene GW112. Contigs 4, 5, 9 and 52 showed homology to carcinoembryonic antigen (SEQ ID NO: 3, 4, 5 and 6, respectively). A representative sampling of these fragments showed over-expression in 85% of colon tumors, with over-expression in normal bone marrow and 3/6 normal colon tissues. Contig 6 (SEQ ID NO: 7), showing homology to the known gene sequence for villin, and was over-expressed in about half of all colon tumors tested, with a limited degree of low level over-expression in normal colon. Contig 12 (SEQ ID NO: 14), showing homology to Chromosome 17, clone hRPC.1171_I_10, also referred to as C798P, was over-expressed in approximately 70% of colon tumors tested, with low over-expression in 1/6 normal colon samples. Contig 14, also referred to as 14261 (SEQ ID NO: 16), showing no significant homology to any known gene, showed over-expression in 44% of colon tumors tested, with low level expression in half of normal colon tissues, as well as small intestine and pancreatic tissue. Contig 18 (SEQ ID NO: 21), showing homology to the known gene for L1-cadherin, showed over-expression in approximately half of colon tumors and low level over-expression in 3/6 normal colon tissues tested. Contig 22 (SEQ ID NO: 23), showing homology to Bumetanide-sensitive Na-K-Cl cotransporter was over-expressed in 70% of colon tumors and no over-expression in all normal tissues tested. Contig 25 (SEQ ID NO: 25), showing homology to macrophage inflammatory protein-3α, was over-expressed in over 40% of colon tumors and in activated PBMC. Contigs 26 and 48 (SEQ ID NOS: 25 and 26), showing homology to the sequence for laminin, was over-expressed in 48% of colon tumors and with low over-expression in stomach tissue. Contig 28 (SEQ ID NO: 29), showing homology to the known gene sequence for Chromosome 16 BAC clone CIT987SK-A-363E6, was over-expressed in 33% of colon tumors tested with normal stomach and 2/6 normal colon tissues showing low level over-expression. Contigs 29, 31 and 35 (SEQ ID NOS: 30, 32 and 33, respetively), also referred to as C751P, an unknown sequence showing limited and partial homology to Rat GSK-3β-interacting protein Axil homolog.and Mus musculus GOB-4 homolog, was over-expressed in 74% of colon tumors and no over-expression in all normal tissues tested. Contig 34 (SEQ ID NO: 35), showing homology to the known sequence for desmoglein 2, was over-expressed in 56% of colon tumors and showed low level over-expression in 1/6 normal colon tissues. Contig 36 (SEQ ID NO: 36), an unknown sequence also referred to as C793P, showed over-expression in 30% of colon tumor tissues tested. Contig 37 and 14287.2 (SEQ ID NOS: 37 and 116), an unknown sequence, but with limited (89%) homology to the known sequence for putative transmembrane protein was over-expressed in 70% of colon tumors, as well as in normal lung tissue and 3/6 normal colon tissues tested. Contig 38, also referred to as C796P and 14219 (SEQ ID NO: 38), showing no significant homology to any known gene, was over-expressed in 38% in colon tumors and no elevated over-expression in any normal tissues. Contig 41 (SEQ ID NO: 40), also referred to as C799P and 14308, an unknown sequence showing no significant homology to any known gene, was over-expressed in 22% of colon tumors. Contig 42, (SEQ ID NO: 41), also referred to as C794P and 14309, an unknown sequence with no significant homology to any known gene, was over-expressed in 63% of colon tumors tested, as well as in 3/6 normal colon tissues. Contig 43 (SEQ ID NO: 42), showing homology to the known sequence for Chromosome 1 specific transcript KIAA0487 was over-expressed in 85% of colon tumors tested and in normal lung and 4/6 normal colon tissues. Contig 49 (SEQ ID NO: 45), showing homology to the known sequence for pump-1, was over-expressed in 44% of colon tumors and no over-expression in all normal tissues tested. Contig 50 (SEQ ID NO: 46), also referred to as C792P and 18323, showing no significant homology to any known gene, was over-expressed in 33% of colon tumors with no detectable over-expression in any normal tissues tested. Contig 51 (SEQ ID NO: 47), also referred to as C795P and 14317 was over-expressed in 11 % of colon tumors.

Additional microarray analysis yielded seven clones showing two or more fold over-expression in the colon tumor probe group as compared to the normal tissue probe group. Three of these clones demonstrated particularly good colon tumor specificity, and are represented by SEQ ID NO: 115, 116 and 120. Specifically, SEQ ID NO: 115, referred to as C791P or 14235, which shows homology to the known gene sequence for H. sapiens chromosome 21 derived BAC containing ets-2 gene, was over-expressed in 89% of colon tumors tested and in 5/6 normal colon tissues, as well as over-expressed at low levels in normal lung and activated PBMC. Microarray analysis for SEQ ID NO: 116 is discussed above. SEQ ID NO: 120, referred to as 14295, showing homology to the known gene sequence for secreted cement gland protein XAG-2 homolog, was over-expressed in 70% of colon tumors and in 5/6 normal colon tissues, as well as low level over-expression in normal small intestine, stomach and lung. All clones showing over-expression in colon tumor were sequenced and these sequences compared to the most recent Genbank database (February 12, 1999). Of the seven clones, three contained sequences that did not share significant homology to any known gene sequences, represented by SEQ ID NO: 116, 117 and 119. To the best of the inventors' knowledge, none of these sequences have been previously shown to be present in colon. The determined cDNA sequences of the remaining clones (SEQ ID NO: 113-115 and 120) were found to show some homology to previously identified genes.

Further analysis identified a clone which was recovered several times by PCR subtraction and by expression screening using a mouse anti-scid antiserum. The determined full length cDNA sequence for this clone is provided in SEQ ID NO: 121, with the corresponding predicted amino acid sequence being provided in SEQ ID NO: 122. This clone is homologous with the known gene Beta IG-H3, as disclosed in U.S. Patent No. 5,444,164. Microarray analysis demonstrated this clone to be over-expressed in 75 to 80% of colon tumors tested (n=27), with no over-expression in normal colon samples (n=6), but with some low level over-expression in other normal tissues tested.

Further analysis of the PCR-subtraction library described above led to the isolation of longer cDNA sequences for the clones of SEQ ID NO: 30, 115, 46, 118, 41, 47, 38, 113, 14 and 40 (known as C751P, C791P, C792P, C793P, C794P, C795P, C796P, C797P, C798P and C799P, respectively). These determined cDNA sequences are provided in SEQ ID NO: 123-132, respectively.

Using PCR subtraction methodology described above with minor modifications, transcripts from a pool of three moderately differentiated colon adenocarcinoma samples were subtracted with a set of transcripts from normal brain, pancreas, bone marrow, liver, heart, lung, stomach and small intestine. Modifications of the above protocol were included at the cDNA digestion steps and in the tester to drive hybridization ratios. In a first subtraction, the restriction enzymes PvuII, DraI, MscI and StuI were used to digest cDNAs, and the tester to driver ratio was 1:40, as suggested by Clontech. In a second subtraction, DraI, MscI and StuI were used for cDNA digestion and a tester to driver ratio of 1:76 was used. Following the PCR amplification steps, the cDNAs were clones into pCR2.1 plasmid vector. The determined cDNA sequences of 167 isolated clones are provided in SEQ ID NO: 205-37.1. These sequences were compared to sequences in the public databases as described above. The sequences of SEQ ID NO: 205, 207, 210-212, 214, 215, 218, 224-226, 228, 233, 234, 236, 238, 241, 242, 245, 246, 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369 and 371 were found to show some homology to previously identified ESTs. The remaining sequences were found to show some homology to previously identified genes.

Using the PCR subtraction technology described above, a cDNA library from a pool of primary colon tumors was subtracted with a cDNA library prepared from normal tissues, including brain, bone marrow, kidney, heart, lung, liver, pancreas, small intestine, stomach and trachea. The determined cDNA sequences for 90 clones isolated in this subtraction are provided in SEQ ID NO: 372-461. Comparison of these sequences with those in the public databases as described above, revealed no homologies to the sequences of SEQ ID NO: 426, 445 and 453. The sequences of SEQ ID NO: 372-378, 380-404, 406, 409-417, 419-423, 425, 427-429, 433-436, 438-441, 443, 446-451, 454, 455 and 457-461 showed some homology to previously identified genes, while the sequences of SEQ ID NO: 379, 405, 407, 408, 418, 424, 430-432, 437, 442, 444, 452 and 456 showed some homology to previously isolated ESTs.

### Example 2

### ISOLATION OF TUMOR POLYPEPTIDES USING SCID-PASSAGED TUMOR RNA

Human colon tumor antigens were obtained using SCID mouse passaged colon tumor RNA as follows. Human colon tumor was implanted in SCID mice and harvested, as described in Patent Application Serial No. 08/556,659 filed 11/13/95, U.S. Patent No. 5,986,170 . First strand cDNA was synthesized from poly A+ RNA from three SCID mouse-passaged colon tumors using a Lambda ZAP Express cDNA synthesis kit (Stratagene). The reactions were pooled and digested with RNase A, T1 and H to cleave the RNA and then treated with NaOH to degrade the RNA. The resulting cDNA was annealed with biotinylated (Vector Labs, Inc., Burlingame, CA) cDNA from a normal resting PBMC plasmid library (constructed from Superscript plasmid System, Gibco BRL), and subtracted with streptavidin by phenol/chloroform extraction. Second strand cDNA was synthesized from the subtracted first strand cDNA and digested with S1 nuclease (Gibco BRL). The cDNA was blunted with Pfu polymerase and EcoRI adaptors (Stratagene) were ligated to the ends. The cDNA was phosphorylated with T4 polynucleotide kinase, digested with restriction endonuclease XhoI, and size selected with Sephacryl S-400 (Sigma). Fractions were pooled, ligated to Lambda ZAP Express arms (Stratagene) and packaged with Gigapack Gold III extract (Stratagene). Random plaques were picked, phagemid was excised, transformed into XLOLR cells (Stratagene) and resulting plasmid DNA (Qiagen Inc., Valencia, CA) was sequenced as described above. The determined cDNA sequences for 17 clones isolated as described above are provided in SEQ ID NO: 133-151, wherein 133 and 134 represent partial sequences of a clone referred to as CoSub-3 and SEQ ID NO: 135 and 136 represent partial sequences of a clone referred to as CoSub-13. These sequences were compared with those in the public databases as described above. The sequences of SEQ ID NO: 139 and 149 showed no significant homologies to any previously identified sequences. The sequences of SEQ ID NO: 138, 140, 141, 142, 143, 148 and 149 showed some homology to previously isolated expressed sequence tags (ESTs). The sequences of SEQ ID NO: 133-137, 144-147, 150 and 151 showed some homology to previously isolated gene sequences.

### Example 3

### USE OF MOUSE ANTISERA TO IDENTIFY DNA SEQUENCES ENCODING COLON TUMOR ANTIGENS

This example illustrates the isolation of cDNA sequences encoding colon tumor antigens by screening of colon tumor cDNA libraries with mouse anti-tumor sera.

A cDNA expression library was prepared from SCID mouse-passaged human colon tumor poly A+ RNA using a Stratagene (La Jolla, CA) Lambda ZAP Express kit, following the manufacturer's instructions. Sera was obtained from the colon tumor-bearing SCID mouse. This serum was injected into normal mice to produce anti-colon tumor serum. Approximately 600,000 PFUs were screened from the unamplified library using this antiserum. Using a goat anti-mouse IgG-A-M (H+L) alkaline phosphatase second antibody developed with NBT/BCIP (BRL Labs.), positive plaques were identified. Phage was purified and phagemid excised for several clones with inserts in a pBK-CMV vector for expression in prokaryotic or eukaryotic cells.

The determined cDNA sequences for 46 of the isolated clones are provided in SEQ ID NO: 152-197. The predicted amino acid sequences for the cDNA sequences of SEQ ID NO: 187, 188, 189, 190, 194, 195 and 197 are provided in SEQ ID NO: 198-204, respectively. The determined cDNA sequences were compared with those in the public database as described above. The sequences of SEQ ID NO: 156, 168, 184, 189, 192 and 196 showed some homology to previously isolated ESTs. The sequences of SEQ ID NO: 152-155, 157-167, 169-182, 183, 185-188, 190, 194, 195 and 197 showed some homology to previously identified genes.

### Example 4

### ISOLATION AND CHARACTERIZATION OF COLON TUMOR POLYPEPTIDES BY CONVENTIONAL SUBTRACTION

Two cDNA libraries were constructed and used to create a subtracted cDNA library as follows.

Using the GibcoBRL Superscript Plasmid System with minor modifications, two cDNA libraries were created. The first library, referred to as CTCL, was prepared from a pool of mRNA samples from three colon adenocarcinoma tissue samples. Two of the samples were described as Duke's stage C and one as Duke's stage B. All three samples were grade III in histological status. A second library (referred to as DriverLibpcDNA3.1+) was prepared from a pool of normal tissues, namely liver, pancreas, skin, bone marrow, resting PBMC, stomach and brain. Both libraries were prepared using the manufacturer's instructions with the following modifications: an EcoRI-NotI 5' cDNA adapter was used instead of the provided reagent; the vector pCDNA3.1 (+) (Invitrogen) was substituted for the pSPORT vector; and the ligated DNA molecules were transformed into ElectroMaxDH10B electrocompetent cells. Clones from the libraries were analyzed by restriction digest and sequencing to determine average insert size, quality of the library and complexity of the library. DNA was prepared from each library and digested.

The driver DNA was biotinylated and hybridized with the colon library tester DNA at a ratio of 10:1. After two rounds of hybridizations, streptavidin incubations and extractions, the remaining colon cDNAs were size-selected by column chromatography and cloned into the pCMV-Script vector from Stratagene. Clones from this subtracted library (referred to as CTCL-S 1) were characterized as described above for the unsubtracted libraries.

The determined cDNA sequences for 18 clones isolated from the CTCL-S1 library are provided in SEQ ID NO: 462-479. Comparison of these sequences with those in the public databases, as described above, revealed no significant homologies to the sequences of SEQ ID NO: 476, 477 and 479. The remaining sequences showed some homology to previously identified genes.

In further studies, a cDNA library was prepared from a pool of mRNA from three metastatic colon adenocarcinomas derived from liver tissue samples. All samples were described as Duke's stage D. Conventional subtraction was performed as described above, using the DriverLibpcDNA3.1+ library described above as the driver. The resulting subtracted library (referred to as CMCL-S1) was characterized by isolating a set of clones for restriction analysis and sequencing.

The determined cDNA sequences for 7 clones isolated from the CMCL-S1 library are provided in SEQ ID NO: 480-486. Comparison of these sequences with those in the public databases revealed no significant homologies to the sequence of SEQ ID NO: 483. The sequences of SEQ ID NO: 480-482 and 484-486 were found to show some homology to previously identified genes.

### Example 5

### SYNTHESIS OF POLYPEPTIDES

Polypeptides may be synthesized on a Perkin Elmer/Applied Biosystems Division 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure fractions, the peptides may be characterized using electrospray or other types of mass spectrometry and by amino acid analysis.

From the foregoing it will be appreciated that, although specific embodiments of the invention have been described herein for purposes of illustration, various modifications may be made without deviating from the spirit and scope of the invention. Accordingly, the invention is not limited except as by the appended claims.

## Claims

1. An isolated polypeptide comprising at least an immunogenic portion of a colon tumor protein, or a variant thereof, wherein the tumor protein comprises an amino acid sequence that is encoded by a polynucleotide sequence selected from the group consisting of:
(a) sequences recited in SEQ ID NO: 8, 2, 15, 16, 22, 24, 30, 32-34, 36, 38, 40, 41, 46-49, 52, 54, 59, 60, 65-69, 79, 89, 90, 93, 99-101, 109-111, 116-119, 123-132, 138-142, 143, 148, 149, 156, 168, 170-182, 184, 189, 191-193, 196, 205, 207, 210-212, 214, 215, 218, 224-226, 228, 233, 234, 236, 238, 241, 242, 245, 246, 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436, 438-441, 443, 446-451, 454, 455, 457-461, 476, 477, 479 and 483;
(b) sequences that hybridize to a sequence of SEQ ID NO: 8, 2, 15, 16, 22, 24, 30, 32-34, 36, 38, 40, 41, 46-49, 52, 54, 59, 60, 65-69, 79, 89, 90, 93, 99-101, 109-111, 116-119, 123-132, 138-142, 143, 148, 149, 156, 168, 170-182, 184, 189, 191-193, 196, 205, 207, 210-212, 214, 215, 218, 224-226, 228, 233, 234, 236, 238, 241, 242, 245, 246, 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436, 438-441, 443,446-451,454,455,457-461, 476, 477, 479 and 483 under moderately stringent conditions; and
(c) a complement of a sequence of (a) or (b).

2. An isolated polypeptide according to claim 1, wherein the polypeptide comprises an amino acid sequence that is encoded by a polynucleotide sequence recited in any one of SEQ ID NO: 8, 2, 15, 16, 22, 24, 30, 32-34, 36, 38, 40, 41, 46-49, 52,54,59,60,65-69,79, 89,90,93,99-101,109-111,116-119,123-132,138-142, 143, 148, 149, 156, 168, 170-182, 184, 189, 191-193, 196, 205, 207, 210-212, 214, 215, 218, 224-226, 228, 233, 234, 236, 238, 241, 242, 245, 246; 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436, 438-441, 443, 446-451, 454, 455, 457-461, 476, 477, 479 and 483 or a complement of any of the foregoing polynucleotide sequences.

3. An isolated polypeptide comprising a sequence recited in any one of SEQ ID NO: 122 and 198-204.

4. An isolated polynucleotide encoding at least 15 amino acid residues of a colon tumor protein, or a variant thereof that differs in one or more substitutions, deletions, additions and/or insertions such that the ability of the variant to react with antigen-specific antisera is not substantially diminished, wherein the tumor protein comprises an amino acid sequence that is encoded by a polynucleotide comprising a sequence recited in any one of SEQ ID NO: 8, 2, 15, 16, 22, 24, 30, 32-34, 36, 38, 40, 41, 46-49, 52, 54, 59, 60, 65-69, 79, 89, 90, 93, 99-101, 109-111, 116-119, 123-132, 138-142, 143, 148, 149, 156, 168, 170-182, 184, 189, 191-193, 196, 205, 207, 210-212, 214, 215, 218, 224-226, 228, 233, 234, 236, 238, 241, 242, 245, 246, 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436, 438-441, 443, 446-451, 454, 455, 457-461, 476, 477, 479 and 483 or a complement of any of the foregoing sequences.

5. An isolated polynucleotide encoding a colon tumor protein, or a variant thereof, wherein the tumor protein comprises an amino acid sequence that is encoded by a polynucleotide comprising a sequence recited in any one of SEQ ID NO: 8, 2, 15, 16, 22, 24, 30, 32-34, 36, 38, 40, 41, 46-49, 52, 54, 59, 60, 65-69, 79, 89, 90, 93, 99-101, 109-111, 116-119, 123-132, 138-142, 143, 148, 149, 156, 168,170-182, 184, 189, 191-193, 196, 205, 207, 210-212, 214, 215, 218, 224-226, 228, 233, 234, 236, 238, 241, 242, 245, 246, 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436,438-441,443,446-451,454,455,457-461,476,477,479 and 483 or a complement of any of the foregoing sequences.

6. An isolated polynucleotide comprising a sequence recited in any one of SEQ ID NO: 8, 2, 15, 16, 22, 24, 30, 32-34, 36, 38, 40, 41, 46-49, 52, 54, 59, 60, 65-69, 79, 89, 90, 93, 99-101, 109-111, 116-119, 123-132, 138-142, 143, 148, 149, 156, 168, 170-182, 184, 189, 191-193, 196, 205, 207, 210-212, 214, 215, 218, 224-226, 228, 233, 234, 236, 238, 241, 242, 245, 246, 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436, 438-441, 443, 446-451, 454, 455, 457-461, 476, 477, 479 and 483, or a sequence that hybridizes thereto under moderately stringent conditions.

7. An isolated polynucleotide complementary to a polynucleotide according to any one of claims 4-6.

8. An expression vector comprising a polynucleotide according to any one of claims-claim 4-7.

9. A host cell transformed or transfected with an expression vector according to claim 8.

10. An isolated antibody, or antigen-binding fragment thereof, that specifically binds to a colon tumor protein that comprises an amino acid sequence that is encoded by a polynucleotide sequence recited in any one of SEQ ID NO: 8, 2, 15, 16, 22, 24, 30, 32-34, 36, 38, 40,41, 46-49, 52, 54, 59, 60, 65-69, 79, 89, 90, 93, 99-101, 109-111, 116-119, 123-132, 138-142, 143, 148, 149, 156, 168, 170-182, 184, 189, 191-193, 196, 205, 207, 210-212, 214, 215, 218, 224-226, 228, 233, 234, 236, 238, 241, 242, 245, 246, 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436, 438-441, 443, 446-451, 454, 455, 457-461, 476, 477, 479 and 483 or a complement of any of the foregoing polynucleotide sequences.

11. A fusion protein comprising at least one polypeptide according to claim 1 or 2.

12. A fusion protein according to claim 11, wherein the fusion protein comprises an expression enhancer that increases expression of the fusion protein in a host cell transfected with a polynucleotide encoding the fusion protein.

13. A fusion protein according to claim 11 or 12, wherein the fusion protein comprises a T helper epitope that is not present within the polypeptide of claim 1, and/or an affinity tag.

14. An isolated polynucleotide encoding a fusion protein according to any one of claims 11 to 13.

15. A pharmaceutical composition comprising a physiologically acceptable carrier and at least one component selected from the group consisting of:
(a) a polypeptide according to claim 1;
(b) a polynucleotide according to claim 4;
(c) an antibody according to claim 10;
(d) a fusion protein according to claim 11; and
(e) a polynucleotide according to claim 14.

16. A vaccine comprising an immunostimulant and at least one component selected from the group consisting of:
(a) a polypeptide according to claim 1;
(b) a polynucleotide according to claim 4;
(c) an antibody according to claim 10;
(d) a fusion protein according to claim 11; and
(e) a polynucleotide according to claim 14.

17. A pharmaceutical composition comprising an antigen-presenting cell that expresses a polypeptide according to claim 1, in combination with a pharmaceutically acceptable carrier or excipient, or a vaccine comprising an antigen-presenting cell that expresses a polypeptide according to claim 1, in combination with an immunosatimulant.

18. A vaccine according to claim 16 wherein the immunostimulant is an adjuvant.

19. A vaccine according to claim 16 wherein the immunostimulant induces a predominantly Type I response.

20. The pharmaceutical composition of claim 15 or the vaccine of claim 19 for use in a method for inhibiting the development of a cancer in a patient, comprising administering to a patient an effective amount of the pharmaceutical composition or vaccine.

21. An antigen-presenting cell that expresses a polypeptide encoded by a polynucleotide recited in any one of SEQ ID NO: 1-121, 123-197 and 205-486, for use in a method for inhibiting the development of a cancer in a patient, comprising administering to a patient an effective amount of the antigen - presenting cell and thereby inhibiting the development of a cancer in the patient.

22. The pharmaceutical composition of claim 17 , wherein the antigen - presenting cell is a dendritic cell or a inacrophoge.

23. The pharmaceutical composition or vaccine of claim 20 or the antigen - presenting cell of claim 21, wherein the cancer is colon cancer.

24. A method for removing tumor cells from a biological sample, comprising contacting a biological sample with T cells that specifically react with a colon tumor protein, wherein the tumor protein comprises an amino acid sequence that is encoded by a polynucleotide sequence selected from the group consisting of:
(i) polynucleotides recited in any one of SEQ ID NO: 1-121, 123-197 and 205-486; and
(ii) complements of the foregoing polynucleotides;
wherein the step of contacting is performed under conditions and for a time sufficient to permit the removal of cells expressing the antigen from the sample.

25. A method according to claim 24, wherein the biological sample is blood or a fraction thereof.

26. A biological sample treated according to the method of claim 24 for use in a method for inhibiting the development of a cancer in a patient, comprising administering to the patient the biological sample.

27. A method for stimulating and/or expanding T cells specific for a colon tumor protein, comprising contacting T cells with at least one component selected from the group consisting of:
(i) a polypeptide according to claim 1;
(ii) a polypeptide encoded by a polynucleotide comprising a sequence provided in any one of SEQ ID NO: 1-121, 123-197 and 205-486;
(iii) a polynucleotide encoding a polypeptide of (i) or (ii); and
(iv) an antigen presenting cell that expresses a polypeptide of (i) or (ii), under conditions and for a time sufficient to permit the stimulation and/or expansion of T cells.

28. An isolated T cell population, comprising T cells prepared according to the method of claim 37.

29. A T cell population according to claim 28 for use in a method for inhibiting the development of a cancer in a patient, comprising administering to the patient an effective amount of the T cell population.

30. A method for preparing T cells comprising the steps of:
incubating CD4⁺ and/or CD8+ T cells isolated from a patient with at least one component selected from the group consisting of:
(i) a polypeptide according to claim 1;
(ii) a polypeptide encoded by a polynucleotide comprising a sequence of any one of SEQ ID NO: 1-121, 123-197 and 205-486;
(iii) a polynucleotide encoding a polypeptide of (i) or (ii); and
(iv) an antigen-presenting cell that expresses a polypeptide of (i) or (ii);
such that T cells proliferate.

31. A method according to Claim 30 further comprising the step of: cloning at least one proliferated cell to provide cloned T cells.

32. T cells prepared according to the method of Claims 30 or 31 for use in a method for inhibiting the development of a cancer in a patient comprising administering to the patient an effective amount of the proliferated or cloned T cells, and thereby inhibiting the development of a cancer in the patient.

33. A method for determining the presence or absence of a cancer in a patient, comprising the steps of:
(a) contacting a biological sample obtained from a patient with a binding agent that binds to a colon tumor protein, wherein the tumor protein comprises an amino acid sequence that is encoded by a polynucleotide sequence selected from the group consisting of:
(i) polynucleotides recited in any one of SEQ ID NO: 1-121, 123-197 and 205-486; and
(ii) complements of the foregoing polynucleotides;
(b) detecting in the sample an amount of polypeptide that binds to the binding agent; and
(c) comparing the amount of polypeptide to a predetermined cut-off value, and therefrom determining the presence or absence of a cancer in the patient.

34. A method for monitoring the progression of a cancer in a patient, comprising the steps of:
(a) contacting a biological sample obtained from a patient at a first point in time with a binding agent that binds to a colon tumor protein, wherein the tumor protein comprises an amino acid sequence that is encoded by a polynucleotide sequence recited in any one of SEQ ID NO: 1-121, 123-197 and 205-486 or a complement of any of the foregoing polynucleotides;
(b) detecting in the sample an amount of polypeptide that binds to the binding agent;
(c) repeating steps (a) and (b) using a biological sample obtained from the patient at a subsequent point in time; and
(d) comparing the amount of polypeptide detected in step (c) to the amount detected in step (b) and therefrom monitoring the progression of the cancer in the patient.

35. A method according to claim 33 or 34, wherein the binding agent is an antibody, preferably a monoclonal antibody.

36. A method according to any one of claims 33 to 35, wherein the cancer is a colon cancer.

37. A method for determining the presence or absence of a cancer in a patient, comprising the steps of:
(a) contacting a biological sample obtained from a patient with an oligonucleotide that hybridizes to a polynucleotide that encodes a colon tumor protein, wherein the tumor protein comprises an amino acid sequence that is encoded by a polynucleotide sequence recited in any one of SEQ ID NO: 1-121, 123-197 and 205-486 or a complement of any of the foregoing polynucleotides;
(b) detecting in the sample an amount of a polynucleotide that hybridizes to the oligonucleotide; and
(c) comparing the amount of polynucleotide that hybridizes to the oligonucleotide to a predetermined cut-off value, and therefrom determining the presence or absence of a cancer in the patient.

38. A method for monitoring the progression of a cancer in a patient, comprising the steps of:
(a) contacting a biological sample obtained from a patient with an oligonucleotide that hybridizes to a polynucleotide that encodes a colon tumor protein, wherein the tumor protein comprises an amino acid sequence that is encoded by a polynucleotide sequence recited in any one of SEQ ID NO: 1-121, 123-197 and 205-486 or a complement of any of the foregoing polynucleotides;
(b) detecting in the sample an amount of a polynucleotide that hybridizes to the oligonucleotide;
(c) repeating steps (a) and (b) using a biological sample obtained from the patient at a subsequent point in time; and
(d) comparing the amount of polynucleotide detected in step (c) to the amount detected in step (b) and therefrom monitoring the progression of the cancer in the patient.

39. A method according to claim 37 or 38, wherein the amount of polynucleotide that hybridizes to the oligonucleotide is determined using a polymerase chain reaction, or a hybridization assay.

40. A diagnostic kit, comprising:
(a) one or more antibodies according to claim 10; and
(b) a detection reagent comprising a reporter group.

41. A kit according to claim 40, wherein the antibodies are immobilized on a solid support.

42. A kit according to claim 40 or 41, wherein the detection reagent comprises an anti-immunoglobulin, protein G, protein A or lectin.

43. A kit according to claim 40 , wherein the reporter group is selected from the group consisting of radioisotopes, fluorescent groups, luminescent groups, enzymes, biotin and dye particles.

44. An oligonucleotide comprising 10 to 40 contiguous nucleotides that hybridize under moderately stringent conditions to a polynucleotide that encodes a colon tumor protein, wherein the tumor protein comprises an amino acid sequence that is encoded by a polynucleotide sequence recited in any one of SEQ ID NO: 8, 2, 15, 16, 22, 24, 30, 32-34, 36, 38, 40, 41, 46-49, 52, 54, 59, 60, 65-69, 79, 89, 90, 93, 99-101, 109-111, 116-119, 123-132, 138-142, 143, 148, 149, 156, 168, 170-182, 184, 189, 191-193, 196, 205, 207, 210-212, 214, 215, 218, 224-226, 228, 233, 234, 236, 238, 241, 242, 245, 246, 248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436, 438-441, 443, 446-451, 454, 455, 457-461, 476, 477, 479 and 483 or a complement of any of the foregoing polynucleotides.

45. A oligonucleotide according to claim 44, wherein the oligonucleotide comprises 10-40 contiguous nucleotides recited in any one of SEQ ID NO: 8, 2, 15, 16, 22, 24, 30, 32-34, 36, 38, 40, 41, 46-49, 52, 54, 59, 60, 65-69, 79, 89, 90, 93, 99-101,109-111,116-119,123-132,138-142,143,148,149, 156, 168, 170-182, 184, 189, 191-193, 196, 205, 207, 210-212, 214, 215, 218, 224-226, 228, 233, 234, 236, 238, 241, 242, 245, 246,248, 250, 253, 254, 256, 259, 260, 262, 263, 266, 267, 270-273, 279, 282, 291, 293, 294, 298, 300, 302, 303, 310-313, 315, 317, 320, 322, 324, 332-335, 345, 347, 356, 358, 361, 362, 366, 369, 371-378, 380-404, 406, 409-417, 419-423, 425, 427-429, 433-436, 438-441, 443, 446-451, 454, 455, 457-461, 476, 477, 479 and 483.

46. A diagnostic kit, comprising:
(a) an oligonucleotide according to claim 45 and
(b) a diagnostic reagent for use in a polymerase chain reaction or hybridization assay.
